(19) Europäisches Patentamt
European Patent Office
Office européen des brevets

(11) **EP 1 765 754 B1**

(12) **EUROPÄISCHE PATENTSCHRIFT**

(45) Veröffentlichungstag und Bekanntmachung des Hinweises auf die Patenterteilung:
**09.12.2015   Patentblatt 2015/50**

(21) Anmeldenummer: **05804913.1**

(22) Anmeldetag: **17.05.2005**

(51) Int Cl.:
*B01J 8/04* *(2006.01)*          *C07C 45/33* *(2006.01)*
*C07C 51/215* *(2006.01)*        *C07C 51/25* *(2006.01)*
*C07C 45/35* *(2006.01)*

(86) Internationale Anmeldenummer:
**PCT/EP2005/005364**

(87) Internationale Veröffentlichungsnummer:
**WO 2006/002713 (12.01.2006 Gazette 2006/02)**

(54) **VERFAHREN ZUR HERSTELLUNG VON ACROLEIN, ODER ACRYLSÄURE ODER DEREN GEMISCH AUS PROPAN**

METHOD FOR THE PRODUCTION OF ACROLEIN, ACRYLIC ACID, OR A MIXTURE THEREOF FROM PROPANE

PROCEDE POUR PRODUIRE DE L'ACROLEINE, DE L'ACIDE ACRYLIQUE OU UN MELANGE DE CES DEUX SUBSTANCES A PARTIR DU PROPANE

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **01.07.2004   US 584469 P**
            **01.07.2004   DE 102004032129**
            **18.03.2005   DE 102005013039**
            **18.03.2005   US 662804 P**

(43) Veröffentlichungstag der Anmeldung:
**28.03.2007   Patentblatt 2007/13**

(73) Patentinhaber: **BASF SE**
**67056 Ludwigshafen (DE)**

(72) Erfinder:
• **MACHHAMMER, Otto**
**68163 Mannheim (DE)**
• **SCHINDLER, Götz-Peter**
**68219 Mannheim (DE)**
• **ADAMI, Christoph**
**69469 Weinheim (DE)**
• **HECHLER, Claus**
**67063 Ludwigshafen (DE)**
• **DIETERLE, Martin**
**68167 Mannheim (DE)**

(56) Entgegenhaltungen:
**WO-A-01/96270**

**Beschreibung**

Vorliegende Erfindung betrifft ein Verfahren zur Herstellung von Acrolein, oder Acrylsäure oder deren Gemisch aus Propan, bei dem man

A) einer ersten Reaktionszone A wenigstens zwei gasförmige, Propan enthaltende Zufuhrströme zuführt, von denen wenigstens einer Frischpropan enthält, und in der Reaktionszone A ihr so zugeführtes Propan einer heterogen katalysierten Dehydrierung unterwirft, wobei ein Propan und Propylen enthaltendes Produktgasgemisch A erhalten wird,

B) aus der Reaktionszone A Produktgasgemisch A herausführt und in einer ersten Trennzone A von den im Produktgasgemisch A enthaltenen, von Propan und Propylen verschiedenen, Bestandteilen wenigstens eine Teilmenge abtrennt, und dabei verbleibendes, Propan und Propylen enthaltendes, Produktgasgemisch A'

C) in einer zweiten Reaktionszone B zur Beschickung wenigstens eines Oxidationsreaktors verwendet und in dem wenigstens einen Oxidationsreaktor das im Produktgasgemisch A' enthaltene Propylen einer (selektiven) heterogen katalysierten Gasphasenpartialoxidation mit molekularem Sauerstoff zu einem Acrolein, oder Acrylsäure, oder deren Gemisch als Zielprodukt sowie überschüssigen molekularen Sauerstoff enthaltenden Produktgasgemisch B unterwirft,

D) aus der Reaktionszone B Produktgasgemisch B herausführt und in einer zweiten Trennzone B im Produktgasgemisch B enthaltenes Zielprodukt abtrennt und von dem dabei verbleibenden, nicht umgesetzten Propan, molekularen Sauerstoff sowie gegebenenfalls nicht umgesetztes Propylen enthaltenden Restgas wenigstens eine nicht umgesetztes Propan, molekularen Sauerstoff und gegebenenfalls nicht umgesetztes Propylen enthaltende Teilmenge als einen der wenigstens zwei Propan enthaltenden Zufuhrströme in die Reaktionszone A rückführt.

Acrylsäure ist eine bedeutende Grundchemikalie, die unter anderem als Monomer zur Herstellung von Polymerisaten Verwendung findet, die beispielsweise in disperser Verteilung in wässrigem Medium befindlich als Bindemittel angewendet werden. Acrolein ist ein bedeutendes Zwischenprodukt, beispielsweise für die Herstellung von Glutardialdehyd, Methionin, Folsäure und Acrylsäure.

Das in der Präambel dieser Schrift beschriebene Verfahren zur Herstellung von Acrolein, oder Acrylsäure oder deren Gemisch aus Propan ist bekannt (z.B. aus der WO 01/96270, der US 2003/0187299 A1 und aus den Schriften DE-A 10245585 sowie DE-A 10246119 und dem in diesen Schriften zitierten Stand der Technik).

Die Verfahren zur Abtrennung von im Produktgasgemisch B enthaltenem Zielprodukt sind dabei dadurch gekennzeichnet, dass man das Zielprodukt z.B. durch absorptive und/oder kondensative Maßnahmen aus der gasförmigen in die kondensierte Phase überführt. Als Absorptionsmittel kommen dabei z.B. Wasser, wässrige Lösung und/oder organische Lösungsmittel in Betracht. Im Rahmen dieser "Kondensation" des Zielproduktes verbleibt normalerweise ein nicht in die kondensierte Phase übergehendes Restgas, das die vergleichsweise schwierig zu kondensierenden Bestandteile des Produktgasgemischs B umfasst. Dies sind üblicherweise vor allem diejenigen Komponenten, deren Siedepunkt bei Normaldruck (1 bar) ≤ -30°C beträgt (ihr Gesamtanteil am Restgas beträgt in der Regel ≥ 70 Vol.%, häufig ≥ 80 Vol.% und vielfach ≥ 90 Vol.-). Dazu gehören in erster Linie nicht umgesetztes Propan, in der Reaktionszone B verbliebener überschüssiger molekularer Sauerstoff sowie gegebenenfalls nicht umgesetztes Propylen. Zusätzlich wird das Restgas in der Regel inerte Verdünnungsgase wie z.B. $N_2$, $CO_2$, Edelgase (He, Ne, Ar etc.), CO sowie in geringem Umfang auch Acrylsäure, Acrolein und/oder $H_2O$ (der Wasserdampfanteil am Restgas kann bis zu 25 Vol.-%, häufig bis zu 20 Vol.-% oder bis zu 10 Vol.-%, vielfach aber auch unter 10 Vol.-% oder unter 5 Vol.-% betragen) enthalten. Dieses vorgenannte Restgas bildet (bezogen auf die darin enthaltene Menge an Propan) die Hauptmenge (normalerweise wenigstens 80 %, bzw. wenigstens 90 %, oder wenigstens 95 % oder mehr) des in der Trennzone B gebildeten Restgases und wird daher in dieser Schrift u.a. auch als Hauptrestgas bezeichnet.

Insbesondere dann, wenn die Kondensation des Zielproduktes durch Absorption mittels eines organischen Lösungsmittels erfolgt, fällt in der Trennzone B in der Regel wenigstens ein zweites nicht umgesetztes Propan sowie gegebenenfalls nicht umgesetztes Propylen enthaltendes restliches Gas an (bezogen auf darin enthaltenes Propan ist seine Menge im Vergleich zur Hauptrestgasmenge normalerweise wesentlich geringer). Dies ist darauf zurückzuführen, dass die sich bildende kondensierte Phase in gewissem Umfang auch nicht umgesetztes Propan sowie gegebenenfalls nicht umgesetztes Propylen aufnimmt. Im weiteren Verlauf der extraktiven, destillativen, kristallisativen und/oder desorptiven Abtrennung des Zielproduktes aus der kondensierten Phase, wird dieses nicht umgesetzte Propan sowie gegebenenfalls Propylen normalerweise als Bestandteil wenigstens einer weiteren Gasphase rückgewonnen und vorzugsweise in die Reaktionszone A rückgeführt. Dies kann z.B. im Gemisch mit dem Hauptrestgas erfolgen (in dieser Schrift dann Gesamtrestgas genannt). Es kann aber auch in Form eigenständiger in die Reaktionszone A rückzuführender Gasströme erfolgen. Letztere können dabei an Sauerstoff frei, oder auch Sauerstoff enthaltend (Nebenrestgas) sein (z.B. wenn er durch Strippen mittels Luft oder am Kopf einer mittels Luft als Polymerisationsinhibitor gespülten Rektifikationskolonne anfällt).

Sowohl Hauptrestgas, Gesamtrestgas als auch Nebenrestgas bilden im Sinne dieser Erfindung in die Reaktionszone A rückführbares nicht umgesetztes Propan, molekularen Sauerstoff und gegebenenfalls nicht umgesetztes Propylen enthaltendes Restgas. An molekularem Sauerstoff freies in der Trennzone B anfallendes nicht umgesetztes Propan sowie gegebenenfalls nicht umgesetztes Propylen enthaltendes restliches Gas kann erfindungsgemäß im Gemisch mit Hauptrestgas und/oder Nebenrestgas (d.h., z.B. als Bestandteil von Gesamtrestgas) und/oder auch eigenständig (in diesem Fall handelt es sich nicht um in die Reaktionszone A im Sinne der Erfindung rückgeführtes Restgas) in die Reaktionszone A rückgeführt werden. Im letzteren Fall kann diese Rückführung ohne jedwede Beschränkung, d.h., z.B. auch als Bestandteil des Reaktionsgasausgangsgemischs der Reaktionszone A erfolgen. Vorzugsweise wird beim erfindungsgemäßen Verfahren die Gesamtmenge der in der Trennzone B anfallenden nicht umgesetztes Propan sowie gegebenenfalls nicht umgesetztes Propylen enthaltenden Gasströme in die Reaktionszone A rückgeführt. Teilmengen können (wie im weiteren Verlauf der Anmeldung noch näher ausgeführt wird) gegebenenfalls aber auch für andere Zwecke, wie z.B. zur Energieerzeugung und/oder Synthesegasherstellung und/oder als Verdünnungsgas in der Reaktionszone B weiterverwendet werden.

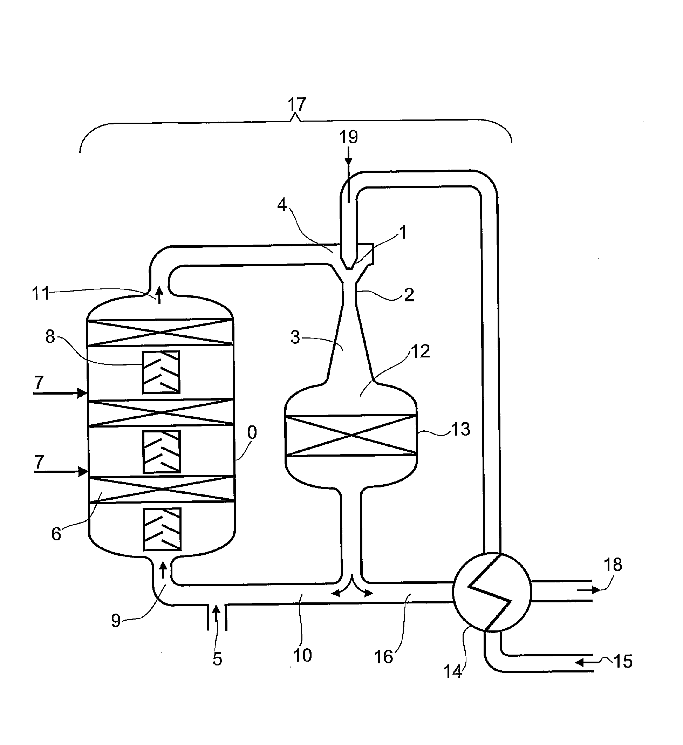

In den vorstehend genannten Schriften des Standes der Technik soll die Rückführung von nicht umgesetztes Propan, molekularen Sauerstoff sowie gegebenenfalls nicht umgesetztes Propylen enthaltendem Restgas in die Reaktionszone A an der selben Stelle wie die Zufuhr der übrigen Propan enthaltenden Zufuhrströme in die Reaktionszone A erfolgen (d.h., als Bestandteil des Reaktionsgasausgangsgemischs; vgl. z.B. Figur 6 in US-2003/0187299 A1). Nachteilig an dieser Verfahrensweise ist, dass der im Restgas enthaltene molekulare Sauerstoff die Selektivität der Propenbildung in der Reaktionszone A mindert und die Selektivität der Nebenproduktbildung an den Kohlenoxiden CO und $CO_2$ infolge partiell erfolgender Vollverbrennung von Propan und/oder Propylen erhöht. Dies gilt insbesondere dann, wenn es sich beim rückzuführenden Restgas um Hauptrestgas handelt.

Die DE-A 10211275 versucht dem vorgenannten Problem dadurch abzuhelfen, dass man zwar wie vorstehend beschrieben verfährt, dabei aber gleichzeitig das in der Reaktionszone A gebildete Produktgasgemisch A in zwei Teilmengen identischer Zusammensetzung aufteilt und eine der beiden Teilmengen als Wasserstoffquelle in die Reaktionszone A zurückführt. Nachteilig ist jedoch, dass die Deaktivierungsrate der Dehydrierkatalysatoren bei dieser Verfahrensweise noch nicht in vollem Umfang zu befriedigen vermag.

Die Aufgabe der vorliegenden Erfindung bestand daher darin, ein gegenüber den vorgenannten Verfahren verbessertes Verfahren zur Herstellung von Acrolein, oder Acrylsäure oder deren Gemisch aus Propan zur Verfügung zu stellen.

Demgemäß wurde ein Verfahren zur Herstellung von Acrolein, oder Acrylsäure oder deren Gemisch aus Propan, bei dem man

A) einer ersten Reaktionszone A wenigstens zwei gasförmige, Propan enthaltende Zufuhrströme zuführt von denen wenigstens einer Frischpropan enthält, und in der Reaktionszone A ihr so zugeführtes Propan einer heterogen katalysierten Dehydrierung unterwirft, wobei ein Propan und Propylen enthaltendes Produktegasgemisch A (in der Regel wird das Produktgasgemisch A auch molekularen Wasserstoff enthalten) erhalten wird,

B) aus der Reaktionszone A Produktgasgemisch A herausführt und in einer ersten Trennzone A von den im Produktgasgemisch A enthaltenen, von Propan und Propylen verschiedenen, Bestandteilen wenigstens eine Teilmenge abtrennt, und dabei verbleibendes, Propan und Propylen enthaltendes, Produktgasgemisch A'

C) in einer zweiten Reaktionszone B zur Beschickung wenigstens eines Oxidationsreaktors verwendet und in dem wenigstens einen Oxidationsreaktor das im Produktgasgemisch A' enthaltene Propylen einer (selektiven) heterogen katalysierten Gasphasenpartialoxidation mit molekularem Sauerstoff zu einem Acrolein, oder Acrylsäure, oder deren Gemisch als Zielprodukt sowie überschüssigen molekularen Sauerstoff enthaltenden Produktgasgemisch B unterwirft,

D) aus der Reaktionszone B Produktgasgemisch B herausführt und in einer zweiten Trennzone B im Produktgasgemisch B enthaltenes Zielprodukt abtrennt und von dem dabei verbleibenden, nicht umgesetztes Propan, molekularen Sauerstoff sowie gegebenenfalls nicht umgesetztes Propylen enthaltenden (Haupt- und/oder Neben- bzw. Gesamt-)Restgas wenigstens eine nicht umgesetztes Propan, molekularen Sauerstoff und gegebenenfalls nicht umgesetztes Propylen enthaltende Teilmenge (vorzugsweise wenigstens 50 Vol.-%, oder wenigstens 75 Vol.-% und ganz besonders bevorzugt die Gesamtmenge (jeweils individuell bezüglich des Gesamtrestgases, des Hauptrestgases und/oder des Nebenrestgases)) als einen der wenigstens zwei Propan enthaltenden Zufuhrströme in die Reaktionszone A rückführt, gefunden, das dadurch gekennzeichnet ist, dass diese Rückführung in die Reaktionszone A längs des Reaktionspfads der heterogen katalysierten Dehydrierung des Propans in der Reaktionszone A so erfolgt, dass an der Zufuhrstelle bereits wenigstens 5 mol-% des der Reaktionszone A über die anderen Zufuhrströme (davor) (insgesamt) zugeführten Propan in der Reaktionszone A dehydrierend umgesetzt sind (bezogen auf einmaligen Durchgang durch die Reaktionszone A).

Die WO 01/96270 betrifft ein Verfahren, das die spezielle Rückführung des erfindungsgemäßen Verfahrens nicht aufweist.

Wird beim erfindungsgemäßen Verfahren nicht die Gesamtmenge des in der Trennzone B (insgesamt) anfallenden (Haupt- und/oder Neben- bzw. Gesamt-)Restgases in die Reaktionszone A rückgeführt, kann diese andere Teilmenge wie bereits erwähnt z.B. zum Zweck der Energie- oder Synthesegaserzeugung oder als Verdünnungsgas in der Reaktionszone B weiterverwendet werden. In der Regel wird man jedoch wenigstens die Hälfte oder zwei Drittel (d.h., 50 Vol-% oder 66,6 Vol.-%), bevorzugt wenigstens drei Viertel und ganz besonders bevorzugt die Gesamtmenge des vorgenannten in der Trennzone B anfallenden (jeweils individuell bezüglich des Haupt- und/oder Neben- bzw. Gesamt-)Restgases erfindungsgemäß in die Reaktionszone A rückführen. Fällt in der Trennzone B nur ein nicht umgesetztes Propan, molekularen Sauerstoff und nicht umgesetztes Propylen enthaltender Restgasstrom an (dies ist häufig der Regelfall), wird dieser erfindungsgemäß bevorzugt vollständig (gegebenenfalls abzüglich einer in die Reaktionszone B als Verdünnungsgas geführten Teilmenge von identischer Zusammensetzung) in die Reaktionszone A rückgeführt. Er kann aber auch in zwei Teilmengen identischer Zusammensetzung aufgeteilt, und, wie vorstehend beschrieben, nur eine Teilmenge in die Reaktionszone A rückgeführt und die andere Teilmenge anderweitig weiterverwendet werden. Fällt in der Trennzone B mehr als ein solcher Restgasstrom an, so können diese Restgasströme (wie bereits erwähnt) erfindungsgemäß gemeinsam (z.B. zusammengeführt) oder auch nur vereinzelt bzw. einzeln in die Reaktionszone A rückgeführt werden. Die prozentualen Rückführungsangaben beziehen sich beim Restgas in dieser Schrift insbesondere auf dessen Gesamtmenge (d.h., auf die Summe aller Restgasströme). Normalerweise besteht (insbesondere Haupt-)Restgas (wie bereits erwähnt) zu $\geq$ 70 Vol.-%, häufig zu $\geq$ 80 Vol.-% und vielfach zur $\geq$ 90 mol-%, meist zu $\geq$ 95 mol-%, oder zu $\geq$ 98 mol-% aus Bestandteilen, deren Siedepunkt bei Normaldruck (1 bar) $\leq$ -30°C beträgt. Erfindungsgemäß kann die Rückführung von in der Trennzone B anfallendem, molekularen Sauerstoff enthaltendem, (Haupt- und/oder Neben- bzw. Gesamt-)Restgas in die Reaktionszone A längs des Reaktionspfads der katalytischen Dehydrierung selbstredend nicht nur an einer einzigen, sondern auch über mehrere hintereinander angeordnete Zufuhrstellen verteilt erfolgen.

Unter Frischpropan wird in dieser Schrift Propan verstanden, das die Reaktionszone A noch nicht durchlaufen hat. In der Regel wird es Roh-Propan (das vorzugsweise die Spezifikationen gemäß DE-A 10246119 und DE-A 10245585 erfüllt) sein, das in geringen Mengen auch von Propan verschiedene Komponenten enthält.

Unter dem Reaktionspfad in der Reaktionszone A wird in dieser Schrift der Strömungsweg des der Reaktionszone A über die von Restgas aus der Trennzone B verschiedenen (anderen) Zuführströme zugeführten Propans durch die Reaktionszone A in Abhängigkeit vom dehydrierenden Umsatz (der Umsatz in der heterogen katalysierten Dehydrierung) dieses Propans verstanden.

Das der Reaktionszone A zugeführte Reaktionsgasausgangsgemisch oder auch Beschickungsgasgemisch soll in dieser Schrift die Summe aller mit dem Frischpropan auf derselben Höhe des Reaktionspfads in der Reaktionszone A derselben zugeführten Gase sein.

Erfindungsgemäß bevorzugt erfolgt die Rückführung des (Haupt- und/oder Neben- bzw. Gesamt-)Restgases längs des Reaktionspfads in der Reaktionszone A in die Reaktionszone A so, dass an der Zufuhrstelle bereits wenigstens 10 oder wenigstens 15 mol%, bevorzugt bereits wenigstens 20 oder wenigstens 25 mol% und ganz besonders bevorzugt wenigstens 30 oder wenigstens 35 mol-% und am besten wenigstens 35 oder wenigstens 40 mol% des der Reaktionszone A über die anderen Zufuhrströme (davor) (insgesamt) zugeführten Propan in der Reaktionszone A (dehydrierend) umgesetzt (Umsatz $Z_U$) sind. In der Regel sind an der Zufuhrstelle der Rückführung des (Haupt- und/oder Neben- bzw. Gesamt-)Restgases in die Reaktionszone A beim erfindungsgemäßen Verfahren weniger als 70 mol-%, häufig weniger als 60 mol-% und vielfach $\leq$ 50 mol-% des der Reaktionszone A über die anderen Zufuhrströme zugeführten Propan in der Reaktionszone A bereits dehydrierend umgesetzt (Umsatz Zu).

Wird beim erfindungsgemäßen Verfahren, wie in der DE-A 10211275 empfohlen, das in der Reaktionszone A gebildete Produktgasgemisch A in zwei Teilmengen identischer Zusammensetzung aufgeteilt und eine der beiden Teilmengen in die Reaktionszone A zurückgeführt (insbesondere dann, wenn diese Rückführung als Bestandteil des der Reaktionszone A zugeführten Reaktionsgasausgangsgemischs erfolgt), so gelten anstelle aller vorgenannten Umsatzzahlen Zu vorzugsweise die wie nachfolgend definierten Umsatzzahlen $Z_U^{Kr}$:

$$Z_u^{Kr} = \frac{Z_U}{1+KGV}.$$

KGV ist dabei das Verhältnis von in die Reaktionszone A rückgeführter Teilmenge des Produktgasgemischs A zu in der Reaktionszone A gebildeter Gesamtmenge an Produktgasgemisch A.

Der Gehalt des in die Reaktionszone A rückgeführten, in der Trennzone B anfallenden, (Haupt- und/oder Neben- bzw. Gesamt-)Restgases an molekularem Sauerstoff wird beim erfindungsgemäßen Verfahren normalerweise 0,5 Vol.-% bis 10 Vol.%, häufig 1 bis 8 Vol.-% und vielfach 2 bis 5 Vol.-% betragen. Er rührt üblicherweise insbesondere daher, dass sich in der Reaktionszone B ein Überschuß an molekularem Sauerstoff (bezogen auf die Stöchiometrie der erwünschten Zielreaktion) in der Regel vorteilhaft auf die Standzeit der Oxidationskatalysatoren und auf die Kinetik der darin ablaufenden selektiven heterogen katalysierten Gasphasenpartialoxidation des Propylens zu Acrolein, oder zu Acrylsäure, oder deren Gemisch auswirkt. Im Unterschied zu den Verhältnissen in der erfindungsgemäßen Reaktionszone A, werden die thermodynamischen Verhältnisse in der Reaktionszone B durch das molare Reaktandenverhältnis im wesentlichen nicht beeinflusst, da die selektive heterogen katalysierte Gasphasenpartialoxidation des Propylens zu Acrolein, oder zu Acrylsäure, oder zu deren Gemisch kinetischer Kontrolle unterliegt.

Das Verhältnis der Propanmenge, die der Reaktionszone A über das aus der Trennzone B stammende rückgeführte (Haupt- und/oder Neben- bzw. Gesamt-)Restgas zugeführt wird, zu der Propanmenge, die der Reaktionszone A über andere Propan enthaltende Zuführströme insgesamt zugeführt wird, wird beim erfindungsgemäßen Verfahren in der Regel 0,1 bis 10, oder 0,5 bis 5, bevorzugt 0,5 bis 1,5 oder 3 bis 5 betragen.

In einer Ausführungsform wird man beim erfindungsgemäßen Verfahren in der Reaktionzone A neben dem im aus der Trennzone B stammenden rückgeführten (Haupt-und/oder Neben- bzw. Gesamt-)Restgas enthaltenen Propan als lediglich zweiten Zufuhrstrom nur noch einen Strom an frischem Roh-Propan (enthält neben Propan noch geringe Mengen an Verunreinigungen) zuführen. Erfindungsgemäß bevorzugt wird dieses Roh-Propan (wie generell in dieser Schrift) die in den Schriften DE-A 10245585 und DE-A 10246119 empfohlenen Spezifikationen aufweisen. Das gleiche gilt für die Spezifikation des Beschickungsgasgemischs der Reaktionszone B beim erfindungsgemäßen Verfahren. Selbstverständlich können beim erfindungsgemäßen Verfahren aber auch Propan (sowie gegebenenfalls Propylen) enthaltende Abgasströme aus anderen als dem erfindungsgemäßen Verfahren als Zufuhrströme in die Reaktionszone A mitverwendet werden (vgl. WO 02/00587; der Gasstrom aus Abschnitt c) dieser WO kann aber auch erst der Reaktionszone B zugeführt werden).

Erfindungswesentlich ist lediglich, dass an der Stelle (auf der Höhe) der Reaktionszone A, an welcher die Zufuhr des (Haupt- und/oder Neben- bzw. Gesamt-)Restgases aus der Trennzone B in die Reaktionszone A hinein erfolgt, wenigstens 5 mol-% des der Reaktionszone A über alle anderen Zufuhrströme zugeführten Propan innerhalb der sich in der Reaktionszone A zu vollziehenden katalytischen Dehydrierung bereits umgesetzt worden sind. Wird vorgenannte Bedingung nicht erfüllt, ist das molare Verhältnis von molekularem Wasserstoff zu molekularem Sauerstoff an der Zuführstelle gemindert, was entweder die Vollverbrennung der involvierten $C_3$-Kohlenwasserstoffe fördert und oder die Standzeit des verwendeten Dehydrierkatalysators mindert und/oder zwingend die Zufuhr von molekularem Wasserstoff aus einer externen Quelle in die Reaktionszone A erforderlich macht. Dies ist vermutlich darauf zurückzuführen, dass sowohl die involvierten $C_3$-Kohlenwasserstoffe als auch der Dehydrierkatalysator selbst in einer Atmosphäre mit einem erhöhten molaren Verhältnis von molekularem Wasserstoff zu molekularem Sauerstoff besser vor Vollverbrennung und thermischer (deaktivierend wirkender) Zersetzung am Dehydrierkatalysator geschützt sind.

Im Unterschied zur exotherm verlaufenden heterogen katalysierten Oxidehydrierung, die durch anwesenden Sauerstoff erzwungen und bei der intermediär kein freier Wasserstoff gebildet wird (der dem zu dehydrierenden Kohlenwasserstoff entrissene Wasserstoff wird unmittelbar als Wasser ($H_2O$) entrissen) bzw. nachweisbar ist, soll unter der erfindungsgemäßen heterogen katalysierten Dehydrierung eine ("konventionelle") Dehydrierung verstanden werden, deren Wärmetönung im Unterschied zur Oxidehydrierung endotherm ist (als Folgeschritt kann eine exotherme Wasserstoffverbrennung im erfindungsgemäßen Verfahren einbezogen sein) und bei der wenigstens intermediär freier molekularer Wasserstoff gebildet wird. Dazu bedarf es in der Regel anderer Reaktionsbedingungen und anderer Katalysatoren als zur Oxidehydrierung.

D.h., das erfindungsgemäße Verfahren verläuft normalerweise unter $H_2$-Entwicklung und dies insbesondere bis zur Rückführung des Restgases aus der Trennzone B in die Reaktionszone A, weshalb das Reaktionsgasgemisch an der zugehörigen Zuführstelle, bezogen auf die in ihm enthaltene molare Menge an Propan, normalerweise einen höheren molaren Wasserstoffgehalt aufweist, als das der Reaktionszone A zugeführte Reaktionsgasausgangsgemisch.

Letzteres trifft beim erfindungsgemäßen Verfahren vorzugsweise auch noch auf das Reaktionsgasgemisch zu, das sich an der Zufuhrstelle durch Vereinigung von aus dem in diese rückgeführten Restgas und dem vor dieser Rückführung an der Zuführstelle vorliegenden Reaktionsgasgemisch bildet.

Im übrigen hat es sich für das erfindungsgemäße Verfahren als günstig erwiesen, wenn innerhalb der Reaktionszone A das molare Verhältnis von im Reaktionsgasgemisch enthaltenem Propylen zu im Reaktionsgasgemisch enthaltenem molekularem Wasserstoff den Wert 10, vorzugsweise den Wert 5, besser den Wert 3 und noch besser den Wert 2 nicht überschreitet. Besonders bevorzugt bewegt sich das vorgenannte Verhältnis bei Werten von 0,5 bzw. 1 bis 2.

Im übrigen kann das Prinzip der heterogen katalysierten Dehydrierung in der Reaktionszone A (und damit die Reaktionszone A selbst) beim erfindungsgemäßen Verfahren wie in den Schriften WO 01/96270, DE-A 3313573, DE-A 10131297, DE-A 10245585, DE-A 10246119 sowie DE-A 10211275 beschrieben gestaltet werden. D.h., bezogen auf den einmaligen Duchgang des der Reaktionszone A zugeführten Beschickungsgasgemischs durch die Reaktionszone A, kann die Reaktionszone A durch gezielten Wärmeaustausch mit außerhalb der Reaktionszone A geführten (fluiden, d.h., flüssig oder gasförmig) Wärmeträgern isotherm gestaltet werden. Sie kann mit gleicher Bezugsbasis aber auch adiabat, d.h., im wesentlichen ohne einen solchen gezielten Wärmeaustausch mit außerhalb der Reaktionszone A geführten Wärmeträgern ausgeführt werden. Im letzteren Fall kann die Bruttowärmetönung, bezogen auf den einmaligen Durchgang des der Reaktionszone A zugeführten Reaktionsgasausgangsgemischs durch die Reaktionszone A, durch Ergreifen von in den vorstehenden Schriften empfohlenen und im Folgenden noch zu beschreibenden Maßnahmen sowohl endotherm (negativ), oder autotherm (im wesentlichen Null) oder exotherm (positiv) gestaltet werden. In erfindungsgemäßer Weise muß lediglich zusätzlich die Rückführung von aus der Trennzone B stammendem Restgas in die Reaktionszone A hinein wie erfindungsgemäß gefordert erfolgen. Ebenso können die in vorgenannten Schriften empfohlenen Katalysatoren beim erfindungsgemäßen Verfahren angewendet werden.

In typischer Weise benötigt die heterogen katalysierte partielle Dehydrierung von Propan zu Propylen vergleichsweise hohe Reaktionstemperaturen. Der dabei erzielbare Umsatz ist normalerweise durch das thermodynamische Gleichgewicht begrenzt. Typische Reaktionstemperaturen betragen 300 bis 800°C bzw. 400 bis 700°C. Pro Molekül an zu Propylen dehydriertem Propan wird dabei ein Molekül Wasserstoff erzeugt.

Hohe Temperaturen und Entfernung des Reaktionsproduktes $H_2$ begünstigen die Gleichgewichtslage im Sinne des Zielprodukts.

Da die heterogen katalysierte Dehydrierreaktion unter Volumenzunahme abläuft, kann der Umsatz durch Erniedrigung des Partialdrucks der Produkte gesteigert werden. Dies läßt sich in einfacher Weise, zum Beispiel durch Dehydrierung bei vermindertem Druck und/oder durch Zumischen von im wesentlichen inerten Verdünnungsgasen, wie zum Beispiel Wasserdampf, erreichen, der für die Dehydrierreaktion im Normalfall ein Inertgas darstellt. Eine Verdünnung mit Wasserdampf bedingt als weiteren Vorteil in der Regel ein vermindertes Verkoken des verwendeten Katalysators, da der Wasserdampf mit gebildetem Koks nach dem Prinzip der Kohlevergasung reagiert. Außerdem kann Wasserdampf als Verdünnungsgas in der nachfolgenden Reaktionszone B mitverwendet werden. Wasserdampf läßt sich aber auch in einfacher Weise teilweise oder vollständig aus dem Produktgasgemisch der Dehydrierung (dem Produktgasgemisch A) abtrennen (zum Beispiel durch Kondensieren), was die Möglichkeit eröffnet, bei der Weiterverwendung des dabei erhältlichen modifizierten Produktgasgemisches (des Produktgasgemischs A') in der Reaktionszone B den Anteil des Verdünnungsgases $N_2$ zu erhöhen. Weitere für die heterogen katalysierte Propandehydrierung geeignete Verdünnungsmittel sind zum Beispiel CO, Methan, Ethan, $CO_2$, Stickstoff und Edelgase wie He, Ne und Ar. Alle genannten Verdünnungsmittel können entweder für sich oder in Form unterschiedlichster Gemische mitverwendet werden. Es ist von Vorteil, dass die genannten Verdünnungsmittel in der Regel auch in der Reaktionszone B geeignete Verdünnungsmittel sind. Generell sind beim erfindungsgemäßen Verfahren sich in der jeweiligen Reaktionszone inert verhaltende (das heißt zu weniger als 5 mol-%, bevorzugt zu weniger als 3 mol-% und noch besser zu weniger als 1 mol-% sich chemisch verändernde) Verdünnungsmittel bevorzugt.

Prinzipiell kommen für die heterogen katalysierte Propandehydrierung alle im Stand der Technik bekannten Dehydrierkatalysatoren in Betracht. Sie lassen sich grob in zwei Gruppen unterteilen. Nämlich in solche, die oxidischer Natur sind (zum Beispiel Chromoxid und/oder Aluminiumoxid) und in solche, die aus wenigstens einem auf einem, in der Regel oxidischen, Träger abgeschiedenen, in der Regel vergleichsweise edlen, Metall (zum Beispiel Platin) bestehen. Unter anderem können damit alle Dehydrierkatalysatoren eingesetzt werden, die in der WO 01/96270, der EP-A 731077, der DE-A 10211275, der DE-A 10131297, der WO 99/46039, der US-A 4 788 371, der EP-A-0 705 136, der WO 99/29420, der US-A 4 220 091, der US-A 5 430 220, der US-A 5 877 369, der EP-A-0 117 146, der DE-A 199 37 196, der DE-A 199 37 105 sowie der DE-A 199 37 107 empfohlen werden. Im besonderen können sowohl der Katalysator gemäß Beispiel 1, Beispiel 2, Beispiel 3, und Beispiel 4 der DE-A 199 37 107 eingesetzt werden.

Dabei handelt es sich um Dehydrierkatalysatoren, die 10 bis 99,9 Gew.-% Zirkondioxid, 0 bis 60 Gew.-% Aluminiumoxid, Siliciumdioxid und/oder Titandioxid und 0,1 bis 10 Gew.-% mindestens eines Elements der ersten oder zweiten Hauptgruppe, eines Elements der dritten Nebengruppe, eines Elements der achten Nebengruppe des Periodensystems der Elemente, Lanthan und/oder Zinn enthalten, mit der Maßgabe, dass die Summe der Gewichtsprozente 100 Gew.-% ergibt.

Besonders geeignet ist auch der in den Beispielen und Vergleichsbeispielen dieser Schrift eingesetzte Dehydrierkatalysator.

Generell kann es sich bei den Dehydrierkatalysatoren um Katalysatorstränge (Durchmesser typisch 1 bis 10 mm, bevorzugt 1,5 bis 5 mm; Länge typisch 1 bis 20 mm, bevorzugt 3 bis 10 mm), Tabletten (vorzugsweise gleiche Abmessungen wie bei den Strängen) und/oder Katalysatorringe (Außendurchmesser und Länge jeweils typisch 2 bis 30 mm oder bis 10 mm, Wandstärke zweckmäßig 1 bis 10 mm, oder bis 5 mm, oder bis 3 mm) handeln.

In der Regel sind die Dehydrierkatalysatoren (insbesondere die in dieser Schrift beispielhaft verwendeten sowie die in der DE-A 19937107 empfohlenen (insbesondere die beispielhaften Katalysatoren dieser DE-A) so beschaffen, dass sie sowohl die Dehydrierung von Propan als auch die Verbrennung von molekularem Wasserstoff zu katalysieren vermögen. Die Wasserstoffverbrennung verläuft dabei im Vergleich zur Dehydrierung des Propans im Fall einer Konkurrenzsituation an den Katalysatoren sehr viel schneller.

Zur Durchführung der heterogen katalysierten Propandehydrierung kommen prinzipiell alle im Stand der Technik bekannten Reaktortypen und Verfahrensvarianten in Betracht. Beschreibungen solcher Verfahrensvarianten enthalten zum Beispiel alle bezüglich der Dehydrierkatalysatoren zitierten Schriften des Standes der Technik sowie der eingangs dieser Schrift zitierte Stand der Technik.

Eine vergleichsweise ausführliche Beschreibung von erfindungsgemäß geeigneten Dehydrierverfahren enthält auch Catalytica® Studies Division, Oxidative Dehydrogenation and Alternative Dehydrogenation Processes, Study Number 4192 OD, 1993, 430 Ferguson Drive, Mountain View, California, 94043-5272 U.S.A..

Charakteristisch für die partielle heterogen katalysierte Dehydrierung von Propan ist, wie bereits gesagt, dass sie endotherm verläuft. Das heißt, die für die Einstellung der erforderlichen Reaktionstemperatur sowie die für die Reaktion benötigte Wärme (Energie) muß entweder dem Reaktionsgasausgangsgemisch vorab und/oder im Verlauf der heterogen katalysierten Dehydrierung zugeführt werden. Gegebenenfalls muß das Reaktionsgasgemisch die benötigte Reaktionswärme sich selbst entziehen.

Ferner ist es für heterogen katalysierte Dehydrierungen von Propan aufgrund der hohen benötigten Reaktionstemperaturen typisch, dass in geringen Mengen schwersiedende hochmolekulare organische Verbindungen, bis hin zum Kohlenstoff, gebildet werden, die sich auf der Katalysatoroberfläche abscheiden und selbige dadurch deaktivieren. Um diese nachteilige Begleiterscheinung zu minimieren, kann das zur heterogen katalysierten Dehydrierung bei erhöhter Temperatur über die Katalysatoroberfläche zu leitende, Propan haltige Reaktionsgasgemisch mit Wasserdampf verdünnt werden. Sich abscheidender Kohlenstoff wird unter den so gegebenen Bedingungen nach dem Prinzip der Kohlevergasung teilweise oder vollständig eliminiert.

Eine andere Möglichkeit, abgeschiedene Kohlenstoffverbindungen zu beseitigen, besteht darin, den Dehydrierkatalysator von Zeit zu Zeit bei erhöhter Temperatur mit einem Sauerstoff enthaltenden Gas (zweckmäßig in Abwesenheit von Kohlenwasserstoffen) zu durchströmen und damit den abgeschiedenen Kohlenstoff quasi abzubrennen. Eine weitgehende Unterdrückung der Bildung von Kohlenstoffablagerungen ist aber auch dadurch möglich, dass man dem heterogen katalysiert zu dehydrierenden Propan, bevor es bei erhöhter Temperatur über den Dehydrierkatalysator geführt wird, molekularen Wasserstoff zusetzt.

Selbstverständlich besteht auch die Möglichkeit, dem heterogen katalysiert zu dehydrierenden Propan ein Gemisch aus Wasserdampf und molekularem Wasserstoff zuzusetzen. Ein Zusatz von molekularem Wasserstoff zur heterogen katalysierten Dehydrierung von Propan mindert auch die unerwünschte Bildung von Allen (Propadien), Propin und Acetylen als Nebenprodukten. Teiloxidation von solchermaßen zugesetztem Wasserstoff vermag gleichfalls benötigte Reaktionswärme zu liefern.

Eine geeignete Reaktorform für die heterogen katalysierte Propandehydrierung in der Reaktionszone A ist der Festbettrohr- beziehungsweise Rohrbündelreaktor. Das heißt, der Dehydrierkatalysator befindet sich in einem oder in einem Bündel von Reaktionsrohren als Festbett. Im Kontaktrohr kann erfindungsgemäß vorteilhaft zentriert ein zweites Rohr verlaufen, das auf unterschiedlichsten Höhen (oder nur auf einer Höhe) Austrittsstellen aufweist (vgl. WO 01/85333 und WO 01/85330), über die das Restgas aus der Trennzone B zugeführt werden kann. Alternativ können die in Kontaktrohrböden eingebundenen Kontaktrohre Unterbrechungsabschnitte (räume) aufweisen, in die das Restgas aus der Trennzone B zudosiert werden kann. Die Reaktionsrohre werden dadurch beheizt, dass im die Reaktionsrohre umgebenden Raum ein Gas, zum Beispiel ein Kohlenwasserstoff wie Methan, verbrannt wird. Günstig ist es, diese direkte Form der Kontaktrohrerwärmung lediglich auf den ersten etwa 20 bis 30% der Festbettschüttung anzuwenden und die verbleibende Schüttungslänge durch die im Rahmen der Verbrennung freigesetzte Strahlungswärme auf die erforderliche Reaktionstemperatur aufzuwärmen. Auf diesem Weg ist eine annähernd isotherme Reaktionsführung erreichbar. Geeignete Reaktionsrohrinnendurchmesser betragen etwa 10 bis 15 cm. Ein typischer Dehydrierrohrbündelreaktor umfaßt 300 bis 1000 Reaktionsrohre. Die Temperatur im Reaktionsrohrinneren bewegt sich im Bereich von 300 bis 700°C, vorzugsweise im Bereich von 400 bis 700°C. Mit Vorteil wird das Reaktionsgasausgangsgemisch dem Rohrreaktor auf die Reaktionstemperatur vorerwärmt zugeführt. Es ist möglich, dass das Produktgasgemisch A das Reaktionsrohr mit einer 50 bis 100°C tiefergelegenen Temperatur verläßt. Diese Ausgangstemperatur kann aber auch höher oder auf gleichem Niveau liegen. Im Rahmen der vorgenannten Verfahrensweise ist die Verwendung von oxidischen Dehydrierkatalysatoren auf der Grundlage von Chrom- und/oder Aluminiumoxid zweckmäßig. Häufig wird man kein Verdünnungsgas mitverwenden, sondern von im wesentlichen alleinigem Roh-Propan als Ausgangsreaktionsgas ausgehen. Auch der Dehydrierkatalysator wird meist unverdünnt angewandt.

Großtechnisch kann man in der Reaktionszone A mehrere (z.B. drei) solcher Rohrbündelreaktoren parallel betreiben. Dabei können sich erfindungsgemäß gegebenenfalls einer (oder zwei) dieser Reaktoren im Dehydrierbetrieb befinden, während in einem zweiten (dritten) Reaktor die Katalysatorbeschickung regeneriert wird, ohne dass der Betrieb in der Reaktionszone B leidet.

Eine solche Verfahrensweise ist beispielsweise bei dem in der Literatur bekannten BASF-Linde Propan-Dehydrierverfahren zweckmäßig. Erfindungsgemäß ist es aber von Bedeutung, dass die Verwendung eines solchen Rohrbündelreaktors ausreichend ist.

Eine solche Verfahrensweise ist auch beim sog. "steam active reforming (STAR) process" anwendbar, der von der Phillips Petroleum Co. entwickelt wurde (vergleiche zum Beispiel US-A 4 902 849, US-A 4 996 387 und US-A 5 389 342). Als Dehydrierkatalysator wird im STAR-Prozess mit Vorteil Promotoren enthaltendes Platin auf Zink (Magnesium) Spinell als Träger angewendet (vergleiche zum Beispiel US-A 5 073 662). Im Unterschied zum BASF-Linde Propan-Dehydrierverfahren wird das zu dehydrierende Propan beim STAR-Prozess mit Wasserdampf verdünnt. Typisch ist ein molares Verhältnis von Wasserdampf zu Propan im Bereich von 4 bis 6. Der Reaktorausgangsdruck liegt häufig bei 3 bis 8 bar und die Reaktionstemperatur wird zweckmäßig zu 480 bis 620°C gewählt. Typische Katalysator(bett)belastungen mit Propan liegen bei 200 bis 4000 $h^{-1}$ (GHSV).

Unter der Belastung eines einen Reaktionsschritt katalysierenden Katalysatorbetts mit Reaktionsgas(ausgangs)gemisch wird dabei die Menge an Reaktionsgas(ausgangs)gemisch in Normlitern (= Nl; das Volumen in Litern, das die entsprechende Reaktionsgas(ausgangs)gemischmenge bei Normalbedingungen, d.h., bei 0°C und 1 atm einnehmen würde) verstanden, die pro Stunde durch einen Liter Katalysatorbett geführt wird. Die Belastung kann auch nur auf einen Bestandteil des Reaktionsgas(ausgangs)gemischs bezogen sein. Dann ist es die Menge dieses Bestandteils in Nl/l•h, die pro Stunde durch einen Liter des Katalysatorbetts geführt wird. Anstelle von Nl/l•h wird häufig verkürzt "$h^{-1}$" geschrieben.

Die heterogen katalysierte Propandehydrierung kann beim erfindungsgemäßen Verfahren auch im Wanderbett gestaltet werden. Beispielsweise kann das Katalysatorwanderbett in einem Radialstromreaktor untergebracht sein. In selbigem bewegt sich der Katalysator langsam von oben nach unten, während das Reaktionsgasgemisch radial fließt. Diese Verfahrensweise wird beispielsweise im sogenannten UOP-Oleflex-Dehydrierverfahren angewandt. Da die Reaktoren bei diesem Verfahren quasi adiabat betrieben werden, ist es zweckmäßig, mehrere Reaktoren als Kaskade hintereinander geschaltet zu betreiben (in typischer Weise bis zu vier). Innerhalb der Kaskade kann das Restgas aus der Trennzone B zugeführt werden. Dadurch lassen sich zu hohe Unterschiede der Temperaturen des Reaktionsgasgemisches am Reaktoreingang und am Reaktorausgang vermeiden (bei der adiabaten Betriebsweise fungiert das Reaktionsgasausgangsgemisch als Wärmeträger, von dessen Wärmeinhalt der Abfall der Reaktionstemperatur abhängig ist) und trotzdem ansprechende Gesamtumsätze erzielen.

Wenn das Katalysatorbett den Wanderbettreaktor verlassen hat, wird es der Regenerierung zugeführt und anschließend wiederverwendet. Als Dehydrierkatalysator kann für dieses Verfahren zum Beispiel ein kugelförmiger Dehydrierkatalysator eingesetzt werden, der im wesentlichen aus Platin auf kugelförmigem Aluminiumoxidträger besteht. Bei der UOP-Variante wird dem zu dehydrierenden Propan Wasserstoff zugefügt, um eine vorzeitige Katalysatoralterung zu vermeiden. Der Arbeitsdruck liegt typisch bei 2 bis 5 atm. Das Wasserstoff zu Propan-Verhältnis (das molare) beträgt zweckmäßig 0,1 bis 1. Die Reaktionstemperaturen liegen bevorzugt bei 550 bis 650°C und die Verweilzeit des Katalysators in einem Reaktor wird zu etwa 2 bis 10 h gewählt.

Bei den beschriebenen Festbettverfahren kann die Katalysatorgeometrie ebenfalls kugelförmig, aber auch zylindrisch (hohl oder voll) oder anderweitig geometrisch gestaltet sein.

Als weitere mögliche Verfahrensvariante für die heterogen katalysierte Propandehydrierung im erfindungsgemäßen Verfahren beschreibt Proceedings De Witt, Petrochem. Review, Houston, Texas, 1992 a, N1 die Möglichkeit einer heterogen katalysierten Propandehydrierung im Wirbelbett.

Erfindungsgemäß können dabei z.B. zwei Wirbelbetten nebeneinander betrieben werden, von denen sich eines ohne negative Auswirkungen auf den Gesamtprozess zeitweise im Zustand der Regenerierung befinden kann. Als Aktivmasse kommt dabei Chromoxid auf Aluminiumoxid zum Einsatz. Der Arbeitsdruck beträgt typisch 1 bis 2 bar und die Dehydriertemperatur liegt in der Regel bei 550 bis 600°C. Die für die Dehydrierung erforderliche Wärme wird dadurch in das Reaktionssystem eingebracht, dass der Dehydrierkatalysator auf die Reaktionstemperatur vorerhitzt wird. Die vorstehende Dehydrierweise ist in der Literatur auch als Snamprogetti-Yarsintez Verfahren bekannt.

Alternativ zu den vorstehend beschriebenen Verfahrensweisen kann die heterogen katalysierte Propandehydrierung beim erfindungsgemäßen Verfahren auch nach einem von ABB Lummus Crest entwickelten Verfahren realisiert werden (vergleiche Proceedings De Witt, Petrochem. Review, Houston, Texas, 1992, P1).

Bei beiden vorgenannten Verfahren kann die erfindungsgemäße Restgaszugabe als einfacher Zufuhrstrom an der anspruchsgemäßen Reaktionspfadstelle erfolgen.

Den bisher beschriebenen heterogen katalysierten Dehydrierverfahren des Propans ist gemein, dass sie bei Propanumsätzen von ≥ 30 mol-% (in der Regel ≤ 60 mol-%) betrieben werden (bezogen auf einmaligen Durchgang durch die Reaktionszone und insgesamt zugeführtes Propan). Erfindungsgemäß von Vorteil ist jedoch, dass es ausreichend ist, einen Propanumsatz von ≥ 5 mol-% bis ≤ 30 mol-% oder ≤ 25 mol-%, zu erzielen. Das heißt, die erfindungsgemäße heterogen katalysierte Propandehydrierung kann in der Reaktionszone A auch bei Propanumsätzen von 10 bis 20 mol-% betrieben werden (die Umsätze beziehen sich auf einmaligen Durchgang durch die Reaktionszone A). Dies rührt unter anderem daher, dass die verbliebene Menge an nicht umgesetztem Propan in der nachfolgenden Reaktionszone B im wesentlichen als inertes Verdünnungsgas fungiert und erfindungsgemäß als Bestandteil des in der Trennzone B anfallenden Restgases weitgehend verlustfrei in die Reaktionszone A rückgeführt werden kann.

Erfindungsgemäß bevorzugt sind jedoch erhöhte Propanumsätze in der Reaktionszone A, da dies die Propionsäurenebenproduktbildung in der Reaktionszone B mindert. Solche vorteilhaften Propanumsätze betragen z.B. 30 oder 40 bis 50 oder 60 mol-% (bezogen auf einmaligen Durchgang durch die Reaktionszone A und insgesamt zugeführtes Propan).

Für die Realisierung der vorgenannten Propanumsätze ist es günstig, die heterogen katalysierte Propandehydrierung bei einem Arbeitsdruck von 0,3 bis 3 bar durchzuführen. Ferner ist es günstig, das heterogen katalytisch zu dehydrierende Propan mit Wasserdampf zu verdünnen. So ermöglicht die Wärmekapazität des Wassers einerseits, einen Teil der Auswirkung der Endothermie der Dehydrierung auszugleichen und andererseits reduziert die Verdünnung mit Wasserdampf den Edukt- und Produktpartialdruck, was sich günstig auf die Gleichgewichtslage der Dehydrierung auswirkt. Ferner wirkt sich die Wasserdampfmitverwendung, wie bereits erwähnt, vorteilhaft auf die Standzeit von Edelmetall enthaltenden Dehydrierkatalysatoren aus. Bei Bedarf kann als weiterer Bestandteil auch molekularer Wasserstoff zugegeben werden. Das molare Verhältnis von molekularem Wasserstoff zu Propan ist dabei in der Regel $\leq 5$. Das molare Verhältnis von Wasserdampf zu Propan kann z.B. $\geq 0$ bis 30, zweckmäßig 0,1 bis 2 und günstig 0,5 bis 1 betragen. Ein Vorteil einer Verfahrensweise mit niederem Propanumsatz ist, dass bei einmaligem Durchgang des Reaktionsgases durch die Reaktionszone A lediglich eine vergleichsweise niedrige Wärmemenge verbraucht wird und zur Umsatzerzielung vergleichsweise niedrige Reaktionstemperaturen ausreichend sind.

Auch kann die Propandehydrierung, wie bereits gesagt, erfindungsgemäß (quasi) adiabat und dabei endotherm durchgeführt werden. Dabei wird das Reaktionsgasausgangsgemisch in der Regel zunächst auf eine Temperatur von 500 bis 700°C (beziehungsweise von 550 bis 650°C) erhitzt (zum Beispiel durch Direktbefeuerung der es umgebenden Wandung). Beim adiabaten Durchgang durch das wenigstens eine Katalysatorbett wird sich das Reaktionsgasgemisch dann je nach Umsatz und Verdünnung um etwa 30°C bis 200°C abkühlen. Hinter der erfindungsgemäßen Eindüsung des Restgases aus der Trennzone B wird in gewissem Umfang Erwärmung erfolgen. Ein Beisein von Wasserdampf als Wärmeträger macht sich auch unter dem Gesichtspunkt einer adiabaten Fahrweise vorteilhaft bemerkbar. Niedrigere Reaktionstemperaturen ermöglichen längere Standzeiten des verwendeten Katalysatorbetts. Höhere Reaktionstemperaturen fördern erhöhte Umsätze.

Prinzipiell ist die heterogen katalysierte Propandehydrierung in der Reaktionszone A, unabhängig davon ob adiabat oder isotherm gefahren, sowohl in einem Festbettreaktor als auch in einem Wanderbett- oder Wirbelbettreaktor durchführbar.

Bemerkenswerterweise kann beim erfindungsgemäßen Verfahren als Reaktionszone A auch im adiabaten Betrieb ein einzelner Schachtofenreaktor als Festbettreaktor ausreichend sein, der vom Reaktionsgasgemisch axial und/oder radial durchströmt wird.

Im einfachsten Fall handelt es sich dabei um ein einziges geschlossenes Reaktionsvolumen, zum Beispiel einen Behälter, dessen Innendurchmesser 0,1 bis 10 m, eventuell auch 0,5 bis 5 m beträgt und in welchem das Katalysatorfestbett auf einer Trägervorrichtung (zum Beispiel ein Gitterrost) aufgebracht ist. Das mit Katalysator beschickte Reaktionsvolumen, das im adiabaten Betrieb im wesentlichen wärmeisoliert ist, wird dabei mit dem heißen, Propan enthaltenden, Reaktionsgas axial durchströmt. Die Katalysatorgeometrie kann dabei sowohl kugelförmig als auch ringförmig oder strangförmig sein. Über in die Katalysatorschüttung eingebrachte Zufuhrleitungen kann das aus der Trennzone B stammende Restgas eingedüst werden. Da in diesem Fall das Reaktionsvolumen durch einen sehr kostengünstigen Apparat zu realisieren ist, sind alle Katalysatorgeometrien, die einen besonders niedrigen Druckverlust aufweisen, zu bevorzugen. Das sind vor allem Katalysatorgeometrien, die zu einem großen Hohlraumvolumen führen oder strukturiert aufgebaut sind, wie zum Beispiel Monolithe bzw. Wabenkörper. Zur Verwirklichung einer radialen Strömung des Propan enthaltenden Reaktionsgases kann der Reaktor zum Beispiel aus zwei in einer Mantelhülle befindlichen, konzentrisch ineinander gestellten, zylindrischen Gitterrosten bestehen und die Katalysatorschüttung in deren Ringspalt angeordnet sein. Im adiabaten Fall wäre die Metallhülle gegebenenfalls wiederum thermisch isoliert.

Als erfindungsgemäße Katalysatorbeschickung für eine heterogen katalysierte Propandehydrierung eignen sich insbesondere auch die in der DE-A 199 37 107 offenbarten, vor allem alle beispielhaft offenbarten Katalysatoren.

Nach längerer Betriebsdauer sind vorgenannte Katalysatoren zum Beispiel in einfacher Weise dadurch regenerierbar, dass man bei einer Eintrittstemperatur von 300 bis 600°C, häufig bei 400 bis 550°C, zunächst in ersten Regenerierungsstufen mit Stickstoff und/oder Wasserdampf (bevorzugt) verdünnte Luft über das Katalysatorbett leitet. Die Katalysator(bett)belastung mit Regeneriergas (z.B. Luft) kann dabei z.B. 50 bis 10000 h$^{-1}$ und der Sauerstoffgehalt des Regeneriergases 0,1 oder 0,5 bis 20 Vol.-% betragen.

In nachfolgenden weiteren Regenerierungsstufen kann unter ansonsten gleichen Regenerierbedingungen als Regeneriergas Luft verwendet werden. Anwendungstechnisch zweckmäßig empfiehlt es sich, den Katalysator vor seiner Regenerierung mit Inertgas (zum Beispiel N$_2$) zu spülen.

Anschließend ist es in der Regel empfehlenswert, noch mit reinem molekularen Wasserstoff oder mit durch Inertgas (vorzusgweise Wasserdampf) verdünntem molekularem Wasserstoff (der Wasserstoffgehalt sollte $\geq 1$ Vol.-% betragen) im ansonsten gleichen Bedingungsraster zu regenerieren.

Die heterogen katalysierte Propandehydrierung mit vergleichsweise geringem Propanumsatz ($\leq 30$ mol-%)

kann in allen Fällen bei den gleichen Katalysator(bett)belastungen (sowohl das Reaktionsgas insgesamt, als auch das in selbigem enthaltende Propan betreffend) betrieben werden wie die Varianten mit hohem Propanumsatz (> 30 mol-%). Diese Belastung mit Reaktionsgas kann zum Beispiel 100 bis 40000 oder bis 10000 h$^{-1}$, häufig 300 bis 7000 h$^{-1}$, das heißt vielfach etwa 500 bis 4000 h$^{-1}$ betragen.

Anwendungstechnisch zweckmäßig läßt sich die Reaktionszone A beim erfindungsgemäßen Verfahren als Hordenreaktor verwirklichen, was die Zudosierung des Restgases aus der Trennzone B zwischen zwei Horden in besonders einfacher Weise ermöglicht.

Dieser enthält räumlich aufeinanderfolgend mehr als ein die Dehydrierung katalysierendes Katalysatorbett. Die Katalysatorbettanzahl kann 1 bis 20, zweckmäßig 2 bis 8, aber auch 3 bis 6 betragen. Mit zunehmender Hordenzahl lassen sich zunehmend leichter erhöhte Propanumsätze erreichen. Die Katalysatorbetten sind vorzugsweise radial oder axial hintereinander angeordnet. Anwendungstechnisch zweckmäßig wird in einem solchen Hordenreaktor der Katalysatorfestbetttyp angewendet.

Im einfachsten Fall sind die Katalysatorfestbetten in einem Schachtofenreaktor axial oder in den Ringspalten von zentrisch ineinandergestellten zylindrischen Gitterrosten angeordnet. Es ist jedoch auch möglich, die Ringspalte in Segmenten übereinander anzuordnen und das Gas nach radialem Durchtritt in einem Segment in das nächste darüber- oder darunterliegende Segment zu führen.

In zweckmäßiger Weise wird das Reaktionsgasgemisch auf seinem Weg von einem Katalysatorbett zum nächsten Katalysatorbett, zum Beispiel durch Überleiten über mit heißen Gasen erhitzte Wärmetauscheroberflächen (z.B. Rippen) oder durch Leiten durch mit heißen Brenngasen erhitzte Rohre, im Hordenreaktor einer Zwischenerhitzung unterworfen.

Wird der Hordenreaktor im übrigen adiabat betrieben, ist es für Propanumsätze ≤ 30 mol-%, vor allem bei Verwendung der in der DE-A 199 37 107 beschriebenen Katalysatoren, insbesondere der beispielhaften Ausführungsformen, ausreichend, das Reaktionsgasgemisch auf eine Temperatur von 450 bis 550°C vorerhitzt in den Dehydrierreaktor zu führen und innerhalb des Hordenreaktors in diesem Temperaturbereich zu halten. Das heißt, die gesamte Propandehydrierung ist so bei äußerst niederen Temperaturen zu verwirklichen, was sich für die Standzeit der Katalysatorfestbetten zwischen zwei Regenerierungen als besonders günstig erweist. Für höhere Propanumsätze wird das Reaktionsgasgemisch zweckmäßig auf höhere Temperaturen vorerhitzt in den Dehydrierreaktor geführt (diese können bis zu 700°C betragen) und innerhalb des Hordenreaktors in diesem erhöhten Temperaturbereich gehalten.

Noch geschickter ist es, die vorstehend geschilderte Zwischenerhitzung auf direktem Weg durchzuführen (autotherme Fahrweise). Dazu wird dem Reaktionsgasgemisch entweder bereits vor Durchströmung des ersten Katalysatorbettes (dann sollte das Reaktionsgasausgangsgemisch molekularen Wasserstoff zugesetzt enthalten) und/oder zwischen den nachfolgenden Katalysatorbetten in begrenztem Umfang molekularer Sauerstoff zugesetzt. So kann (in der Regel durch die Dehydrierkatalysatoren selbst katalysiert) eine begrenzte Verbrennung von im Reaktionsgasgemisch enthaltenem, im Verlauf der heterogen katalysierten Propandehydrierung gebildetem und/oder dem Reaktionsgasgemisch zugesetztem molekularem Wasserstoff bewirkt werden (es kann auch anwendungstechnisch zweckmäßig sein, im Hordenreaktor Katalysatorbetten einzufügen, die mit Katalysator beschickt sind, der besonders spezifisch (selektiv) die Verbrennung von Wasserstoff katalysiert (als solche Katalysatoren kommen zum Beispiel jene der Schriften US-A 4 788 371, US-A 4 886 928, US-A 5 430 209, US-A 5 530 171, US-A 5 527 979 und US-A 5 563 314 in Betracht; beispielsweise können solche Katalysatorbetten in alternierender Weise zu den Dehydrierkatalysator enthaltenden Betten im Hordenreaktor untergebracht sein). Die dabei freigesetzte Reaktionswärme ermöglicht so auf quasi autotherme (die Bruttowärmetönung ist im wesentlichen Null) Weise eine nahezu isotherme Betriebsweise der heterogen katalysierten Propandehydrierung. Mit zunehmender gewählter Verweilzeit des Reaktionsgases im Katalysatorbett ist so eine Propandehydrierung bei abnehmender oder im wesentlichen konstanter Temperatur möglich, was besonders lange Standzeiten zwischen zwei Regenerierungen ermöglicht.

Generell sollte erfindungsgemäß eine wie vorstehend beschriebene Sauerstoffeinspeisung so vorgenommen werden, dass der Sauerstoffgehalt des Reaktionsgasgemisches, bezogen auf die darin enthaltene Menge an molekularem Wasserstoff, 0,5 bis 50 bzw. bis 30, vorzugsweise 10 bis 25 Vol.-% beträgt. Als Sauerstoffquelle kommen dabei sowohl reiner molekularer Sauerstoff oder mit Inertgas, zum Beispiel CO, $CO_2$, $N_2$ und/oder Edelgase, verdünnter Sauerstoff, insbesondere aber auch Luft, sowie Stickoxide in Betracht. Die resultierenden Verbrennungsgase wirken in der Regel zusätzlich verdünnend und fördern dadurch die heterogen katalysierte Propandehydrierung.

Im Rahmen der erfindungsgemäßen Verfahrensweise bildet das aus der Trennzone B in die Reaktionszone A rückgeführte (Haupt- und/oder Neben- bzw. Gesamt-)Restgas eine besonders reichhaltige Quelle an molekularem Sauerstoff, die erfindungsgemäß dem Reaktionsgasgemisch im Hordenreaktor in der Regel frühestens nach Durchlaufern des ersten Katalysatorbetts zugefügt würde.

Die Isothermie der heterogen katalysierten Propandehydrierung läßt sich dadurch weiter verbessern, dass man im Hordenreaktor in den Räumen zwischen den Katalysatorbetten geschlossene, vor ihrer Füllung günstigerweise aber nicht notwendigerweise evakuierte, Einbauten (zum Beispiel rohrförmige), anbringt. Derartige Einbauten können auch ins jeweilige Katalysatorbett gestellt werden. Diese Einbauten enthalten geeignete Feststoffe oder Flüssigkeiten, die oberhalb einer bestimmten Temperatur verdampfen oder schmelzen und dabei Wärme verbrauchen und dort, wo diese Temperatur unterschritten wird, wieder kondensieren und dabei Wärme freisetzen.

Selbstredend lässt sich die Reaktionszone A des erfindungsgemäßen Verfahrens auch wie in der DE-A 10211275 beschrieben (als "Schlaufenvariante") realisieren, die einen integralen Bestandteil dieser Patentanmeldung bildet.

D.h., eine Ausführungsform des erfindungsgemäßen Verfahrens ist ein Verfahren, bei dem man der Reaktionszone A wenigstens drei gasförmige, Propan enthaltende, Zufuhrströme zuführt, von denen wenigstens einer Frischpropan enthält und wenigstens einer aus der Trennzone B in die Reaktionszone A rückgeführtes, molekularen Sauerstoff, nicht umgesetztes Propan und (in der Reaktionszone B) gegebenenfalls nicht umgesetztes Propylen enthaltendes (Haupt- und/oder Neben- bzw. Gesamt-)Restgas ist, und in der Reaktionszone A ihr so zugeführtes Propan einer heterogen katalysierten (partiellen) Dehydrierung unterwirft (prinzipiell kommen dazu alle in dieser Schrift genannten und ausgeführten heterogen katalysierten Dehydrierverfahren in Betracht), wobei ein Propan und Propylen enthaltendes Produktgasgemisch A erhalten wird, mit der Maßgabe, dass man

a) das Produktgasgemisch A in zwei Teilmengen identischer Zusammensetzung aufteilt und eine der beiden Teilmengen (als Dehydrierkreisgas) als einen der wenigstens drei Propan enthaltenden Zufuhrströme in die Reaktionszone A rückführt (vorzugsweise als Bestandteil des Beschickungsgasgemischs (Reaktionsgasausgangsgemischs) der Reaktionszone A) und von der anderen Teilmenge an Produktgasgemisch A (vorzugsweise die gesamte andere Teilmenge) in der ersten Trennzone A erfindungsgemäß weiterbehandelt, und

b) die Zuführung des (Haupt- und/oder Neben- bzw. Gesamt-)Restgases aus der Trennzone B in die Reaktionszone A längs des Reaktionspfads der heterogen katalysierten Dehydrierung des Propans so vornimmt, dass an der Zuführstelle bereits wenigstens 5 mol-%, oder wenigstens 10 mol-%, vorzugsweise wenigstens 15 mol-%, oder wenigstens 20 mol-%, besonders bevorzugt wenigstens 25 mol-%, oder wenigstens 30 mol-%, ganz besonders bevorzugt wenigstens 35 mol-%, oder wenigstens 40 mol-%, und am Besten wenigstens 45 mol-%, oder wenigstens 50 mol-% (in der Regel jedoch weniger als 70 mol-%, häufig weniger als 60 mol-%, und oft $\leq$ 50 mol-%) des der Reaktionszone A über die anderen Zuführströme (insgesamt) zugeführten Propan in der Reaktionszone A bereits dehydrierend umgesetzt sind (besonders bevorzugte Verfahrensvarianten sind dabei wiederum jene, bei denen die vorgenannten Umsatzzahlen Zu jeweils durch $Z_U^{Kr} = (Z_U/1 + KGV)$ ersetzt sind). Dabei kann die Zuführung des (Haupt- und/oder Neben- bzw. Gesamt-)Restgases aus der Trennzone B in die Reaktionszone A an einer Stelle, oder auch auf mehrere hintereinander angeordnete Stellen verteilt erfolgen.

Zweckmäßig wird man wenigstens 10 Vol.-%, bzw. 20 Vol.-% des Produktgasgemischs A in die Reaktionszone A rückführen. Vorteilhaft wird die als Kreisgas in die Reaktionszone A rückgeführte Menge an Produktgasgemisch A jedoch nicht mehr als 90 Vol.-%, bzw. 80 Vol.-% des Produktgasgemischs A betragen. D.h., die als Kreisgas in die Reaktionszone A rückgeführte Teilmenge des Produktgasgemischs A kann somit z.B. 20 bis 80 Vol.-%, oder 30 bis 70 Vol.-%, oder 40 bis 60 Vol.-% oder auch 50 Vol.-% des Produktgasgemischs A betragen. Besonders vorteilhaft ist der Mengenanteil 50 bis 70 Vol.-%.

Eine Möglichkeit, das der Reaktionszone A zugeführte Reaktionsgasausgangsgemisch auf die für die heterogen katalysierte Propandehydrierung in der Reaktionszone A benötigte Reaktionstemperatur zu erwärmen, besteht auch darin, ihm molekularen Wasserstoff zuzusetzen und diesen mittels molekularem Sauerstoff, zum Beispiel an geeigneten spezifisch wirkenden Verbrennungskatalysatoren (zum Beispiel den in dieser Schrift genannten), zu verbrennen (zum Beispiel durch einfaches Überleiten und/oder Durchleiten), und mittels der so freigesetzten Verbrennungswärme die Erwärmung auf die gewünschte Reaktionstemperatur zu bewirken. Die resultierenden Verbrennungsprodukte wie $CO_2$, $H_2O$ sowie das den für die Verbrennung benötigten molekularen Sauerstoff gegebenenfalls begleitende $N_2$ bilden vorteilhaft inerte Verdünnungsgase.

Wird längs des Reaktionspfades der heterogen katalysierten Dehydrierung in der Reaktionszone A bereits vorab der Rückführung des molekularen Sauerstoff enthaltenden Restgases aus der Trennzone B ein anderes molekularen Sauerstoff enthaltendes Gas in die Reaktionszone A geführt, wird, unabhängig von der konkreten Ausgestaltung der Reaktionszone A, erfindungsgemäß zweckmäßig darauf geachtet, dass solchermaßen vorab zugeführter molekularer Sauerstoff den Wasserstoffgehalt im Reaktionsgasgemisch in der Reaktionszone A bis zur Zuführstelle des rückzuführenden molekularen Sauerstoff enthaltenden Restgases aus der Trennzone B nicht zu sehr abgesenkt.

Erfindungsgemäß kann das aus der Trennzone B in die Reaktionszone A rückgeführte Restgas auch das einzige der Reaktionszone A zugeführte molekularen Sauerstoff enthaltende Gas sein (unabhängig von der konkreten Ausgestaltung der Reaktionszone A). Dies ist jedoch nicht der Regelfall der erfindungsgemäßen Verfahrensweise.

In erfindungsgemäß geschickter Weise wird man die Reaktionszone A mit der Maßgabe gestalten und betreiben, dass sie aus einem ersten und aus einem zweiten Abschnitt besteht (nachfolgend "Zwei-Abschnitt-Variante" genannt), wobei man

I. dem ersten Abschnitt der Reaktionszone A wenigstens einen, gasförmiges Propan enthaltenden Zufuhrstrom zuführt, der Frischpropan enthält, und in diesem ersten Abschnitt der Reaktionszone A das ihm so zugeführte Propan einer heterogen katalysierten Dehydrierung unterwirft, wobei ein Propan, Propylen und molekularer Wasserstoff enthaltendes Produktgasgemisch A* erhalten wird, bei dessen Erhalt wenigstens 5 mol-%, oder wenigstens 10 mol-%, vorteilhaft wenigstens 15 mol-%, oder wenigstens 20 mol-%, besonders vorteilhaft wenigstens 25 mol-%, oder wenigstens 30 mol-%, ganz besonders vorteilhaft wenigstens 35 mol-%, oder wenigstens 40 mol-%, und noch besser wenigstens 45 mol-%, oder wenigstens 50 mol-% (in der Regel jedoch weniger als 70 mol-%, oder weniger als 60 mol-%, oder ≤ 50 mol-%) des dem ersten Abschnitt der Reaktionszone A (insgesamt) zugeführten Propan in diesem ersten Abschnitt (der Reaktionszone A) dehydrierend (heterogen katalysiert) umgesetzt worden sind und das vorzugsweise, bezogen auf die darin enthaltene molare Menge an Propan, eine größere molare Menge an molekularem Wasserstoff enthält, als das dem ersten Abschnitt der Reaktionszone A zugeführte Reaktionsgasausgangsgemisch;

II. dem Produktgasgemisch A* anschließend molekularen Sauerstoff, nicht umgesetztes Propan und gegebenenfalls (in der Reaktionszone B) nicht umgesetztes Propylen enthaltendes (Haupt- und/oder Neben- bzw. Gesamt-)Restgas aus der Trennzone B zuführt (erfindungsgemäß vorteilhaft wenigstens die Hälfte, bevorzugt wenigstens zwei Drittel oder drei Viertel und ganz besonders bevorzugt die Gesamtmenge (gegebenenfalls abzüglich einer als Verdünnungsgas in die Reaktionszone B rückgeführten Teilmenge identischer Zusammensetzung) des vorgenannten in der Trennzone B anfallenden (jeweils individuell bezogen auf Haupt- und/oder Neben- bzw. Gesamt-)Restgases) und das dabei resultierende Reaktionsgasgemisch A* dem zweiten Abschnitt der Reaktionszone A zuführt, und

III. in diesem zweiten Abschnitt der Reaktionszone A unter Ausbildung des Propan und Propylen enthaltenden Produktgasgemischs A im Reaktionsgasgemisch A* enthaltenen molekularen Sauerstoff mit im Reaktionsgasgemisch A* enthaltenem molekularen Wasserstoff heterogen katalysiert zu Wasser verbrennt (vorteilhaft wenigstens 25 mol-%, bevorzugt wenigstens 50 mol-%, besonders bevorzugt wenigstens 75 mol-%, besser wenigstens 90 mol-% und am Besten wenigstens 95 mol-% der im Reaktionsgasgemisch A* enthaltenen Menge an molekularem Sauerstoff) und im Reaktionsgasgemisch A* enthaltenes Propan gegebenenfalls heterogen katalysiert zu Propylen dehydriert (in der Regel weniger als 40 mol-% bzw. weniger als 30 mol-%, meist weniger als 20 mol-%, häufig weniger als 10 mol-%, und oft weniger als 5 mol-% des im Reaktionsgasgemisch A* enthaltenen Propan). Vorgenannte Mengenverhältnisse können durch Anpassen der Ausmaße des zugehörigen Katalysatorbetts eingeregelt werden;

IV. dann Produktgasgemisch A aus der Reaktionszone A herausgeführt und wie beschrieben in der ersten Trennzone A des erfindungsgemäßen Verfahrens erfindungsgemäß weiterbehandelt (gegebenenfalls nicht in die erste Trennzone A geführtes Produktgasgemisch A kann z.B. zur Energieerzeugung verbrannt und/oder zur Erzeugung von Synthesegas etc. verwendet werden).

Als Katalysatoren kommen für den zweiten Abschnitt der Reaktionszone A dabei all diejenigen Katalysatoren in Betracht, die in dieser Schrift auch für die heterogen katalysierte Dehydrierung empfohlen werden und insbesondere auch für den ersten Abschnitt der Reaktionszone A geeignet sind, da sie, wie Eingangs dieser Schrift bereits erwähnt, in der Regel auch die Verbrennung von molekularem Wasserstoff zu katalysieren vermögen (dies trifft insbesondere auf die Katalysatoren der DE-A 19937107 (insbesondere die dort beispielhaft ausgeführten) zu; in einer Konkurrenzsituation zwischen heterogen katalysierter Propandehydrierung und heterogen katalysierter Wasserstoffverbrennung verläuft letztere in der Regel wesentlich schneller und dominiert die erstere). Selbstredend kommen für den zweiten Abschnitt der Reaktionszone A aber auch solche Katalysatoren in Betracht, die spezifisch für die selektive Verbrennung von molekularem Wasserstoff maßgeschneidert sind. Solche Katalysatoren sind z.B. jene der Schriften US-A 4788371, US-A 4886928, US-A 5430203, US-A 5530171, US-A 5527979 und US-A 5563314.

Grundsätzlich kann der erste Abschnitt der Reaktionszone A sowohl isotherm als auch adiabat gestaltet werden. Im letzteren Fall ist eine auf den einmaligen Durchgang des dem ersten Abschnitt der Reaktionszone A zugesetzten (zugeführten) Reaktionsgasausgangsgemischs durch den ersten Abschnitt der Reaktionszone A bezogene endotherme bis autotherme Bruttowärmetönung über den ersten Reaktionsabschnitt der Reaktionszone A bevorzugt.

Die Reaktionsbedingungen im zweiten Abschnitt der Reaktionszone A (Reaktionstemperatur (z.B. 400 bzw. 500 bis 800 bzw. 700°C), Reaktionsdruck (z.B. 1 bis 10, bzw. bis 5, bzw. bis 3 bar) und Belastung des Katalysatorbetts mit Reaktionsgasgemisch (z.B. 500 (oder weniger) bis 80000 (oder mehr) Nl/l•h) können grundsätzlich ähnlich wie im ersten Abschnitt der Reaktionszone A gewählt werden.

Prinzipiell kann auch der zweite Abschnitt der Reaktionszone A isotherm oder adiabat gestaltet werden. Im letzteren Fall ist eine auf den einmaligen Durchgang des dem zweiten Abschnitt der Reaktionszone A zugesetzten (zugeführten) Reaktionsgasausgangsgemischs (das Reaktionsgasgemisch A*) durch den zweiten Abschnitt der Reaktionszone A bezogene exotherme Bruttowärmetönung über den zweiten Reaktionsabschnitt der Reaktionszone A bevorzugt.

Erfindungsgemäß bevorzugt werden sowohl der erste als auch der zweite Abschnitt der Reaktionszone A adiabat gestaltet, wobei die Kombination "endotherme bis autotherme Bruttowärmetönung" im ersten Reaktionsabschnitt und "exotherme Bruttowärmetönung" im zweiten Reaktionsabschnitt besonders bevorzugt ist.

Besonders vorteilhaft ist dabei eine Ausgestaltung des ersten Abschnitts der Reaktionszone A in Hordenstruktur, die erfindungsgemäß bevorzugt in adiabater Ausgestaltung endotherm bis autotherm betrieben wird. Die Hordenstruktur enthält ein oder mehrere räumlich aufeinanderfolgende mit Dehydrierkatalysator bestückte Katalysatorbetten. Die Anzahl der Katalysatorbetten kann 1 bis 20, zweckmäßigerweise 2 bis 8, insbesondere 3 bis 6 betragen (für hohe Dehydrierumsätze im ersten Abschnitt der Reaktionszone A ist eine große Zahl von Katalysatorbetten vorteilhaft).

Die Katalysatorbetten werden vorzugsweise radial oder axial vom Reaktionsgas durchströmt.

Im einfachsten Fall sind die Katalysatorfestbetten in einem Reaktor axial oder in den Ringspalten von zentrisch ineinander gestellten zylindrischen Gitterrosten hintereinander angeordnet. Dabei kann es sich beim Reaktor z.B. um einen Schachtofen handeln. Die Durchführung des ersten Abschnitts der Reaktionszone A in einem einzelnen Schachtofen ist ebenso möglich wie die Realisierung der gesamten Reaktionszone A in einem Hordenreaktor.

Würde dem Reaktionsgas vor und/oder nach Eintritt in den ersten Abschnitt der Reaktionszone A an keiner Stelle molekularer Sauerstoff enthaltendes Gas zugesetzt, würde das Reaktionsgas im in Horden strukturierten ersten Abschnitt der Reaktionszone A auf seinem Weg von einem Katalysatorbett zum nächsten Katalysatorbett zweckmäßig einer Zwischenerhitzung unterworfen, z.B. durch Überleiten über mit heißen Gasen erhitzte Wärmeaustauscheroberflächen, z.B. Wärmeaustauscherrippen, oder durch Durchleiten durch mit heißen Brenngasen beheizte Einbauten, z.B. Rohre.

Im Rahmen des erfindungsgemäßen Verfahrens wird vorstehend geschilderte Zwischenerhitzung jedoch vorzugsweise wenigstens teilweise auf direktem Weg durchgeführt. Dazu wird dem Reaktionsgas entweder bereits vor Durchströmen des ersten Katalysatorbetts und/oder zwischen den nachfolgenden Katalysatorbetten in begrenztem Umfang ein molekularen Sauerstoff enthaltendes Gas (vorzugsweise Luft oder Gemische aus Sauerstoff und Inertgasen wie $CO_2$, $N_2$ und Edelgase, oder reiner $O_2$) zugesetzt.

An einem, oder an mehreren, oder gegebenenfalls an allen, Katalysatorbetten wird so in begrenztem Umfang dem Reaktionsgas vorab zugesetzter und/oder im Verlauf der Dehydrierung gebildeter molekularer Wasserstoff verbrannt. Die dabei freigesetzte Reaktionswärme ermöglicht so bei adiabater Gestaltung eine im wesentlichen autotherme Betriebsweise des ersten Abschnitts der Reaktionszone A. Bezogen auf die im Reaktionsgas enthaltene Menge an molekularem Wasserstoff sollte die zugesetzte molekulare Sauerstoffmenge 0,5 bis 50 bzw. bis 30 Vol.-% (vorzugsweise 10 bis 25 Vol.-%) betragen. Infolge der hohen Verbrennungswärme des Wasserstoffs ist eine Teilverbrennung vergleichsweise geringer Mengen desselben für den vorgenannten Zweck in der Regel ausreichend.

In einer Ausführungsform der Erfindung erfolgt eine Zwischeneinspeisung von sauerstoffhaltigem Gas gegebenenfalls vor jeder Horde des Hordenreaktors. In einer weiteren Ausführungsform des erfindungsgemäßen Verfahrens erfolgt die Einspeisung von sauerstoffhaltigem Gas vor jeder Horde außer der ersten Horde. In einer weiteren Ausführungsform des erfindungsgemäßen Verfahrens ist hinter jeder Sauerstoffeinspeisungsstelle eine Schüttung aus spezifischem, für Zwecke von $H_2$-Oxidationen geeignetem Oxidationskatalysator vorhanden, gefolgt von einer Schüttung aus Dehydrierkatalysator. Bei Bedarf kann gegebenenfalls zusätzlich vor jeder Horde externer molekularer Wasserstoff (in reiner oder in mit Inertgas verdünnter Form) zugeführt werden. In einer weniger bevorzugten Ausführungsform können die Katalysatorbetten auch Mischungen aus Dehydrier- und $H_2$-Oxidations-Katalysatoren enthalten.

Erfindungswesentlich ist jedoch, dass es beim erfindungsgemäßen Verfahren keiner Einspeisung von externem molekularen Wasserstoff in notwendiger Weise bedarf. Die Dehydriertemperatur in der Hordenstruktur (im Hordenreaktor) beträgt in der Regel 400 bis 800°C, der Druck im allgemeinen 0,2 bis 10 bar, bevorzugt 0,5 bis 4 bar und besonders bevorzugt 1 bis 3 bar. Die Gesamtgasbelastung (GHSV) liegt in der Regel bei 500 bis 10000 $h^{-1}$, bei Hochlastfahrweise auch bis zu 80000 $h^{-1}$, regelmäßig bei 30000 bis 40000 $h^{-1}$.

Beisein von Wasserdampf ist förderlich für die Katalysatorstandzeit.

Partialdruckerniedrigung der Reaktanden durch Druckminderung, Verbrennung von in der Dehydrierung gebildetem Wasserstoff und/oder Inertverdünnung, hohe Temperaturen und große Hordenzahl fördern den Umsatz der Dehydrierung innerhalb des ersten Abschnitts der Reaktionszone A.

Für den zweiten Abschnitt der Reaktionszone A wird vorzugsweise der gleiche Reaktortyp wie für den ersten Abschnitt der Reaktionszone A angewandt. Allerdings enthält er in der Regel lediglich ein Katalysatorbett. Vorzugsweise werden in beiden Abschnitten einfache Schachtöfen eingesetzt.

Das molare Verhältnis von molekularem Sauerstoff zu molekularem Wasserstoff kann (im Reaktionsgasgemisch A*) im zweiten Abschnitt der Reaktionszone A 1:2 bis 1:10, vorzugsweise 1:2 bis 1:4 betragen. D.h., im zweiten Abschnitt der Reaktionszone A kann im wesentlichen der gesamte im Reaktionsgemisch A* enthaltene molekulare Sauerstoff mit molekularem Wasserstoff zu Wasser verbrannt werden, so dass das dabei gebildete Produktgasgemisch A weitgehend frei von molekularem Sauerstoff ist. Beispielsweise kann das so gebildete Produktgasgemisch A weniger als 5 Vol.-%, oder weniger als 3 Vol.-%, oder weniger als 1 Vol.-%, vielfach sogar weniger als 0,5 oder weniger als 0,2 Vol.-% molekularen Sauerstoff enthalten. Bevorzugt ist ein verschwindender Gehalt an molekularem Sauerstoff.

Der erste Abschnitt der Reaktionszone A und der zweite Abschnitt der Reaktionszone A können sowohl in voneinander getrennten Reaktoren, als auch in einem einzigen Reaktor (z.B. in einem Hordenreaktor, z.B. in Schachtofenausführung) untergebracht sein.

In erfindungsgemäß noch eleganterer Weise lässt sich die Reaktionszone A als Kombination der vorstehend beschriebenen "Schlaufenvariante" und der "Zwei-Abschnitt-Variante" wie folgt gestalten und betreiben.

Dabei wird man das wie vorstehend beschrieben in der "Zwei-Abschnitt-Variante" im zweiten Abschnitt der Reaktionszone A ausgebildete Produktgasgemisch A zusätzlich in zwei Teilmengen identischer Zusammensetzung aufteilen und eine der beiden Teilmengen (als Dehydrierkreisgas) als ein weiterer Propan enthaltender Zufuhrstrom in den ersten Abschnitt der Reaktionszone A rückführen (bevorzugt als Bestandteil des Reaktionsgasausgangsgemischs des ersten Abschnitts der Reaktionszone A) und von der anderen Teilmenge an Produktgasgemisch A (vorzugsweise die gesamte andere Teilmenge) in der ersten Trennzone A erfindungsgemäß weiterbehandeln.

Die Rückführung von Produktgasgemisch A in den ersten Abschnitt der Reaktionszone A ist erfindungsgemäß insofern vorteilhaft, als das im zweiten Abschnitt der Reaktionszone A gebildete Produktgasgemisch A infolge der Verbrennung von molekularem Sauerstoff mit molekularem Wasserstoff einerseits normalerweise (zumindest bei adiabat exothermem Betrieb des zweiten Abschnitts der Reaktionszone A) eine erhöhte Temperatur und andererseits, ebenfalls infolge der Sauerstoffverbrennung, einen geringen Sauerstoffgehalt und einen nennenswerten Wasserdampfgehalt aufweist. Die Wasserstoffgehalte sind in der Regel gleichfalls gering. Insbesondere bei kleineren Dehydrierumsätzen im ersten Abschnitt der Reaktionszone A kann dem Reaktionsgasgemisch A* vorab seiner Zuführung in den zweiten Abschnitt der Reaktionszone A ergänzend externer molekularer Wasserstoff (das ist molekularer Wasserstoff, der weder Bestandteil von in die Reaktionszone A rückgeführtem Kreisgas ist, noch in der Reaktionszone A (oder in einer der anderen Reaktions-/Trennzonen des erfindungsgemäßen Verfahrens) selbst gebildet wird) zugegeben werden, um danach im zweiten Abschnitt der Reaktionszone A z.B. die erwünschte Temperatur des Reaktionsgasgemischs A (sowie Eliminierung von molekularem Sauerstoff in selbigem) zu erreichen.

Gemäß Vorgenanntem eignet sich solchermaßen als Bestandteil des Reaktionsgasausgangsgemischs in den ersten Abschnitt der Reaktionszone A rückgeführtes Produktgasgemisch A in besonderer Weise, um das dem ersten Abschnitt der Reaktionszone A zugeführte Reaktionsgasausgangsgemisch auf Reaktionstemperatur zu erwärmen, ohne gleichzeitig das Reaktionsgasausgangsgemisch durch bereits enthaltenen molekularen Wasserstoff thermodynamisch bzw. durch enthaltenen molekularen Sauerstoff kinetisch zu belasten. Der im rückgeführten Produktgasgemisch A enthaltene Wasserdampf fördert die heterogen katalysierte Dehydrierung im ersten Abschnitt der Reaktionszone A zusätzlich und macht eine separate Zufuhr von externem Wasserdampf in der Regel überflüssig.

D.h., die erfindungsgemäße Verfahrensweise besticht u.a. dadurch, dass sie eine erfindungsgemäße Betriebsweise mit befriedigender Standzeit ermöglicht, ohne dass es in irgendeiner der Reaktions- bzw. Trennzonen der Zufuhr von externem Wasserdampf (das ist Wasserdampf, der in keiner der Reaktions-/Trennzonen des erfindungsgemäßen Verfahrens gebildet wird) bedarf.

Der in die erste Trennzone A geführte Teil des im zweiten Abschnitts der Reaktionszone A gebildeten Produktgasgemischs A eignet sich aufgrund seiner erhöhten Temperatur gleichzeitig in hervorragender Weise dazu, durch indirekten Wärmeaustausch in einem Gaskühler sowohl zunächst das der Reaktionszone A zugeführte Frischpropan als auch das aus der Trennzone B in die Reaktionszone A rückgeführte Restgas (oder nur eines der beiden Gase) (im wesentlichen auf die erwünschte Reaktionstemperatur) anzuwärmen, und sich selbst gleichzeitig in für die Erfordernisse in der ersten Trennzone A vorteilhafter Weise abzukühlen.

Erfindungsgemäß zweckmäßig wird man wenigstens 10 Vol.-% bzw. 20 Vol.-% des im zweiten Abschnitt gebildeten Produktgasgemischs A in den ersten Abschnitt der Reaktionszone A rückführen. Erfindungsgemäß vorteilhaft wird die als Kreisgas in den ersten Abschnitt der Reaktionszone A rückgeführte Teilmenge des Produktgasgemischs A jedoch nicht mehr als 90 Vol.-% bzw. 80 Vol.-% betragen. D.h., die als Kreisgas in den ersten Abschnitt der Reaktionszone A rückgeführte Teilmenge des Produktgasgemischs A kann bei der vorgenannten Verfahrensweise z.B. 20 bis 80 Vol.-%, oder 30 bis 70 Vol.-%, oder 40 bis 60 Vol.-%, oder auch 50 Vol.-% des gebildeten Produktgasgemischs A betragen. Besonders vorteilhaft ist ein (Dehydrier)Kreisgasmengenanteil von 50 bis 70 Vol.-%.

In ganz besonders eleganter Weise lässt sich die Rückführung von vorgenanntem Produktgasgemisch A in den ersten Abschnitt der Reaktionszone A wie folgt gestalten.

Zunächst führt man dem Produktgasgemisch A* das molekularen Sauerstoff, Propan und gegebenenfalls (in der Reaktionszone B) nicht umgesetztes Propylen enthaltende (Haupt- und/oder Neben- bzw. Gesamt-)Restgas aus der Trennzone B nach dem Prinzip einer mit diesem Restgas als Treibstrahl betriebenen Strahlpumpe (Figur 1 der DE-A 10211275 erläutert dieses Prinzip, es soll in dieser Schrift auch das Prinzip der Ejektorstrahldüsen umfassen) zu, wobei die Förderrichtung des durch eine Treibdüse (1) über eine Mischstrecke (2) und einen Diffusor (3) entspannten Treibstrahls in den zweiten Abschnitt der Reaktionszone A sowie die Saugwirkung (Saugrichtung) des Saugstutzens (4) in Richtung des ersten Abschnitts der Reaktionszone A weist und die Verbindung "Saugstutzen-Mischstrecke-Diffusor" die alleinige Verbindung zwischen den beiden Abschnitten der Reaktionszone A bildet.

Im Saugstutzen wird dadurch ein Unterdruck erzeugt, durch den aus dem ersten Abschnitt der Reaktionszone A Produktgasgemisch A* angesaugt und bei gleichzeitiger Vermischung mit dem Treibstrahl als Reaktionsgasgemisch A* durch die Mischstrecke (das Mischrohr) über den Diffusor transportiert und in den zweiten Abschnitt der Reaktionszone A entlassen wird (die numerischen Adressen beziehen sich auf die Fig. 1 dieser Schrift).

In notwendiger Weise wird man den Druck des Treibstrahls so wählen (die Druckeinstellung wird normalerweise mittels eines Radialverdichters bzw. (in selteneren Fällen) mittels eines Gebläses (normalerweise ein Axialverdichter mit niedrigem Druckverhältnis von Enddruck zu Ausgangsdruck) erfolgen), dass der Druck des sich im zweiten Abschnitt der Reaktionszone A ausbildenden Produktgasgemischs A oberhalb des Drucks, des sich im ersten Abschnitt der Reaktionszone A ausbildenden Produktgasgemischs A* liegt. Eine einfache Rohrverbindung zwischen dem Ausgang des zweiten Abschnitts der Reaktionszone A und dem Eingang in den ersten Abschnitt der Reaktionszone A, die man zweckmäßig als T-Stück mit Mengenteiler ausführen wird, um eine Teilmenge des Produktgasgemischs A in die erste Trennzone A weiterzuleiten, ist dann normalerweise ausreichend, um eine in den ersten Abschnitt der Reaktionszone A rückführende (dem Druckgefälle folgende) Kreisströmung (eine Art natürlicher Umlauf) des anderen Teils des Produktgemischs A zu erzeugen.

Eine ganz besonders bevorzugte Ausführungsform der Reaktionszone A des erfindungsgemäßen Verfahrens gestaltet sich daher wie folgt und ist schematisch in der Figur 1 dieser Schrift, auf die sich alle numerischen Adressen beziehen, graphisch dargestellt:

Die Reaktionszone A (17) wird dabei wie bereits beschrieben als Kombination der vorstehend beschriebenen "Schlaufenvariante" und "Zwei-Abschnitt-Variante" gestaltet und betrieben, wobei man zusätzlich folgende Maßgaben erfüllt:

I. dem ersten Abschnitt (0) der Reaktionszone A wenigstens einen, gasförmiges Propan enthaltenden Zufuhrstrom zuführt, der Frischpropan (5) enthält, und in diesem ersten Abschnitt der Reaktionszone A das ihm zugeführte Propan einer heterogen katalysierten (partiellen) Dehydrierung unterwirft, wobei ein Propan, Propylen und molekularen Wasserstoff enthaltendes Produktgasgemisch A* erhalten wird, bei dessen Erhalt wenigstens 5 mol-%, oder wenigstens 10 mol-%, vorteilhaft wenigstens 15 mol-%, oder wenigstens 20 mol-%, besonders vorteilhaft wenigstens 25 mol-%, oder wenigstens 30 mol-%, ganz besonders vorteilhaft wenigstens 35 mol-%, oder wenigstens 40 mol-%, und noch besser wenigstens 45 mol-%, oder wenigstens 50 mol-% (in der Regel jedoch weniger als 70 mol-%, oder weniger als 60 mol-%, oder ≤ 50 mol-%) des dem ersten Abschnitt der Reaktionszone A (insgesamt) zugeführten Propan in diesem ersten Abschnitt dehydrierend umgesetzt worden sind (bei Schlaufenfahrweise in der Reaktionszone A sind bevorzugte Verfahrensvarianten dabei wiederum jene, bei denen die vorgenannten Umsatzzahlen Zu jeweils durch $Z_U^{Kr} = (Z_U/1 + KGV)$ ersetzt sind);

dabei wird der erste Abschnitt der Reaktionszone A im wesentlichen adiabat betrieben (d.h. über außerhalb des ersten Abschnitts der Reaktionszone A befindliche Wärmeträger wird dem ersten Abschnitt der Reaktionszone A im wesentlichen weder (gezielt) Wärme zugeführt noch entnommen);

bezogen auf einmaligen Durchgang des Beschickungsgasgemischs des ersten Abschnitts der Reaktionszone A durch den ersten Abschnitt der Reaktionszone A ist die Bruttowärmetönung über den ersten Abschnitt der Reaktionszone A endotherm (negativ) bis autotherm (im wesentlichen Null);

vorzugsweise wird der erste Abschnitt der Reaktionszone A in Hordenstruktur ausgeführt;

vorzugsweise enthält die Hordenstruktur (6) 2 bis 20, besonders bevorzugt 2 bis 10 und ganz besonders bevorzugt 2 bis 6 Katalysatorbetten, die vorzugsweise axial (z.B. in einem Schachtofen) oder radial (z.B. in den Ringspalten von zentrisch ineinander gestellte zylindrischen Gitterrosten) angeordnet sind und vom Reaktionsgas in entsprechender Weise axial oder radial durchströmt werden;

die Hordenstruktur kann z.B. in einem einzigen Reaktor oder in einer Hintereinanderschaltung von Reaktoren verwirklicht sein;

die Katalysatorbetten sind vorzugsweise so mit Katalysatoren (z.B. jenen der DE-A 19937107, insbesondere den beispielhaft ausgeführten) beschickt, dass beim Durchströmen des Katalysatorbetts mit einem Reaktionsgas, dessen Zusammensetzung eine Konkurrenzreaktion zwischen heterogen katalysierter Wasserstoffverbrennung und heterogen katalysierter Propandehydrierung zulässt, in Strömungsrichtung zunächst die heterogen katalysierte Wasserstoffverbrennung schneller verläuft;

vorzugsweise wird dem dem ersten Abschnitt der Reaktionszone A zugeführten Reaktionsgasausgangsgemisch weder externer molekularer Sauerstoff, noch externer molekularer Wasserstoff, noch externer molekularer Wasserdampf zugeführt (unter einem an irgendeiner Stelle der Reaktionszone A (oder einer anderen Reaktions-/Trennzone des erfindungsgemäßen Verfahrens) extern zugeführten Gas soll in dieser Schrift generell Gas verstanden werden, das weder in der Reaktionszone A (noch in einer der anderen Reaktions-/Trennzonen des erfindungsgemäßen Verfahrens) selbst gebildet wird, noch in Kreisfahrweise aus der Reaktionszone A herauskommend unmittelbar und/oder mittelbar in die Reaktionszone A rückgeführt wird);

vorzugsweise wird dem Reaktionsgas(ausgangs)gemisch (des ersten Abschnitts der Reaktionszone A), nachdem es in Strömungsrichtung das erste Katalysatorbett durchlaufen hat, vor jedem Durchströmen eines dem ersten Katalysatorbett in Strömungsrichtung nachfolgenden Katalysatorbett des ersten Abschnitts der Reaktionszone A in begrenztem Umfang ein molekularen Sauerstoff enthaltendes Gas zugesetzt, wobei die dem Reaktionsgasgemisch so jeweils zugeführte Sauerstoffmenge jeweils so begrenzt wird, dass der resultierende Sauerstoffgehalt des Reaktionsgasgemischs, bezogen auf den in ihm enthaltenen Wasserstoff, 0,5 bis 50 bzw. bis 30 Vol.-%, vorzugsweise 10 bis 25 Vol.-% beträgt;

als ein solches Sauerstoff enthaltendes Gas wird vorzugsweise Luft (7) verwendet; es kann jedoch auch reiner Sauerstoff oder ein Gemisch aus Sauerstoff und Inertgasen wie $CO_2$, $N_2$ und/oder Edelgasen oder Stickoxid verwendet werden;

nach Zusatz des Sauerstoff enthaltenden Gases durchläuft das Reaktionsgas vorzugsweise noch einen statischen Mischer (8), bevor es in das relevante Katalysatorbett eintritt;

die Temperaturen innerhalb des ersten Abschnitts der Reaktionszone A werden vorzugsweise auf 400 bis 700°C gehalten;

der Arbeitsdruck innerhalb des ersten Abschnitts der Reaktionszone A liegt vorzugsweise bei 0,5 bis 10 bar;

das dem ersten Abschnitt der Reaktionszone A zugeführte Reaktionsgasausgangsgemisch (9) (seine Temperatur beträgt vorzugsweise 400 bis 700°C (z.B. 560°C), sein Druck liegt bevorzugt bei 0,5 bis 10 bar) weist vorzugsweise nachfolgende Gehalte auf:

10 bis 35 (z.B. 19 oder 28) Vol.-% Propan,
3 bis 10 (z.B. 7,5 oder 5,5) Vol.% Propen,
5 bis 20 (z.B. 9 oder 12) Vol.-% Wasserdampf,
0,01 bis 6 (z.B. 3,9 oder 4,3) Vol.-% molekularer Wasserstoff und
0 bis 0,02 Vol.-% molekularer Sauerstoff;

die Belastung der Katalysatorbetten mit Reaktionsgas beträgt häufig 250 bis 5000 $h^{-1}$ (bei Hochlastfahrweise auch bis 40000 $h^{-1}$) vorzugsweise 10000 bis 25000 Nl/l•h, besonders bevorzugt 15000 bis 20000 Nl/l•h;

um das dem ersten Abschnitt der Reaktionszone A zugeführte Frischpropan auf die Reaktionstemperatur zu erwärmen, wird dieses vorteilhaft in indirektem Wärmeaustausch (in einem Gaskühler üblicher Bauart) zum aus dem zweiten Abschnitt der Reaktionszone A in die Trennzone A geführten heißen Produktgasgemisch A geführt;

außer den in dieser Schrift bereits genannten, werden dem ersten Abschnitt der Reaktionszone A vorzugsweise keine weiteren externen Gase zugeführt;

das im ersten Abschnitt der Reaktionszone A gebildete Produktgasgemisch A* weist vorzugsweise nachfolgende Gehalte auf:

8 bis 32 Vol.-% (z.B. 16 oder 24 Vol.-%) Propan,
3 bis 12 Vol.-% (z.B. 9 oder 6 Vol.-%) Propen,
5 bis 23 Vol.-% (z.B. 11 oder 15 Vol.-%) Wasserdampf,
0,1 bis 6 Vol.-% (z.B. 1 oder 3 Vol.-%) molekularer Wasserstoff, und
0 bis 0,05 Vol.-% molekularer Sauerstoff;

es weist vorzugsweise eine Temperatur von 500 bis 750°C (z.B. 580°C) und vorteilhaft einen Arbeitsdruck von 0,05 bis 10 bar auf.

II. dem Produktgemisch A* anschließend molekularen Sauerstoff, Propan und gegebenenfalls (in der Reaktionszone B) nicht umsetztes Propylen enthaltendes (Haupt- und/oder Neben- bzw. Gesamt-)Restgas aus der Trennzone B zuführt (erfindungsgemäß vorteilhaft wenigstens die Hälfte, bevorzugt wenigstens drei Vierteil und ganz besonders bevorzugt die Gesamtmenge des vorgenannten in der Trennzone B anfallenden Restgases) und das dabei resultierende Reaktionsgasgemisch A* dem zweiten Abschnitt der Reaktionszone A zuführt;

das dem Produktgasgemisch A* zugeführte Restgas enthält in der Regel 0,5 Vol.-% bis 10 Vol.-%, häufig 1 bis 8 Vol.-% und bevorzugt 2 bis 5 Vol.-% an molekularem Sauerstoff;

die Gehalte des dem Produktgasgemisch A* zugeführten (Haupt- und/oder Neben- bzw. Gesamt-)Restgases sind vorzugsweise wie folgt:

10 bis 40 Vol.-% (z.B. 15 oder 32 Vol.-%) Propan,
0 bis 1 Vol.-% Propen,
> 0 bis 5 Vol.-% (z.B. 3 oder 4 Vol.-%) molekularer Sauerstoff,
1 bis 10 Vol.-% (z.B. 2 Vol.-%) Wasserdampf, und

0 bis 0,5 Vol.-% (z.B. 0 bis 0,3 oder 0 bis 0,1 Vol.-%) molekularer Wasserstoff;

vorzugsweise wird dem Produktgasgemisch A* das Restgas in solchen Mengen zugeführt, dass der Sauerstoffgehalt des resultierenden Reaktionsgasgemischs A*, bezogen auf den in ihm enthaltenen Wasserstoff, 15 bis 50 Vol.-%, vorteilhaft 25 bis 50 Vol.-%, und besonders vorteilhaft 30 oder 40 bis 50 Vol.% beträgt; das Reaktionsgasgemisch A* (12) weist vorzugsweise nachfolgende Gehalte auf:

10 bis 38 Vol.-% (z.B. 16 oder 28 Vol.-%) Propan,
3 bis10 Vol.-% (z.B. 5 Vol.-%) Propen,
0 bis 0,05 Vol.-% molekularer Sauerstoff,
3 bis 20 Vol.-% (z.B. 7 oder 12 Vol.-%) Wasserdampf, und
0,1 bis 6 Vol.-% (z.B. 4 Vol.-%) molekularer Wasserstoff.

die Temperatur des Reaktionsgasgemischs A* beträgt vorteilhaft 300 bis 600°C (z.B. 400 bis 500°C), der Druck des Reaktionsgasgemischs A* beträgt vorteilhaft 0,5 bzw. 0,6 bis 12 bar;
bevorzugt führt man dem Produktgasgemisch A* (11) das (Haupt- und/oder Neben- bzw. Gesamt-)Restgas aus der Trennzone B nach dem Prinzip einer mit diesem Restgas als Treibstrahl betriebenen Strahlpumpe zu, wobei die Förderrichtung des durch eine Treibdüse (1) über eine Mischstrecke (2) und einen Diffusor (3) entspannten Treibstrahls in den zweiten Abschnitt der Reaktionszone A (13) sowie die Saugrichtung (die Saugwirkung) des Saugstutzens (4) in Richtung des ersten Abschnitts der Reaktionszone A weist und die Verbindung "Saugstutzen-Mischstrecke-Diffusor" die alleinige Verbindung zwischen dem ersten und dem zweiten Abschnitt der Reaktionszone A bildet;
vorzugsweise weist der Treibstrahl eine Temperatur von 400 bzw. 500 bis 600°C und einen Druck von 3 bis 6 bar (vorzugsweise 4 bis 5 bar) auf;
vorzugsweise wird das aus der Trennzone B in die Reaktionszone A geführte Restgas (15) durch indirekten Wärmetausch (14) mit in die Trennzone A geführtem heißem Produktgasgemisch A (16) auf diese Temperatur erwärmt (vorzugsweise nachdem das Frischpropan bereits mit diesem Produktgasgemisch A erwärmt worden ist);
vorzugsweise wird das aus der Trennzone B in die Reaktionszone A geführte Restgas mittels eines Radial- bzw. Axialverdichters auf vorgenannten Druck verdichtet;
vorzugsweise enthält das Reaktionsgasgemisch A* außer den in dieser Schrift genannten Gasströmen keine weiteren Gasströme zugesetzt;

III. im zweiten Abschnitt der Reaktionszone A unter Ausbildung des Propan und Propylen enthaltenden Produktgasgemisch A im Reaktionsgasgemisch A* enthaltenen molekularen Sauerstoff mit darin enthaltenem molekularem Wasserstoff heterogen katalysiert zu Wasser verbrennt (vorteilhaft wenigstens 25 mol-%, bevorzugt wenigstens 50 mol-%, besonders bevorzugt wenigstens 75 mol-%, besser wenigstens 90 mol-% und am Besten wenigstens 95 mol-% der im Reaktionsgasgemisch A* enthaltenen Menge an molekularem Sauerstoff) und gegebenenfalls im Reaktionsgasgemisch A* enthaltenes Propan heterogen katalysiert zu Propylen dehydriert (in der Regel weniger als 40 mol-% bzw. weniger als 30 mol-%, meist weniger als 20 mol-%, häufig weniger als 10 mol-%, und oft weniger als 5 mol-% des im Reaktionsgasgemisch A* enthaltenen Propan);
dabei wird der zweite Abschnitt der Reaktionszone A im wesentlichen adiabat betrieben (d.h., über außerhalb des zweiten Abschnitts der Reaktionszone A befindliche Wärmeträger wird dem zweiten Abschnitt der Reaktionszone A im wesentlichen weder (gezielt) Wärme zugeführt noch entnommen);
bezogen auf einmaligen Durchgang des Reaktionsgasgemischs A* durch den zweiten Abschnitt der Reaktionszone A ist die Bruttowärmetönung über den zweiten Abschnitt der Reaktionszone A exotherm (um diese positive Wärmetönung zu gewährleisten, kann es insbesondere im Fall von vergleichsweise geringen Dehydrierumsätzen im ersten Abschnitt der Reaktionszone zweckmäßig sein, den Wasserstoffgehalt des Reaktionsgasgemischs A* durch Zufuhr von externem Wasserstoff zu erhöhen; anwendungstechnisch zweckmäßig wird man diese Zufuhr von molekularem Wasserstoff vorteilhaft bereits ins Produktgasgemisch A* hinein vornehmen, sie kann aber z.B. auch in das rückgeführte Restgas hinein erfolgen (19) oder in das resultierende Reaktionsgasgemisch A*);
außer den in dieser Schrift bereits genannten, werden dem zweiten Abschnitt der Reaktionszone A vorzugsweise keine weiteren externen Gase zugeführt;
die Temperaturen innerhalb des zweiten Abschnitts der Reaktionszone A werden vorzugsweise auf 400 bis 750°C (bzw. 500 bis 700°C) gehalten;
der Arbeitsdruck innerhalb des zweiten Abschnitts der Reaktionszone A liegt vorzugsweise bei 0,5 bis 12 bar;
für den zweiten Abschnitt der Reaktionszone A sind z.B. diejenigen Katalysatoren geeignet, die in dieser Schrift auch für die heterogen katalysierte Dehydrierung im ersten Abschnitt der Reaktionszone A als geeignet empfohlen werden, da sie wie eingangs dieser Schrift bereits erwähnt, in der Regel auch die Verbrennung von molekularem Wasserstoff zu katalysieren vermögen (dies trifft insbesondere auf die Katalysatoren der DE-A 19937107 (insbesondere auf die dort beispielhaft aufgeführten) zu, weshalb diese Katalysatoren ganz besonders bevorzugt in beiden Abschnitten der Reaktionszone A zum Einsatz kommen; in einer Konkurrenzsituation zwischen heterogen katalysierter Propandehydrierung und heterogen katalysierter Wasserstoffverbrennung verläuft letztere an den genannten Katalysatoren in der Regel wesentlich schneller und dominiert die erstere); selbstredend kommen für den zweiten Abschnitt der Reaktionszone A aber auch solche Katalysatoren in Betracht, die spezifisch für die selektive Verbrennung von molekularem Wasserstoff maßgeschneidert sind; solche Katalysatoren sind z.B. jene der Schriften US-A 4788371, US-A 4886928, US-A 5430203, US-A 5530171, US-A 5527979 und US-A 5563314;

als Reaktoren kommen für den zweiten Abschnitt der Reaktionszone A grundsätzlich dieselben in Betracht, wie für den ersten Abschnitt der Reaktionszone A;

vorzugsweise enthält der Reaktor für den zweiten Abschnitt der Reaktionszone A keine Vielzahl von Katalysatorbetten, sondern nur ein Katalysatorbett;

anwendungstechnisch zweckmäßig ist dieses eine Katalysatorbett in einem einfachen Schachtofen untergebracht und wird axial oder radial durchströmt;

die Belastung des vorgenannten Katalysatorbetts mit Reaktionsgasgemisch A* beträgt in der Regel 500 bis 80000 Nl/l•h, vorzugsweise 10000 bis 50000 Nl/l•h, besonders bevorzugt 20000 bis 40000 Nl/l•h (bei nur geringen Dehydrierumsätzen im zweiten Abschnitt der Reaktionszone A und vorwiegend ausschließlicher Verbrennung von molekularem Sauerstoff mit molekularem Wasserstoff kann die vorgenannte Belastung auch bis zu 200000 Nl/l•h betragen);

mit Vorteil sind der erste und der zweite Abschnitt der Reaktionszone A in ein und demselben Reaktor untergebracht;

anwendungstechnisch vorteilhaft ist der zweite Abschnitt der Reaktionszone A eine mit Katalysator beschickte Horde in einem Hordenreaktor, dessen übrige Horden dem ersten Abschnitt der Reaktionszone A zugeordnet sind;

das im zweiten Abschnitt der Reaktionszone A gebildete Produktgasgemisch A weist vorzugsweise nachfolgende Gehalte auf:

7 bis 30 Vol.-% (z.B. 12 oder 24 Vol.-%) Propan,
3 bis 12 Vol.-% (z.B. 6 oder 8 Vol.-%) Propen,
0 bis 0,05 Vol.-% molekularer Sauerstoff,
5 bis 22 Vol.-% (z.B. 10 oder 13 Vol.-%) Wasserdampf und
0,1 bis 6 Vol.-% (z.B. 4 Vol.-%) molekularer Wasserstoff.

die Temperatur des Produktgasgemischs A beträgt vorteilhaft 400 bis 700°C; der Druck des Reaktionsgasgemischs beträgt vorteilhaft 0,4 bis 10 bar und liegt vorzugsweise gleichzeitig oberhalb des Drucks des Produktgasgemischs A*;

IV. das im zweiten Abschnitt der Reaktionszone A ausgebildete Produktgasgemisch A in zwei Teilmengen identischer Zusammensetzung aufteilt und die eine der beiden Teilmengen (10) als ein weiterer Propan enthaltender Zufuhrstrom (als Dehydrierkreisgas) in den ersten Abschnitt der Reaktionszone A rückführt (bevorzugt als Bestandteil des Reaktionsgasausgangsgemischs des ersten Abschnitts der Reaktionszone A und vorzugsweise in natürlichem Umlauf dem zum Druck am Ausgang des ersten Abschnitts der Reaktionszone A bestehenden Druckgefälle folgend; die Vermischung mit Frischpropan erfolgt vorzugsweise mittels statischem Mischer vor dem in Strömungsrichtung befindlichen ersten Katalysatorbett des ersten Abschnitts der Reaktionszone A) und die andere Teilmenge (18) an Produktgasgemisch A (vorzugsweise die gesamte andere Teilmenge) in der ersten Trennzone A erfindungsgemäß weiterbehandelt;

vorzugsweise führt man die andere Teilmenge (18) an Produktgasgemisch A über einen indirekten Wärmeaustauscher (14) in die Trennzone A, um in selbigem das Frischpropan und/oder das Restgas aus der Trennzone B (auf die gewünschte Temperatur) zu erwärmen.

Das im Rahmen der erfindungsgemäßen heterogen katalysierten Propandehydrierung in der Reaktionszone A gebildete Produktgasgemisch A enthält beim erfindungsgemäßen Verfahren in der Regel Propan, Propen, molekularen Wasserstoff, $N_2$, $H_2O$, Methan, Ethan, Ethylen, Buten-1, sonstige Butene (z.B. iso-Buten) und andere $C_4$-Kohlenwasserstoffe (n-Butan, iso-Butan, Butadien etc.), CO und $CO_2$, im Regelfall aber auch Oxygenate wie Alkohole, Aldehyde und Carbonsäuren (normalerweise mit $\leq$ 9 C-Atomen). Weiterhin können in geringen Mengen aus dem Restgas herrührende Bestandteile enthalten sein, wie z.B. Nebenkomponenten die in der Reaktionszone B gebildet werden, aber auch in den Trennzonen A und/oder B verwendetes Absorptionsmittel. Es wird sich in der Regel bei einem Druck von 0,3 bis 10 bar befinden und häufig eine Temperatur von 400 bis 650 bzw. bis 500°C, in günstigen Fällen von 450 bis 500°C aufweisen.

Während die EP-A 117 146, die DE-A 3 313 573 und die US-A 3 161 670 empfehlen, das in der heterogen katalysierten Propandehydrierung gebildete Produktgasgemisch A als solches zur Beschickung der Reaktionszone B zu verwenden, ist es erfindungsgemäß erforderlich, aus dem Produktgasgemisch A vor seiner Weiterverwendung zur Beschickung der Reaktionszone B als Nebenkomponentenauslass wenigstens eine Teilmenge der darin enthaltenen, von Propan und Propylen verschiedenen, Bestandteile, abzutrennen. Dabei sind die Erfordernisse der DE-A 10211275 zu beachten. Enthält das Produktgasgemisch A noch nennenswerte Mengen an molekularem Wasserstoff, wird vorgenannte Abtrennung in der Regel mit wenigstens einer Teilabtrennung des Wasserstoffs einhergehen oder man wird vorteilhaft eine solche Wasserstoffabtrennung vorab durchführen.

Letzteres kann z.B. dadurch erfolgen, dass man das Produktgasgemisch A, gegebenenfalls nachdem man es zuvor in einem indirekten Wärmetauscher abgekühlt hat (zweckmäßigerweise wird, wie bereits erwähnt, in der Reaktionszone A die dabei entnommene Wärme zum Erhitzen eines für das erfindungsgemäße Verfahren benötigten Feed-Gases verwendet) über eine, in der Regel zu einem Rohr (in Betracht kommt aber auch ein Platten- oder ein Wickelmodul) gestaltete, Membran leitet, die lediglich für den molekularen Wasserstoff durchlässig ist. Der so abgetrennte molekulare Wasserstoff kann bei Bedarf teilweise in die heterogen katalysierte Dehydrierung von Propan (d.h., in die Reaktionszone A) rückgeführt und/oder einer sonstigen Verwertung zugeführt werden. Beispielsweise kann er in Brennstoffzellen verbrannt werden. Alternativ kann eine partielle oder vollständige Wasserstoffabtrennung auch durch partielle Kondensation, Adsorption und/oder Rektifikation (vorzugsweise unter Druck) vorgenommen werden. Die Teil- oder Vollabtrennung von molekularem Wasserstoff aus Produktgasgemisch A kann beim erfindungsgemäßen Verfahren auch durch außerhalb der Reaktionszone A vorzunehmende selektive (z.B. heterogen katalysierte) Verbrennung desselben mit molekularem Sauerstoff vorgenommen werden. Das sich dabei bildende Reaktionswasser kann entweder teilweise oder vollständig abgetrennt oder im Gasgemisch belassen werden, da es in der Reaktionszone B als inertes Verdünnungsgas zu fungieren vermag. Diesbezüglich geeignete Katalysatoren offenbaren beispielsweise die US-A 4 788 371, US-A 4 886 928, US-A 5 430 209, US-A 5 5530171, US-A 5 527979 und US-A 5 563314.

Erfindungsgemäß zweckmäßig wird man wenigstens 10 mol-%, oder wenigstens 25 mol-%, häufig wenigstens 35 mol-%, oder wenigstens 50 mol-%, vielfach wenigstens 75 mol-% und oft die Gesamtmenge des im aus der Reaktionszone A herausgeführten Produktgasgemisch A enthaltenen molekularen Wasserstoff vorab- und/oder co- abtrennen, bevor das verbleibende Produktgasgemisch A' zur Beschickung der Reaktionszone B verwendet wird. Es ist jedoch ein ganz besonders vorteilhaftes Merkmal der vorstehend zuletzt beschriebenen, ganz besonders bevorzugten Betriebsweise der Reaktionszone A, nämlich der Kombination aus "Schlaufenvariante" und "Zwei-Abschnitt-Variante", mit adiabat endothermer Gestaltung des ersten Abschnitts und adiabat exothermer Gestaltung des zweiten Abschnitts (jeweils der Reaktionszone A), dass dort das Produktgasgemisch A bereits weitgehend frei von molekularem Wasserstoff anfällt, der im Rahmen der Reaktionszone A gebildete molekulare Wasserstoff noch in der Reaktionszone A nahezu quantitativ vorteilhaft genutzt wird und der dabei gebildete Wasserdampf als Bestandteil der in den ersten Abschnitt der Reaktionszone A rückgeführten Teilmenge des Produktgasgemischs A gleichfalls einer vorteilhaften Nutzung zugeführt wird. Bei Bedarf kann aus dem Produktgasgemisch A vor seiner Weiterverarbeitung in der Reaktionszone B auch eventuell darin enthaltenes Wasser teilweise oder vollständig abgetrennt (z.B. Auskondensieren) werden. Selbstredend kann bei Bedarf im Rahmen der Abtrennung von molekularem Wasserstoff und/oder Wasserdampf auch eine Abtrennung anderer, von Propan und Propylen verschiedener, Bestandteile des Produktgasgemisches A vorgenommen werden.

Eine einfache Möglichkeit dazu besteht z.B. darin, das vorzugsweise abgekühlte (vorzugsweise auf Temperaturen von 10 bis 100 bzw. 70°C), Produktgasgemisch A, z.B. bei einem Druck von 0,1 bis 50 bar, vorzugsweise 5 bis 15 bar und einer Temperatur von z.B. 0 bis 100°C, vorzugsweise 20 bis 40°C, mit einem (vorzugsweise hochsiedenden) organischen Lösungsmittel (vorzugsweise ein hydrophobes), in welchem Propan und Propylen (gegenüber den anderen Bestandteilen des Produktgasgemischs A zweckmäßig bevorzugt) absorbiert werden, in Kontakt zu bringen (z.B. durch einfaches Durchleiten). Durch nachfolgende Desorption, Rektifikation und/oder Strippung mit einem bezüglich der Reaktionszone B sich inert verhaltenden und/oder in dieser Reaktionszone als Reaktand benötigten Gas (z.B. Luft oder ein anderes Gemisch aus molekularem Sauerstoff und Inertgas) werden das Propan und Propylen im Gemisch in gereinigter Form rückgewonnen und zur Beschickung der Reaktionszone B verwendet (vorzugsweise wird wie im Vergleichsbeispiel 1 beschrieben vorgegangen) Das gegebenenfalls molekularen Wasserstoff enthaltende Abgas der Absorption kann man z.B. wieder einer Membrantrennung unterwerfen und dann, bei Bedarf, den abgetrennten Wasserstoff in der heterogen katalysierten Propandehydrierung in der Reaktionszone A mitverwenden.

Allerdings ist der C3-Kohlenwasserstoffe/C4-Kohlenwasserstoffe Trennfaktor beim vorstehenden Trennverfahren vergleichsweise begrenzt und für die in der DE-A 10245585 beschriebenen Bedürfnisse häufig nicht ausreichend.

Als Alternative für den beschriebenen Trennschritt via Absorption ist für die erfindungsgemäßen Zwecke daher häufig eine Druckwechseladsorption oder eine Druckrektifikation bevorzugt.

Als Absorptionsmittel für die vorstehend beschriebene absorptive Abtrennung eignen sich grundsätzlich alle Absorptionsmittel, die in der Lage sind, Propan und Propylen zu absorbieren. Bei dem Absorptionsmittel handelt es sich vorzugsweise um ein organisches Lösungsmittel, das vorzugsweise hydrophob und/oder hochsiedend ist. Vorteilhafterweise hat dieses Lösungsmittel einen Siedepunkt (bei einem Normaldruck von 1 atm) von mindestens 120°C, bevorzugt von mindestens 180°C, vorzugsweise von 200 bis 350°C, insbesondere von 250 bis 300°C, stärker bevorzugt von 260 bis 290°C. Zweckmäßigerweise liegt der Flammpunkt (bei einem Normaldruck von 1 bar) über 110°C. Allgemein eignen sich als Absorptionsmittel relativ unpolare organische Lösungsmittel, wie zum Beispiel aliphatische Kohlenwasserstoffe, die vorzugsweise keine nach außen wirkende polare Gruppe enthalten, aber auch aromatische Kohlenwasserstoffe. Allgemein ist es erwünscht, dass das Absorptionsmittel einen möglichst hohen Siedepunkt bei gleichzeitig möglichst hoher Löslichkeit für Propan und Propylen hat. Beispielhaft als Absorptionsmittel genannt seien aliphatische Kohlenwasserstoffe, zum Beispiel $C_8$-$C_{20}$-Alkane oder -Alkene, oder aromatische Kohlenwasserstoffe, zum Beispiel Mittelölfraktionen aus der Paraffindestillation oder Ether mit sperrigen (sterisch anspruchvollen) Gruppen am O-Atom, oder Gemische davon, wobei diesen ein polares Lösungsmittel, wie zum Beispiel das in der DE-A 43 08 087 offenbarte 1,2-Dimethylphthalat zugesetzt sein kann. Weiterhin eignen sich Ester der Benzoesäure und Phthalsäure mit geradkettigen, 1 bis 8 Kohlenstoffatome enthaltenden Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Diphenyl, Diphenylether und Gemische aus Diphenyl und Diphenylether oder deren Chlorderivate und Triarylalkene, zum Beispiel 4-Methyl-4'-benzyl-diphenylmethan und dessen Isomere 2-Methyl-2'-benzyl-diphenyl-methan, 2-Methyl-4'-benzyldiphenylmethan und 4-Methyl-2'-benzyl-diphenylmethan und Gemische solcher Isomerer. Ein geeignetes Absorptionsmittel ist ein Lösungsmittelgemisch aus Diphenyl und Diphenylether, bevorzugt in der azeotropen Zusammensetzung, insbesondere aus etwa 25 Gew.-% Diphenyl (Biphenyl) und etwa 75 Gew.-% Diphenylether, beispielsweise das im Handel erhältliche Diphyl® (z.B von der Bayer Aktiengesellschaft bezogenes). Häufig enthält dieses Lösungsmittelgemisch ein Lösungsmittel wie Dimethylphthalat in einer Menge von 0,1 bis 25 Gew.-%, bezogen auf das gesamte Lösungsmittelgemisch, zugesetzt. Besonders geeignete Absorptionsmittel sind auch Octane, Nonane, Decane, Undecane, Dodecane, Tridecane, Tetradecane, Pentadecane, Hexadecane, Heptadecane und Octadecane, wobei sich insbesondere Tetradecane als besonders geeignet erwiesen haben. Günstig ist es, wenn das verwendete Absorptionsmittel einerseits den oben genannten Siedepunkt erfüllt, andererseits gleichzeitig aber ein nicht zu hohes Molekulargewicht aufweist. Mit Vorteil beträgt das Molekulargewicht des Absorptionsmittels ≤ 300 g/mol. Geeignet sind auch die in DE-A 33 13 573 beschriebenen Paraffinöle mit 8 bis 16 Kohlenstoffatomen. Beispiele für geeignete Handelsprodukte sind von der Firma Haltermann vertriebene Produkte wie Halpasole i, wie Halpasol 250/340 i und Halpasol 250/275 i, sowie Druckfarbenöle unter den Bezeichnungen PKWF und Printosol. Bevorzugt sind an Aromaten freie Handelsprodukte, z.B. solche des Typs PKWFaf.

Die Durchführung der Absorption unterliegt keinen besonderen Beschränkungen. Es können alle dem Fachmann gängigen Verfahren und Bedingungen eingesetzt werden. Vorzugsweise wird das Gasgemisch bei einem Druck von 1 bis 50 bar, bevorzugt 2 bis 20 bar, stärker bevorzugt 5 bis 15 bar, und einer Temperatur von 0 bis 100°C, insbesondere von 20 bis 50 bzw. 40°C, mit dem Absorptionsmittel in Kontakt gebracht. Die Absorption kann sowohl in Kolonnen als auch in Quenchapparaten vorgenommen werden. Dabei kann im Gleichstrom oder im Gegenstrom (ist bevorzugt) gearbeitet werden. Geeignete Absorptionskolonnen sind zum Beispiel Bodenkolonnen (mit Glocken- und/oder Siebböden), Kolonnen mit strukturierten Packungen (zum Beispiel Blechpackungen mit einer spezifischen Oberfläche von 100 bis 1000, oder bis 750 m²/m³, zum Beispiel Mellapak® 250 Y) und Füllkörperkolonnen (zum Beispiel mit Raschig-Füllkörpern gefüllte). Es können aber auch Riesel- und Sprühtürme, Graphitblockabsorber, Oberflächenabsorber wie Dickschicht- und Dünnschichtabsorber sowie Tellerwäscher, Kreuzschleierwäscher und Rotationswäscher verwendet werden. Zudem kann es günstig sein, die Absorption in einer Blasensäule mit und ohne Einbauten stattfinden zu lassen.

Die Abtrennung des Propans und Propylens von dem Absorptionsmittel kann durch Strippung, Entspannungsverdampfung (Flashen) und/oder Destillation erfolgen.

Die Abtrennung des Propans und Propylens von dem Absorptionsmittel erfolgt vorzugsweise durch Strippen und/oder Desorption. Die Desorption kann in üblicher Weise über eine Druck- und/oder Temperaturänderung durchgeführt werden, vorzugsweise bei einem Druck von 0,1 bis 10 bar, insbesondere 1 bis 5 bar, stärker bevorzugt 1 bis 3 bar, und einer Temperatur von 0 bis 200°C, insbesondere 20 bis 100°C, stärker bevorzugt 30 bis 70°C, besonders bevorzugt 30 bis 50°C. Ein für die Strippung geeignetes Gas ist beispielsweise Wasserdampf, bevorzugt sind jedoch insbesondere Sauerstoff-/Stickstoff-Mischungen, beispielsweise Luft. Bei der Verwendung von Luft beziehungsweise Sauerstoff-/Stickstoff-Mischungen, bei denen der Sauerstoffgehalt über 10 Vol.-% liegt, kann es sinnvoll sein, vor und/oder während des Strippprozesses ein Gas zuzusetzen, das den Explosionsbereich reduziert. Besonders geeignet dafür sind Gase mit einer spezifischen Wärmekapazität ≥ 29 J/mol•K bei 20°C, wie zum Beispiel Methan, Ethan, Propan, Propen, Benzol, Methanol, Ethanol, sowie Ammoniak, Kohlendioxid, und Wasser. C4-Kohlenwasserstoffe sind erfindungsgemäß bevorzugt als solche Zusätze jedoch zu vermeiden. Besonders geeignet für die Strippung sind auch Blasensäulen mit und ohne Einbauten.

Die Abtrennung des Propans und Propylens von dem Absorptionsmittel kann auch über eine Destillation bzw. Rektifikation erfolgen, wobei die dem Fachmann geläufigen Kolonnen mit Packungen, Füllkörpern oder entsprechenden Einbauten verwendet werden können. Bevorzugte Bedingungen bei der Destillation bzw. Rektifikation sind ein Druck von 0,01 bis 5 bar, insbesondere 0,1 bis 4 bar, stärker bevorzugt 1 bis 3 bar, und eine Temperatur (im Sumpf) von 50 bis 300°C, insbesondere 150 bis 250°C.

Ein durch Strippen aus dem Absorptionsmittel gewonnenes Gasgemisch kann vor seiner Verwendung zur Beschickung der Reaktionszone B noch einer weiteren Verfahrensstufe zugeführt werden, um z.B. die Verluste an mitgestripptem Absorptionsmittel zu reduzieren (z.B. Abscheidung in Demistern und/oder Tiefenfiltern) und die Reaktionszone B so gleichzeitig vor Absorptionsmittel zu schützen oder um die Trennwirkung zwischen C3-/C4-Kohlenwasserstoffen weiter zu verbessern. Eine solche Abtrennung des Absorptionsmittels kann nach allen dem Fachmann bekannten Verfahrensschritten erfolgen. Eine im Rahmen des erfindungsgemäßen Verfahrens bevorzugte Ausführungsform einer solchen Abtrennung ist z.B. das Quenchen des Ausgangsstromes aus der Strippvorrichtung mit Wasser. In diesem Fall wird das Absorptionsmittel aus diesem beladenen Ausgangsstrom mit Wasser herausgewaschen und der Ausgangsstrom gleichzeitig mit Wasser beladen. Diese Wäsche beziehungsweise das Quenchen kann z.B. am Kopf einer Desorptionskolonne über einem Flüssigkeits-Fangboden durch Gegensprühen von Wasser oder in einem eigenen Apparat erfolgen.

Zur Unterstützung des Trenneffektes können im Quenchraum die Quenchoberfläche vergößernde Einbauten installiert werden, wie sie dem Fachmann von Rektifikationen, Absorptionen und Desorptionen her bekannt sind.

Wasser ist insofern ein bevorzugtes Waschmittel, da es in der nachfolgenden wenigstens einen Partialzone normalerweise nicht stört. Nachdem das Wasser das Absorptionsmittel aus dem mit Propan und Propylen beladenen Ausgangstrom herausgewaschen hat, kann das Wasser/Absorptionsmittel-Gemisch einer Phasentrennung zugeführt und der behandelte Ausgangstrom unmittelbar der Reaktionszone B zugeführt werden.

Sowohl das im wesentlichen C3-frei gestrippte Absorptionsmittel als auch das in der Phasentrennung rückgewonnene Absorptionsmittel können für den Absorptionszweck wiederverwendet werden.

Das in einer wie beispielhaft beschrieben durchführbaren Abtrennung anfallende, das Propan und Propen enthaltende Gasgemisch kann nun in an sich bekannter Weise zur Beschickung einer heterogen katalysierten Gasphasenpartialoxidation des Propylens zu Acrolein und/oder Acrylsäure eingesetzt werden, wie es z.B. in der WO 01/96270 und in den Schriften DE-A 10245585, DE-A 10246119, DE-A 10313210, DE-A 10313214, DE-A 10313213, DE-A 10313212, DE-A 10308824, DE-A 10313208 und DE-A 10211275 beschrieben ist. Als Oxidationsmittel kann dabei reiner molekularer Sauerstoff, Luft, mit Sauerstoff angereicherte Luft oder jedes sonstige Gemisch aus Sauerstoff und Inertgas zugesetzt werden.

Dabei wird man beim erfindungsgemäßen Verfahren die Zusammensetzung des Beschickungsgasgemisches (vgl. DE-A 10245585 und DE-A 10246119) für die Reaktionszone B so einstellen, dass es die nachfolgenden molaren Verhältnisse erfüllt:

Propan : Propen : $N_2$ : $O_2$ : $H_2O$ : sonstige = 0,5 bis 20 : 1 : 0,1 bis 40 : 0,1 bis 10 : 0 bis 20 : 0 bis 1.

Mit Vorteil betragen die vorgenannten molaren Verhältnisse erfindungsgemäß = 1 bzw. 2 bis 10 : 1 : 0,5 bis 20 : 0,5 bis 5 : 0,01 bis 10 : 0 bis 1.

Günstig ist es auch, wenn die vorgenannten molaren Verhältnisse erfindungsgemäß = 3 bis 6 : 1 : 1 bis 10 : 1 bis 3 : 0,1 bis 2 : 0 bis 0,5 betragen.

In an sich bekannter Weise läuft die heterogen katalysierte Gasphasen-Partialoxidation von Propylen zu Acrylsäure mit molekularem Sauerstoff in zwei längs der Reaktionskoordinate aufeinanderfolgenden Schritten ab, von denen der erste zum Acrolein und der zweite vom Acrolein zur Acrylsäure führt.

Dieser Reaktionsablauf in zwei zeitlich aufeinanderfolgenden Schritten eröffnet in an sich bekannter Weise die Möglichkeit, die Reaktionszone B des erfindungsgemäßen Verfahrens in diesem Fall zweistufig, d.h., in zwei hintereinander angeordneten Oxidationszonen auszuführen, wobei in jeder der beiden Oxidationszonen der zu verwendende oxidische Katalysator in optimierender Weise angepasst werden kann (auch kann die Reaktion auf der Stufe des Acrolein abgebrochen werden). So wird für die erste Oxidationszone (Propylen → Acrolein) in der Regel ein Katalysator auf der Basis von die Elementkombination Mo-Bi-Fe enthaltenden Multimetalloxiden bevorzugt, während für die zweite Oxidationszone (Acrolein → Acrylsäure) normalerweise Katalysatoren auf der Basis von die Elementkombination Mo-V enthaltenden Multimetalloxiden bevorzugt werden.

Entsprechende Multimetalloxidkatalysatoren für die beiden Oxidationszonen sind vielfach vorbeschrieben und dem Fachmann wohlbekannt. Beispielsweise verweist die EP-A 253 409 auf Seite 5 auf entsprechende US-Patente.

Günstige Katalysatoren für die beiden Oxidationszonen offenbaren auch die DE-A 4 431 957, die DE-A 102004025445 und die DE-A 4431949. Dieses gilt insbesondere für jene der allgemeinen Formel I in den beiden vorgenannten Schriften. Besonders vorteilhafte Katalysatoren für die beiden Oxidationszonen offenbaren die Schriften DE-A 10325488, DE-A 10325487, DE-A 10353954, DE-A 10344149, DE-A 10351269, DE-A 10350812, DE-A 10350822.

Die vorgenannten Schriften offenbaren auch erfindungsgemäß geeignete Gasphasenpartialoxidationsverfahren.

Für den ersten Schritt der Partialoxidation, die heterogen katalysierte Gasphasen-Partialoxidation von Propylen zu Acrolein, kommen, wie bereits gesagt, prinzipiell alle Mo, Bi und Fe enthaltenden Multimetalloxidmassen in Betracht.

Dies sind insbesondere die Multimetalloxidaktivmassen der allgemeinen Formel I der DE-A 19955176, die Multimetalloxidaktivmassen der allgemeinen Formel I der DE-A 19948523, die Multimetalloxidaktivmassen der allgemeinen Formeln I, II und III der DE-A 10101695, die Multimetalloxidaktivmassen der allgemeinen Formeln I, II und III der DE-A 19948248 und die Multimetalloxidaktivmassen der allgemeinen Formeln I, II und III der DE-A 19955168 sowie die in der EP-A 700714 genannten Multimetalloxidaktivmassen.

Ferner eignen sich für diesen Oxidationsschritt die Mo, Bi und Fe enthaltenden Multimetalloxidkatalysatoren die in den Schriften DE-A 10046957, DE-A 10063162, DE-C 3338380, DE-A 19902562, EP-A 15565, DE-C 2380765, EP-A 807465, EP-A 279374, DE-A 3300044, EP-A 575897, US-A 4438217, DE-A 19855913, WO 98/24746, DE-A 19746210 (diejenigen der allgemeinen Formel II), JP-A 91/294239, EP-A 293224 und EP-A 700714 offenbart werden. Dies gilt insbesondere für die beispielhaften Ausführungsformen in diesen Schriften, unter denen jene der EP-A 15565, der EP-A 575897, der DE-A 19746210 und der DE-A 19855913 besonders bevorzugt werden. Besonders hervorzuheben sind in diesem Zusammenhang ein Katalysator gemäß Beispiel 1 c aus der EP-A 15565 sowie ein in entsprechender Weise herzustellender Katalysator, dessen Aktivmasse jedoch die Zusammensetzung $Mo_{12}Ni_{6,5}Zn_2Fe_2Bi_1P_{0,0065}K_{0,06}O_x \cdot 10SiO_2$ aufweist. Ferner sind hervorzuheben das Beispiel mit der laufenden Nr. 3 aus der DE-A 19855913 (Stöchiometrie: $Mo_{12}Co_7Fe_3Bi_{0,6}K_{0,08}Si_{1,6}O_x$) als Hohlzylindervollkatalysator der Geometrie 5 mm x 3 mm x 2 mm (Außendurchmesser x Höhe x Innendurchmesser) sowie der Multimetalloxid II - Vollkatalysator gemäß Beispiel 1 der DE-A 19746210. Ferner wären die Multimetalloxid-Katalysatoren der US-A 4438217 zu nennen. Letzteres gilt insbesondere dann, wenn diese Hohlzylinder eine Geometrie 5,5 mm x 3 mm x 3,5 mm, oder 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Höhe x Innendurchmesser) aufweisen. Weitere mögliche Katalysatorgeometrien sind in diesem Zusammenhang Stränge (z.B. 7,7 mm Länge und 7 mm Durchmesser; oder 6,4 mm Länge und 5,7 mm Durchmesser).

Eine Vielzahl der für den Schritt vom Propylen zum Acrolein geeigneten Multimetalloxidaktivmassen lässt sich unter der allgemeinen Formel IV,

$$Mo_{12}Bi_aFe_bX^1_cX^2_dX^3_eX^4_fO_n \qquad (IV),$$

in der die Variablen nachfolgende Bedeutung aufweisen:

$X^1 =$ Nickel und/oder Kobalt,
$X^2 =$ Thallium, ein Alkalimetall und/oder ein Erdalkalimetall,
$X^3 =$ Zink, Phosphor, Arsen, Bor, Antimon, Zinn, Cer, Blei und/oder Wolfram,
$X^4 =$ Silicium, Aluminium, Titan und/oder Zirkonium,

a= 0,5 bis 5,
b= 0,01 bis 5, vorzugsweise 2 bis 4,
c= 0 bis 10, vorzugsweise 3 bis 10,
d= 0 bis 2, vorzugsweise 0,02 bis 2,
e= 0 bis 8, vorzugsweise 0 bis 5,
f= 0 bis 10 und

n= eine Zahl, die durch die Wertigkeit und Häufigkeit der von Sauerstoff verschiedenen Elemente in IV bestimmt wird,

subsummieren.

Sie sind in an sich bekannter Weise erhältlich (siehe z.B. die DE-A 4023239) und werden üblicherweise in Substanz zu Kugeln, Ringen oder Zylindern geformt oder auch in Gestalt von Schalenkatalysatoren, d.h., mit der Aktivmasse beschichteten vorgeformten, inerten Trägerkörpern, eingesetzt. Selbstverständlich können sie aber auch in Pulverform als Katalysatoren angewendet werden.

Prinzipiell können Aktivmassen der allgemeinen Formel IV in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, $NH_3$, CO und/oder $H_2$) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, AmmoniumSalze und/oder Hydroxide in Betracht (Verbindungen wie $NH_4OH$, $(NH_4)_2CO_3$, $NH_4NO_3$, $NH_4CHO_2$, $CH_3COOH$, $NH_4CH_3CO_2$ und/oder Ammoniumoxalat, die spätestens beim späteren Calcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidaktivmassen IV kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die Multimetalloxidaktivmassen der allgemeinen Formel IV können für den Schritt "Propylen → Acrolein" sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine besonders günstige Hohlzylindergeometrie beträgt 5 mm x 3 mm x 2 mm (Außendurchmesser x Länge x Innendurchmesser), insbesondere im Fall von Vollkatalysatoren.

Selbstverständlich kann die Formgebung der pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist. Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Sie verhalten sich bezüglich der dem erfindungsgemäßen Verfahren unterliegenden Zielreaktion in der Regel im wesentlichen inert. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit, deren Durchmesser 1 bis 10 mm bzw. bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Für den Schritt vom Propylen zum Acrolein zu verwendende Multimetalloxidaktivmassen sind ferner Massen der allgemeinen Formel V,

$$[Y^1{}_{a'}Y^2{}_{b'}O_{x'}]_p[Y^c{}_{c'}Y^4{}_{d'}Y^5{}_{e'}Y^6{}_{f'}Y^7{}_{g'}Y^2{}_{h'}O_{y'}]_q \qquad (V),$$

in der die Variablen folgende Bedeutung haben:

$Y^1$ = nur Wismut oder Wismut und wenigstens eines der Elemente Tellur, Antimon, Zinn und Kupfer,

$Y^2$ = Molybdän oder Molybdän und Wolfram,

$Y^3$ = ein Alkalimetall, Thallium und/oder Samarium,

$Y^4$ = ein Erdalkalimetall, Nickel, Kobalt, Kupfer, Mangan, Zink, Zinn, Cadmium und/oder Quecksilber,

$Y^5$ = Eisen oder Eisen und wenigstens eines der Elemente Chrom und Cer,

$Y^6$ = Phosphor, Arsen, Bor und/oder Antimon,

$Y^7$ = ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

a' = 0,01 bis 8,

b' = 0,1 bis 30,

c' = 0 bis 4,

d' = 0 bis 20,

e' > 0 bis 20,

f' = 0 bis 6,

g' = 0 bis 15,

h' = 8 bis 16,

x',y'= Zahlen, die durch die Wertigkeit und Häufigkeit der von Sauerstoff verschiedenen Elemente in V bestimmt werden und

p,q = Zahlen, deren Verhältnis p/q 0,1 bis 10 beträgt,

enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Y^1_{a'}Y^2_{b'}O_{x'}$, deren Größtdurchmesser (längste durch den Schwerpunkt des Bereichs gehende direkte Verbindungsstrecke zweier auf der Oberfläche (Grenzfläche) des Bereichs befindlicher Punkte) 1 nm bis 100 $\mu$m, häufig 10 nm bis 500 nm oder 1 $\mu$m bis 50 bzw. 25 $\mu$m, beträgt.

Besonders vorteilhafte erfindungsgemäße Multimetalloxidmassen V sind solche, in denen $Y^1$ nur Wismut ist.

Unter diesen werden wiederum jene bevorzugt, die der allgemeinen Formel VI,

$$[Bi_{a''}Z^2_{b''}O_{x''}]_{p''} [Z^2_{12}Z^3_{c''}Z^4_{d''}Fe_{e''}Z^5_{f''}Z^6_{g''}Z^7_{h''}O_{y''}]_{q''} \qquad (VI),$$

in der die Varianten folgende Bedeutung haben:

$Z^2$ = Molybdän oder Molybdän und Wolfram,

$Z^3$ = Nickel und/oder Kobalt,

$Z^4$ = Thallium, ein Alkalimetall und/oder ein Erdalkalimetall,

$Z^5$ = Phosphor, Arsen, Bor, Antimon, Zinn, Cer und/oder Blei,

$Z^6$ = Silicium, Aluminium, Titan und/oder Zirkonium,

$Z^7$ = Kupfer, Silber und/oder Gold,

a" = 0,1 bis 1,

b" = 0,2 bis 2,

c" = 3 bis 10,

d" = 0,02 bis 2,

e" = 0,01 bis 5, vorzugsweise 0,1 bis 3,

f" = 0 bis 5,

g" = 0 bis 10,

h" = 0 bis 1,

x",y"= Zahlen, die durch die Wertigkeit und Häufigkeit der von Sauerstoff verschiedenen Element in VI bestimmt werden,

p",q"= Zahlen, deren Verhältnis p"/q" 0,1 bis 5, vorzugsweise 0,5 bis 2 beträgt,

entsprechen, wobei diejenigen Massen VI ganz besonders bevorzugt werden, in denen $Z^2_{b''}$ = (Wolfram)$_{b''}$ und $Z^2_{12}$ = (Molybdän)$_{12}$ ist.

Ferner ist es von Vorteil, wenn wenigstens 25 mol-% (bevorzugt wenigstens 50 mol-% und besonders bevorzugt 100 mol-%) des gesamten Anteils $[Y^1_{a'}Y^2_{b'}O_{x'}]_p$ ($[Bi_{a''}Z^2_{b''}O_{x''}]_{p''}$) der erfindungsgemäß geeigneten Multimetalloxidmassen V (Multimetalloxidmassen VI) in den erfindungsgemäß geeigneten Multimetalloxidmassen V (Multimetalloxidmassen VI) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung $Y^1_{a'}Y^2_{b'}O_{x'}$ $[Bi_{a''}Z^2_{b''}O_{x''}]$ vorliegen, deren Größtdurchmesser im Bereich 1 nm bis 100 $\mu$m liegt.

Hinsichtlich der Formgebung gilt bezüglich Multimetalloxidmassen V-Katalysatoren das bei den Multimetalloxidmassen IV-Katalysatoren Gesagte.

Die Herstellung von Multimetalloxidmassen V-Aktivmassen ist z.B. in der EP-A 575897 sowie in der DE-A 19855913 beschrieben.

Die vorstehend empfohlenen inerten Trägermaterialien kommen u.a. auch als inerte Materialien zur Verdünnung und/oder Abgrenzung der entsprechenden Katalysatorfestbetten, bzw. als deren schützende und/oder das Gasgemisch aufheizende Vorschüttung in Betracht.

An dieser Stelle sei erwähnt, daß sich alle Katalysatoren und Multimetalloxidmassen, die für den Schritt vom Propylen zum Acrolein als geeignet empfohlen wurden, sich grundsätzlich auch für die Partialammoxidation von Propylen zu Acrylnitril eignen.

Für den zweiten Schritt, die heterogen katalysierte Gasphasen-Partialoxidation von Acrolein zu Acrylsäure, kommen, wie bereits gesagt, prinzipiell alle Mo und V enthaltenden Multimetalloxidmassen als Aktivmassen in Betracht, z.B. jene der DE-A 10046928.

Eine Vielzahl derselben, z.B. diejenigen der DE-A 19815281, lässt sich unter der allgemeinen Formel VII,

$$Mo_{12}V_aX^1_bX^2_cX^3_dX^4_eX^5_fX^6_gO_n \qquad (VII),$$

in der die Variablen folgende Bedeutung haben:

$X^1 =$  W, Nb, Ta, Cr und/oder Ce,
$X^2 =$  Cu, Ni, Co, Fe, Mn und/oder Zn,
$X^3 =$  Sb und/oder Bi,
$X^4 =$  eines oder mehrere Alkalimetalle,
$X^5 =$  eines oder mehrere Erdalkalimetalle,
$X^6 =$  Si, Al, Ti und/oder Zr,

$a =$  1 bis 6,
$b =$  0,2 bis 4,
$c =$  0,5 bis 18,

$d =$  0 bis 40,
$e =$  0 bis 2,
$f =$  0 bis 4,
$g =$  0 bis 40 und
$n =$  eine Zahl, die durch die Wertigkeit und Häufigkeit der von Sauerstoff verschiedenen Elemente in VII bestimmt wird,

subsummieren.

Erfindungsgemäß bevorzugte Ausführungsformen innerhalb der aktiven Multimetalloxide VII sind jene, die von den nachfolgenden Bedeutungen der Variablen der allgemeinen Formel VII erfasst werden:

$X^1 =$  W, Nb, und/oder Cr,
$X^2 =$  Cu, Ni, Co, und/oder Fe,
$X^3 =$  Sb,
$X^4 =$  Na und/oder K,
$X^5 =$  Ca, Sr und/oder Ba,
$X^6 =$  Si, Al, und/oder Ti,

$a =$  1,5 bis 5,
$b =$  0,5 bis 2,
$c =$  0,5 bis 3,
$d =$  0 bis 2,
$e =$  0 bis 0,2,
$f =$  0 bis 1 und
$n =$  eine Zahl, die durch die Wertigkeit und Häufigkeit der von Sauerstoff verschiedenen Elemente in VII bestimmt wird.

Erfindungsgemäß ganz besonders bevorzugte Multimetalloxide VII sind jedoch jene der allgemeinen Formel VII

$$Mo_{12}V_{a'}Y^1_{b'}Y^2_{c'}Y^5_{f'}Y^6_{g'}O_{n'} \qquad (VIII),$$

mit

$Y^1 =$  W und/oder Nb,
$Y^2 =$  Cu und/oder Ni,
$Y^5 =$  Ca und/oder Sr,
$Y^6 =$  Si und/oder Al,

a' =     2 bis 4,

b' =     1 bis 1,5,

c'=     1 bis 3,

f =     0 bis 0,5

g' =     0 bis 8 und

n' =     eine Zahl, die durch die Wertigkeit und Häufigkeit der von Sauerstoff verschiedenen Elementen in VIII bestimmt wird.

Die erfindungsgemäß geeigneten Multimetalloxidaktivmassen (VII) sind in an sich bekannter, z.B. in der DE-A 4335973 oder in der EP-A 714700 offenbarter, Weise erhältlich.

Prinzipiell können für den Schritt "Acrolein → Acrylsäure" geeignete Multimetalloxidaktivmassen, insbesondere solche der allgemeinen Formel VII, in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemische aus Inertgas und reduzierenden Gasen wie $H_2$, $NH_3$, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen VII kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen VII kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die resultierenden Multimetalloxidmassen, insbesondere jene der allgemeinen Formel VII, können für die Acroleinoxidation sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm (z.B. 8,2 mm oder 5,1 mm) betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist.

Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 μm, bevorzugt im Bereich 50 bis 500 μm und besonders bevorzugt im Bereich 150 bis 250 μm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder mit Splittauflage, bevorzugt werden. Geeignet ist die Verwendung von im wesentlichen unporösen, oberflächenrauhen, kugelförmigen Trägern aus Steatit, deren Durchmesser 1 bis 10 mm bzw. bis 8 mm, bevorzugt 4 bis 5 mm beträgt. D.h., geeignete Kugelgeometrien können Durchmesser von 8,2 mm oder von 5,1 mm aufweisen. Geeignet ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepass(vgl. EP-A 714 700).

Günstige für den Schritt "Acrolein → Acrylsäure" zu verwendende Multimetalloxidaktivmassen sind ferner Massen der allgemeinen Formel IX,

$$[D]_p[E]_q \qquad (IX),$$

in der die Variablen folgende Bedeutung haben:

D = $Mo_{12}V_{a''}Z^1_{b''}Z^2_{c''}Z^3_{d''}Z^4_{e''}Z^5_{f''}Z^6_{g''}O_{x''}$,
E = $Z^1_{12}Cu_{h''}H_{i''}Q_{y''}$
$Z^1$ = W, Nb, Ta, Cr und/oder Ce,
$Z^2$ = Cu, Ni, Co, Fe, Mn und/oder Zn,
$Z^3$ = Sb und/oder Bi,
$Z^4$ = Li, Na, K, Rb, Cs und/oder H
$Z^5$ = Mg, Ca, Sr und/oder Ba,
$Z^6$ = Si, Al, Ti und/oder Zr,
$Z^7$ = Mo, W, V, Nb und/oder Ta, vorzugsweise Mo und/oder W

a" = 1 bis 8,
b" = 0,2 bis 5,
c" = 0 bis 23,
d" = 0 bis 50,
e" = 0 bis 2,
f" = 0 bis 5,
g" = 0 bis 50,
h" = 4 bis 30,
i" = 0 bis 20 und
x",y" = Zahlen, die durch die Wertigkeit und Häufigkeit der von Sauerstoff verschiedenen Element in IX bestimmt werden und
p,q = von Null verschiedene Zahlen, deren Verhältnis p/q 160:1 bis 1:1 beträgt,

und die dadurch erhältlich sind, dass man eine Multimetalloxidmasse E

$$Z^7_{12}Cu_{h''}H_{i''}O_{y''} \qquad (E),$$

in feinteiliger Form getrennt vorbildet (Ausgangsmasse 1) und anschließend die vorgebildete feste Ausgangsmasse 1 in eine wäßrige Lösung, eine wäßrige Suspension oder in ein feinteiliges Trockengemisch von Quellen der Elemente Mo, V, $Z^1$, $Z^2$, $Z^3$, $Z^4$, $Z^5$, $Z^6$, die die vorgenannten Elemente in der Stöchiometrie D

$$Mo_{12}V_{a''}Z^1_{b''}Z^2_{c''}Z^3_{d''}Z^4_{e''}Z^5_{f''}Z^6_g \qquad (D),$$

enthält (Ausgangsmasse 2), im gewünschten Mengenverhältnis p:q einarbeitet, die dabei gegebenenfalls resultierende wässrige Mischung trocknet, und die so gegebene trockene Vorläufermasse vor oder nach ihrer Trocknung zur gewünschten Katalysatorgeometrie bei Temperaturen von 250 bis 600°C calciniert.

Bevorzugt sind die Multimetalloxidmassen IX, bei denen die Einarbeitung der vorgebildeten festen Ausgangsmasse 1 in eine wässrige Ausgangsmasse 2 bei einer Temperatur < 70°C erfolgt. Eine detaillierte Beschreibung der Herstellung von Multimetalloxidmassen VI-Katalysatoren enthalten z.B. die EP-A 668104, die DE-A 19736105, die DE-A 10046928, die DE-A 19740493 und die DE-A 19528646.

Hinsichtlich der Formgebung gilt bezüglich Multimetalloxidmassen IX-Katalysatoren das bei den Multimetalloxidmassen VII-Katalysatoren Gesagte.

Für den Schritt "Acrolein → Acrylsäure" in hervorragender Weise geeignete Multimetalloxidkatalysatoren sind auch jene der DE-A 19815281, insbesondere mit Multimetalloxidaktivmassen der allgemeinen Formel I dieser Schrift.

Vorteilhaft werden für den Schritt vom Propylen zum Acrolein Vollkatalysatorringe und für den Schritt vom Acrolein zur Acrylsäure Schalenkatalysatorringe verwendet.

Die Durchführung des ersten Schrittes der Partialoxidation, vom Propylen zum Acrolein, kann mit den beschriebenen Katalysatoren z.B. in einem Einzonen-Vielkontaktrohr-Festbettreaktor erfolgen, wie ihn die DE-A 4431957 beschreibt.

Als Oxidationsmittel wird Sauerstoff verwendet. Wird als inertes Verdünnungsgas $N_2$ gewählt, erweist sich die Verwendung von Luft als Sauerstoffquelle besonders vorteilhaft.

Häufig wird bei einem Propylen : Sauerstoff: indifferente Gase (einschließlich Wasserdampf) Volumen (N1)-Verhältnis von 1:(1,0 bis 3,0):(5 bis 25), vorzugsweise 1:(1,7 bis 2,3):(10 bis 15) gearbeitet. Der Reaktionsdruck liegt üblicherweise im Bereich von 1 bis 3 bar und die Gesamtraumbelastung beträgt vorzugsweise 1500 bis 4000 bzw. 6000 Nl/l•h oder mehr. Die Propylenlast beträgt typisch 90 bis 200 Nl/l•h oder bis 300 Nl/l•h oder mehr.

Bevorzugt wird der Einzonen-Vielkontaktrohr-Festbettreaktor mit dem Beschickungsgasgemisch von oben angeströmt. Als Wärmeaustauschmittel wird zweckmäßig eine Salzschmelze, bevorzugt bestehend aus 60 Gew.-% Kaliumnitrat ($KNO_3$) und 40 Gew.-% Natriumnitrit ($NaNO_2$), oder aus 53 Gew.-% Kaliumnitrat ($KNO_3$), 40 Gew.-% Natriumnitrit ($NaNO_2$) und 7 Gew.-% Natriumnitrat ($NaNO_3$), eingesetzt.

Über den Reaktor betrachtet können Salzschmelze und Reaktionsgasgemisch sowohl im Gleichstrom als auch im Gegenstrom geführt werden. Die Salzschmelze selbst wird bevorzugt mäanderförmig um die Kontaktrohre geführt.

Strömt man die Kontaktrohre von oben nach unten an, ist es zweckmäßig, die Kontaktrohre von unten nach oben wie folgt mit Katalysator zu beschicken (für die Anströmung von unten nach oben wird die Beschickungsabfolge in zweckmäßiger Weise umgekehrt):

- zunächst auf einer Länge von 40 bis 80 bzw. bis 60 % der Kontaktrohrlänge entweder nur Katalysator, oder ein Gemisch aus Katalysator und Inertmaterial, wobei letzteres, bezogen auf die Mischung, einen Gewichtsanteil von bis zu 20 Gew.-% ausmacht (Abschnitt C);

- daran anschließend auf einer Länge von 20 bis 50 bzw. bis 40 % der Gesamtrohrlänge entweder nur Katalysator, oder ein Gemisch aus Katalysator und Inertmaterial, wobei letzteres, bezogen auf die Mischung, einen Gewichtsanteil von bis zu 40 Gew.-% ausmacht (Abschnitt B); und

- abschließend auf einer Länge von 10 bis 20 % der Gesamtrohrlänge eine Schüttung aus Inertmaterial (Abschnitt A), die vorzugsweise so gewählt wird, dass sie einen möglichst geringen Druckverlust bedingt.

Bevorzugt ist der Abschnitt C unverdünnt.

Vorgenannte Beschickungsvariante ist insbesondere dann zweckmäßig, wenn als Katalysatoren solche gemäß Beispiel 1 der DE-A 10046957 oder gemäß Beispiel 3 der DE-A 10046957 und als Inertmaterial Ringe aus Steatit mit der Geometrie 7 mm x 7 mm x 4 mm (Außendurchmesser x Höhe x Innendurchmesser) verwendet werden. Hinsichtlich der Salzbadtemperatur gilt das in der DE-A 4431957 Gesagte.

Die Durchführung des ersten Schrittes der Partialoxidation, vom Propylen zum Acrolein, kann mit den beschriebenen Katalysatoren aber auch z.B. in einem Zweizonen-Vielkontaktrohr-Festbettreaktor erfolgen, wie es die DE-A 19910506 beschreibt. In beiden vorstehend beschriebenen Fällen liegt der erzielte Propenumsatz bei einfachem Durchgang normalerweise bei Werten $\geq$ 90 mol%, bzw. $\geq$ 95 mol-%. Die Durchführung des zweiten Schrittes der Partialoxidation, vom Acrolein zur Acrylsäure, kann mit den beschriebenen Katalysatoren z.B. in einem Einzonen-Vielkontaktrohr-Festbettreaktor durchgeführt werden, wie es die DE-A 4431949 beschreibt. In der Regel wird das Produktgemisch der Propylenpartialoxidation zu Acrolein als solches (gegebenenfalls nach erfolgter Zwischenkühlung desselben), d.h., ohne Nebenkomponentenabtrennung, in die Acroleinoxidation zu Acrylsäure geführt.

Der für den zweiten Schritt der Partialoxidation benötigte Sauerstoff wird dabei bevorzugt als Luft zugesetzt und in der Regel dem Produktgasgemisch der Propylenpartialoxidation unmittelbar zugegeben (er kann aber auch schon im Reaktionsgasausgangsgemisch für die Propylenpartialoxidation enthalten sein).

In der Regel ist das Beschickungsgasgemisch einer solchen Acroleinpartialoxidation dann wie folgt zusammengesetzt: Acrolein : Sauerstoff: Wasserdampf: Inertgas-Volumenverhältnis (NI) von 1:(1 bis 3):(0 bis 20):(3 bis 30), vorzugsweise von 1:(1 bis 3):(0,5 bis 10):(7 bis 18).

Der Reaktionsdruck beträgt in der Regel auch hier 1 bis 3 bar und die Gesamtraumbelastung liegt vorzugsweise bei 1000 bis 3800, bzw. 4800 Nl/l·h oder mehr. Die Acroleinlast beträgt typisch 80 bis 190, bzw. 290 Nl/l·h oder mehr.

Bevorzugt wird der Einzonen-Vielkontaktrohr-Festbettreaktor mit dem Beschickungsgasgemisch ebenfalls von oben angeströmt. Als Wärmeaustauschmittel wird auch in der zweiten Stufe in zweckmäßiger Weise eine Salzschmelze, bevorzugt aus 60 Gew.-% Kaliumnitrat ($KNO_3$) und 40 Gew.-% Natriumnitrit ($NaNO_2$) oder aus 53 Gew.-% Kaliumnitrat ($KNO_3$), 40 Gew.-% Natriumnitrit ($NaNO_2$) und 7 Gew.-% Natriumnitrat (Na-$NO_3$) bestehend, eingesetzt. Über den Reaktor betrachtet können Salzschmelze und Reaktionsgasgemisch sowohl im Gleichstrom als auch im Gegenstrom geführt werden. Die Salzschmelze selbst wird bevorzugt mäanderförmig um die Kontaktrohre geführt.

Strömt man die Kontaktrohre von oben nach unten an, ist es zweckmäßig, die Kontaktrohre von unten nach oben wie folgt zu beschicken:

- zunächst auf einer Länge von 50 bis 80 bzw. bis 70 % der Kontaktrohrlänge entweder nur Katalysator oder ein Gemisch aus Katalysator und Inertmaterial, wobei letzteres, bezogen auf die Mischung, einen Gewichtsanteil von bis zu 20 Gew.-% ausmacht (Abschnitt C);

- daran anschließend auf einer Länge von 20 bis 40 % der Gesamtrohrlänge entweder nur Katalysator, oder ein Gemisch aus Katalysator und Inertmaterial, wobei letzteres, bezogen auf die Mischung, einen Gewichtsanteil von bis zu 50 bzw. bis zu 40 Gew.-% ausmacht (Abschnitt B); und

- abschließend auf einer Länge von 5 bis 20 % der Gesamtrohrlänge eine Schüttung aus Inertmaterial (Abschnitt A), die vorzugsweise so gewählt wird, dass sie einen möglichst geringen Druckverlust bedingt.

Bevorzugt ist der Abschnitt C unverdünnt. Wie ganz allgemein bei der heterogen katalysierten Gasphasenpartialoxidation von Acrolein zu Acrylsäure (insbesondere bei hohen Acroleinbelastungen des Katalysatorbetts und hohen Wasserdampfgehalten des Beschickungsgasgemischs), kann der Abschnitt B auch aus zwei aufeinanderfolgenden Katalysatorverdünnungen bestehen (zum Zweck der Minimierung von Heißpunkttempatur und Heißpunkttemperatursensitivität). Von unten nach oben zunächst mit bis zu 20 Gew.-% Inertmaterial und daran anschließend mit > 20 Gew.-% bis 50 bzw. bis 40 Gew.-% Inertmaterial. Der Abschnitt C ist dann bevorzugt unverdünnt.

Für eine Anströmung der Kontaktrohre von unten nach oben, wird die Kontaktrohrbeschickung in zweckmäßigerweise umgekehrt.

Vorgenannte Beschickungsvariante ist insbesondere dann zweckmäßig, wenn als Katalysatoren solche gemäß Herstellungsbeispiel 5 der DE-A 10046928 oder solche gemäß DE-A 19815281 und als Inertmaterial Ringe aus Steatit mit der Geometrie 7 mm x 7 mm x 4 mm oder 7 mm x 7 mm x 3 mm (jeweils Außendurchmesser x Höhe x Innendurchmessser) verwendet werden. Hinsichtlich der Salzbadtemperatur gilt das in der DE-A 44 319 49 Gesagte. Sie wird in der Regel so gewählt, daß der erzielte Acroleinumsatz bei einfachem Durchgang normalerweise $\geq$ 90 mol-%, bzw. $\geq$ 95 mol-% oder $\geq$ 99 mol-% beträgt.

Die Durchführung des zweiten Schrittes der Partialoxidation, vom Acrolein zur Acrylsäure, kann mit den beschriebenen Katalysatoren aber auch z.B. in einem Zweizonen-Vielkontaktrohr-Festbettreaktor erfolgen, wie es die DE-19910508 beschreibt. Für den Acroleinumsatz gilt das oben Genannte. Auch im Fall der Durchführung dieses zweiten Schrittes in einem Zweizonen-Vielkontaktrohr-Festbettreaktor wird man zur Erzeugung des Beschickungsgasgemisches zweckmäßig unmittelbar das Produktgasgemisch einer auf den ersten Schritt gerichteten Partialoxidation (gegebenenfalls nach erfolgter Zwischenkühlung desselben) verwenden (wie sie vorstehend beschrieben wurde). Der für den zweiten Schritt der Partialoxidation benötigte Sauerstoff wird dabei bevorzugt als Luft zugesetzt und im zweiten Fall dem Produktgasgemisch des ersten Schritts der Partialoxidation unmittelbar zugegeben.

Bei einer zweistufigen Fahrweise mit unmittelbarer Weiterverwendung des Produktgasgemisches des ersten Schritts der Partialoxidation zur Beschickung des zweiten Schritts der Partialoxidation, wird man in der Regel zwei Einzonen-Vielkontaktrohr-Festbettreaktoren (bei hoher Reaktandenbelastung des Katalysatorbetts ist, wie ganz allgemein gültig, eine Gleichstromfahrweise zwischen Reaktionsgas und Salzbad (Wärmeträger) über den Rohrbündelreaktor betrachtet bevorzugt) der zwei Zweizonen-Vielkontaktrohr-Festbettreaktoren hintereinanderschalten. Eine gemischte Hintereinanderschaltung (Einzonen/Zweizonen oder umgekehrt) ist auch möglich.

Zwischen den Reaktoren kann sich ein Zwischenkühler befinden, der gegebenenfalls Inertschüttungen enthalten kann, die eine Filterfunktion ausüben können. Die Salzbadtemperatur von Vielkontaktrohrreaktoren für den ersten Schritt der Partialoxidation von Propylen zu Acrylsäure beträgt in der Regel 300 bis 400°C. Die Salzbadtemperatur von Vielkontaktrohrreaktoren für den zweiten Schritt der Partialoxidation von Propylen zu Acrylsäure, die Partialoxidation von Acrolein zu Acrylsäure, beträgt meist 200 bis 350°C. Außerdem werden die Wärmeaustauschmittel (bevorzugt Salzschmelzen) normalerweise in solchen Mengen durch die relevanten Vielkontaktrohr-Festbettreaktoren geführt, dass der Unterschied zwischen ihrer Eingangs- und ihrer Ausgangstemperatur in der Regel $\leq$ 5°C beträgt. Wie bereits erwähnt, können beide Schritte der Partialoxidation von Propylen zu Acrylsäure aber auch wie in der DE-A 10121592 beschrieben in einem Reaktor an einer Beschickung vollzogen werden.

Es sei auch nochmals erwähnt, daß ein Teil des Beschickungsgasgemisches für den ersten Schritt ("Propylen → Acrolein") aus der Partialoxidation kommendes Kreisgas (Restgas) sein kann.

Hierbei handelt es sich, wie bereits gesagt, um ein molekularen Sauerstoff enthaltendes Gas, das nach der Zielproduktabtrennung (Acrolein- und/oder Acrylsäureabtrennung) vom Produktgasgemisch der Partialoxidation verbleibt und teilweise als inertes Verdünnungsgas in die Beschickung für den ersten und/oder gegebenenfalls zweiten Schritt der Partialoxidation von Propylen zu Acrolein und/oder Acrylsäure rückgeführt werden kann.

Bevorzugt wird solches Propan, molekularen Sauerstoff und gegebenenfalls nicht umgesetztes Propylen enthaltendes Kreisgas (Restgas) jedoch ausschließlich auf erfindungsgemäße Weise in die Reaktionszone A des erfindungsgemäßen Verfahrens rückgeführt.

Es sei auch noch erwähnt, dass eine erfindungsgemäße Partialoxidation so durchgeführt werden kann, dass man zunächst ein Reaktionsgasgemisch über die Katalysatorbeschickung leitet, das keinen Sauerstoff enthält. In diesem Fall wird der für die Partialoxidation benötigte Sauerstoff als Gittersauerstoff zur Verfügung gestellt. In einem nachfolgenden Regenerierschritt mit einem Sauerstoff enthaltenden Gas (z.B. Luft, mit Sauerstoff angereicherte Luft oder an Sauerstoff entreicherte Luft) wird das Katalysatorbett regeneriert, um nachfolgend wiederum für ein an Sauerstoff freies Reaktionsgasgemisch zur Verfügung zu stehen usw..

Zusammenfassend bildet nochmals ein Rohrbündelreaktor innerhalb dessen sich die Katalysatorbeschickung längs der einzelnen Kontaktrohre mit Beendigung des ersten Reaktionsschrittes entsprechend ändert (derartige als Reaktionszone B erfindungsgemäß geeignete Propylenpartialoxidationen lehren z.B. die EP-A 911313, die EP-A 979813, die EP-A 990636 und die DE-A 2830765) die einfachste Realisierungsform zweier Oxidationszonen für die beiden Schritte der Partialoxidation von Propylen zu Acrylsäure. Gegebenenfalls wird dabei die Beschickung der Kontaktrohre mit Katalysator durch eine Inertschüttung unterbrochen.

Vorzugsweise werden die beiden Oxidationszonen jedoch in Form zweier hintereinander geschalteter Rohrbündelsysteme realisiert. Diese können sich in einem Reaktor befinden, wobei der Übergang von einem Rohrbündel zum anderen Rohrbündel von einer nicht im Kontaktrohr untergebrachten (zweckmäßigerweise begehbaren) Schüttung aus Inertmaterial gebildet werden. Während die Kontaktrohre in der Regel von einem Wärmeträger umspült werden, erreicht dieser eine wie vorstehend beschrieben angebrachte Inertschüttung nicht. Mit Vorteil werden daher die beiden Kontaktrohrbündel in räumlich voneinander getrennten Reaktoren untergebracht. In der Regel befindet sich dabei zwischen den beiden Rohrbündelreaktoren ein Zwischenkühler, um eine gegebenenfalls erfolgende Acroleinnachverbrennung im Produktgasgemisch, das die erste Oxidationszone verlässt, zu mindern. Anstelle von Rohrbündelreaktoren können auch Plattenwärmetauscherreaktoren mit Salz- und/oder Siedekühlung, wie sie z.B. die DE-A 19 929 487 und die DE-A 19 952 964 beschreiben, eingesetzt werden.

Die Reaktionstemperatur in der ersten Oxidationszone liegt in der Regel bei 300 bis 450°C, bevorzugt bei 320 bis 390°C. Die Reaktionstemperatur in der zweiten Oxidationszone liegt in der Regel bei 200 bis 370°C, häufig bei 220 bis 330°C. Der Reaktionsdruck beträgt in beiden Oxidationszonen zweckmäßig 0,5 bis 5, vorteilhaft 1 bis 3 bar. Die Belastung (NI/l·h) der Oxidationskatalysatoren mit Reaktionsgas beträgt in beiden Oxidationszonen häufig 1500 bis 2500 h-1 bzw. bis 4000 h-1. Die Belastung mit Popylen kann dabei bei 100 bis 200 oder 300 und mehr NI/l·h liegen.

Prinzipiell können die beiden Oxidationszonen beim erfindungsgemäßen Verfahren so gestaltet werden, wie es z.B. in der DE-A 19 837 517, der DE-A 19 910 506, der DE-A 19 910 508 sowie der DE-A 19 837 519 beschrieben ist. Üblicherweise wird die externe Temperierung in den beiden Oxidationszonen, gegebenenfalls in Mehrzonenreaktorsystemen, in an sich bekannter Weise an die spezielle Reaktionsgasgemischzusammensetzung sowie Katalysatorbeschickung angepasst.

Der für die erfindungsgemäß erforderliche Reaktionszone B als Oxidationsmittel insgesamt benötigte molekulare Sauerstoff kann im Fall einer zweistufigen Propylenpartialoxidation zu Acrylsäure dem Beschickungsgasgemisch der Propylenoxidationsstufe in seiner Gesamtmenge vorab zugegeben werden. Selbstverständlich kann aber auch nach der Propylenoxidationsstufe mit Sauerstoff ergänzt werden. Letzteres ist bei der Acrylsäureherstellung bevorzugt.

Vorzugsweise wird dabei in der ersten Oxidationszone (Propylen → Acrolein) ein molares Verhältnis Propylen : molekularer Sauerstoff von 1 : 1 bis 3, häufig 1 : 1,5 bis 2 eingestellt. Ähnliche numerische Werte eignen sich für das molare Verhältnis Acrolein: molekularer Sauerstoff in der zweiten Oxidationszone (1 : 0,5 bis 1,5 wäre bevorzugt) für die Partialoxidation von Acrolein zu Acrylsäure.

In beiden Oxidationszonen wirkt sich dabei ein Überschuss an molekularem Sauerstoff in der Regel vorteilhaft auf die Kinetik der Gasphasenoxidation aus. Im Unterschied zu den Verhältnissen in der Reaktionszone A des erfindungsgemäßen Verfahrens werden die thermodynamischen Verhältnisse in der Reaktionszone B durch das molare Reaktandenverhältnis im wesentlichen nicht beeinflusst, da die heterogen katalysierte Gasphasen-Partialoxidation des Propylens zu Acrylsäure kinetischer Kontrolle unterliegt. Prinzipiell kann daher z.B. in der ersten Oxidationszone auch das Propylen gegenüber dem molekularen Sauerstoff im molaren Überschuss vorgelegt werden. In diesem Fall kommt dem überschüssigen Propylen faktisch die Rolle eines Verdünnungsgases zu.

Prinzipiell ist die heterogen katalysierte Gasphasen-Partialoxidation von Propylen zu Acrylsäure aber auch in einer einzigen Oxidationszone realisierbar. In diesem Fall erfolgen beide Reaktionsschritte in einem Oxidationsreaktor der mit einem Katalysator beschickt ist, der die Umsetzung beider Reaktionsschritte zu katalysieren vermag. Selbstredend kann sich auch die Katalysatorbeschickung innerhalb der Oxidationszone längs der Reaktionskoordinate kontinuierlich oder abrupt ändern. Natürlich kann bei einer Ausführungsform der erfindungsgemäß anzuwendenden wenigstens einen Partialoxidation in Gestalt zweier hintereinandergeschalteter Oxidationszonen aus dem das die erste Oxidationszone verlassenden Produktgasgemisch in selbigem enthaltenes, in der ersten Oxidationszone als Nebenprodukt entstandenes, Kohlenoxid und Wasserdampf bei Bedarf vor der Weiterleitung in die zweite Oxidationszone teilweise oder vollständig abgetrennt werden. Vorzugsweise wird man erfindungsgemäß eine Verfahrensweise wählen, die solch einer Abtrennung nicht bedarf.

Als Quelle für den in der wenigstens einen Partialoxidation benötigten molekularen Sauerstoff, der z.B dem Produktgasgemisch A' vor dessen Verwendung zur Beschickung der Reaktionszone B zugemischt wird, kommen sowohl reiner molekularer Sauerstoff als auch mit Inertgas wie $CO_2$, CO, Edelgasen, $N_2$ und/oder gesättigten Kohlenwasserstoffen verdünnter molekularer Sauerstoff oder Stickoxid in Betracht.

In zweckmäßiger Weise wird man wenigstens zur Deckung eines Teilbedarfs an molekularem Sauerstoff Luft als Sauerstoffquelle verwenden.

Durch Zudosieren von kalter Luft zu heißem Produktgasgemisch A' kann im Rahmen des erfindungsgemäßen Verfahrens auch auf direktem Weg eine Abkühlung desselben bewirkt werden.

Im Langzeitbetrieb beider Reaktionszonen A, B wird normalerweise eine Minderung der Katalysatorqualität (insbesondere Aktivität und Selektivität) der in beiden Reaktionszonen angewandten Katalysatoren einhergehen. Dem kann einerseits dadurch entgegengewirkt werden, dass man das jeweilige Katalysatorbett von Zeit zu Zeit regeneriert (dazu kann in den Oxidationszonen z.B. auch Restgas bei erhöhter Temperatur über das jeweilige Katalysatorbett der Oxidationskatalysatoren geführt werden), wie es z.B. in den Schriften DE-A 10351269, DE-A 10350812 und DE-A 10350822 beschrieben ist. Auch kann die Reaktionstemperatur über die Betriebsdauer erhöht werden, um eine geminderte Katalysatoraktivität auszugleichen. In überraschender Weise hat es sich jedoch in beiden Reaktionszonen A, B als vorteilhaft erwiesen, den jeweiligen Arbeitsdruck in der Gasphase, bezogen auf eine identische Belastung des jeweiligen Katalysatorbetts mit Reaktionsgasgemisch in Nl/l·h, während der Betriebsdauer des Katalysatorbetts zu erhöhen, um der Deaktivierung der Katalysatorbetten weiter entgegenzuwirken, wie es z.B. in der DE-A 102004025445 beschrieben ist. Zu diesem Zweck kann beispielsweise hinter jeder der beim erfindungsgemäßen Verfahren anzuwendenden Reaktions- und Trennzonen eine Druckregelvorrichtung (im einfachsten Fall eine Drosselvorrichtung, z.B. ein Drosselventil oder auch ein Drallregler) angebracht werden (z.B. auch partielle durchlässige Lochblenden, deren Löcher sukzessive teilweise oder vollständig verschlossen werden können. Selbstverständlich kann sich aber auch nur hinter einer der genannten Zonen eine solche Druckregelvorrichtung befinden. D.h., prinzipiell ist es völlig ausreichend, die Druckregelvorrichtung irgendwo in den weiteren Strömungsweg des Reaktionsgases einzubringen, von wo aus sich die Druckerhöhung durch Rückstau in die Reaktionszonen fortpflanzen kann. Typische vorgenannte Druckerhöhungen (die kontinuierlich gemäß erfolgender Deaktivierung, aber auch diskontinuierlich vorgenommen werden können) können im Verlauf der Betriebsdauer bis zu 3000 mbar und mehr betragen.

Das die erfindungsgemäß anzuwendende Reaktionszone B verlassende Produktgasgemisch B ist im Fall einer Herstellung von Acrolein und/oder Acrylsäure in der Regel im wesentlichen zusammengesetzt aus dem Zielprodukt Acrolein oder Acrylsäure oder dessen Gemisch mit Acrolein, nicht umgesetztem molekularem Sauerstoff, Propan, nicht umgesetztem Propylen, molekularem Stickstoff, als Nebenprodukt entstandenem und/oder als Verdünnungsgas mitverwendetem Wasserdampf, als Nebenprodukt und/oder als Verdünnungsgas mitverwendeten Kohlenoxiden, sowie geringen Mengen sonstiger niederer Aldehyde, niederer Alkancarbonsäuren (z.B. Essigsäure, Ameisensäure und Propionsäure) sowie Maleinsäureanhydrid, Benzaldehyd, aromatische Carbonsäuren und aromatische Carbonsäureanhydride (z.B. Phthalsäureanhydrid und Benzoesäure), gegebenenfalls weiteren Kohlenwasserstoffen, wie z.B. C4-Kohlenwasserstoffe (z.B. Buten-1 und eventuell sonstige Butene), und anderer inerter Verdünnungsgase.

Das Zielprodukt kann aus dem Produktgasgemisch B in an sich bekannter Weise in einer Trennzone B abgetrennt werden (z.B. durch partielle oder vollständige sowie gegebenenfalls fraktionierende Kondensation der Acrylsäure oder durch Absorption von Acrylsäure in Wasser oder in einem hochsiedenden hydrophoben organischen Lösungsmittel oder durch Absorption von Acrolein in Wasser oder in wässrigen Lösungen niederer Carbonsäuren sowie anschließende Aufarbeitung der Kondensate und/oder Absorbate; erfindungsgemäß bevorzugt wird das Produktgasgemisch B fraktionierend kondensiert werden; vgl. z.B. EP-A 1388533, EP-A 1388532, DE-A 10235847, EP-A 792867, WO 98/01415, EP-A 1015411, EP-A 1015410, WO 99/50219, WO 00/53560, WO 02/09839, DE-A 10235847, WO 03/041833, DE-A 10223058, DE-A 10243625, DE-A 10336386, EP-A 854129, US-A 4317926, DE-A 19837520, DE-A 19606877, DE-A 190501325, DE-A 10247240, DE-A 19740253, EP-A 695736, EP-A 982287, EP-A 1041062, EP-A 117146, DE-A 4308087, DE-A 4335172, DE-A 4436243, DE-A 19 924 532, DE-A 10332758 sowie DE-A 19 924 533). Eine Acrylsäureabtrennung kann auch wie in der EP-A 982287, der EP-A 982289, DE-A 10336386, der DE-A 10115277, der DE-A 19606877, der DE-A 19740252, der DE-A 19627847, der DE-A 10053086 und der EP-A 982288 vorgenommen werden. Vorzugsweise wird wie in Fig. 7 der WO/0196271 bzw. in den Ausführungsbeispielen der vorliegenden Anmeldung abgetrennt.

Gemeinsames Merkmal der vorgenannten Trennverfahren ist (wie eingangs bereits erwähnt), dass am Kopf der jeweiligen trennwirksame Einbauten enthaltenden Trennkolonne, in deren unteren Teil das Produktgemisch B, normalerweise nach vorheriger direkter und/oder indirekter Kühlung desselben, zugeführt wird, normalerweise ein Restgasstrom (Hauptrestgas) verbleibt, der im wesentlichen diejenigen Bestandteile des Produktgasgemischs B enthält, deren Siedepunkt bei Normaldruck (1 bar) $\leq$ -30°C beträgt (d.h., die schwer kondensierbaren oder auch leicht flüchtigen Bestandteile).

Im unteren Teil der Trennkolonne fallen normalerweise die schwerer flüchtigen Bestandteile des Produktgasgemischs B, einschließlich des jeweiligen Zielprodukts, in kondensierter Phase an.

Die Restgasbestandteile sind in erster Linie Propan, gegebenenfalls in der Reaktionszone B nicht umgesetztes Propylen, molekularer Sauerstoff sowie häufig die in den Reaktionszonen A, B mitverwendeten inerten Verdünnungsgase, vorzugsweise insbesondere Stickstoff und Kohlendioxid. Wasserdampf kann je nach angewandtem Trennverfahren im Restgas nur noch in Spuren oder in Mengen von bis zu 20 Vol.-% oder mehr enthalten sein.

Von diesem (Haupt)Restgas wird erfindungsgemäß wenigstens eine (vorzugsweise (Haupt)Restgaszusammensetzung aufweisende) Propan, molekularen Sauerstoff und gegebenenfalls (in der Reaktionszone B) nicht umgesetztes Propylen enthaltende Teilmenge (vorzugsweise die Gesamtmenge, gegebenenfalls aber auch nur die Hälfte, oder zwei Drittel, oder drei Viertel dieser Gesamtmenge) als ein Propan enthaltender Zufuhrstrom in die Reaktionszone A rückgeführt. (Haupt)Restgasteilmengen können aber auch, wie bereits beschrieben, in die Reaktionszone B rückgeführt und/oder zum Zweck der Energieerzeugung verbrannt werden.

Bei der Aufarbeitung der kondensierten Phase (zum Zweck der Abtrennung des Zielproduktes) können erfindungsgemäß (wie bereits erwähnt) weitere restliche Gase anfallen, da normalerweise versucht wird, die insgesamt im Produktgasgemisch A enthaltene Menge an nicht umgesetztem Propan in die Reaktionszone A zurückzuführen und im Rahmen der Zielabtrennung wiederzugewinnen. Diese enthalten in der Regel zwar noch Propan sowie gegebenenfalls Propylen, häufig jedoch keinen molekularen Sauerstoff mehr (enthalten sie noch molekularen Sauerstoff werden sie in dieser Schrift als Nebenrestgase bezeichnet). Sie können daher gegebenenfalls mit dem Hauptrestgas zu einem Gesamtrestgas vereint in die erfindungsgemäße Reaktionszone A rückgeführt werden. Es ist aber auch eine separate Verwertung solcher weiterer restliche Gase möglich. Beispielsweise können sie, soweit sie an molekularem Sauerstoff frei sind, in der Regel nach Verdichtung, auch als Bestandteil des Reaktionsgasausgangsgemischs der Reaktionszone A in die Reaktionszone A rückgeführt werden. Auch kann ihre Rückführung in die Reaktionszone A an anderer Stelle erfolgen, wenn sie an molekularem Sauerstoff im wesentlichen frei sind.

Durch die vorzugsweise vollständige Rückführung des (Haupt- und/oder Neben- bzw. Gesamt-)Restgases aus der Trennzone B, die eine Verschaltung von mehreren Trennkolonnen sowie gegebenenfalls weiterer von Kolonnen verschiedener Trennvorrichtung umfassen kann, in die Reaktionszone A, erfolgt bei kontinuierlicher Ausführung des erfindungsgemäßen Verfahrens so eine kontinuierliche Umsetzung von Propan zu Acrylsäure und/oder Acrolein.

Erfindungsgemäß wesentlich ist dabei, dass durch die erfindungsgemäße Rückführung des (Haupt- und/oder Neben- bzw. Gesamt-)Restgases aus der Trennzone B in die Reaktionszone A in letzterer eine Überführung von Propan zu Propylen mit nahezu hundertprozentiger Selektivität erreichbar ist.

Die Vorteilhaftigkeit der erfindungsgemäßen Verfahrensweise ist dabei sowohl bei niederen (≤ 30 mol-%) als auch bei hohen (≥ 30 mol-%) Dehydrierumsätzen (bezogen auf einmaligen Durchgang des Reaktionsgasgemischs durch die Reaktionszone A) gegeben. Dies rührt im wesentlichen daher, dass mit hohen Dehydrierumsätzen in der Reaktionszone A in der Regel hohe Propylenanteile im Produktgasgemisch A und daraus resultierend auch erhöhte Propylenströme im Beschickungsgasgemisch der Reaktionszone B einhergehen. Letzteres führt normalerweise zu erhöhten Restsauerstoffströmen im (Haupt)restgas, die wiederum dazu geeignet sind, die mit den erhöhten Dehydrierumsätzen in der Reaktionszone A einhergehenden erhöhten Wasserstoffmengen zu verbrennen. D.h., in vorteilhafter Weise beinhaltet des erfindungsgemäße Verfahren einen sich so selbst optimierenden Regelmechanismus. Mit höheren Propanumsätzen sind allerdings geringere Gesamtgasbelastungen der Katalysatorbeschickung in der Reaktionszone B möglich. Generell ist es erfindungsgemäß günstig, wenn der Wasserstoffstrom im Reaktionsgasgemisch der Reaktionszone A an der Zufuhrstelle für das in die Reaktionszone A rückzuführende Restgas in einem wenigstens stöchiometrischen Verhältnis zum Sauerstoffstrom im rückgeführten Restgas steht.

Das erfindungsgemäße Verfahren ist in entsprechender Weise anwendbar, wenn in der Reaktionszone B eine partielle Ammoxidation von Propen zu Acrylnitril durchgeführt wird. Es ist auch dann entsprechend anwendbar, wenn in der Reaktionszone A das Propan durch iso-Butan ersetzt wird und das dabei resultierende iso-Buten in entsprechender Weise in der Reaktionszone B zu Methacrolein und/oder Methacrylsäure partialoxidiert wird.

Vergleichsbeispiele und Beispiele

Vergleichsbeispiel 1

A) Gestaltung der Reaktionszone A (der Dehydrierstufe)

Die Dehydrierstufe bestand aus drei hintereinander geschalteten identischen Rohrreaktoren, die in identischer Weise mit Dehydrierkatalysator beschickt waren.

Der einzelne Rohrreaktor war ein Stahlrohr (Edelstahl der DIN Werkstoffnummer 1.4841) der Länge 1300 mm, der Wanddicke 3,6 mm und des Innendurchmessers 53,1 mm. Die Rohrreaktoren wurden vom Reaktionsgasgemisch jeweils von oben nach unten durchströmt.

Am unteren Ende jedes Rohrreaktors befand sich ein Tragerost aus demselben Edelstahl. Auf dem Tragerost befand sich von unten nach oben die folgende Beschickung:

175 mm    Schüttlänge aus Steatitkugeln (Durchmesser 4-5 mm) des Steatits C-220 der Fa. CeramTec;

| 21 mm | Schüttlänge aus Steatitkugeln (Durchmesser 1,5-2,5 mm) des Steatits C-220 der Fa. CeramTec; |
|---|---|

210 mm    Schüttlänge Dehydrierkatalysator (Pt/Sn-Legierung, die mit den Elementen Cs, K und La in oxidischer Form promoviert war und die auf die äußere und innere Oberfläche von $ZrO_2 \cdot SiO_2$ Mischoxidträgerstränglingen (mittlere Länge (gaußverteilt im Bereich von 3 mm bis 12 mm mit Maximum bei ca. 6 mm): 6 mm, Durchmesser: 2 mm) in der Elementstöchiometrie (Massenverhältnisse einschließlich Träger) $Pt_{0,3}Sn_{0,6}La_{3,0}Cs_{0,5}K_{0,2}$ $(ZrO_2)_{88,3}(SiO_2)_{7,1}$ aufgebracht war (Katalysatorvorläuferherstellung und Aktivierung zum aktiven Katalysator wie in Beispiel 4 der DE-A 10 219 879).

21 mm    Schüttlänge Steatitkugeln (Durchmesser 1,5-2,5 mm) des Steatits C-220 der Fa. CeramTec; und

abschließend auf der Restlänge des Rohrreaktors wieder eine Schüttung aus Steatitkugeln (Durchmesser 4-5 mm) des Steatits C-220 der Fa. CeramTec.

Jeder der Rohrreaktoren war außen im Sinne einer Vorheizstrecke auf den ersten 500 mm Rohrlänge von oben nach unten (zum Tragerost hin) in zwei, eine Gleichverteilung der zugeführten Wärmemenge gewährleistenden, Halbschalen aus Kupfer (Schalendicke = 200 mm) eingelegt, die mittels einer sie im vollen Umfang umgebenden Heizmanschette (Fa. Horst, DE-Heidelberg, 500 mm Länge, 100 mm Innendurchmesser) elektrisch beheizt wurden.

Von unten nach oben war jeder der Rohrreaktoren im Sinne einer adiabaten Strecke auf einer Länge von 600 mm in jeweils zwei Paare von thermisch isolierend wirkenden Halbschalen (Dicke einer Halbschale = 25 mm) aus MPS-Super G der Fa. Microtherm in DE, die um 90° gegeneinander versetzt übereinander angebracht waren, eingebracht. Die isolierend wirkenden Halbschalen waren ihrerseits von einer zylindrischen Einhüllenden aus Edelstahl (Außendurchmesser = 173 mm, Innendurchmesser = 167 mm) umgeben, an die zum Zweck der Begleitheizung eine Heizmanschette (Länge = 675 mm, Innendurchmesser = 173 mm) der Fa. Horst, DE-Heidelberg, angelegt war. Auf diese Weise konnte auf der adiabaten Strecke der Wärmefluss aus der Umgebung in das Reaktionsrohr hinein und aus dem Reaktionsrohr heraus in die Umgebung minimiert werden.

In jedes Reaktionsrohr war mittig zusätzlich eine 1370 mm lange Thermohülse eingeführt (Außendurchmesser: 6mm, Innendurchmesser: 4mm), in die ein Mehrfach-Thermoelement (vom unteren Reaktorende nach oben alle 4 cm insgesamt 10 Messstellen, Dicke 3,2mm) eingeführt war.

Vor jeden einzelnen Rohrreaktor war ein mit Steatitringen (aus Steatit C-220 der Fa. CeramTec und von der Geometrie 7 mm x 3 mm x 3 mm = Außendurchmesser x Innendurchmesser x Höhe) gefülltes Stahlrohr der Länge 1300 mm als Erhitzer geschaltet. In ihm wurde das Reaktionsgasgemisch jeweils auf die Eintrittstemperatur des nachfolgenden Rohreaktors vorgeheizt und gleichzeitig ideal durchmischt. Zu diesem Zweck wurden die Erhitzerrohre (Edelstahl der DIN Werkstoffnummer 1.4841, Wanddicke 3,6 mm, Innendurchmessers 53,1 mm) auf einer rohrmittigen Länge von 1200 mm mittels um sie angelegter Heizmanschetten der Fa. Horst, DE-Heidelberg elektrisch beheizt. Die Verbindung zwischen Erhitzer und Rohrreaktoren wurde durch mit üblichen Wärmedämmmaterialien thermisch isolierten Edelstahlrohren (Edelstahl der DIN Werkstoffnummer 1.4841, Außendurchmesser 21.3 mm, Innendurchmesser 16.1 mm, Länge ca. 700 mm) bewerkstelligt.

Vor dem Eintritt des Reaktionsgasgemischs in den jeweiligen Erhitzer war ein Zufuhrhahn angebracht, über den dem Reaktionsgasgemisch jeweils Druckluft zugeführt werden konnte. Beschrieben wird nachfolgend der stationäre Betrieb.

Dem ersten Dehydrierreaktor wurde ein Reaktionsgasausgangsgemisch aus 395 g/h Roh-Propan (erster Propan enthaltender Zufuhrstrom), 500 g/h Wasser und 3649 g/h (Gesamt)Restgas als Kreisgas (zweiter Propan enthaltender Zufuhrstrom) mit einer Temperatur von 400°C und einem Druck von 2,8 bar zugeführt.

Das Roh-Propan enthielt:

|  | Vol.-% |
|---|---|
| Methan | 0 |
| Ethan | 0,31 |
| Ethen | 0,004 |
| Propan | 96,29 |
| Propen (Propylen) | 0,05 |
| $H_2$ | 0 |
| $O_2$ | 0 |
| $N_2$ | 0,94 |

(fortgesetzt)

|  | Vol.-% |
|---|---|
| CO | 0 |
| $CO_2$ | 0 |
| iso-Butan | 1,84 |
| n-Butan | 0,56 |
| trans-Buten | 0,0003 |
| iso-Buten | 0 |
| cis-Buten | 0,0014 |
| 1-Buten | 0,004 |
| Butadien | 0 |

Das (Gesamt)Restgas (Kreisgas) enthielt:

|  | Vol.-% |
|---|---|
| Methan | 0,002 |
| Ethan | 0,083 |
| Ethen | 0,021 |
| Propan | 26,56 |
| Propen | 0,129 |
| $H_2$ | 0,033 |
| $O_2$ | 3,01 |
| $N_2$ | 67,38 |
| CO | 0,47 |
| $CO_2$ | 1,92 |
| iso-Butan | 0,31 |
| n-Butan | 0,065 |
| trans-Buten | 0 |
| iso-Buten | 0,0004 |
| cis-Buten | 0,0014 |
| 1-Buten | 0,0003 |
| Butadien | 0 |

Die Bestimmung der Zusammensetzung des Roh-Propans und aller anderen Gaszusammensetzungen erfolgte gaschromatographisch [HP 6890 mit Chem-Station, Detektoren: FID, WLD, Trennsäulen: $Al_2O_3$/KCL (Chrompack), Carboxen 1010 (Supelco)]. Bei Wasserdampf enthaltenden Gasgemischen wurde dieser vorab der gaschromatographischen Analyse durch Abkühlen und gegebenenfalls Entspannen in einem Wasserabscheider auskondensiert. Das nicht kondensierte Gas wurde analysiert und auf dieses trocken gerechnete Gas (d.h., die im eigentlich zu analysierenden Gasgemisch enthaltene Wasserdampfmenge blieb unberücksichtigt) beziehen sich alle Angaben.

Das Reaktionsgasausgangsgemisch wurde in einem Verdampfer erzeugt, der dem ersten Erhitzer vorgeschaltet war. Der Verdampfer selbst war ebenfalls wie ein Erhitzer ausgeführt. Ihm wurden 395 g/h gasförmiges Roh-Propan (65°C, 5 bar), 3649 g/h (Gesamt)Restgas (Kreisgas) (50°C, 2,8 bar) und 500 g/h Wasser (20°C, 3 bar) zugeführt. Die Beheizung des Verdampfers war auf eine Gasgemischaustrittstemperatur von 200°C geregelt. Der Verdampfer war mit dem ersten Erhitzer in entsprechender Weise verbunden wie die Reaktoren an die Erhitzer angebunden waren.

Die Beheizung des ersten Erhitzers war so geregelt, dass das vom Verdampfer in den ersten Erhitzer geleitete Gasgemisch den ersten Erhitzer mit einer Temperatur von 400°C verließ (die dazu erforderliche Wandtemperatur betrug ca. 440°C). Dann wurde das Reaktionsgasausgangsgemisch in den ersten Rohrreaktor geführt und in der Vorheizstrecke desselben auf eine Reaktionszoneneintrittstemperatur von 423,3°C weiter erhitzt.

Die Temperatur des Reaktionsgasgemischs durchlief beim Durchgang durch den ersten Rohrreaktor ein Maximum (Heißpunkttemperatur genannt) von 549,1°C (die hier angegebenen quantitativen Angaben beziehen sich auf den Betriebszustand nach 200 Betriebsstunden; im weiteren Verlauf des Betriebs wurden die verschiedenen Temperaturen so nachgezogen, dass der auf Einmaldurchgang bezogene Umsatz und die Raum-Zeit-Ausbeute im wesentlichen konstant blieben; in entsprechender Weise wurde auch bereits in den ersten 200 Betriebsstunden verfahren), das im Verlauf des kontinuierlichen Betriebs der Versuchsanlage infolge allmählicher Katalysatordeaktivierung in Strömungsrichtung wanderte (die Wanderungsgeschwindigkeit betrug ca. 0,03 mm/h).

Das den ersten Dehydrierreaktor verlassende Reaktionsgasgemisch wies folgende Gehalte auf:

|  | Vol.-% |
| --- | --- |
| Methan | 0,010 |
| Ethan | 0,103 |
| Ethen | 0,023 |
| Propan | 27,2 |
| Propen | 3,05 |
| $H_2$ | 3,3 |
| $O_2$ | 0 |
| $N_2$ | 62,2 |
| CO | 0,053 |
| $CO_2$ | 3,36 |
| iso-Butan | 0,34 |
| n-Butan | 0,077 |
| trans-Buten | 0,050 |
| iso-Buten | 0,001 |
| cis-Buten | 0,004 |
| 1-Buten | 0,0004 |
| Butadien | 0,0015 |

Seine Temperatur betrug 509,1 °C und sein Druck lag bei 2,55 bar.

Vor dem Eintritt in den nachfolgenden Erhitzer wurden dem Reaktionsgasgemisch 130 Nl/h Druckluft zudosiert (23°C, 4,2 bar).

Dann wurde das Reaktionsgasgemisch mittels der elektrischen Beheizungsmöglichkeiten des Erhitzers (Wandtemperatur ca. 540°C) und der Vorheizstrecke des nachfolgenden (zweiten) Reaktionsrohres (Wandtemperatur ca. 560°C) auf 477°C (Eintritt 2. Reaktionszone) erwärmt. Der Druck des Reaktionsgasgemischs an dieser Stelle lag bei 2,55 bar.

Beim Durchgang durch den zweiten Rohrreaktor durchlief die Temperatur des Reaktionsgasgemischs ein Maximum von 515,5°C, das im Verlauf des kontinuierlichen Betriebs der Versuchsanlage infolge allmählicher Katalysatordeaktivierung in Strömungsrichtung wanderte (die Wanderungsgeschwindigkeit betrug ca. 0,1 mm/h). Das den zweiten Dehydrierreaktor verlassende Reaktionsgasgemisch wies folgende Gehalte auf:

|  | Vol % |
| --- | --- |
| Methan | 0,015 |
| Ethan | 0,11 |

(fortgesetzt)

|  | Vol % |
|---|---|
| Ethen | 0,023 |
| Propan | 24,0 |
| Propen | 4,78 |
| $H_2$ | 3,40 |
| $O_2$ | 0 |
| $N_2$ | 63,84 |
| CO | 0,068 |
| $CO_2$ | 3,29 |
| iso-Butan | 0,28 |
| n-Butan | 0,062 |
| trans-Buten | 0,084 |
| iso-Buten | 0,001 |
| cis-Buten | 0,007 |
| 1-Buten | 0,0003 |
| Butadien | 0,0016 |

Seine Temperatur betrug 493,8°C und sein Druck lag bei 2,5 bar.

Vor dem Eintritt in den nachfolgenden Erhitzer wurden dem Reaktionsgasgemisch 195 Nl/h Druckluft zudosiert (23°C, 4,2 bar).

Dann wurde das Reaktionsgasgemisch mittels der elektrischen Beheizungsmöglichkeiten des Erhitzers (Wandtemperatur ca.480°C) und der Vorheizstrecke des nachfolgenden (dritten) Reaktionsrohres (Wandtemperatur ca. 480°C) auf 464°C (Eintritt 3. Reaktionszone) erwärmt. Der Druck des Reaktionsgasgemischs an dieser Stelle lag bei 2,5 bar.

Beim Durchgang durch den dritten Rohrreaktor durchlief die Temperatur des Reaktionsgasgemischs ein Maximum von 492,8°C, das im Verlauf des kontinuierlichen Betriebs der Versuchsanlage infolge allmählicher Katalysatordeaktivierung in Strömungsrichtung wanderte (die Wanderungsgeschwindigkeit betrug ca. 0,1 mm/h). Das den dritten Dehydrierreaktor verlassende Reaktionsgasgemisch wies folgende Gehalte auf:

|  | Vol % |
|---|---|
| Methan | 0,020 |
| Ethan | 0,11 |
| Ethen | 0,027 |
| Propan | 22,76 |
| Propen | 5,33 |
| $H_2$ | 2,48 |
| $O_2$ | 0 |
| $N_2$ | 65,5 |
| CO | 0,042 |
| $CO_2$ | 3,25 |
| iso-Butan | 0,26 |
| n-Butan | 0,056 |
| trans-Buten | 0,093 |

(fortgesetzt)

|  | Vol % |
|---|---|
| iso-Buten | 0,001 |
| cis-Buten | 0,008 |
| 1-Buten | 0,0003 |
| Butadien | 0,002 |

Seine Temperatur betrug 480,4°C und sein Druck lag bei 2,45 bar.

Damit ergab sich über die Dehydrierstufe ein auf den Einmaldurchgang des Reaktionsgasausgangsgemischs bezogener Gesamtdehydrierumsatz des Propans von 19,83 mol-%.

Bei fortgeschrittener Deaktivierung der Dehydrierkatalysatorschüttungen wurde das Verfahren unterbrochen und diese wie in der DE-A 10028582 beschrieben regeneriert. Dies war im wesentlichen immer dann der Fall, wenn die Heißpunkttemperatur in allen drei Rohrreaktoren ca. 580°C betrug.

In überraschender Weise schritt die Deaktivierung der Dehydrierkatalysatorschüttungen bei erhöhtem Arbeitsdruck langsamer voran.

B) Gestaltung der Trennzone A

Durch Direktkühlung mit versprühtem gekühltem Wasser (T = 20°C) wurde das den dritten Dehydrierreaktor verlassende Produktgasgemisch (Produktgasgemisch A) in einem Direktkühler (Quench) im Gleichstrom auf 40°C abgekühlt (das dabei gasförmig verbleibende Gemisch wurde in der Gegenrichtung zur Produktgaseinströmrichtung aus dem Wasserquench herausgeführt). Etwa 75 Gew.-% des im Produktgasgemisch enthaltenen Wasserdampf (Wasserdampf wurde dem Reaktionsgasausgangsgemisch der Reaktionszone A zugesetzt und bildete sich in der Dehydrierstufe durch Verbrennung von Wasserstoff sowie möglicherweise Kohlenwasserstoff mit Luftsauerstoff; die Verbrennungswärme hielt die Reaktionstemperatur im Reaktionsgasgemisch der Reaktionszone A weitgehend aufrecht) kondensierte bei der Direktkühlung aus. Das sich dabei bildende Kondensat wurde mittels Standregelung aus dem Wasserquench heraus- und seiner Entsorgung zugeführt. Im übrigen wurde das zur Direktkühlung eingesetzte Wasser im Kreis geführt (gepumpt), durch indirekten Wärmeaustausch rückgekühlt und zum Zweck der Direktkühlung wieder versprüht.

Anstelle der beschriebenen Direktkühlung mit Wasser, kann das Produktgasgemisch aus der Dehydrierstufe auch zunächst dadurch abgekühlt werden, dass man mit dem Produktgasgemisch in einem indirekten Wärmeaustauscher (z.B. in einem Rohrbündelwärmeaustauscher im Gleichstrom oder im Gegenstrom) das der Dehydrierstufe zuzuführende Reaktionsgasausgangsgemisch aufwärmt (auf 400°C). Das Produktgasgemisch der Dehydrierstufe kühlt dabei von etwa 500°C auf ca. 200 bis 300°C ab.

Eine weitere Abkühlung des Produktgasgemisches der Dehydrierstufe kann dadurch erfolgen, dass es in einem indirekten Wärmeaustauscher dazu verwendet wird, das Ausgangsgasgemisch für die nachfolgend noch zu beschreibende Partialoxidation des in der Dehydrierstufe erzeugten Propens aufzuwärmen, und/oder dass es dazu verwendet wird, die im weiteren noch zu beschreibende Absorberabgaskühlung beim, vorzugsweise zweistufigen, Expandieren desselben mittels Expansionsturbinen auszugleichen, oder durch Vorabanwärmung zu kompensieren.

Danach befindet sich das Produktgasgemisch bei einer Temperatur von etwa 180°C. Anschließend kann mittels Luft- und/oder Oberflächenwasserkühler auf eine Temperatur im Bereich von 30°C bis 60°C abgekühlt werden.

In die Kühler integrierte oder diesen nachgeschaltete Tropfenabscheider sammeln das beim Abkühlen auskondensierte Wasser und führen es standgeregelt der Entsorgung zu.

Das so abgekühlte und von Wasserdampf entlastete, bei einem Druck von etwa 2 bar befindliche Produktgasgemisch der Dehydrierstufe wurde nachfolgend auf einen Druck von 10 bis 13 bar verdichtet.

In anwendungstechnisch zweckmäßiger Weise wurde die Verdichtung zweistufig durchgeführt, um zu hohe Verdichtungstemperaturen zu vermeiden (diesem Zweck diente auch schon die vorab durchgeführte Abkühlung; die Wasserdampfabscheidung entlastet die aufzuwendende Verdichterleistung zusätzlich). In der ersten Stufe wurde auf einen Druck von 4 bis 4,5 bar verdichtet. Die Austrittstemperatur des Gasgemisches betrug beim Verlassen des Verdichters etwa 115°C.

In einem nach geschalteten indirekten Wärmeaustauscher (Luftkühler oder Oberflächenwasserkühler) wurde das Gasgemisch wieder auf 40 bis 60°C abgekühlt, wobei weitere Wasserdampfkondensation erfolgte. Tropfenabscheider sammelten das Kondensat und ließen es standgeregelt aus.

In der zweiten Verdichterstufe wurde ausgehend von einem Druck von etwa 4 bar auf einen Enddruck von 10 bis 13 bar verdichtet. Die Austrittstemperatur beim Verlassen des Verdichters betrug etwa 126°C.

In zwei weiteren nachgeschalteten indirekten Wärmeaustauschern (zunächst ein Luftkühler (ist normalerweise ein Rohrbündelwärmetauscher; das zu kühlende Gas durchströmt zweckmäßig das Rohrinnere) und dann ein Oberflächenwasserkühler) wurde das verdichtete Gasgemisch zunächst auf 40 bis 60°C und dann auf 30°C abgekühlt. Dabei auskondensierendes Wasser wurde wieder mittels Tropfenabscheidern abgeschieden und herausgeführt. Beim Verlassen des zweiten Verdichters enthält das Gasgemisch nur noch 0,2 Gew.-% Wasser. Der geringe Wassergehalt vermeidet eine Zweiphasigkeit in der nachfolgenden Absorption und der hohe Druck mindert die zum Zweck der Absorption benötigte Menge an Absorptionsmittel.

Während großtechnisch Turboverdichter (nicht ölgeschmierte, trocken laufende, berührungsfreie Verdichter) zum Zweck der Verdichtung eingesetzt werden (z.B. vom Typ 12 MH 4B, der Fa. Mannesmann DEMAG, DE), wurden hier Membran-Verdichter MV 3459 II der Fa. Sera verwendet. Prinzipiell können die Verdichter sowohl über Elektromotoren als auch über Dampf- oder Gasturbinen angetrieben werden. Häufig ist der Antrieb über Dampfturbinen der wirtschaftlichste. Das wie beschrieben verdichtete und abgekühlte Gasgemisch wurde direkt oberhalb des Sumpfs einer Absorptionskolonne zugeführt (ca. 4220 g/h). Es wies nachfolgende Gehalte auf:

|  | Vol.-% |
|---|---|
| Stickstoff | 64,39 |
| Sauerstoff | 0,27 |
| Propan | 23,21 |
| Propen | 5,08 |
| Methan | 0,02 |
| Ethan | 0,11 |
| n-Butan | 0,06 |
| iso-Butan | 0,17 |
| n-Butene | 0,04 |
| iso-Buten | 0,10 |
| 1,3-Butadien | 0,00 |
| Wasserstoff | 3,32 |
| Kohlenmonoxid | 0,04 |
| Kohlendioxid | 3,16 |

Die Absorptionskolonne bestand aus Edelstahl 1.4571. Der Kolonneninnendurchmesser betrug 80 mm, die Wanddicke 4 mm und die Kolonnenlänge 1,70 m.

Etwa 70 % des Volumens der Absorptionskolonne waren mit Packungselementen der Fa. Montz (Montz BSH-750, spezifische Oberfläche 750 $m^2/m^3$) als trennwirksame Einbauten gefüllt. Die Packungselemente folgten unmittelbar aufeinander und begannen auf der Höhe von 1/5 der Kolonnenlänge von unten. Die Absorptionskolonne war von außen weder gekühlt noch beheizt. Am Kolonnenkopf wurde mit einer Aufgabetemperatur von 30°C technisches Tetradecan der Fa. Haltermann, DE, vom Typ PKWF 4/7 af als Absorptionsmittel aufgegeben (gaschromatographische Analyse mittels FID-Detektion ergab zu Beginn (frisch) nachfolgende GC-Flächen-% Zusammensetzung:

n-Tridecan 7,6 %,
n-Tetradecan 47,3 %,
n-Pentadecan 7,0 %,
n-Hexadecan 3,2 %,
n-Heptadecan 14,1 % und
Restsumme 20,7 %;

diese Zusammensetzung änderte sich im Verlauf (nach ca. 3000 $h^{-1}$) des kontinuierlichen Verfahrensbetriebs auf folgende Werte:

n-Tridecan 2,6 %,
n-Tetradecan 39,5 %,
n-Pentadecan 9,4 %,

n-Hexadecan 4,8 %,
n-Heptadecan 23,4 und
Restsumme 20,3 %).

Die Berieselungsdichte lag bei 15 m$^3$ Absorptionsmittel pro m$^2$ freie Querschnittsfläche und Stunden (= 25 kg/h Tetradecan).

Der aus der Absorptionskolonne zur Verbrennung geführte Abgasstrom enthielt noch 1700 Vol.-ppm Propan und 400 Vol.-ppm Propen. Großtechnisch wird dieser Abgasstrom über einen Expander (z.B. eine Expansionsturbine) in die Verbrennung geführt, um den Großteil der in der zweistufigen Verdichtung aufgewendeten Verdichtungsleistung rückzugewinnen und in die zweistufige Verdichtung rückzuführen. In zweckmäßiger Weise wird die Expansion auch zweistufig durchgeführt, um unerwünschte Kondensation zu vermeiden. Die bei der Entspannung gewonnene mechanische Energie kann sowohl direkt als Mit- oder Hauptantrieb für einen der Verdichter und/oder zur Erzeugung von Strom genutzt werden.

Bevor das entspannte Absorberabgas zur Verbrennung geführt wird, kann es großtechnisch zweckmäßig sein, den darin enthaltenen Wasserstoff abzutrennen. Dies kann z.B. dadurch erfolgen, dass man das Abgas über eine, in der Regel zu einem Rohr gestaltete, Membran leitet, die lediglich für den molekularen Wasserstoff durchlässig ist. Der so abgetrennte molekulare Wasserstoff kann z.B. in die heterogen katalysierte Dehydrierung rückgeführt oder einer sonstigen Verwertung (z.B. in Brennstoffzellen) zugeführt werden.

Prinzipiell kann die Wasserstoffabtrennung auch durch partielle Kondensation, Adsorption und/oder Rektifikation (vorzugsweise unter Druck, z.B. als Druckwechseladsorption) vorgenommen werden. Großtechnisch ist es zu dem in der Regel zweckmäßig, das Absorberabgas durch das im weiteren noch zu beschreibende Sauerwasser zu führen, um dieses einzuengen.

Das Absorbat enthielt folgende Gehalte (Gew.-% bezogen auf das Gewicht des Absorbats):

|  | Gew.-% |
| --- | --- |
| Stickstoff | 0,094 |
| Propan | 2,65 |
| Propen | 0,55 |
| Ethan | 0,006 |
| n-Butan | 0,009 |
| iso-Butan | 0,024 |
| n-Butene | 0,006 |
| iso-Buten | 0,014 |
| Kohlendioxid | 0,074 |
| Ethen | 0,001 |
| Tetradekan | ca. Rest bis zu 100 Gew.-% |

Bevor das Absorbat zum Kopf der nachfolgenden Desorptionskolonne geführt wurde, wurde es in einem indirekten Wärmeaustauscher auf 36 bis 37°C erwärmt. Als Wärmeträger wurde der flüssige Ablauf aus der Desorptionskolonne verwendet, der eine Temperatur von 37°C aufwies.

Anschließend wurde das Absorbat (dies kann z.B. in einer inversen Pumpe oder mittels eines Ventils ausgeführt werden) auf einen Druck von 2,5 bis 3 bar entspannt (die im Fall der inversen Pumpe dabei frei gesetzte mechanische Energie wird zweckmäßig zur Rückverdichtung von in der Desorptionskolonne befreitem Absorptionsmittel mitverwendet) und das dabei erzeugte zweiphasige Gemisch am Kopf in die Desorptionskolonne geführt.

In die Desorptionskolonne (Innendurchmesser = 80 mm, Länge = 1,70 m, Wanddicke = 4 mm) wurde von unten im Gegenstrom zum vom Desorptionskolonnenkopf ablaufenden Absorbat mit einem Druck von 3 bar Luft geführt (1340 Nl/h). Die am Kopf aufgegebene Absorbatmenge betrug 35 l/h. Das unten aus der Desorptionskolonne herausgeführte, im wesentlichen von desorbierten Komponenten befreite, Absorptionsmittel wurde durch indirekten Wärmeaustausch mit Absorbat gekühlt, auf den in der Absorptionskolonne geforderten Druck verdichtet, durch nochmaligen indirekten Wärmeaustausch (großtechnisch zweckmäßig mit Oberflächenwasser) auf 30°C gekühlt und dann zum Kopf der Absorptionskolonne rückgeführt. Als trennwirksame Einbauten enthielt die Desorptionskolonne strukturierte Blechpackungen der Fa. Montz (Montz BSH-750, spezifische Oberfläche 750 m$^2$/m$^3$). Um Absorptionsmittel zurückzuhalten wurde der aus der Desorptionskolonne (gegebenenfalls über einen mechanischen Tropfenabscheider) geführte Gasstrom mit Wasser gewaschen. D.h., er wurde durch ein Packungselement der Fa. Montz (Montz BSH-750, spezifische Oberfläche 750 m$^2$/m$^3$) geführt, auf das Wasser (70 l/h) mit einer Temperatur von 18 °C im Gegenstrom aufgegeben wurde. Unterhalb der Packung war ein Fangboden (Kaminboden) angebracht, von dem die wässrige Phase herausgeführt werden konnte. In einem Phasenscheider wurde in eine wässrige Phase und in eine organische Phase getrennt. Die sehr geringe Menge organische Phase wurde mit dem zum Kopf der Absorptionskolonne rückgeführten Absorptionsmittelstrom vereinigt. Die wässrige Phase wurde rückgekühlt und mit Frischwasser ergänzt (um Verdampfungsverluste auszugleichen) wieder auf das Packungselement aufgegeben. Das Waschen erfolgte der Desorptionskolonne aufgesetzt.

Aus dem Waschabschnitt wurde der gewaschene Gasstrom über mechanische Tropfenabscheider (abgeschiedene Flüssigphase wird in die Wäsche rückgeführt) mit den nachfolgenden Gehalten herausgeführt (wird in der Reaktionszone A der Dehydrierumsatz höher gewählt, kann der nachfolgende Propengehalt auch bei 8 bis 12 Vol.-% liegen; beispielsweise kann der gewaschene Gasstrom auch 15 Vol.-% Propan, 10 Vol.-% Propen und 14,5 Vol.-% O$_2$ aufweisen):

|  | Vol.-% |
|---|---|
| Stickstoff | 48,11 |
| Sauerstoff | 12,20 |
| Propan | 30,53 |
| Propen | 6,68 |
| Ethan | 0,09 |
| n-Butan | 0,08 |
| iso-Butan | 0,21 |
| n-Butene | 0,05 |
| iso-Buten | 0,13 |
| Wasserstoff | 0,04 |
| Kohlenmonoxid | 0,00 |
| Kohlendioxid | 0,85 |
| Wasser | 1,00 |
| Ethen | 0,02 |

Die Temperatur des Gasgemischs wurde durch indirekten Wärmeaustausch auf 250°C erhöht und das Gasgemisch mit der vorgenannten Zusammensetzung in einer Menge von 2260 Nl/l·h und einem Eingangsdruck von 2,1 bar als neues Reaktionsgasausgangsgemisch in die nachfolgende Partialoxidationsvorrichtung geführt.

C) Gestaltung der Reaktionszone B (der Partialoxidationsstufe)

1. Erster Festbettreaktor für den Schritt der Partialoxidation des Propens (Propylens) zu Acrolein

| Verwendetes Wärmeaustauschmittel: | Salzschmelze, bestehend aus |
|---|---|

53 Gew.-% Kaliumnitrat,

40 Gew.-% Nariumnitrit und

7 Gew.-% Natriumnitrat.

| Abmessung des Kontaktrohres: | 4200 mm Gesamtlänge, |
|---|---|

26 mm Innendurchmesser,

30 mm Außendurchmesser,

(fortgesetzt)

2 mm Wandstärke.

**Reaktor:** Besteht aus einem doppelwandigen Zylinder aus Edelstahl (zylindrisches Führungsrohr, umgeben von einem zylindrischen Außenbehälter). Die Wanddicken betrugen überall 2 bis 5 mm.

Der Innendurchmesser des äußeren Zylinders betrug 168 mm. Der Innendurchmesser des Führungsrohres betrug ca. 60 mm.

Oben und unten war der doppelwandige Zylinder durch einen Deckel beziehungsweise Boden abgeschlossen.

Das Kontaktrohr war durch das zylindrische Führungsrohr geführt im zylindrischen Behälter so untergebracht, dass es am oberen bzw. unteren Ende desselben (abgedichtet) durch den Deckel bzw. Boden jeweils um 250 mm herausgeführt war.

Das Wärmeaustauschmittel war im zylindrischen Behälter eingeschlossen. Um über die gesamte im zylindrischen Behälter befindliche Kontaktrohrlänge (3700 mm) möglichst gleichmäßige thermische Randbedingungen an der Außenwand des Kontaktrohres zu gewährleisten, wurde das Wärmeaustauschmittel durch Einperlen von Stickstoff im zylindrischen Behälter umgewälzt.

Mittels des aufsteigenden Stickstoffs wurde das Wärmeaustauschmittel im zylindrischen Führungsrohr von unten nach oben befördert, um dann im Zwischenraum zwischen zylindrischem Führungsrohr und zylindrischem Außenbehälter wieder nach unten zu strömen (eine Umwälzung gleicher

Güte kann auch durch Umpumpen (z.B. Propellerpumpen) erreicht werden). Durch eine auf den Außenmantel aufgebrachte elektrische Heizung konnte die Temperatur des Wärmeaustauschmittels auf das gewünschte Niveau geregelt werden. Im übrigen bestand Luftkühlung.

**Reaktorbeschickung:** Über den Reaktor betrachtet wurden Salzschmelze und Reaktionsgasgemisch im Gegenstrom geführt. Das Reaktionsgasgemisch trat oben in den Reaktor ein. Es wurde jeweils mit einer Temperatur von 250°C ins Reaktionsrohr geführt.

Die Salzschmelze trat unten mit einer Temperatur $T^{ein}$ = 320°C in das zylindrische Führungsrohr ein und oben mit einer Temperatur $T^{aus}$ aus dem zylindrischen Führungsrohr aus. Der Unterschied zwischen $T^{ein}$ und $T^{aus}$ betrug etwa 2°C. $T^{mittel} = (T^{ein} + T^{aus})/2$.

**Kontaktrohrbeschickung:** Abschnitt A: 50 cm Länge
**(von oben nach unten)** Vorschüttung aus Steatit-Ringen (Steatit C 220 der Fa. Ceram.Tec) der Geometrie 7 mm x 7 mm x 4 mm (Außendurchmesser x Länge x Innendurchmesser).

Abschnitt B: 100 cm Länge
Kontaktrohrbeschickung mit einem homogenen Gemisch aus 30 Gew.-% an Steatit-Ringen (Steatit C 220 der Fa. CeramTec) der Geometrie 5 mm x 3 mm x 2 mm (Außendurchmesser x Länge x Innendurchmessser) und 70 Gew.-% Vollkatalysator aus Abschnitt C.

Abschnitt C: 170 cm Länge
Katalysatorbeschickung mit ringförmigem (5 mm x 3 mm x 2 mm = Außendurchmesser x Länge x Innendurchmesser) Vollkatalysator gemäß Beispiel 1 der DE-A 10046957.

Abschnitt D: 50 cm Länge

Nachschüttung aus Steatit-Ringen (Steatit C 220 der Fa. CeramTec) der Geometrie 7 mm x 7 mm x 4 mm (Außendurchmesser x Länge x Innendurchmesser).

2. Zwischenkühlung und Sauerstoffzwischeneinspeisung

Das den ersten Festbettreaktor verlassende Produktgasgemisch wurde zum Zweck der Zwischenkühlung (indirekt mittels Luft) durch ein Verbindungsrohr (Länge = 400 mm, Innendurchmesser = 26 mm, Wanddicke = 2 mm, Material = Edelstahl) geführt, das, auf einer Länge von 200 mm zentriert untergebracht, mit einer Inertschüttung aus Steatitkugeln (Steatit der Fa. CeramTec) des Durchmessers 5 bis 6 mm beschickt und unmittelbar an das Kontraktrohr des ersten Festbettreaktors angeflanscht war.

Das Gasgemisch trat mit einer Temperatur von mehr als 310°C in das Verbindungsrohr ein und verließ es mit einer Temperatur von etwa 140°C. Anschließend wurden dem Gasgemisch als Sauerstoffquelle 290 Nl/h an komprimierter Luft zugemischt.

Das dabei resultierende, an einem statischen Mischer vermischte, Beschickungsgasgemisch wurde mit einer Temperatur von 220°C dem Festbettreaktor für den Schritt der Partialoxidation des Acroleins zu Acrylsäure zugeführt.

3. Zweiter Festbettreaktor für den Schritt der Partialoxidation des Acroleins zu Acrylsäure

Es wurde ein Festbettreaktor verwendet, der mit jenem für den ersten Schritt baugleich war. Salzschmelze und Reaktionsgasgemisch wurden über den Reaktor betrachtet im Gleichstrom geführt. Die Salzschmelze trat unten ein, das Reaktionsgasgemisch ebenfalls.

Die Kontaktrohrbeschickung (von unten nach oben) war:

Abschnitt A: 20 cm Länge

Vorschüttung aus Steatit-Ringen (Steatit C 220 der Fa. CeramTec) der Geometrie 7 mm x 7 mm x 4 mm (Außendurchmesser x Länge x Innendurchmesser).

Abschnitt B: 100 cm Länge

Katalysatorbeschickung mit einem homogen Gemisch aus 30 Gew.-% an Steatit-Ringen (Steatit C 220 der Fa. CeramTec) der Geometrie 7 mm x 3 mm x 4 mm

(Außendurchmesser x Länge x Innendurchmesser) und 70 Gew.-% Schalenkatalysator aus Abschnitt C.

Abschnitt C: 200 cm Länge

Katalysatorbeschickung mit ringförmigem

(7 mm x 3 mm x 4 mm = Außerdurchmesser x Länge x Innendurchmesser) Schalenkatalysator gemäß Herstellungsbeispiel 5 der DE-A 10046928 (an dieser Stelle können auch dazu analoge und in entsprechender Weise hergestellte Schalenkatalysatoren eingesetzt werden, deren Aktivmasse jedoch eine Stöchiometrie von $Mo_{12}V_{2,8}W_{1,2}Cu_{2,4}O_x$ oder von $Mo_{12}V_{3,5}W_{1,3}Cu_{2,4}O_x$ aufweist).

Abschnitt D: 50 cm Länge

Nachschüttung aus Steatit-Ringen (Steatit C 220 der Fa. CeramTec) der Geometrie 7 mm x 7 mm x 4 mm (Außendurchmesser x Länge x Innendurchmesser).

Der zweite Reaktor wurde mit ca. 3850 g/h an Beschickungsgasgemisch belastet. $T^{mit-tel}$ ist wie für den ersten Festbettreaktor definiert und betrug 274°C.

Der Propenumsatz im ersten Reaktor betrug 97,7 mol-% und der Acroleinumsatz im zweiten Reaktor betrug 99,4 mol-%.

Die Gehalte des den zweiten Festbettreaktor mit einer Temperatur von 283°C und einem Druck von 1,8 bar verlassenden Produktgasgemisches (Produktgasgemisch B) waren:

|  | Vol.-% |
|---|---|
| Stickstoff | 52,87 |

(fortgesetzt)

|  | Vol.-% |
|---|---|
| Sauerstoff | 3,03 |
| Propan | 27,48 |
| Propen | 0,14 |
| Methan | 0 |
| Ethan | 0,07 |
| n-Butan | 0,08 |
| iso-Butan | 0,34 |
| n-Butene | 0 |
| iso-Buten | 0 |
| 1,3-Butadien | 0 |
| Wasserstoff | 0,03 |
| Kohlenmonoxid | 0,42 |
| Kohlendioxid | 1,85 |
| Wasser | 7,92 |
| Acrolein | 0,03 |
| Acrylsäure | 5,3 |
| Essigsäure | 0,18 |
| Ameisensäure | 0,01 |
| Formaldehyd | 0,17 |
| Benzaldehyd | 0 |
| Maleinsäureanhydrid | 0,04 |
| Ethen | 0,02 |

Die in den beiden Reaktionsstufen verwendeten Katalysatoren können auch durch die in den Beispielen der DE-A 10351269 verwendeten Katalysatoren ersetzt werden. Der Katalysator der ersten Reaktionsstufe kann auch durch die Katalysatoren der Beispiele und Vergleichsbeispiele der DE-A 10344149 ersetzt werden. Der Katalysator der zweiten Reaktionsstufe kann auch durch die Katalysatoren der Beispiele der DE-A 10360057 und DE-A 10360058 ersetzt werden.

D) Gestaltung der Trennzone B (Abtrennung des Zielproduktes Acrylsäure aus dem Produktgasgemisch der Partialoxidation)

In einem Direktkühler wurde das heiße Produktgasgemisch (das Produktgasgemisch B) durch Eindüsen einer Quenchflüssigkeit (enthaltend 57,4 Gew.-% Diphenylether, 20,7 Gew.-% Diphenyl und 20 Gew.-% o-Dimethylphthalat) der Temperatur 140 bis 150°C, die auch als Absorptionsmittel verwendet wurde, auf ca. 180°C abgekühlt. Im unteren Teil des Direktkühlers wurde die Quenchflüssigkeit gesammelt, entnommen und über einen Wärmeaustauscher, in welchem sie rückgekühlt wurde, in den oberen Teil des Quenchgefäßes rückgesprüht. Das Produktgasgemisch wurde dem Direktkühler in die gesammelte Quenchflüssigkeit getaucht zugeführt. Das abgekühlte Produktgasgemisch strömte dann in die nachfolgende Absorptionskolonne. Im Quenchumlauf reicherten sich hochsiedende Nebenprodukte an, die ausgeschleust werden mussten. Aus dem Quenchumlauf wurde deshalb einmal je Tag diskontinuierlich eine Teilmenge von 1 kg Quenchflüssigkeit in einen Behälter abgelassen, der mit Hilfe eines Rotationsverdampfers diskontinuierlich aufgearbeitet wurde. Das gereinigte Absorptionsmittel wurde wieder in den Quenchumlauf geführt und die Verluste an Absorptionsmittel mit einem Gemisch aus Diphenylether, Diphenyl und o-Dimethylphthalat ergänzt.

Das abgekühlte Produktgasgemisch wurde oberhalb des Sumpfes in die Absorptionskolonne geführt. Die Absorptionskolonne war 5200 mm lang, hatte einen Innendurchmesser von 50 mm und enthielt Kuehni (CH) Rombopak 9M Packungen. Die Absorptionskolonne war aus Glas gefertigt und wies aus Gründen der Temperierung einen segmentierten Glasdoppelmantel auf. Oberhalb der ersten Packung, von unten betrachtet, war ein Druckentkopplungsventil installiert (eine einstellbare Drossel), mit dessen Hilfe ein Druckverlust über die Absorptionskolonne (wie er großtechnisch auftreten würde) von 100 bis 300 mbar simuliert wurde. Die Absorptionskolonne wurde mit einem Kopfdruck von 1,35 bar betrieben. Der Glasdoppelmantel war in fünf voneinander getrennte Segmente unterteilt, über die das gewünschte Temperaturprofil aufgeprägt wurde.

Von unten nach oben wiesen die Segmente die folgenden Temperaturen auf (Thermostatisierung mittels umgepumptem Wasser):

> 1. Segment, 1020 mm Länge, 90-115°C;
> 2. Segment, 1250 mm Länge, 88°C;
> 3. Segment, 950 mm Länge, ca. 60°C;
> 4. Segment, 950 mm Länge, 50-55°C;
> 5. Segment, 1250 mm Länge, 18°C.

Unterhalb der obersten Packung war ein Fangboden, von welchem wasserreiches Kondensat (Sauerwasser) abgezogen wurde. Es wurde über einen Wärmeaustauscher auf 20°C abgekühlt über die oberste Packung in die Absorptionskolonne rückgeführt und die überschüssige Menge (das Reaktionswasser) standgeregelt ausgeschleust. Die ausgelassene Sauerwassermenge wurde einem Phasenscheider zugeführt. Die organische Phase wurde über die zweite Packung in die Absorptionskolonne rückgeführt, die wässrige Phase wurde entsorgt. Unmittelbar unterhalb des Fangbodens wurden 3,5 l/h Absorptionsflüssigkeit mit einer Temperatur von 40°C aufgegeben.

Das am Kopf der Absorptionskolonne ausgelassene (Haupt)Restgas (T = 18 °C, P = 1.35 bar) wies folgende Gehalte auf:

|  | Vol.-% |
|---|---|
| Stickstoff | 61,17 |
| Sauerstoff | 3,51 |
| Propan | 31,80 |
| Propen | 0,16 |
| Methan | 0 |
| Ethan | 0,09 |
| n-Butan | 0,09 |
| iso-Butan | 0,39 |
| n-Butene | 0 |
| iso-Buten | 0 |
| Butadiene | 0 |
| Wasserstoff | 0,04 |
| Kohlenmonoxid | 0,49 |
| Kohlendioxid | 2,12 |
| Wasser | 0,09 |
| Acrolein | 0,03 |
| Acrylsäure | 0 |
| Ameisensäure | 0 |
| Formaldehyd | 0 |
| Benzaldehyd | 0 |
| Maleinsäureanhydrid | 0 |
| Ethen | 0,02 |

Durch indirekten Wärmetausch wurde das (Haupt)Restgas auf 40°C erwärmt (um unerwünschte Kondensation auszuschließen), mittels eines Verdichters KNF Neuberger Typ PM 17739-1200 (großtechnisch ein mittels Elektromotor angetriebener Turboverdichter, z.B. vom Typ 12MH4B, der Fa. Mannesmann DEMAG, DE) auf 2,75 bar verdichtet und als Kreisgasanteil (als erster Anteil des (Gesamt)Restgases) zur Beschickung des ersten Dehydrierreaktors in die Reaktionszone A rückgeführt.

Aus dem Sumpf der Absorptionskolonne wurden 3,9 l/h Sumpfflüssigkeit stand geregelt abgezogen. Ein zweiter, kleinerer Strom von ca. 0,5 l/h wurde in den Direktkühler für das heiße Produktgasgemisch zurückgeführt.

Der Sumpfaustrag (er kann zunächst noch einer wie in der DE-A 10336386 beschriebenen Strippung mit einer Teilmenge an Restgas (vorzugsweise zuvor im wesentlichen an Acrylsäure frei gewaschen) unterworfen werden; das beladene Strippgas kann dann wie in der DE-A 10336386 beschrieben in die Absorptionskolonne rückgeführt werden) wurde auf den Kopf einer Abtriebskolonne (50 mm Innendurchmesser, 2000 mm Länge, aus Glas, Doppelmantel zum Zweck der Temperierung (150°C), 20 Siebböden (Lochdurchmesser 6,5 mm, Dreiecksteilung) geführt. Dort dampften bei etwa 200 mbar und 150°C alle Leichtsieder (insbesondere Propan und Propen), der Hauptteil der Acrylsäure, Mittelsieder (z.B. Essigsäure) sowie ein Teil des Absorptionsmittels ab. Aus dem Sumpf der Abtriebskolonne wurde mittels einer Pumpe (Hermetic, 500 l/h) ein Umlaufstrom (200 l/h) entnommen und über einen Wärmeaustauscher (Zwangsumlaufentspannungsverdampfer) in die Abtriebskolonne rückgeführt (mit 190°C). Der am Sumpf ablaufende, gering beladene Strom an Lösemittel ging zurück zur Acrylsäureabsorptionskolonne und wurde unterhalb des Fangbodens, an welchem das Reaktionswasser (Sauerwasser) ausgeschleust wurde, wieder zugeführt.

Die der Abtriebskolonne entweichenden Brüden wurden in eine an Einbauten freie Kolonne geführt, um sie in ihrem Kondensat aufsteigen zu lassen. Letzteres wurde in einer Menge von 100 l/h aus dem Sumpf dieser Kolonne entnommen, über einen Wärmeaustauscher geführt, in dem die Kondensationswärme entzogen wurde, und anschließend mit einer Temperatur von 20°C am Kopf dieser Kolonne rückgeführt.

Aus dem Sumpf dieser Kolonne wurden ferner 600 ml/h abzogen (enthält das Zielprodukt Acrylsäure, Wasser und noch Restabsorptionsmittel), aus denen die Acrylsäure bei Bedarf in an sich bekannter Weise abgetrennt werden kann. Am Kopf der an Einbauten freien Kolonne war nochmals ein Sauerwasserquench aufgesetzt, der durch einen Sammelboden von der Kolonne abgetrennt war. Das auf dem Sammelboden abgezogene Kondensat wurde mit dem aus der Absorptionskolonne (für den Produktgasstrom der Partialoxidation) entnommenen Sauerwässer gemischt und auf 18°C gekühlt in den Sauerwasserquench rückgeführt. Überschüssiges Sauerwasser wurde ausgetragen. Das aus dem Sauerwasserquench entweichende Gas wurde als weiteres Propan und nicht umgesetztes Propylen enthaltendes restliches Gas nach Verdichtung als weiterer Kreisgasanteil (zweiter Anteil des (Gesamt)Restgases) zur Beschickung des ersten Dehydrierreaktors in die Reaktionszone A rückgeführt.

Vergleichsbeispiel 2

Es wurde wie im Vergleichsbeispiel 1 verfahren. Das den dritten Dehydrierreaktor verlassende Produktgasgemisch A wurde jedoch durch einen Strömungsteiler in zwei Hälften identischer Zusammensetzung aufgeteilt und die eine Hälfte in die Trennzone A und die andere Hälfte als Bestandteil des Reaktionsgasausgangsgemischs des ersten Dehydrierreaktors in die Reaktionszone A rückgeführt.

Dazu wurde das (Gesamt) Restgas auf einen Druck von 4 bar verdichtet und mit dem so verdichteten (Gesamt) Restgas als Treibstrahl eine Strahlpumpe betrieben, wobei die Förderrichtung des durch eine Treibdüse über eine Mischstrecke und einen Diffusor auf 3 bar entspannten Treibstrahls in den dem ersten Erhitzer vorgeschalteten Verdampfer sowie der Saugstutzen in Richtung der einen Strömungshälfte des Produktgemischs A wies und die Verbindung "Saugstutzen - Mischstrecke -Diffusor" die alleinige Verbindung zwischen der zurückzuführenden Hälfte des Produktgemisches A und dem dem ersten Erhitzer vorgeschalteten Verdampfer war.

Bereits nach einer kontinuierlichen Betriebsdauer von 10 h begannen die Dehydrierkatalysatoren an Aktivität zu verlieren.

Vergleichsbeispiel 3 (alle Drücke sind wie stets in dieser Schrift Absolutdrücke, soweit nichts anderes explizit erwähnt wird)

A) Gestaltung der Reaktionszone A (der Dehydrierstufe)

Die Dehydrierstufe bestand aus drei hintereinander geschalteten identischen Rohrreaktoren, die in identischer Weise mit Dehydrierkatalysator beschickt waren.

Der einzelne Rohrreaktor war ein Stahlrohr (Edelstahl der DIN Werkstoffnummer 1.4841) der Länge 1300 mm, der Wanddicke 3,6 mm und des Innendurchmessers 53,1 mm. Die Rohrreaktoren wurden vom Reaktionsgasgemisch jeweils von oben nach unten durchströmt.

Am unteren Ende jedes Rohrreaktors befand sich ein Tragerost aus demselben Edelstahl. Auf dem Tragerost befand sich von unten nach oben die folgende Beschickung:

| 175 mm | Schüttlänge aus Steatitkugeln (Durchmesser 4-5 mm) des Steatits C-220 der Fa. CeramTec; |
|---|---|
| 21 mm | Schüttlänge aus Steatitkugeln (Durchmesser 1,5-2,5 mm) des Steatits C-220 der Fa. CeramTec; |
| 210 mm | Schüttlänge Dehydrierkatalysator (Pt/Sn-Legierung, die mit den Elementen Cs, K und La in oxidischer Form promoviert war und die auf die äußere und innere Oberfläche von $ZrO_2 \cdot SiO_2$ Mischoxidträgersträglingen (mittlere Länge (gaußverteilt im Bereich von 3 mm bis 12 mm mit Maximum bei ca. 6 mm): 6 mm, Durchmesser: 2 mm) in der Elementstöchiometrie (Massenverhältnisse einschließlich Träger) $Pt_{0,3}Sn_{0,6}La_{3,0}Cs_{0,5}K_{0,2}$ $(ZrO_2)_{88,3}(SiO_2)_{7,1}$ aufgebracht war (Katalysatorvorläuferherstellung und Aktivierung zum aktiven Katalysator wie in Beispiel 4 der DE-A 10 219 879). |
| 21 mm | Schüttlänge Steatitkugeln (Durchmesser 1,5-2,5 mm) des Steatits C-220 der Fa. CeramTec; und |

abschließend auf der Restlänge des Rohrreaktors wieder eine Schüttung aus Steatitkugeln (Durchmesser 4-5 mm) des Steatits C-220 der Fa. CeramTec.

Jeder der Rohrreaktoren war außen im Sinne einer Vorheizstrecke auf den ersten 500 mm Rohrlänge von oben nach unten (zum Tragerost hin) in zwei, eine Gleichverteilung der zugeführten Wärmemenge gewährleistenden, Halbschalen aus Kupfer (Schalendicke = 200 mm) eingelegt, die mittels einer sie im vollen Umfang umgebenden Heizmanschette (Fa. Horst, DE-Heidelberg, 500 mm Länge, 100 mm Innendurchmesser) elektrisch beheizt wurden.

Von unten nach oben war jeder der Rohrreaktoren im Sinne einer adiabaten Strecke auf einer Länge von 600 mm in jeweils zwei Paare von thermisch isolierend wirkenden Halbschalen (Dicke einer Halbschale = 25 mm) aus MPS-Super G der Fa. Microtherm in DE, die um 90° gegeneinander versetzt übereinander angebracht waren, eingebracht. Die isolierend wirkenden Halbschalen waren ihrerseits von einer zylindrischen Einhüllenden aus Edelstahl (Außendurchmesser = 173 mm, Innendurchmesser = 167 mm) umgeben, an die zum Zweck der Begleitheizung eine Heizmanschette (Länge = 675 mm, Innendurchmesser = 173 mm) der Fa. Horst, DE-Heidelberg, angelegt war. Auf diese Weise konnte auf der adiabaten Strecke der Wärmefluss aus der Umgebung in das Reaktionsrohr hinein und aus dem Reaktionsrohr heraus in die Umgebung minimiert werden.

In jedes Reaktionsrohr war mittig zusätzlich eine 1370 mm lange Thermohülse eingeführt (Außendurchmesser: 6mm, Innendurchmesser: 4mm), in die ein Mehrfach-Thermoelement (vom unteren Reaktorende nach oben alle 4 cm insgesamt 10 Messstellen, Dicke 3,2mm) eingeführt war.

Vor jeden einzelnen Rohrreaktor war ein mit Steatitringen (aus Steatit C-220 der Fa. CeramTec und von der Geometrie 7 mm x 3 mm x 3 mm = Außendurchmesser x Innendurchmesser x Höhe) gefülltes Stahlrohr der Länge 1300 mm als Erhitzer geschaltet. In ihm wurde das Reaktionsgasgemisch jeweils auf die Eintrittstemperatur des nachfolgenden Rohreaktors vorgeheizt und gleichzeitig ideal durchmischt. Zu diesem Zweck wurden die Erhitzerrohre (Edelstahl der DIN Werkstoffnummer 1.4841, Wanddicke 3,6 mm, Innendurchmessers 53,1 mm) auf einer rohrmittigen Länge von 1200 mm mittels um sie angelegter Heizmanschetten der Fa. Horst, DE-Heidelberg elektrisch beheizt. Die Verbindung zwischen Erhitzer und Rohrreaktoren wurde durch mit üblichen Wärmedämmmaterialien thermisch isolierten Edelstahlrohren (Edelstahl der DIN Werkstoffnummer 1.4841, Außendurchmesser 21.3 mm, Innendurchmesser 16.1mm, Länge ca. 700 mm) bewerkstelligt.

Vor dem Eintritt des Reaktionsgasgemischs in den jeweiligen Erhitzer war ein Zufuhrhahn angebracht, über den dem Reaktionsgasgemisch jeweils Druckluft zugeführt werden konnte. Beschrieben wird nachfolgend der stationäre Betrieb.

Dem ersten Dehydrierreaktor wurde ein Reaktionsgasausgangsgemisch aus 300 g/h Roh-Propan (erster Propan enthaltender Zufuhrstrom), 375 g/h Wasser und 3768 g/h (Gesamt)Restgas als Kreisgas (zweiter Propan enthaltender Zufuhrstrom) mit einer Temperatur von 400°C und einem Druck von 2,6 bar absolut zugeführt (im großtechnischen Betrieb würde der Eingangsdruck zweckmäßig ca. 0,5 bar höher gewählt, um dem erhöhten Druckverlust (bedingt durch höhere Strömungsgeschwindigkeiten) in der Reaktionszone A Rechnung zu tragen).

Das Roh-Propan enthielt:

|  | Vol.-% |
|---|---|
| Methan | 0 |
| Ethan | 0,156 |
| Ethen | 0 |
| Propan | 96,18 |

(fortgesetzt)

|  | Vol.-% |
|---|---|
| Propen (Propylen) | 0,002 |
| $H_2$ | 0 |
| $O_2$ | 0 |
| $N_2$ | 1,70 |
| CO | 0 |
| $CO_2$ | 0 |
| iso-Butan | 1,245 |
| n-Butan | 0,711 |
| trans-Buten | 0,0005 |
| iso-Buten | 0 |
| cis-Buten | 0,0015 |
| 1-Buten | 0,0048 |
| Butadien | 0 |

Das (Gesamt)Restgas (Kreisgas) enthielt:

|  | Vol.-% |
|---|---|
| Methan | 0,009 |
| Ethan | 0,088 |
| Ethen | 0,038 |
| Propan | 29,56 |
| Propen | 0,122 |
| $H_2$ | 0,050 |
| $O_2$ | 3,35 |
| $N_2$ | 64,05 |
| CO | 0,538 |
| $CO_2$ | 1,85 |
| iso-Butan | 0,234 |
| n-Butan | 0,098 |
| trans-Buten | 0,00005 |
| iso-Buten | 0,00051 |
| cis-Buten | 0,00144 |
| 1-Buten | 0,00048 |
| Butadien | 0,0087 |

Die Bestimmung der Zusammensetzung des Roh-Propans und aller anderen Gaszusammensetzungen erfolgte gaschromatographisch [HP 6890 mit Chem-Station, Detektoren: FID, WLD, Trennsäulen: $Al_2O_3$/KCL (Chrompack), Carboxen 1010 (Supelco)]. Bei Wasserdampf enthaltenden Gasgemischen wurde dieses vorab der gaschromatographischen Analyse durch Abkühlen und gegebenenfalls Entspannen in einem Wasserabscheider auskondensiert. Das nicht kondensierte verbleibende Gas wurde analysiert und auf dieses trocken gerechnete Gas (d.h., die im eigentlich zu analysierenden Gasgemisch enthaltene Wasserdampfmenge blieb unberücksichtigt) beziehen sich alle Angaben.

Das Reaktionsgasausgangsgemisch wurde in einem Verdampfer erzeugt, der dem ersten Erhitzer vorgeschaltet war. Der Verdampfer selbst war ebenfalls wie ein Erhitzer ausgeführt. Ihm wurden 300 g/h gasförmiges Roh-Propan (65°C, 5 bar), 3768 g/h (Gesamt)Restgas (Kreisgas) (50°C, 2,8 bar) und 375 g/h Wasser (20°C, 3 bar) zugeführt. Die Beheizung des Verdampfers war auf eine Gasgemischaustrittstemperatur von 200°C geregelt. Der Verdampfer war mit dem ersten Erhitzer in entsprechender Weise verbunden wie die Reaktoren an die Erhitzer angebunden waren.

Die Beheizung des ersten Erhitzers war so geregelt, dass das vom Verdampfer in den ersten Erhitzer geleitete Gasgemisch den ersten Erhitzer mit einer Temperatur von 400°C verließ (die dazu erforderliche Wandtemperatur betrug ca. 440°C). Dann wurde das Reaktionsgasausgangsgemisch in den ersten Rohrreaktor geführt und in der Vorheizstrecke desselben auf eine Reaktionszoneneintrittstemperatur von 460°C weiter erhitzt.

Die Temperatur des Reaktionsgasgemischs durchlief beim Durchgang durch den ersten Rohrreaktor eine Maximum (Heißpunkttemperatur genannt) von 549,1°C (die hier angegebenen quantitativen Angaben beziehen sich auf den Betriebszustand nach 200 Betriebsstunden; im weiteren Verlauf des Betriebs wurden die verschiedenen Temperaturen so nachgezogen, dass der auf Einmaldurchgang bezogene Umsatz und die Raum-Zeit-Ausbeute im wesentlichen konstant blieben; in entsprechender Weise wurde auch bereits in den ersten 200 Betriebsstunden verfahren), das im Verlauf des kontinuierlichen Betriebs der Versuchsanlage infolge allmählicher Katalysatordeaktivierung in Strömungsrichtung wanderte (die Wanderungsgeschwindigkeit betrug ca. 0,03 mm/h).

Das den ersten Dehydrierreaktor verlassende Reaktionsgasgemisch wies folgende Gehalte auf:

|  | Vol.-% |
|---|---|
| Methan | 0,045 |
| Ethan | 0,109 |
| Ethen | 0,042 |
| Propan | 30,3 |
| Propen | 2,88 |
| $H_2$ | 5,00 |
| $O_2$ | 0 |
| $N_2$ | 59,13 |
| CO | 0,06 |
| $CO_2$ | 3,24 |
| iso-Butan | 0,257 |
| n-Butan | 0,116 |
| trans-Buten | 0,05 |
| iso-Buten | 0,001 |
| cis-Buten | 0,004 |
| 1-Buten | 0,001 |
| Butadien | 0,003 |

Seine Temperatur betrug 509°C und sein Druck lag bei ca. 2,56 bar.

Vor dem Eintritt in den nachfolgenden Erhitzer wurden dem Reaktionsgasgemisch 80 Nl/h Druckluft zudosiert (23°C, 4,2 bar).

Dann wurde das Reaktionsgasgemisch mittels der elektrischen Beheizungsmöglichkeiten des Erhitzers (Wandtemperatur ca. 540°C) und der Vorheizstrecke des nachfolgenden (zweiten) Reaktionsrohres (Wandtemperatur ca. 560°C) auf 465°C (Eintritt 2. Reaktionszone) erwärmt. Der Druck des Reaktionsgasgemischs an dieser Stelle lag bei 2,56 bar.

Beim Durchgang durch den zweiten Rohrreaktor durchlief die Temperatur des Reaktionsgasgemischs ein Maximum von ca. 560°C, das im Verlauf des kontinuierlichen Betriebs der Versuchsanlage infolge allmählicher Katalysatordeaktivierung in Strömungsrichtung wanderte (die Wanderungsgeschwindigkeit betrug ca. 0,1 mm/h). Das den zweiten Dehydrierreaktor verlassende Reaktionsgasgemisch wies folgende Gehalte auf:

|  | Vol % |
|---|---|
| Methan | 0,078 |
| Ethan | 0,144 |
| Ethen | 0,063 |
| Propan | 26,6 |
| Propen | 4,94 |
| $H_2$ | 6,43 |
| $O_2$ | 0 |
| $N_2$ | 58,58 |
| CO | 0,384 |
| $CO_2$ | 3,58 |
| iso-Butan | 0,22 |
| n-Butan | 0,094 |
| trans-Buten | 0,063 |
| iso-Buten | 0,001 |
| cis-Buten | 0,01 |
| 1-Buten | 0 |
| Butadien | 0,004 |

Seine Temperatur betrug ca. 493°C und sein Druck lag bei ca. 2,52 bar.

Vor dem Eintritt in den nachfolgenden Erhitzer wurden dem Reaktionsgasgemisch 98 NI/h Druckluft zudosiert (23°C, 4,2 bar).

Dann wurde das Reaktionsgasgemisch mittels der elektrischen Beheizungsmöglichkeiten des Erhitzers (Wandtemperatur ca. 540°C) und der Vorheizstrecke des nachfolgenden (dritten) Reaktionsrohres (Wandtemperatur ca. 540°C) auf 521 °C (Eintritt 3. Reaktionszone) erwärmt. Der Druck des Reaktionsgasgemischs an dieser Stelle lag bei 2,52 bar.

Beim Durchgang durch den dritten Rohrreaktor durchlief die Temperatur des Reaktionsgasgemischs ein Maximum von 570°C, das im Verlauf des kontinuierlichen Betriebs der Versuchsanlage infolge allmählicher Katalysatordeaktivierung in Strömungsrichtung wanderte (die Wanderungsgeschwindigkeit betrug ca. 0,1 mm/h). Das den dritten Dehydrierreaktor verlassende Reaktionsgasgemisch wies folgende Gehalte auf:

|  | Vol % |
|---|---|
| Methan | 0,1046 |
| Ethan | 0,144 |
| Ethen | 0,0743 |
| Propan | 25,22 |
| Propen | 5,51 |
| $H_2$ | 4,69 |
| $O_2$ | 0 |
| $N_2$ | 60,10 |
| CO | 0,237 |
| $CO_2$ | 3,54 |
| iso-Butan | 0,201 |
| n-Butan | 0,085 |

(fortgesetzt)

|  | Vol % |
|---|---|
| trans-Buten | 0,070 |
| iso-Buten | 0,0013 |
| cis-Buten | 0,011 |
| 1-Buten | 0,0004 |
| Butadien | 0,0056 |

Seine Temperatur betrug 480,4°C und sein Druck lag bei 2,48 bar. Damit ergab sich über die Dehydrierstufe ein auf den Einmaldurchgang des Reaktionsgasausgangsgemischs bezogener Gesamtdehydrierumsatz des Propans von 19,91 mol-%.

Bei fortgeschrittener Deaktivierung der Dehydrierkatalysatorschüttungen wurde das Verfahren unterbrochen und diese wie in der DE-A 10028582 beschrieben regeneriert. Dies war im wesentlichen immer dann der Fall, wenn die Heißpunkttemperatur in allen drei Rohrreaktoren ca. 580°C betrug.

In überraschender Weise schritt die Deaktivierung der Dehydrierkatalysatorschüttungen bei erhöhtem Arbeitsdruck langsamer voran.

B) Gestaltung der Trennzone A

Durch Direktkühlung mit versprühtem gekühltem Wasser (T = 20°C) wurde das den dritten Dehydrierreaktor verlassende Produktgasgemisch (Produktgasgemisch A) in einem Direktkühler (Quench) im Gleichstrom auf 40°C abgekühlt (das dabei gasförmig verbleibende Gemisch wurde in der Gegenrichtung zur Produktgaseinströmrichtung aus dem Wasserquench herausgeführt). Etwa 75 Gew.-% des im Produktgasgemisch enthaltenen Wasserdampf (Wasserdampf wurde dem Reaktionsgasausgangsgemisch der Reaktionszone A zugesetzt und bildete sich in der Dehydrierstufe durch Verbrennung von Wasserstoff sowie möglicherweise Kohlenwasserstoff mit Luftsauerstoff; die Verbrennungswärme hielt die Reaktionstemperatur im Reaktionsgasgemisch der Reaktionszone A weitgehend aufrecht) kondensierte bei der Direktkühlung aus. Das sich dabei bildende Kondensat wurde mittels Standregelung aus dem Wasserquench heraus- und seiner Entsorgung zugeführt. Im übrigen wurde das zur Direktkühlung eingesetzte Wasser im Kreis geführt (gepumpt), durch indirekten Wärmeaustausch rückgekühlt und zum Zweck der Direktkühlung wieder versprüht.

Anstelle der beschriebenen Direktkühlung mit Wasser, kann das Produktgasgemisch aus der Dehydrierstufe auch zunächst dadurch abgekühlt werden, dass man mit dem Produktgasgemisch in einem indirekten Wärmeaustauscher (z.B. in einem Rohrbündelwärmeaustauscher im Gleichstrom oder im Gegenstrom) das der Dehydrierstufe zuzuführende Reaktionsgasausgangsgemisch aufwärmt (z.B. auf eine Temperatur im Bereich von 350 bis 450°C). Das Produktgasgemisch der Dehydrierstufe kühlt dabei in entsprechendem Maß von der hohen Austrittstemperatur der Dehydrierstufe (z.B. von 450 bis 550°C) auf ca. 200 bis 300°C ab.

Eine weitere Abkühlung des Produktgasgemisches der Dehydrierstufe kann dadurch erfolgen, dass es in einem indirekten Wärmeaustauscher dazu verwendet wird, das Ausgangsgasgemisch für die nachfolgend noch zu beschreibende Partialoxidation des in der Dehydrierstufe erzeugten Propens aufzuwärmen, und/oder dass es dazu verwendet wird, die im weiteren noch zu beschreibende Absorberabgaskühlung beim, vorzugsweise zweistufigen, Expandieren desselben mittels Expansionsturbinen auszugleichen, oder durch Vorabanwärmung zu kompensieren.

Danach befindet sich das Produktgasgemisch bei einer Temperatur von etwa 180°C. Anschließend kann mittels Luft- und/oder Oberflächenwasserkühler auf eine Temperatur im Bereich von 30°C bis 60°C abgekühlt werden.

In die Kühler integrierte oder diesen nachgeschaltete Tropfenabscheider sammeln das beim Abkühlen auskondensierte Wasser und führen es standgeregelt der Entsorgung zu.

Das so abgekühlte und von Wasserdampf entlastete, bei einem Druck von etwa 2 bar befindliche Produktgasgemisch der Dehydrierstufe wurde nachfolgend auf einen Druck von 10 bis 13 bar verdichtet.

In anwendungstechnisch zweckmäßiger Weise wurde die Verdichtung zweistufig durchgeführt, um zu hohe Verdichtungstemperaturen zu vermeiden (diesem Zweck diente auch schon die vorab durchgeführte Abkühlung; die Wasserdampfabscheidung entlastet die aufzuwendende Verdichterleistung zusätzlich). In der ersten Stufe wurde auf einen Druck von 4 bis 4,5 bar verdichtet. Die Austrittstemperatur des Gasgemisches betrug beim Verlassen des Verdichters etwa 115°C.

In einem nach geschalteten indirekten Wärmeaustauscher (Luftkühler oder Oberflächenwasserkühler) wurde das Gasgemisch wieder auf 40 bis 60°C abgekühlt, wobei weitere Wasserdampfkondensation erfolgte. Tropfenabscheider sammelten das Kondensat und schleusten es standgeregelt aus.

In der zweiten Verdichterstufe wurde ausgehend von einem Druck von etwa 4 bar auf einen Enddruck von 10 bar verdichtet (hier kann gegebenenfalls auch auf einen Druck von bis zu 13 bar und mehr verdichtet werden). Die Austrittstemperatur beim Verlassen des Verdichters betrug etwa 126°C.

In zwei weiteren nachgeschalteten indirekten Wärmeaustauschern (zunächst ein Luftkühler (ist normalerweise ein Rohrbündelwärmetauscher; das zu kühlende Gas durchströmt zweckmäßig das Rohrinnere) und dann ein Oberflächenwasserkühler) wurde das verdichtete Gasgemisch zunächst auf 40 bis 60°C und dann auf 30°C abgekühlt. Dabei auskondensierendes Wasser wurde wieder mittels Tropfenabscheidern abgeschieden und herausgeführt. Beim Verlassen des zweiten Verdichters enthält das Gasgemisch nur noch ca. 0,2 Gew.-% Wasser. Der geringe Wassergehalt verringert den Wasseranfall und vermeidet so durch eine Zweiphasigkeit der Flüssigkeit bedingte Betriebsprobleme in der nachfolgenden Absorption und der hohe Druck mindert die zum Zweck der Absorption benötigte Menge an Absorptionsmittel.

Während großtechnisch Turboverdichter (nicht ölgeschmierte, trocken laufende, berührungsfreie Verdichter) zum Zweck der Verdichtung eingesetzt werden (z.B. vom Typ 12 MH 4B, der Fa. Mannesmann DEMAG, DE), wurden hier Membran-Verdichter MV 3459 II der Fa. Sera verwendet. Prinzipiell können die Verdichter sowohl über Elektromotoren als auch über Dampf- oder Gasturbinen angetrieben werden. Häufig ist der Antrieb über Dampfturbinen der wirtschaftlichste. Das wie beschrieben verdichtete und abgekühlte Gasgemisch wurde direkt oberhalb des Sumpfs einer Absorptionskolonne zugeführt (ca. 4650 g/h). Es wies nachfolgende Gehalte auf:

|  | Vol.-% |
| --- | --- |
| Stickstoff | 61,22 |
| Sauerstoff | 0,25 |
| Propan | 24,23 |
| Propen | 5,07 |
| Methan | 0,02 |
| Ethan | 0,11 |
| Ethen | 0,03 |
| n-Butan | 0,06 |
| iso-Butan | 0,09 |
| n-Butene | 0,04 |
| iso-Buten | 0,10 |
| 1,3-Butadien | 0,00 |
| Wasserstoff | 5,57 |
| Kohlenmonoxid | 0,05 |
| Kohlendioxid | 3,15 |

Die Absorptionskolonne bestand aus Edelstahl 1.4571. Der Kolonneninnendurchmesser betrug 80 mm, die Wanddicke 4 mm und die Kolonnenlänge 1,70 m.

Etwa 70 % des Volumens der Absorptionskolonne waren mit Packungselementen der Fa. Montz (Montz BSH-750, spezifische Oberfläche 750 $m^2/m^3$) als trennwirksame Einbauten gefüllt. Die Packungselemente folgten unmittelbar aufeinander und begannen auf der Höhe von 1/5 der Kolonnenlänge von unten. Die Absorptionskolonne war von außen weder gekühlt noch beheizt. Am Kolonnenkopf wurde mit einer Aufgabetemperatur von 30°C technisches Tetradecan der Fa. Haltermann, DE, vom Typ PKWF 4/7 af als Absorptionsmittel aufgegeben (gaschromatographische Analyse mittels FID-Detektion ergab zu Beginn (frisch) nachfolgende GC-Flächen% Zusammensetzung:

n-Tridecan 7,6 %,
n-Tetradecan 47,3 %,
n-Pentadecan 7,0 %,
n-Hexadecan 3,2 %,
n-Heptadecan 14,1 % und
Restsumme 20,7 %;

diese Zusammensetzung änderte sich im Verlauf (nach ca. 3000 h$^{-1}$) des kontinuierlichen Verfahrensbetriebs auf folgende Werte:

n-Tridecan 2,6 %,
n-Tetradecan 39,5 %,
n-Pentadecan 9,4 %,
n-Hexadecan 4,8 %,
n-Heptadecan 23,4 und
Restsumme 20,3 %).

Die Berieselungsdichte lag bei 15 m$^3$ Absorptionsmittel pro m$^2$ freie Querschnittsfläche und Stunden (= 28 kg/h Tetradecan).

Der aus der Absorptionskolonne zur Verbrennung geführte Abgasstrom enthielt noch 950 Vol.-ppm Propan und 250 Vol.-ppm Propen. Großtechnisch wird dieser Abgasstrom über einen Expander (z.B. eine Expansionsturbine) in die Verbrennung geführt, um den Großteil der in der zweistufigen Verdichtung aufgewendeten Verdichtungsleistung rückzugewinnen und in die zweistufige Verdichtung rückzuführen. In zweckmäßiger Weise wird die Expansion auch zweistufig durchgeführt, um unerwünschte Kondensation zu vermeiden. Die bei der Entspannung gewonnene mechanische Energie kann sowohl direkt als Mit- oder Hauptantrieb für einen der Verdichter und/oder zur Erzeugung von Strom genutzt werden.

Bevor das entspannte Absorberabgas zur Verbrennung geführt wird, kann es großtechnisch zweckmäßig sein, den darin enthaltenen Wasserstoff abzutrennen. Dies kann z.B. dadurch erfolgen, dass man das Abgas über eine, in der Regel zu einem Rohr gestaltete, Membran leitet, die lediglich für den molekularen Wasserstoff durchlässig ist. Der so abgetrennte molekulare Wasserstoff kann z.B. in die heterogen katalysierte Dehydrierung rückgeführt oder einer sonstigen Verwertung (z.B. in Brennstoffzellen) zugeführt werden.

Prinzipiell kann die Wasserstoffabtrennung auch durch partielle Kondensation, Adsorption und/oder Rektifikation (vorzugsweise unter Druck) vorgenommen werden. Großtechnisch ist es zu dem in der Regel zweckmäßig, das Absorberabgas durch das im weiteren noch zu beschreibende Sauerwasser zu führen, um dieses einzuengen.

Das Absorbat enthielt folgende Gehalte (Gew.-% bezogen auf das Gewicht des Absorbats):

|  | Gew.-% |
|---|---|
| Stickstoff | 0,147 |
| Propan | 4,58 |
| Propen | 0,915 |
| Ethan | 0,07 |
| n-Butan | 0,015 |
| iso-Butan | 0,023 |
| n-Butene | 0,009 |
| iso-Buten | 0,024 |
| Kohlendioxid | 0,086 |
| Ethen | 0,000 |
| Tetradekan | ca. Rest bis zu 100 Gew.-% |

Bevor das Absorbat zum Kopf der nachfolgenden Desorptionskolonne geführt wurde, wurde es in einem indirekten Wärmeaustauscher auf 60°C erwärmt.

Anschließend wurde das Absorbat (dies kann z.B. in einer inversen Pumpe oder mittels eines Ventils ausgeführt werden) auf einen Druck von 2,7 bar entspannt (die im Fall der inversen Pumpe dabei frei gesetzte mechanische Energie wird zweckmäßig zur Rückverdichtung von in der Desorptionskolonne befreitem Absorptionsmittel mitverwendet) und das dabei erzeugte zweiphasige Gemisch am Kopf in die Desorptionskolonne geführt.

In die Desorptionskolonne (Innendurchmesser = 80 mm, Länge = 1,70 m, Wanddicke = 4 mm) wurde von unten im Gegenstrom zum vom Desorptionskolonnenkopf ablaufenden Absorbat mit einem Druck von 3 bar Luft geführt (1269 Nl/h). Die am Kopf aufgegebene Absorbatmenge betrug 33,7 l/h. Das unten aus der Desorptionskolonne herausgeführte, im wesentlichen von desorbierten Komponenten befreite, Absorptionsmittel wurde durch indirekten Wärmeaustausch gekühlt, auf den in der Absorptionskolonne geforderten Druck verdichtet, durch nochmaligen indirekten Wärmeaustausch (großtechnisch zweckmäßig mit Oberflächenwasser) auf 16°C gekühlt und dann zum Kopf der Absorptionskolonne rückgeführt. Als trennwirksame Einbauten enthielt die Desorptionskolonne strukturierte Blechpackungen der Fa. Montz (Montz BSH-750, spezifische Oberfläche 750 $m^2/m^3$). Um Absorptionsmittel zurückzuhalten wurde der aus der Desorptionskolonne (gegebenenfalls über einen mechanischen Tropfenabscheider) geführte Gasstrom mit Wasser gewaschen. D.h., er wurde durch ein Packungselement der Fa. Montz (Montz BSH-750, spezifische Oberfläche 750 $m^2/m^3$) geführt, auf das Wasser (70 l/h) mit einer Temperatur von 18 °C im Gegenstrom aufgegeben wurde. Unterhalb der Packung war ein Fangboden (Kaminboden) angebracht, von dem die wässrige Phase herausgeführt werden konnte. In einem Phasenscheider wurde in eine wässrige Phase und in eine organische Phase getrennt. Die sehr geringe Menge organische Phase wurde mit dem zum Kopf der Absorptionskolonne rückgeführten Absorptionsmittelstrom vereinigt. Die wässrige Phase wurde rückgekühlt und mit Frischwasser ergänzt (um Verdampfungsverluste auszugleichen) wieder auf das Packungselement aufgegeben. Das Waschen erfolgte der Desorptionskolonne aufgesetzt.

Aus dem Waschabschnitt wurde der gewaschene Gasstrom über mechanische Tropfenabscheider (abgeschiedene Flüssigphase wird in die Wäsche rückgeführt) mit den nachfolgenden Gehalten herausgeführt (wird in der Reaktionszone A der Dehydrierumsatz höher gewählt, kann der nachfolgende Propengehalt auch bei 8 bis 12 Vol.-% liegen; beispielsweise kann der gewaschene Gasstrom auch 15 Vol.-% Propan, 10 Vol.-% Propen und 14,5 Vol.-% $O_2$ aufweisen):

|  | Vol.-% |
| --- | --- |
| Stickstoff | 46,69 |
| Sauerstoff | 11,84 |
| Propan | 32,53 |
| Propen | 6,81 |
| Ethan | 0,07 |
| n-Butan | 0,08 |
| iso-Butan | 0,12 |
| n-Butene | 0,05 |
| iso-Buten | 0,13 |
| Wasserstoff | 0,07 |
| Kohlenmonoxid | 0,00 |
| Kohlendioxid | 0,61 |
| Wasser | 1,00 |
| Ethen | 0,00 |

Die Temperatur des Gasgemischs wurde durch indirekten Wärmeaustausch auf 250°C erhöht und das Gasgemisch mit der vorgenannten Zusammensetzung in einer Menge von 2128 Nl/l·h und einem Eingangsdruck von 2,1 bar als neues Reaktionsgasausgangsgemisch in die nachfolgende Partialoxidationsvorrichtung geführt.

C) Gestaltung der Reaktionszone B (der Partialoxidationsstufe)

1. Erster Festbettreaktor für den Schritt der Partialoxidation des Propens (Propylens) zu Acrolein

| Verwendetes Wärmeaustauschmittel: | Salzschmelze, bestehend aus |
| --- | --- |
|  | 53 Gew.-% Kaliumnitrat, |
|  | 40 Gew.-% Nariumnitrit und |
|  | 7 Gew.-% Natriumnitrat. |

(fortgesetzt)

| Abmessung des Kontaktrohres: | 4200 mm Gesamtlänge, |
| --- | --- |
| | 26 mm Innendurchmesser, |
| | 30 mm Außendurchmesser, |
| | 2 mm Wandstärke. |
| Reaktor: | Besteht aus einem doppelwandigen Zylinder aus Edelstahl (zylindrisches Führungsrohr, umgeben von einem zylindrischen Außenbehälter). Die Wanddicken betrugen überall 2 bis 5 mm. |
| | Der Innendurchmesser des äußeren Zylinders betrug 168 mm. Der Innendurchmesser des Führungsrohres betrug ca. 60 mm. |
| | Oben und unten war der doppelwandige Zylinder durch einen Deckel beziehungsweise Boden abgeschlossen. |
| | Das Kontaktrohr war durch das Führungsrohr geführt im zylindrischen Behälter so untergebracht, dass es am oberen bzw. unteren Ende desselben (abgedichtet) durch den Deckel bzw. Boden jeweils um 250 mm herausgeführt war. |
| | Das Wärmeaustauschmittel war im zylindrischen Behälter eingeschlossen. Um über die gesamte im zylindrischen Behälter befindliche Kontaktrohrlänge (3700 mm) möglichst gleichmäßige thermische Randbedingungen an der Außenwand des Kontaktrohres zu gewährleisten, wurde das Wärmeaustauschmittel durch Einperlen von Stickstoff im zylindrischen Behälter umgewälzt. |
| | Mittels des aufsteigenden Stickstoffs wurde das Wärmeaustauschmittel im zylindrischen Führungsrohr von unten nach oben befördert, um dann im Zwischenraum zwischen zylindrischem Führungsrohr und zylindrischem Außenbehälter wieder nach unten zu strömen (eine Umwälzung gleicher Güte kann auch durch Umpumpen (z.B. Propellerpumpen) erreicht werden). Durch eine auf den Außenmantel aufgebrachte elektrische Heizung könnte die Temperatur des Wärmeaustauschmittels auf das gewünschte Niveau geregelt werden. Im übrigen bestand Luftkühlung. |
| Reaktorbeschickung: | Über den Reaktor betrachtet wurden Salzschmelze und Reaktionsgasgemisch im Gegenstrom geführt. Das Reaktionsgasgemisch trat oben in den Reaktor ein. Es wurde jeweils mit einer Temperatur von 250°C ins Reaktionsrohr geführt. |
| | Die Salzschmelze trat unten mit einer Temperatur $T^{ein}$ = 335°C in das zylindrische Führungsrohr ein und oben mit einer Temperatur $T^{aus}$ aus dem zylindrischen Führungsrohr aus. Der Unterschied zwischen $T^{ein}$ und $T^{aus}$ betrug etwa 2°C. $T^{mittel} = (T^{ein} + T^{aus})/2$. |
| Kontaktrohrbeschickung: (von oben nach unten) | Abschnitt A: 50 cm Länge <br> Vorschüttung aus Steatit-Ringen (Steatit C 220 der Fa. Ceram.Tec) der Geometrie 7 mm x 7 mm x 4 mm (Außendurchmesser x Länge x Innendurchmesser). |
| | Abschnitt B: 100 cm Länge <br> Kontaktrohrbeschickung mit einem homogenen Gemisch aus 30 Gew.-% an Steatit-Ringen (Steatit C 220 der Fa. CeramTec) der Geometrie 5 mm x 3 mm x 2 mm (Außendurchmesser x Länge x Innendurchmessser) und 70 Gew.-% Vollkatalysator aus Abschnitt C. |

(fortgesetzt)

Abschnitt C: 170 cm Länge
Katalysatorbeschickung mit ringförmigem (5 mm x 3 mm x 2 mm = Außendurchmesser x Länge x Innendurchmesser) Vollkatalysator gemäß Beispiel 1 der DE-A 10046957.

Abschnitt D: 50 cm Länge
Nachschüttung aus Steatit-Ringen (Steatit C 220 der Fa. CeramTec) der Geometrie 7 mm x 7 mm x 4 mm (Außendurchmesser x Länge x Innendurchmesser).

2. Zwischenkühlung und Sauerstoffzwischeneinspeisung

Das den ersten Festbettreaktor verlassende Produktgasgemisch wurde zum Zweck der Zwischenkühlung (indirekt mittels Luft) durch ein Verbindungsrohr (Länge = 400 mm, Innendurchmesser = 26 mm, Wanddicke = 2 mm, Material = Edelstahl) geführt, das, auf einer Länge von 200 mm zentriert untergebracht, mit einer Inertschüttung aus Steatitkugeln (Steatit der fa. CeramTec) des Durchmessers 5 bis 6 mm beschickt und unmittelbar an das Kontraktrohr des ersten Festbettreaktors angeflanscht war.

Das Gasgemisch trat mit einer Temperatur von mehr als 310°C in das Verbindungsrohr ein und verließ es mit einer Temperatur von etwa 140°C. Anschließend wurden dem Gasgemisch als Sauerstoffquelle 269 NI/h an komprimierter Luft zugemischt.

Das dabei resultierende, an einem statischen Mischer vermischte, Beschickungsgasgemisch wurde mit einer Temperatur von 220°C dem Festbettreaktor für den Schritt der Partialoxidation des Acroleins zu Acrylsäure zugeführt.

3. Zweiter Festbettreaktor für den Schritt der Partialoxidation des Acroleins zu Acrylsäure

Es wurde ein Festbettreaktor verwendet, der mit jenem für den ersten Schritt baugleich war. Salzschmelze und Reaktionsgasgemisch wurden über den Reaktor betrachtet im Gleichstrom geführt. Die Salzschmelze trat unten ein, das Reaktionsgasgemisch ebenfalls.

Die Kontaktrohrbeschickung (von unten nach oben) war:

Abschnitt A: 20 cm Länge
Vorschüttung aus Steatit-Ringen (Steatit C 220 der Fa. CeramTec) der Geometrie 7 mm x 7 mm x 4 mm (Außendurchmesser x Länge x Innendurchmesser).

Abschnitt B: 100 cm Länge
Katalysatorbeschickung mit einem homogenen Gemisch aus 30 Gew.-% an Steatit-Ringen (Steatit C 220 der Fa. CeramTec) der Geometrie 7 mm x 3 mm x 4 mm
(Außendurchmesser x Länge x Innendurchmesser) und 70 Gew.-% Schalenkatalysator aus Abschnitt C.

Abschnitt C: 200 cm Länge
Katalysatorbeschickung mit ringförmigem
(7 mm x 3 mm x 4 mm = Außerdurchmesser x Länge x Innendurchmesser) Schalenkatalysator gemäß Herstellungsbeispiel 5 der DE-A 10046928 (an dieser Stelle können auch dazu analoge und in entsprechender Weise hergestellte Schalenkatalysatoren eingesetzt werden, deren Aktivmasse jedoch eine Stöchiometrie von $Mo_{12}V_{2,8}W_{1,2}Cu_{2,4}O_x$ oder von $Mo_{12}V_{3,5}W_{1,3}Cu_{2,4}O_x$ aufweist).

Abschnitt D: 50 cm Länge
Nachschüttung aus Steatit-Ringen (Steatit C 220 der Fa. CeramTec) der Geometrie 7 mm x 7 mm x 4 mm (Außendurchmesser x Länge x Innendurchmesser).

Der zweite Reaktor wurde mit ca. 3607 g/h an Beschickungsgasgemisch belastet. $T^{mittel}$ ist wie für den ersten Festbettreaktor definiert und betrug 284°C.

Der Propenumsatz im ersten Reaktor betrug 98,0 mol-% und der Acroleinumsatz im zweiten Reaktor betrug ca. 99,8 mol-%.

Die Gehalte des den zweiten Festbettreaktor mit einer Temperatur von 281 °C und einem Druck von 1,8 bar verlassenden Produktgasgemisches (Produktgasgemisch B) waren:

|  | Vol.-% |
|---|---|
| Stickstoff | 51,54 |
| Sauerstoff | 2,3 |
| Propan | 29,20 |
| Propen | 0,110 |
| Methan | 0 |
| Ethan | 0,077 |
| n-Butan | 0,101 |
| iso-Butan | 0,236 |
| n-Butene | 0 |
| iso-Buten | 0,001 |
| 1,3-Butadien | 0,009 |
| Wasserstoff | 0,05 |
| Kohlenmonoxid | 0,596 |
| Kohlendioxid | 1,72 |
| Wasser | 8,21 |
| Acrolein | 0,009 |
| Acrylsäure | 5,28 |
| Essigsäure | 0,240 |
| Propionsäure | 0,002 |
| Ameisensäure | 0,019 |
| Formaldehyd | 0,198 |
| Benzaldehyd | 0,005 |
| Maleinsäureanhydrid | 0,047 |
| Methacrolein | 0,020 |
| Methacrylsäure | 0,011 |
| Ethen | 0,032 |

Die in den beiden Reaktionsstufen verwendeten Katalysatoren können auch durch die in den Beispielen der DE-A 10351269 verwendeten Katalysatoren ersetzt werden. Der Katalysator der ersten Reaktionsstufe kann auch durch die Katalysatoren der Beispiele und Vergleichsbeispiele der DE-A 10344149 ersetzt werden. Der Katalysator der zweiten Reaktionsstufe kann auch durch die Katalysatoren der Beispiele der DE-A 10360057 und DE-A 10360058 ersetzt werden. Ferner kann die Partialoxidation als Hochlastverfahren durchgeführt werden, wie es in der DE-A 10313210, der DE-A 10313213, der DE-A 10313212, der DE-A 10313211, der DE-A 10313208, der DE-A 10313209 und in der DE-A 10313214 und dem in diesen Schriften gewürdigten Stand der Technik beschrieben ist.

D) Gestaltung der Trennzone B (Abtrennung des Zielproduktes Acrylsäure aus dem Produktgasgemisch der Partialoxidation)

In einem Direktkühler wurde der heiße Produktgasgemischstrom (das Produktgasgemisch B) im Gleichstrom (nach Vereinigung mit dem weiter unten beschriebenen beladenen Stripgasstrom) durch Eindüsen einer Quenchflüssigkeit (enthaltend 57,4 Gew.-% Diphenylether, 20,7 Gew.-% Diphenyl, 20 Gew.-% o-Dimethylphthalat (DMP) und als Restmenge bis zu 100 Gew.-% Phenothiazin (bis zu 1 Gew.-%) sowie Hochsieder (z.B. Acrylsäureoligomere)) der Temperatur 140 bis 150°C, die auch als Absorptionsmittel verwendet wurde, auf ca. 180°C abgekühlt. Im unteren Teil des Direktkühlers wurde die nicht verdampfte Quenchflüssigkeit gesammelt, entnommen und über einen Wärmeaustauscher, in welchem sie wieder auf Vorlauftemperatur gebracht wurde, in den oberen Teil des Quenchgefäßes rückgesprüht. Der Produktgasgemischstrom B (vereint mit dem beladenen Stripgas) und die zu Strahlen verdüste Quenchflüssigkeit wurden dem Direktkühler parallel zugeführt. Der abgekühlte Produktgasgemischstrom B strömte dann in die nachfolgende Absorptionskolonne. Im Quenchumlauf reicherten sich hochsiedende Nebenprodukte an, die ausgeschleust werden mussten. Aus dem Quenchumlauf wurde deshalb einmal je Tag diskontinuierlich eine Teilmenge von 1 kg Quenchflüssigkeit in einen Behälter abgelassen, der mit Hilfe eines Rotationsverdampfers (8 mbar, 180°C) diskontinuierlich aufgearbeitet wurde. Das dabei gewonnene gereinigte Absorptionsmittelkondensat wurde dem weiter unten beschriebenen Pufferbehälter für den Sumpfablauf aus der Restgas-Edelstahl-Druck-Waschkolonne zugeführt und dabei einmal je Woche die beim Aufarbeiten im Rotationsverdampfer erlittenen Verluste an Absorptionsmittel von rund 2,9 g/h durch Zusatz von frischem Gemisch bestehend aus Diphenylether (21,2 Gew-%), Diphenyl (58,8 Gew-%) (beide zusammen = Diphyl®) und o-Dimethylphthalat (20 Gew.-%) zum Kondensat ersetzt.

Das abgekühlte Produktgasgemisch B wurde oberhalb des Sumpfes und unterhalb der ersten Packung in die Absorptionskolonne geführt. Die Absorptionskolonne (ohne Sumpf) war 5420 mm lang, hatte einen Innendurchmesser von 50 mm und enthielt auf insgesamt 3,3 m Länge Kuehni (CH) Rhombopak 9M Packungen.

Die Absorptionskolonne war aus Glas gefertigt und wies aus Gründen der Temperierung einen segmentierten Glasdoppelmantel auf. Unmittelbar oberhalb der zweiten Packung, von unten betrachtet, war ein Druckentkopplungsventil installiert (eine einstellbare Drossel), mit dessen Hilfe ein Druckverlust über die Absorptionskolonne (wie er großtechnisch auftreten würde) von 100 bis 300 mbar simuliert werden konnte. Die Absorptionskolonne wurde mit einem Kopfdruck von 1,30 bar und einem Sumpfdruck von 1,40 bar betrieben. Die Sumpftemperatur betrug ca. 113°C. Der Glasdoppelmantel war in fünf aufeinanderfolgende getrennt betriebene Segmente unterteilt, über die der Absorptionskolonne das nachfolgende Temperaturprofil aufgeprägt wurde. Dieser Segmentierung folgte auch die innere Struktur der Absorptionskolonne.

Die Segmente wiesen von unten nach oben die folgenden Temperaturen auf (ihre Thermostatisierung erfolgte jeweils mittels umgepumptem Wasser, bzw. im 1.Segment mit Wärmeträgeröl der entsprechenden Temperatur) und waren wie folgt gestaltet:

1. Segment: 1020 mm Länge (nach oben gerichtet an der Zufuhrstelle des Produktgasgemischs B beginnend), 113°C, 0,4 m Rhombopak zwischen der unmittelbar oberhalb des Sumpfes der Absorptionskolonne befindlichen Zufuhrstelle des Produktgasgemischs B und der Zufuhrstelle von aus dem Kolonnensumpf kontinuierlich entnommener und in die Kolonne rezierkulierter (mit ca. 113 °C, in einer Menge von ca. 200 l/h) Sumpfflüssigkeit und 0,6 m Rhombopak oberhalb dieser Aufgabestelle. Der Abstand zwischen beiden Rhombopaks betrug ca. 2 cm.

2. Segment: 1250 mm Länge, 75°C, am unteren Ende befand sich das Druckentkopplungsventil und oberhalb des Druckentkopplungsventils bis zur Zufuhrstelle des Leichtsiederkondensatstroms aus der weiter unten beschriebenen Reindestillation befanden sich 0,5 m Rhombopak.

3. Segment: 950 mm Länge, 60°C, 0,6 m Rhombopak, die unmittelbar oberhalb der Leichtsiederkondensataufgabestelle des 2. Segments beginnen.

4. Segment: 950 mm Länge, 50-55°C, 0,6 m Rhombopak, die unmittelbar unterhalb der Aufgabestelle des im folgenden beschriebenen Absorptionsmittelhauptstroms enden.

5. Segment: 1250 mm Länge, 20°C, 0,6m Rhombopak zwischen dem unmittelbar oberhalb der Absorptionsmittelaufgabe im 4. Segment für den Ablauf des Sauerwassers (im folgenden beschrieben) angebrachten Fangbodens und der Aufgabestelle von rezierkuliertem Sauerwasser am Kopf der Absorptionskolonne.

Vom vorgenannten im 5.Segment befindlichen Fangboden wurde kontinuierlich wasserreiches Kondensat (Sauerwasser) abgezogen (ca. 70,2 l/h). Es wurde über einen Wärmetauscher indirekt auf 20°C abgekühlt und überwiegend oberhalb der obersten Rhombopak-Packung wieder in die Absorptionskolonne rückgeführt (70 Liter/h). Der nicht rückgeführte Anteil des entnommenen Sauerwassers (das Reaktionswasser) wurde standgeregelt ausgeschleust und einer Sauerwasserextraktionsstufe zugeführt. In dieser wurde das ausgeschleuste Sauerwasser in einem auf Umgebungstemperatur gehaltenen Glasrührgefäß (1 Liter Volumen, 80 mm Durchmesser) bei Umgebungsdruck mit dem kleineren Teilstrom des (weiter unten beschriebenen) gering beladenen (mit Acrylsäure und Nebenkomponenten) Absorptionsmittels vereinigt und gemischt. Die resultierende Mischung wurde in einen ebenfalls auf Umgebungstemperatur gehaltenen gläsernen Phasenscheidebehälter (7,5 Liter Volumen, 150 mm Durchmesser) durch freien Überlauf kontinuierlich abgelassen. Dort trennte sich das übergelaufene flüssige Gemisch in zwei flüssige Phasen, wobei kurzkettige saure Nebenkomponenten (z.B. Essigsäure) bevorzugt in die spezifisch leichtere wässrige Phase und Acrylsäure bevorzugt in die spezifisch schwerere organische Phase übergehen. Die organische Phase wird mittels einer zwangsfördernden Membrandosierpumpe mit dem grösseren Teilstrom des gering beladenen Absorptionsmittels vereinigt und dieser Gesamtstrom (ca. 3,0 l/h, 40°C) wie bereits beschrieben direkt unterhalb des Sauerwasserfangbodens als Absorptionsmittelhauptstrom der Absorptionskolonne zugeführt.

Das die Absorptionskolonne am Kopf verlassende (Haupt)Restgas (T = 20 °C, P = 1,30 bar) wies folgende Gehalte auf:

|  | Vol.-% |
|---|---|
| Stickstoff | 59,9 |
| Sauerstoff | 2,67 |
| Propan | 33,93 |
| Propen | 0,128 |
| Methan | 0 |
| Ethan | 0,09 |
| n-Butan | 0,118 |
| iso-Butan | 0,274 |
| n-Butene | 0 |
| iso-Buten | 0,001 |
| Butadiene | 0,010 |
| Wasserstoff | 0,058 |
| Kohlenmonoxid | 0,693 |
| Kohlendioxid | 1,97 |
| Acrolein | 0,009 |
| Ethen | 0,037 |
| alle vorstehenden Gehalte sind wasserfrei gerechnet |  |
| Wasser | 1,78 (bezogen auf alles) |

Durch indirekten Wärmetausch wurde das (Haupt)Restgas auf 40°C erwärmt (um unerwünschte Kondensation auszuschließen), mittels eines Membranverdichters (großtechnisch ein mittels Elektromotor angetriebener Turboverdichter, z.B. vom Typ 12MH4B, der Fa. Mannesmann DEMAG, DE) auf 2,70 bar verdichtet und etwa 78 Vol.-% als Kreisgas ((Gesamt)Restgas) zur Beschickung des ersten Dehydrierreaktors in die Reaktionszone A rückgeführt.

Etwa 22 Vol.-% des verdichteten (Haupt)Restgases,wurden unter Entspannung auf 1,6 bar abgezweigt und in einer Edelstahl-Druck-Waschkolonne (Werkstoff 1.4571, Innendurchmesser 30 mm, Füllung: 2 m Rhombopak 9M) bei ca. 1,6 bar und ca. 51 °C mit dem weiter unten beschriebenen Gesamtstrom an geringst beladenem Absorptionsmittel im Gegenstrom gewaschen (das geringst beladene Absorptionsmittel wurde oben aufgegeben und das Gas unten in die Kolonne geführt).

In einer weiteren Edelstahl-Druckkolonne (Werkstoff 1.4571, Innendurchmesser 30 mm, Füllung: 3 m Rhombopak 9M, Doppelmantel mit Öl temperiert = Strippkolonne) wurde dieser gewaschene Gasstrom bei ca. 1,5 bar und ca. 119 bis 125 °C genutzt, um aus dem beladenen Absorptionsmittelstrom, welcher aus dem Sumpf der Absorptionskolonne kontinuierlich abgezogen wurde (ca. 3,5 l/h, ca. 113°C), leichtsiedende Komponenten auszustrippen (z.B. so wie in der DE-A 10336386 beschrieben). Der dabei resultierende mit Leichtsiedern (leichter flüchtig als Acrylsäure) beladene Gasstrom (beladenes Strippgas) wurde in einer auf 170 °C temperierten Doppelmantelleitung (Durchmesser 15 mm, Aussenmantel Edelstahlwellschlauch) aufgeheizt und am Eingang des Quenchgefässes mit dem heissen Produktgasgemischstrom B vereinigt. Am unteren Ende der Edelstahl-Druck- Waschkolonne wurde der Absorptionsmittelstrom gering beladen stand geregelt in den bereits erwähnten Pufferbehälter (Glas, 5 Liter Volumen) abgelassen (in diesen Behälter wird, wie bereits beschrieben, auch das im Rotationsverdampfer anfallende Absorptionsmittelkondensat geleitet).

Vom Pufferbehälter wurde mittels einer Membranpumpe ein grösserer Teilstrom von 2,5 l/h an gering beladenem Absorptionsmittel zur Absorptionskolonne geführt und dort wie bereits beschrieben als Bestandteil des Absorptionsmittelhauptstroms unmittelbar unterhalb des Fangbodens für die Sauerwasserausschleusung im Segment 4 aufgegeben. Der kleinere Teilstrom von 460 ml/h an gering beladenem Absorptionsmittel wurde, wie ebenfalls bereits beschrieben, mittels einer weiteren Membranpumpe in das Glasrührgefäß der Sauerwasserextraktionsstufe geleitet.

Aus dem Sumpfumlauf (ca. 100 l/h, Druckluftmembranpumpe) der Strippkolonne wurden ca. 3,5 kg Sumpfflüssigkeit über einen Edelstahl-Gewebefilter und ein Regelventil stand geregelt abgezogen und zum Zweck der Reindestillation der Vakuumdestillationseinheit zugeführt.

Die Vakuumdestillationeinheit bestand aus einer verspiegelten und vakuumisolierten Glaskolonne (Reinkolonne) mit 50 mm Innendurchmesser und 4000 mm Länge. Mittels Zwangsentspannungssumpfumlauf (ca. 250 l/h, Peripheralradkreiselpumpe) wurde eine Sumpftemperatur von 191 °C aufrechterhalten (p = 4 bar). Der Absolutdruck im Sumpf betrug ca. 230 mbar, der Kopfdruck lag bei 100 mbar. Zum Zweck der Polymerisationsinhibierung wurden oberhalb des Sumpfspiegels 52 Nl/h Luft zugeführt.

Zwischen dem Sumpf der Vakuumdestillationskolonne und dem Zufluss des produktbeladenen Stroms aus dem Sumpfumlauf der Strippkolonne in die Vakuumdestillationskolonne waren zunächst 6 Glockenböden (Bodenabstand: 5 cm) und darüber weitere 15 Glockenböden (Bodenabstand: 5 cm) angebracht, oberhalb deren die Möglichkeit einer Probenahme mittels einer Miniatur-Membrandosierpumpe bestand.

Oberhalb dieser Probenahmestelle waren 10 Siebböden (je Boden 6 äquidistante Löcher mit Durchmesser 6,5 mm) angebracht (Bodenabstand: 5 cm), die sich nach oben bis zu einem Fangboden erstreckten, von dem kontinuierlich ca. 364 g/h gereinigte Acrylsäure als Zielpodukt ausgeschleust und nach Abkühlung in einem Vorratsbehälter gelagert wurden.

Nebenkomponentengehalte des Zielproduktes waren:

| | |
|---|---|
| Acrylsäure (Reinheit, ohne Inhibitorgehalt) | 99,54 Gew.-% |
| alle Nebenkomponentenanteile sind als auf die enthaltene Acrylsäure bezogene Gewichtsmengen angegeben | |
| Essigsäure | 0,186% |
| Propionsäure | 269 ppm |
| Maleinsäureanhydrid | 406 ppm |
| Formaldehyd | 344 ppm |
| Benzaldehyd | 186 ppm |
| Methacrylsäure | 1597 ppm |
| Wasser | 342 ppm |

Auf den obersten der unterhalb des Zielproduktabzugs befindlichen Siebböden wurde eine weitere Menge (732 ml/h) an vom Fangboden ausgetragener Acrylsäure unter Beibehalt ihrer Entnahmetemperatur von ca. 75 °C in die Destillationskolonne rückgeführt.

Oberhalb des Zielproduktaustrags befanden sich weitere 10 Siebböden (je Boden 6 Löcher mit Durchmesser 5,5 mm, Bodenabstand: 5 cm), die es gestatteten, noch vorhandene Leichtsieder zum Kolonnenkopf hin anzureichern. Oberhalb dieser Siebböden war ein weiterer Fangboden angebracht, um das Leichtsiederkondensat, welches durch indirekte Kühlung bedingte Kondensation im Kolonnenkopf (26 °C, 100 mbar Absolutdruck) anfällt, aufzufangen. Die Temperatur auf dem Fangboden betrug 73 °C. Das Leichtsiederkondensat enthielt:

| | |
|---|---|
| Acrylsäure | 98,42 Gew.-% |
| Essigsäure | 1,02 Gew.-% |
| Wasser | 0,427 Gew.-% |
| Methacrolein | 0,012 Gew.-% |
| Methacrylsäure | 0,021 Gew.-% |
| Diphyl | 0,009 Gew.-% |
| Propionsäure | 0,024Gew.-% |
| Furan-2-aldehyd | 0,010 Gew.-% |

(fortgesetzt)

| Allylacrylat | 0,009 Gew.-% |
|---|---|
| Acrolein | 0,002 Gew.-% |

Von dem vom Fangboden entnommenen Leichtsiederkondensatgesamtstrom wurde ein Hauptstrom von 570 ml/h unterhalb des Leichtsiederkondensatfangbodens als Rücklauf wieder in die Destillationskolonne eingespeist. Der dabei verbleibende Leichtsiederkondensatreststrom von 190 ml/h wurde auf 40°C abgekühlt und oberhalb des 2.Segments der Acrylsäureabsorptionskolonne selbiger zugeführt. Zur inhibierenden Wandbenetzung wurde im obersten Bereich des Reinkolonnenkopfs ein mit 0,5 Gew.-% Phenothiazin stabilisierter Zielproduktstrom von 51 ml/h mit der Temperatur 25°C über eine 4-Loch-Vollstrahldüse eingedüst.

Der am Kopf der Reinkolonne mittels einer Membranvakuumpumpe abgezogene Gasstrom bestand in der Hauptsache aus Inertgasen und Leichtsiedern. In einer auf 8 °C gekühlten Kühlfalle konnten aus ihm noch 4,6 g/h kondensierbare Leichtsieder- Restkomponenten abgetrennt wurden. Dieses flüssig abgeschiedene Restkomponentenkondensat enthielt:

| Acrylsäure | 89,65 Gew.-% |
|---|---|
| Essigsäure | 2,45 Gew.-% |
| Wasser | 7,24 Gew.-% |
| Methacrolein | 0,197 Gew.-% |
| Metharylsäure | 0,029 Gew.-% |
| Diphyl | 0,059 Gew.-% |
| Propionsäure | 0,020 Gew.-% |
| Furan-3-aldehyd | 0,021 Gew.-% |
| Allylacrylat | 0,007 Gew.-% |
| Acrolein | 0,037 Gew.-% |

Der abzüglich des Restkomponentenkondensats verbliebene "Inertgas"strom wies folgende Zusammensetzung auf:

| | Vol.-% |
|---|---|
| Stickstoff | Rest zu 100% |
| Sauerstoff | 1,20 |
| Propan | 18,8 |
| Propen | 0,08 |
| Methan | 0,004 |
| Ethan | 0,052 |
| n-Butan | 0,069 |
| iso-Butan | 0,146 |
| n-Butene | 0 |
| iso-Buten | 0,0 |
| Butadiene | 0,010 |
| Kohlenmonoxid | 0,372 |
| Kohlendioxid | 1,88 |
| Ethen | 0,022 |

Er wurde oberhalb des Druckentkopplungsventils und unterhalb der darüber befindlichen Rhomopak-Packung in das 2. Segment der Absorptionskolonne rückgeführt.

Am Sumpf der Reinkolonne wurde standgeregelt das von Acrysäure weitgehend befreite Absorptionsmittel abgezogen und als geringstbeladener Absorptionsmittelgesamtstrom zu der bereits beschriebenen Edelstahl-Druck-Waschkolonne geleitet, wobei die Druckerhöhung der Sumpfumlaufpumpe der Reinkolonne zum Austrag aus dem Vakuum genutzt wurde.

Das geringstbeladene Absorptionsmittel enthielt:

|  | Gew.-% |
|---|---|
| Essigsäure | 0,012 |
| Furan-2-aldehyd | 0,0000 |
| Propionsäure | 0,0000 |
| Benzaldehyd | 0,097 |
| Acrylsäure | 0,056 |
| Methacrylsäure | 0,017 |
| Diphyl | 77,5 |
| DMP | 20,0 |
| Benzoesäure | 0,642 |
| Di-Acrylsäure | 0,910 |
| Wasser | 0,0283 |

Zur Minderung von Eduktverlusten sowie zur Erreichung einer hohen Ausbeute wurden alle mit Edukt oder Zielprodukt beladenen Analysengasströme in einem Glasbehälter (0,5 Liter) zusammengeführt und mittels einer Kleinmembranpumpe der ersten Verdichterstufe der Trennzone A zugeführt und vor dem dortigen Verdichter mit dem abgekühlten Produktgasgemisch der Dehydrierstufe vereinigt und anschließend verdichtet. Bezogen auf eingesetztes Propan wurde so eine Ausbeute an Acrylsäure von 78,9 mol-% erzielt.

Die als Zielprodukt gewonnene Roh-Acrylsäure kann wie in der Schrift EP-A 616998 (unter Beachtung der Lehre der EP-A 912486) oder wie in der Schrift DE-A 19606877 (die Mutterlaugenrückführung kann in die Absorptions- und/oder in die Reinkolonne hinein erfolgen) oder wie in der Schrift EP-A 648732 beschrieben kristallisativ oder rektifikativ zu Reinacrylsäure weitergereinigt werden, die dann zur Herstellung von Wasser superabsorbierenden Polymerisaten in an sich bekannter Weise radikalisch polymerisiert werden kann. Sowohl die gewonnene Roh-Acrylsäure als auch die gewonnene Reinacrylsäure eignen sich in hervorragender Weise zu Herstellung von Estern der Acrylsäure, z.B. zur Herstellung von Alkylacrylaten.

Vergleichsbeispiel 4

Ähnlich wie in den Vergleichsbeispielen 1 und 3 wird in einer Reaktionszone A samt nachgeschalteter Trennzone A ein Reaktionsgasausgangsgemisch für eine Reaktionszone B erzeugt, das folgende Gehalte aufweist:

| Bestandteil | Gew.-% (bezogen auf Gesamtmenge) |
|---|---|
| Wasser | 1,10 |
| Tetradekan | 0,0101 |
| n-Butan | 0,091 |
| iso-Butan | 0,47 |
| 1-Buten | 0,054 |
| iso-Buten | 0,176 |
| Propan | 40,04 |
| Propen | 8,56 |

(fortgesetzt)

| Bestandteil | Gew.-% (bezogen auf Gesamtmenge) |
|---|---|
| $CO_2$ | 1,44 |
| $N_2$ | 36,9 |
| $O_2$ | 10,92 |
| Methan | 0,002 |
| Ethan | 0,204 |
| Ethylen | 0,034 |
| Wasserstoff | 0 |

Die Reaktionszone B besteht aus zwei parallel betriebenen Tandemreaktorsystemen (Reaktorstrassen) von denen jedes wie das Tandemreaktorsystem aus dem Ausführungsbeispiel der DE-A 103 51 269 aufgebaut und mit Katalysator beschickt ist. Jede der beiden Reaktionsstrassen wird mit 61022 $Nm^3$/h des vorgenannten Reaktionsgasausgangsgemischs beschickt. Die Zwischenlufteinspeisung zwischen den beiden Partialoxidationsstufen beträgt je Strasse 11200 $Nm^3$/h. In beiden Partialoxidationsstufen werden Reaktionsgas und Salzschmelze über den Reaktor betrachtet im Gegenstrom geführt.

Die Belastung der ersten Reaktionsstufe mit Propylen liegt bei etwa 110 NI/l•h. Die Eintrittstemperatur des Salzbads in der ersten Reaktionsstufe beträgt 346°C, die Austrittstemperatur des Salzbads aus der ersten Reaktionsstufe beträgt 349°C. Die Eintrittstemperatur des Salzbads in die zweite Reaktionsstufe beträgt 272°C. Die Austrittstemperatur des Salzbads aus der zweiten Reaktionsstufe beträgt 275°C. Das Reaktionsgasausgangsgemisch wird dem Erststufenreaktor mit 300°C zugeführt. Die Gaseintrittstemperatur in die zweite Reaktionsstufe beträgt 230°C. Die Salzschmelzepumpleistungen sind wie in der DE-A 103 51 269 angegeben. Der Eingangsdruck in die erste Reaktionsstufe beträgt 1,9 bar. Der Eingangsdruck in die zweite Reaktionsstufe beträgt 1,4 bar. Der Propylenumsatz in der ersten Reaktionsstufe beträgt 97 mol-% (bezogen auf Einmaldurchgang). Der Acroleinumsatz in der zweiten Reaktionsstufe beträgt 99,3 mol-% (bezogen auf Einmaldurchgang).

Die beiden Produktgasgemische verlassen die beiden zweiten Partialoxidationsstufen mit einer Temperatur von 275°C. Sie werden zu einem Gesamtstrom von 145000 $Nm^3$/h zusammengeführt, der folgende, auf seine Gesamtmenge bezogenen, Gehalte aufweist:

| | |
|---|---|
| Stickstoff | 42,75 Gew.-%, |
| Sauerstoff | 3,0 Gew.%, |
| Kohlenoxide | 3,11 Gew-%, |
| Wasser | 4,87 Gew.%, |
| Acrylsäure | 10,84 Gew.-%, |
| Essigsäure | 0,30 Gew.-%, |
| Acrolein | 0,06 Gew.-%, |
| Diphyl | 0 Gew.-%, |
| Ameisensäure | 0,03 Gew.-%, |
| Formaldehyd | 0,09 Gew.-%, |
| Propionsäure | 0,004 Gew.-%, |
| Furfurale | 0,003 Gew.-%, |
| Allylacrylat | 0,001 Gew-% |
| Benzaldehyd | 0,009 Gew.-%, |
| Maleinsäureanhydrid | 0,127 Gew-%, |
| Benzoesäure | 0,015 Gew.-%, |
| Methacrylsäure | 0,01 Gew-%, |
| Methacrolein | 0,015 Gew-%, |
| Phthalsäureanhydrid | 0,001 Gew-%, |
| Methan | 0,001 Gew-%, |
| Wasserstoff | 0 Gew-%, |
| Ethan | 0,17 Gew-%, |

(fortgesetzt)

| | |
|---|---|
| Ethen | 0,03 Gew-%, |
| Butane | 0,47 Gew-%, |
| Butene | 0,14 Gew-%, |
| Propan | 33,8 Gew-% und |
| Propen | 0,14 Gew.-%. |

Abkühlung des Produktgasgemischs B

Das Produktgasgemisch B wird in einen Direktkühler üblicher Bauart mit einem Durchmesser von 2,6 m und einer Höhe von 14,5 m geführt, der aus austenitischem Stahl (Werkstoff 1.4571) gerfertigt ist. Durch Teilverdampfung des Absorptionsmittels, das dem Sumpf der Absorptionskolonne entnommen wird, wird das Produktgasgemisch auf eine Temperatur von 165,5 °C abgekühlt. Das Kühlmedium wird dabei im Gleichstrom zum abzukühlenden Produktgasgemisch B geführt und über eine Prallplattendüse in den Direktkondensator aufgegeben.

Das abgekühlte Produktgasgemisch B wird zusammen mit dem nicht verdampften Absorptionsmittel in den Sumpf der nachfolgenden Absorptionseinheit geführt.

Absorptionseinheit

Die Absorptionseinheit ist als Bodenkolonne mit 45 Ventilböden (wobei die Ventildeckel der einzelnen Ventile nicht montiert sind) und 3 Kaminböden ausgeführt und wird mit einem Kopfdruck von 1,2 bar betrieben. Eine Ausführung mit Siebböden oder Regensiebböden als trennwirksame Einbauten in der Bodenkolonne ist ebenfalls denkbar.

Der unterste Boden der Absorptionskolonne ist als doppelwandiger Kaminboden ausgeführt. Unterhalb dieses Bodens wird das die Produktgasdirektkühlung verlassende Gasgemisch vom in der Kühlvorrichtung nicht verdampften Absorptionsmittel abgetrennt.

Das die Direktkühlung verlassende Gemisch aus nicht verdampftem Absorptionsmittel und Produktgasgemisch wird mit einem Tangentialimpuls in die Absorptionskolonne aufgegeben. Ein Zyklonring verhindert einen direkten Tropfenmitriss in die Kamine des untersten Bodens.

Die Sumpftemperatur der Absorptionseinheit beträgt 162,7 °C. Aus dem Sumpf der Absorptionseinheit werden 1067 m$^3$/h Sumpfflüssigkeit mit folgender Zusammensetzung abgezogen:

| | |
|---|---|
| Diphyl (Gemisch aus Diphenyl und Diphenylether im Gewichtsverhältnis 2,8:1) | 54,27 Gew.-%, |
| Dimethylphthalat | 36,97 Gew.%, |
| Diacrylsäure | 0,95 Gew.-%, |
| Acrylsäure | 4,75 Gew.-%, |
| Phenothiazin | 0,65 Gew.-%, |
| Phthalsäureanhydrid | 0,1 Gew.-%, |
| Benzoesäure | 1,87 Gew.-%, |
| Maleinsäureanhydrid | 0,24 Gew.-%, |
| Benzaldehyd | 0,11 Gew.-%, |
| Ameisensäure | 0,005 Gew.-%, |
| Acrolein | 0,002 Gew.-%, |
| Wasser | 0,01 Gew.-%, |
| Essigsäure | 0,04 Gew.-% und |
| Furfurale | 0,003 Gew.-%. |

Die Sumpfflüssigkeit (Flüssigkeitsablauf der Absorptionseinheit) wird überwiegend als Kühlmittel mit Hilfe einer Kreiselpumpe in die Direktkühlung des Produktgasgemischs B zurückgeführt. Eine Teilmenge von 0,3 % bezogen auf die Gesamtmenge, wird entnommen und einer Destillationseinheit zugeführt.

Der Zulauf des frischen bzw. gering beladenen Absorptionsmittels in die Absorptionskolonne erfolgt auf den 34. Boden von unten gezählt, unmittelbar unterhalb des 3. Kaminbodens. Dieser Zulauf an gering beladenem Absorptionsmittel besteht aus einem polymerisationsinhibitorhaltigen Teilstrom von 86,6 Gew.-% bezogen auf die Gesamtzulaufmenge, der aus der im folgenden noch zu beschreibenden zur Kreisgaswäsche eingesetzten Wascheinheit entspringt, und aus einem Teilstrom von 13,4 Gew.-% bezogen auf die Gesamtzulaufmenge aus der Sauerwasserextraktion.

Die Temperatur des Zulaufstroms beträgt 51,9 °C.

Vom untersten Boden der Absorptionseinheit, der als Fangboden (Kaminboden) ausgebildet ist, werden (als weiterer Flüssigkeitsablauf der Absorptionskolonne) 1728 m³/h des mit Acrylsäure beladenen Absorptionsmittels (des Absorbats) entnommen. Die Entnahmetemperatur beträgt 119,6 °C. Das über eine Kreiselpumpe abgezogenene Absorbat enthält die nachfolgenden Bestandteile (die Mengenanteile sind auf die Gesamtmenge bezogen) :

| | |
|---|---|
| Diphyl | 52,55 Gew.-%, |
| Dimethylphthalat | 12,11 Gew.%, |
| Diacrylsäure | 1,38 Gew.-%, |
| Acrylsäure | 30,1 Gew.-%, |
| Phenothiazin | 0,03 Gew.-%, |
| Phthalsäureanhydrid | 0,08 Gew.-%, |
| Benzoesäure | 1,22 Gew.-%, |
| Maleinsäureanhydrid | 1,06 Gew.-%, |
| Benzaldehyd | 0,61 Gew.-%, |
| Ameisensäure | 0,03 Gew.-%, |
| Acrolein | 0,007 Gew.-%, |
| Wasser | 0,20 Gew.-%, |
| Essigsäure | 0,28 Gew.-%, |
| Propan | 0,19 Gew-% |
| Propionsäure | 0,01 Gew.-%, |
| Formaldehyd | 0,001 Gew.-%, |
| Allylacrylat | 0,005 Gew.-%, |
| Furfurale | 0,02 Gew.-% und |
| Methacrylsäure | 0,07 Gew-%. |

11, 4 Gew.-% bezogen auf die Gesamtmenge des Absorbat wird einer Desorptionseinheit zugeführt, 3,1 Gew.-% bezogen auf die Gesamtmenge werden unmittelbar dem Sumpf der Absorptionskolonne zugeführt und 85,5 Gew.-% werden über in Reihe geschaltete Wärmetauscher, in welchen es um 15 K abgekühlt wird, auf den sechsten Boden von unten gezählt in die Absorptionseinheit rückgeführt.

Die im ersten als Rohrbündelwärmetauscher ausgeführten Wärmetauscher abgeführte Wärme wird zur Teilverdampfung des in der noch zu beschreibenden Kondensationseinheit anfallenden Sauerwassers verwendet. Die Austrittstemperatur des diesen Wärmetauscher verlassenden Absorbats beträgt 113,3 °C. Die weitere Abkühlung des Absorbats auf die Zulauftemperatur der Rückführung erfolgt in Luftkühlern. Die Böden zwischen dem ersten Kaminboden bis zu diesem Zulaufboden sind als 4-flutige Ventilböden (Stahl VV12 ohne Deckel) ausgeführt. Der Abstand zwischen zwei Böden beträgt 0,7 m. Die Böden oberhalb dieses Zulaufboden bis zum 13. Boden von unten gezählt sind als 2-flutige Ventilböden (Stahl VV12 ohne Deckel) ausgeführt und sind in einem Abstand von 0,5 m montiert.

Oberhalb des 13. Bodens von unten gezählt ist ein weiterer als doppelwandiger Kaminboden ausgeführter Fangboden angebracht und über eine Kreiselpumpe wird ein flüssiger Abzugsstrom mit einer Temperatur von 71,8 °C entnommen. 9 Gew.-% der an dieser Stelle entnommenen Gesamtmenge werden unmittelbar unterhalb dieses zweiten Fangbodens als Rücklauf aufgegeben, wobei 1 Gew.-% bis 10 Gew.-% bezogen auf diese Rücklaufmenge dazu genutzt werden, den zweiten Fangboden über Düsen zu besprühen. Die verbleibende abgezogene Menge wird mit Hilfe von Luftkühlern auf 59,4 °C abgekühlt und auf den 19. Boden von unten gezählt aufgegeben. Die Böden zwischen dem zweiten Fangboden bis zum 19. Boden von unten gezählt sind als 4-flutige Ventilböden (Stahl VV12 ohne Deckel) ausgeführt, wobei der Abstand zwischen den Böden 0,6 m beträgt.

In den Abzugstrom des zweiten Fangbodens wird auch der über den Kopf der nachfolgend noch zu beschreibenden Rektifikationseinheit entnommene acrylsäurehaltige Leichtsiederstrom eingespeist. Des weiteren eignet sich diese Stelle im großtechnischen Verfahren auch zur Einspeisung acrylsäurehaltiger Stoffströme, wie beispielsweise nicht spezifikationsgerechte rohe Acrylsäure oder acrylsäurehaltige Stoffströme aus anderen Verfahrenstufen, wie beispielsweise aus der Reinacrylsäureherstellung durch Destillation oder Kristallisation.

Die Böden oberhalb des 19. Bodens von unten gezählt bis zur Einspeisestelle des frischen bzw. gering beladenen Absorptionsmittels in die Absorptionskolonne sind als 2-flutige Ventilböden (Stahl VV12 ohne Deckel) mit einem Abstand zwischen den Böden von jeweils 0,5 m ausgeführt. Oberhalb des 34. Bodens von unten gezählt befindet sich ein dritter als doppelwandiger Kaminboden ausgeführter Fangboden.

Das verbleibende, nicht in das Absorptionsmittel absorbierte Restproduktgasgemisch, das vor allem Leichtsieder und Nichtkondensierbare enthält, wird in einer Kondensationseinheit abgekühlt, die oberhalb des dritten Fangbodens anschließt. Die Kondensationseinheit ist als Direktkühlung ausgeführt und enthält zehn 2-flutige Ventilböden (Stahl VV12 ohne Deckel), die einen Abstand zueinander von 0,5 m aufweisen.

Zur direkten Kühlung wird am dritten Fangboden bereits kondensierte wässrige Leichtsiederfraktion abgezogen. 2 Gew.-% bezogen auf die gesamte am dritten Fangboden abgezogene Menge des Sauerwasserkondensats werden mit einer Temperatur von 45 °C ausgeschleust und einer Sauerwasserextraktion zugeführt. Das ausgeschleuste Sauerwasser hat im wesentlichen folgende Gehalte:

| | |
|---|---|
| Acrylsäure | 4,02 Gew.-%, |
| Essigsäure | 5,04 Gew.%, |
| Wasser | 69,84 Gew.-%, |
| Diphyl | 12,95 Gew.-%, |
| Dimethylphthalat | 2,51 Gew.-%, |
| Formaldehyd | 0,02 Gew.-%, |
| Diacrylsäure | 0,2 Gew.-%, |
| Allylacrylat | 0,004 Gew.-%, |
| Furfurale | 0,02 Gew.-%, |
| Propionsäure | 0,0015 Gew.-%, |
| Ameisensäure | 0,62 Gew.-%, |
| Benzaldehyd | 0,84 Gew.-%, |
| Maleinsäureanhydrid | 0,05 Gew.-%, |
| Benzoesäure | 0,25 Gew.-%, |
| Maleinsäure | 1,40 Gew.-%, |
| Phthalsäureanhydrid | 0,01 Gew.-%, |
| Phenothiazin | 0,007 Gew.-% , |
| Acrolein | 0,02 Gew.-%., |
| Methacrylsäure | 0,05 Gew-%, |
| Methacrolein | 0,005 Gew-%, |
| Butane | 0,21 Gew-% und |
| Butene | 0,08 Gew-%. |

Das nicht ausgeschleuste Sauerwasserkondensat wird in einem Rohrbündelwärmetauscher auf 33 °C abgekühlt. 79 Gew.-% bezogen auf die Menge des auf 33°C abgekühlten Sauerwasserkondensats werden auf den 40. Boden von unten gezählt aufgegeben. Die verbleibende Menge des Sauerwasserkondensats wird in einem weiteren Wärmetauscher auf 16°C abgekühlt. Dieser Wärmetauscher ist energetisch mit der für die Verdampfung des flüssigen Propans notwendigen Verdampfereinheit gekoppelt.

Das bei der Sauerwasserkondensation gasförmig verbleibende Restgasgemisch verlässt die Absorptionseinheit mit einer Temperatur von 29°C und enthält im wesentlichen folgende Bestandteile:

| | |
|---|---|
| Stickstoff | 50,40 Gew.-%, |
| Sauerstoff | 3,57 Gew.%, |
| Kohlenoxide | 3,65 Gew-%, |
| Wasser | 1,46 Gew.%, |
| Essigsäure | 0,058 Gew.-%, |
| Acrylsäure | 0,041 Gew.-%, |
| Acrolein | 0,067 Gew.-%, |
| Diphyl | 0,008 Gew.-%, |
| Ameisensäure | 0,003 Gew.-%, |
| Ethan | 0,20 Gew-%, |
| Ethen | 0,03 Gew-%, |
| Butene | 0,15 Gew-%, |
| Butane | 0,55 Gew-%, |
| Propan | 39,62 Gew.-% und |

(fortgesetzt)

| | |
|---|---|
| Propen | 0,17 Gew.-%. |

Der bei der Sauerwasserkondensation verbleibende Restgasstrom (Kreisgas) wird über einen Demister aus der Kolonne geführt und in einem Rohrbündelwärmetauscher um 6 K überhitzt. Dadurch wird eine mögliche Kondensation in den Abgasgasleitungen vermieden. Dieser Restgasstrom wird durch einen elektrisch angetriebenen Turboverdichter auf einen Druck von 3,3 bar verdichtet.

Von diesem Restgasstrom werden 20 Vol.-% bezogen auf die Gesamtmenge des Restgasstroms als Strippgas in einer noch zu beschreibenden Waschkolonne verwendet. Der verbleibende Restgasstrom wird in die Reaktionszone A zur heterogen katalysierten Propandehydrierung rückgeführt.

Desorptionseinheit

Das vom untersten Fangboden der Absorptionseinheit effektiv ausgeschleuste Absorbat wird einer Desorptionseinheit zugeführt, um es in dieser von in ihm noch enthaltenen Leichtsiedern zu befreien. Das Absorbat wird zunächst in einem mit Dampf beheizten Rohrbündelwärmetauscher auf 130 °C erwärmt und dann einer Desorptionskolonne am Kopf derselben zugeführt. Dabei werden über Düsen eingesprühte 5 Gew.-% Absorbat, bezogen auf die Gesamtmenge des der Desorptionskolonne zugeführten Absorbats, dazu benutzt, die Kolonnenwandungen, das Mannloch sowie die Kolonnenhaube zu befeuchten.

Als Kolonneneinbauten werden in der Desorptionskolonne 38 Regensiebböden verwendet auf denen Wellenbrecher montiert sind. Dabei beträgt der Lochdurchmesser der Böden 30 mm von Boden 1 von unten gezählt bis zum Boden 9, 25 mm am Boden 10 und 20 mm von Boden 11 bis zum Kopf der Kolonne. Der Bodenabstand zwischen den einzelnen Böden beträgt 0,5 m. Der Kopfdruck der Desorptionskolonne beträgt 1,83 bar.

Die Wärmezufuhr im Sumpf der Kolonne erfolgt über zwei parallel betriebene Verdampfer mit Zwangsumlauf.

Als Strippgas wird der Desorptionskolonne ein Teilstrom des Restgasstroms vom Kopf der Absorptionseinheit, nachdem er in der noch zu beschreibenden Waschkolonne gewaschen wurde, unmittelbar im Sumpf zugeführt. Das Strippgas wird dabei vor Eintritt in die Desorptionskolonne mit einem Teilstrom der am Sumpf der Desorptionskolonne entnommenen und im Verdampfer erwärmten Sumpfflüssigkeit vermischt und in einem Rohrbündelwärmetauscher auf 120 °C vorgewärmt. Der Teilstrom der Sumpfflüssigkeit der mit dem Strippgas vermischt wird beträgt 9 Gew.-% bezogen auf die Gesamtmenge der abgezogenen Sumpfflüssigkeit. Wahlweise kann auf diese Vermischung mit erwärmter Sumpfflüssigkeit auch verzichtet werden.

Das im Kolonnensumpf der Desorptionskolonne anfallende mit Acrylsäure beladene Absorptionsmittel enthält:

| | |
|---|---|
| Diphyl | 62,8 Gew.-%, |
| Dimethylphthalat | 14,7 Gew.%, |
| Diacrylsäure | 1,8 Gew.-%, |
| Acrylsäure | 17,1 Gew.-%, |
| Essigsäure | 0,04 Gew.-%, |
| Ameisensäure | 0,004 Gew.-%, |
| Propionsäure | 0,006 Gew-%, |
| Phenothiazin | 0,04 Gew.-%, |
| Phthalsäureanhydrid | 0,1 Gew.-%, |
| Benzoesäure | 1,5 Gew.-%, |
| Maleinsäureanhydrid | 1,1 Gew.-%, |
| Methacrylsäure | 0,06 Gew-%, |
| Benzaldehyd | 0,62 Gew.-% und |
| Furfural | 0,02 Gew.-%. |

Den Zwangsumlaufverdampfern wird die aus dem Sumpf der Kolonne entnommene Sumpfflüssigkeit mit einer Temperatur von 131,5 °C zugeführt und auf 150 °C erwärmt.

Ein Teilstrom von 63 Gew.-% bezogen auf die Gesamtmenge der entnommenen Sumpfflüssigkeit wird in die Desorptionskolonne auf den 8. Boden von unten gezählt zurückgeführt.

Das am Kopf der Desorptionskolonne anfallende mit Leichtsiedern beladene Strippgas wird in den zur Abkühlung des Produktgasgemischs B benutzten Direktkühler zurückgeführt. Das rückgeführte beladene Strippgas beinhaltet auch den Flüssigkeitsmitriss des obersten Bodens der Desorptionskolonne und enthält im wesentlichen folgende Bestandteile:

| | |
|---|---|
| Stickstoff | 26,75 Gew.-%, |
| Sauerstoff | 1,89 Gew.%, |
| Kohlenoxide | 1,94 Gew.-%, |
| Acrylsäure | 36,84 Gew.-%, |
| Diphyl | 7,57 Gew-%, |
| Wasser | 0,95 Gew.-%, |
| Essigsäure | 0,56 Gew.-%, |
| Dimethylphthalat | 1,39 Gew.-%, |
| Formaldehyd | 0,0 Gew.-%, |
| Acrolein | 0,01 Gew.-%, |
| Ameisensäure | 0,05 Gew.-%, |
| Propionsäure | 0,01 Gew.-%, |
| Allylacrylat | 0,01 Gew.-%, |
| Diacrylsäure | 0,16 Gew.-%, |
| Phthalsäureanhydrid | 0,0 Gew.-%, |
| Benzoesäure | 0,15 Gew.-%, |
| Maleinsäureanhydrid | 0,47 Gew.-%, |
| Benzaldehyd | 0,27 Gew.-%, |
| Furfurale | 0,01 Gew.-%, |
| Ethan | 0,1 Gew-%, |
| Propan | 20,4 Gew-%, |
| Propen | 0,1 Gew-%, |
| Butene | 0,06 Gew-% und |
| Butane | 0,23 Gew-%. |

Rektifikationseinheit

Der aus der Desorptionseinheit nach den Zwangsumlaufverdampfern entnommene Teilstrom, der hauptsächlich aus Acrylsäure, Diphyl und Dimethylphthalat besteht, wird in eine einen Verstärkungs- und einen Abtriebsteil umfassende Rektifikationseinheit geführt.

Die Rektifikationseinheit besteht aus 43 Regensiebböden auf welchen Wellenbrecher montiert sind, wobei die Böden unterschiedliche Lochdurchmesser aufweisen: 50 mm vom 1. Boden von unten gezählt bis zum 10. Boden, 25 mm vom 11. Boden bis zum 13. Boden und 14 mm vom 14. Boden bis zum obersten Boden. Der Abstand zwischen den einzelnen Böden beträgt jeweils 0,4 m. Der Abstand zwischen 8. und 9. Boden von unten gezählt beträgt 1 m.

Die Kolonne wird mit einem Kopfdruck von 106 mbar betrieben. Der Zulauf des beladenen, teilweise von Leichtsiedern in der Desorptionseinheit befreiten Absorptionsmittels erfolgt über eine Ringleitung mit mehreren Stutzen auf den 8. Boden von unten gezählt.

Unterhalb des 1. Bodens wird über eine Ringleitung mit mehreren Einspeisestellen der Kolonne 430 Nm$^3$/h Luft zugeführt.

Die Wärmezufuhr im Kolonnensumpf erfolgt über zwei parallel betriebene außenliegende Umlaufverdampfer mit Zwangsumlauf. Die Sumpftemperatur der Rektifikationseinheit beträgt typisch 188°C. Die in den Sumpf der Rektifikationsolonne kondensierende Schwersiederfraktion enthält im wesentlichen folgende Bestandteile:

| | |
|---|---|
| Diphyl | 75,6 Gew.-%, |
| Dimethylphthalat | 17,7 Gew.%, |
| Diacrylsäure | 1,85 Gew.-%, |
| Acrylsäure | 0,79 Gew.-%, |
| Phenothiazin | 0,05 Gew.-%, |
| Phthalsäureanhydrid | 0,1 Gew.-%, |
| Benzoesäure | 1,75 Gew.-%, |
| Maleinsäureanhydrid | 1,3 Gew.-%, |
| Methacrylsäure | 0,06 Gew.-%, |

(fortgesetzt)

| | |
|---|---|
| Benzaldehyd | 0,74 Gew.-% und |
| Furfurale | 0,015 Gew.-%. |

Die der Rektifikationseinheit entnommene, das schwersiedende Absorptionsmittel einkodensiert enthaltende, Sumpfflüssigkeit wird zu 83 Gew.-%, bezogen auf den Zulauf in die Rektifikationseinheit, ausgeschleust, und teilweise über einen Wärmetauscher in den Sumpfbereich der Rektifikationskolonne zurückgeführt. Die ausgeschleuste Schwersiederfraktion wird über einen Feststoffabscheider (Zyklon) und gegebenenfalls durch frisches Absorptionsmittel (Diphyl und Dimethylphthalt) ergänzt, der Wascheinheit zugeführt. Ein kleiner Teilstrom von 1,2 Gew-% bezogen auf die Gesamtmenge der ausgeschleusten Sumpfflüssigkeit wird dem Sumpfbereich der Absorption zugeführt.

27 Böden oberhalb des Zulaufs in die Rektifikationskolonne wird über Seitenabzug eine rohe Acrylsäure entnommen. Die Entnahme der rohen Acrylsäure erfolgt über einen Regensiebboden mit integrierter Mittelabzugstasse. Die entnommene rohe Acrylsäure enthält im wesentlichen folgende Bestandteile:

| | |
|---|---|
| Acrylsäure | 99,66 Gew.-%, |
| Essigsäure | 0,135 Gew.%, |
| Wasser | 0,007 Gew.-%, |
| Ameisensäure | 0,002 Gew.-%, |
| Propionsäure | 0,036 Gew.-%, |
| Furfurale | 0,022 Gew.-%, |
| Allyacrylat | 0,011 Gew.-%, |
| Benzaldehyd | 0,008 Gew.-%, |
| Maleinsäureanhydrid | 0,008 Gew.-%, |
| Methacrylsäure | 0,06 Gew-%, |
| Diacrylsäure | 0,02 Gew.-% und |
| Phenothiazin | 0,025 Gew.-%. |

Die entnommene rohe Acrylsäure wird mittels zweier in Reihe geschalteter Wärmetauscher auf 25°C abgekühlt. Von der entnommenen rohen Acrylsäure werden 15,5 Gew.-% bezogen auf den Zulauf in die Rektifikationskolonne ausgeschleust und ein kleinerer Teilstrom wird als Lösemittel für den Polymerisationsinhibitor verwendet.

Die Abkühlung des am Kopf der Rektifikationskolonne abgetrennten Leichtsiederstroms erfolgt zweistufig durch zwei Direktkühler. Beide Stufen sind als Gleichstromquench ausgeführt, wobei das Kondensat der ersten Stufe eine Temperatur von 52 °C und das Kondensat der zweiten Stufe eine Temperatur von 24,5 °C hat. Die Brüdenleitung der zweiten Kondensationsstufe führt auf die Vakuumeinheit. Dabei werden zwei Flüssigkeitsringpumpen parallel betrieben, wobei gemäß DE-A-10143565 als Sperrflüssigkeit das Kondensat aus der zweiten Kondensationsstufe eingesetzt wird. In einem nachfolgenden Behälter wird die flüssige Phase, in erster Linie die Ringflüssigkeit, von den nicht kondensierbaren Anteilen getrennt, die das Abgas der Rektifikationseinheit bilden. Dieses hat im wesentlichen folgende Zusammensetzung:

| | |
|---|---|
| Stickstoff | 48 Gew.-%, |
| Sauerstoff | 14,5 Gew.%, |
| Acrylsäure | 2,3 Gew.-%, |
| Essigsäure | 0,09 Gew.-%, |
| Wasser | 0,25 Gew.-%, |
| Acrolein | 0,001 Gew.-%, |
| Ameisensäure | 0,04 Gew.-%, |
| Allylacrylat | 0,004 Gew.-%, |
| Kohlenoxide | 0,03 Gew.-%, |
| Propan | 33,65 Gew-%, |
| Propen | 0,03 Gew-%, |
| Butane | 0,85 Gew-% und |
| Butene | 0,21 Gew-%. |

Das Abgas der Rektifikationseinheit wird zusammen mit anderen Produktionsrückständen verbrannt. Bei hohen Propangehalten dieses Vakuumabgas ist es jedoch auch denkbar, diesen Strom in die Absorption zurückzuführen.

Der über Kopf der Rektifikationskolonne abgetrennte Leichtsiederstrom enthält im wesentlichen folgende Komponenten:

| | |
|---|---|
| Acrylsäure | 98,44 Gew.-%, |
| Essigsäure | 0,94Gew.%, |
| Wasser | 0,35 Gew.-%, |
| Phenothiazin | 0,028 Gew.-%, |
| Diacrylsäure | 0,027 Gew.-%, |
| Allylacrylat | 0,05 Gew.-%, |
| Furfurale | 0,006 Gew.-%, |
| Propionsäure | 0,034 Gew.-%, |
| Methacrylsäure | 0,01 Gew-%, |
| Ameisensäure | 0,07 Gew.-% und |
| Acrolein | 0,001 Gew.-%. |

Ein Teilstrom der als Leichtsiederfraktion entnommenen Flüssigkeit von 47,6 Gew.-% bezogen auf den Zulauf in die Rektifikationskolonne wird als Rücklauf verwendet, der über mehrere Düsen aufgegeben wird. Dabei wird ein kleiner Teil auch dazu benutzt, mittels Düsen die Kolonnenhaube sowie die Brüdenleitung mit inhibitorhaltigem Kondensat zu besprühen. 1,3 Gew-% der Leichtsiederfraktion, bezogen auf den Zulauf in die Rektifikationskolonne, wird ausgeschleust und in die Absorptionseinheit wie dort beschrieben zurückgeführt.

Als Polymerisationsinhibitor wird Phenothiazin verwendet. Der Inhibitor wird als Lösung mit einer Inhibitorkonzentration von 1,1 Gew-% in der über Seitenabzug wie beschrieben abgetrennten rohen Acrylsäure angesetzt und dem kondensierten Leichtsiederrücklauf sowie den Kondensaten der beiden Direktkühlungsstufen der Rektifikationseinheit kontinuierlich zugesetzt. Der feste Stabilisator wird in Form von Schuppen oder Pellets über Förderschnecken in den Stabilisatoransatzbehälter eindosiert.

Wascheinheit

Die Waschkolonne wird mit einem Kopfdruck von 3 bar betrieben. Als trennwirksame Einbauten werden Regensiebböden mit einem Lochdurchmesser von 30 mm verwendet. 30 dieser Böden sind mit einem Bodenabstand von 0,4 m montiert.

Als Waschflüssigkeit wird die Sumpfflüssigkeit aus der Rektifikationseinheit verwendet. Die Waschflüssigkeit wird am Kopf der Waschkolonne mit einer Temperatur von 50 °C aufgegeben. Unterhalb des untersten Bodens wird das aus der Absorptionseinheit kommende Kreisgas eingespeist, nachdem es zuvor auf die Eintrittstemperatur von 50°C abgekühlt wurde. Die zugeführte Kreisgasmenge beträgt 37 Gew-% der zugeführten Waschflüssigkeitsmenge.

Die im Kolonnensumpf anfallende Flüssigkeit enthält im wesentlichen folgende Komponenten:

| | |
|---|---|
| Diphyl | 75 Gew.-%, |
| Dimethylphthalat | 17,6 Gew.%, |
| Diacrylsäure | 1,9 Gew.-%, |
| Acrylsäure | 0,8 Gew.-%, |
| Maleinsäureanhydrid | 1,3 Gew.-%, |
| Phenothiazin | 0,05 Gew.-%, |
| Phthalsäureanhydrid | 0,1 Gew.-%, |
| Benzoesäure | 1,7 Gew.-%, |
| Acrolein | 0,02 Gew.-%, |
| Wasser | 0,17 Gew.-%, |
| Essigsäure | 0,02 Gew.-%, |
| Benzaldehyd | 0,74 Gew.-%, |
| Furfurale | 0,015 Gew-%, |
| Methacrylsäure | 0,06 Gew-% und |
| Propan | 0,5 Gew-%. |

Die in der Wascheinheit anfallende Sumpfflüssigkeit wird ausgeschleust und in zwei Teilmengen im Gewichtsverhältnis 7,2 zu 1 aufgeteilt. Der größere Teilstrom wird wie beschrieben in die Absorptionskolonne aufgegeben, während der kleiner Teilstrom der Sauerwasserextraktion zugeführt wird.

Das gewaschene Kreisgas enthält im wesentlichen noch folgende Bestandteile:

| | |
|---|---|
| Stickstoff | 51,62 Gew.-%, |
| Sauerstoff | 3,66 Gew.%, |
| Kohlenoxide | 3,73 Gew-%, |
| Wasser | 1,01 Gew.-%, |
| Acrylsäure | 0,02 Gew.-%, |
| Diphyl | 0,03 Gew.-%, |
| Benzaldehyd | 0,01 Gew.-%, |
| Maleinsäureanhydrid | 0,015 Gew.-%, |
| Propan | 38,96 Gew.-%, |
| Propen | 0,16 Gew.-%, |
| Butane | 0,42 Gew-%, |
| Butene | 0,11 Gew-% und |
| Ethan | 0,2 Gew-%. |

Das gewaschene Kreisgas wird als Stripgas in die beschriebene Desorptionseinheit geleitet.

Sauerwasserextraktion

Das Sauerwasserkondensat aus der Absorptionseinheit wird in einer Extraktionseinheit mit dem ihr zugeführten Teil des Flüssigkeitsablaufs aus der Wascheinheit extrahiert. Die Extraktionseinheit besteht aus einem Rührbehälter mit einem zweistufigen Impellerrührer sowie einem liegenden Absetzbehälter. Zu- und Ablauf im Absetzbehälter sind durch Einbauten quer zur Strömungsrichtung voneinander getrennt. Die Eintrittstemperatur des Sauerwasserkondensats in die Extraktionseinheit beträgt 45 °C. Das Massenverhältnis von Menge Sumpfablauf der Wascheinheit und Sauerwasser aus der Sauerwasserkondensation beträgt 0,8 zu 1.

Das in der Extraktionseinheit anfallende wässrige Extrakt nimmt aus dem Sumpfablauf der Wascheinheit polare Bestandteile wie Diacrylsäure (Michael - Addukt) und Maleinsäureanhydrid auf, wobei letzteres dabei hydrolysiert wird. Es enthält im wesentlichen folgende Komponenten:

| | |
|---|---|
| Wasser | 70 - 95 Gew.-%, |
| Essigsäure | 2 -10 Gew.%, |
| Acrylsäure | 1 - 5 Gew.-%, |
| Formaldehyd | 2 -10 Gew-%, |
| Maleinsäure | 1 - 5 Gew.-%, |
| Diacrylsäure | 1 - 5 Gew.-%, |
| Diphyl | 0,01 - 0,05 Gew.-%, |
| Dimethylphthalat | 0,1 -1 Gew.-%, |
| Allylacrylat | 0,0005 -0,002 Gew.-%, |
| Furfurale | 0,001 - 0.001 Gew.-%, |
| Ameisensäure | 0,2 - 2 Gew.-%, |
| Benzaldehyd | 0,01 - 0,05 Gew.-%, |
| Benzoesäure | 0,05 - 0,2 Gew.-%, |
| Maleinsäure | 0,5 - 3 Gew.-% und |
| Phthalsäureanhydrid | 0,01 - 0,1 Gew.-%. |

Die ausgeschleuste wässrige Phase wird zusammen mit den anderen Produktionsrückständen verbrannt. Vorab seiner Verbrennung wird das Sauerwasser teilverdampft. Die dazu erforderliche Wärme wird über Rohrbündelwärmetauscher der Absorptionseinheit entnommen, der dort wie beschrieben auf dem ersten Fangboden anfallendes Kondensat abkühlt.

Die in der Extraktionseinheit anfallende organische Phase (das Raffinat) enthält im wesentlichen folgende Komponenten:

| | |
|---|---|
| Diphyl | 76,3 Gew.-%, |
| Dimethylphthalat | 17,2 Gew.%, |
| Acrylsäure | 1,1 Gew.-%, |
| Maleinsäureanhydrid | 0,35 Gew-%, |
| Diacrylsäure | 0,85 Gew.-%, |
| Phenothiazin | 0,05 Gew.-%, |
| Phthalsäureanhydrid | 0,1 Gew.-%, |
| Benzoesäure | 1,55 Gew.-%, |
| Methacrylsäure | 0,03 Gew.-%, |
| Propionsäure | 0,02 Gew-% |
| Benzaldehyd | 1,16 Gew.-%, |
| Ameisensäure | 0,05 Gew.-%, |
| Acrolein | 0,04 Gew.-%, |
| Wasser | 0,7 Gew.-%, |
| Essigsäure | 0,41 Gew.-% und |
| Furfurale | 0,02 Gew.-%. |

Die ausgeschleuste organische Phase wird in der Absorptionseinheit wie dort beschrieben zugeführt.

Schwersiederdestillation

Die beschriebene Teilmenge des Sumpfablaufs der Absorptionseinheit wird einer Destillationseinheit zugeführt und in dieser durch Erwärmen in eine Schwersiederfraktion und eine Leichtsiederfraktion aufgetrennt. Die Destillationseinheit ist einstufig ausgeführt und wird bei einem Druck von 90 mbar betrieben. Eine technische Ausführung in einer Destillationskolonne mit dem Fachmann bekannten trennwirksamen Einbauten ist aber auch denkbar.

Die Wärmezufuhr erfolgt über einen außenliegenden Zwangsumlaufverdampfer. Die Überhitzung der umlaufenden Flüssigkeit beträgt dort 5 K. In der Destillationseinheit stellt sich eine Temperatur von 188 °C ein.

Aus der Umlaufmenge des Zwangsumlaufverdampfers wird eine Teilmenge entnommen und nach Verdünnung mit einem geeigneten Verdünnungsmittel (z.B. Dimethylformamid oder Methanol) gemeinsam mit anderen Produktionsrückständen verbrannt.

Die Schwersiederfraktion enthält im wesentlichen folgende Komponenten:

| | |
|---|---|
| Diphyl | 25,8 Gew.-%, |
| Dimethylphthalat | 43,2 Gew.%, |
| Phenothiazin | 29,1 Gew.-%, |
| Acrylsäure | 0,13 Gew.-%, |
| Diacrylsäure | 0,6 Gew.-%, |
| Phthalsäureanhydrid | 0,3 Gew.-%, |
| Benzoesäure | 1 Gew.-%, |
| Maleinsäureanhydrid | 0,02 Gew.-% und |
| Benzaldehyd | 0,01 Gew.-%. |

Von der Schwersiederfraktion werden 1,7 Gew.-% bezogen auf die der Destillationseinheit zugeführte Zulaufmenge ausgeschleust und verbrannt.

Die in der Destillationseinheit in die Dampfphase überführte Leichtsiederfraktion wird durch eine Kombination aus direkter und indirekter Kühlung abgekühlt und kondensiert. Dazu wird bereits kondensierte Leichtsiederfraktion im Brüden der Destillationseinheit versprüht und gemeinsam mit dem nicht kondensierten Brüden mittels eines Rohrbündelwärmetauschers gekühlt und die gekühlte Flüssigkeit oberhalb des Wärmetauschers im Brüden versprüht. Der den Kühler verlassende Stoffstrom weist eine Temperatur von 52 °C auf und wird in einem Behälter in Gas- und Flüssigphase getrennt.

Die in der Destillationseinheit anfallende flüssige Leichtsiederfraktion enthält im wesentlichen folgende Komponenten:

| | |
|---|---|
| Acrylsäure | 4,5 Gew.-%, |
| Diphyl | 54,8 Gew.%, |
| Dimethylphthalat | 36,7 Gew.-%, |
| Diacrylsäure | 1,25 Gew.-%, |
| Essigsäure | 0,04 Gew.-%, |
| Wasser | 0,01 Gew.-%, |
| Ameisensäure | 0,005 Gew.-%, |
| Phenothiazin | 0,165 Gew.-%, |
| Benzaldehyd | 0,12 Gew.-%, |
| Maleinsäureanhydrid | 0,24 Gew.-%, |
| Benzoesäure | 1,89 Gew.-%, |
| Phthalsäureanhydrid | 0,14 Gew.-%, |
| Furfurale | 0,003 Gew.-% und |
| Acrolein | 0,002 Gew.-%. |

Über eine Kreiselpumpe wird die flüssige Leichtsiederfraktion wie beschrieben oberhalb des Rohrbündelwärmeübertragers wie beschrieben versprüht. 98,2 Gew.-% der als Leichtsiederfraktion entnommenen Flüssigkeit bezogen auf die der Destillationseinheit zugeführte Zulaufmenge, wird ausgeschleust und in die Absorptionseinheit unterhalb des ersten Kaminbodens zurückgeführt.

Das die Destillationseinheit gasförmig verlassende Abgas enthält im wesentlichen folgende Komponenten:

| | |
|---|---|
| Acrylsäure | 4,8 Gew.-%, |
| Essigsäure | 0,2 Gew.%, |
| Wasser | 2,2 Gew.-%, |
| Diphyl | 0,8 Gew.%, |
| Dimethylphthalat | 0,1 Gew.-%, |
| Formaldehyd | 0,13 Gew.-%, |
| Acrolein | 0,13 Gew.-%, |
| Propionsäure | 0,002 Gew.-%, |
| Furfurale | 0,003 Gew.-%, |
| Benzaldehyd | 0,07 Gew.-%, |
| Maleinsäureanhydrid | 0,1 Gew.-%, |
| Benzoesäure | 0,01 Gew.-%, |
| Phthalsäureanhydrid | 0,01 Gew.-%, |
| Diacrylsäure | 0 Gew.-%, |
| Ameisensäure | 0,02 Gew.-%, |
| Allylacrylat | 0,003Gew.-%, |
| Butane | 1,1 Gew-%, |
| Butene | 0,3 Gew-% und |
| Propan | 89,9 Gew-%. |

Dieses Abgas wird zusammen mit den anderen Produktionsrückständen verbrannt.

Die gewonnene rohe Acrylsäure kann in an sich bekannter Weise in weiteren Verfahrensstufen zu einer reinen Acrylsäure weiterverarbeitet werden. Geeignet sind hierzu ein einstufiges oder, bei einer rohen Acrylsäure mit signifikanten Anteilen an leichtsiedenden Nebenkomponenten, ein zweistufiges destillatives Verfahren mit vorheriger Aldehydbehandlung durch eine mindestens eine primäre Aminogruppe enthaltende Verbindung (wie Hydrazin oder Aminoguanidinhydrogencarbonat) gemäß den Lehren von EP-A 270 999, EP-A 648 732, sowie auch ein kristallisatives Verfahren gemäß den Lehren von EP-A 616 998, EP-A 792 867, EP-A 1 189 861 und WO 98/01404.

Wird die reine Acrylsäure in einer kristallisativen Verfahrensstufe wie einer Schichtkristallisation gewonnen, so fällt hierbei neben der reinen Acrylsäure auch ein acrylsäurehaltiger Mutterlaugen-/-säurenstrom an, der wie oben beschrieben in die Absorptionseinheit zur Gewinnung der rohen Acrylsäure zurückgeführt werden kann.

Beispiel 1

Es wurde wie im Vergleichsbeispiel 1 verfahren. Das (Gesamt) Restgas wurde jedoch nicht in den dem ersten Erhitzer vorgeschalteten Verdampfer, sondern auf der entsprechenden Druck verdichtet gemeinsam mit den 195 Nl/h Druckluft in den dem dritten Dehydrierreaktor vorgeschalteten Erhitzer geführt.

Der Gehalt des den dritten Dehydrierreaktor verlassenden Reaktionsgasgemischs an $CO_2$ sank in der Folge von 3,25 Vol.-% auf 1,6 Vol.-%.

Dies weist aus, dass die erfindungsgemäße Verfahrensweise mit einer auf weniger als die Hälfte verringerten Vollbrennung der involvierten $C_3$-Kohlenwasserstoffe einhergeht.

Ferner konnte diese erfindungsgemäße Verfahrensweise über einen Zeitraum von 7500 Betriebsstunden im wesentlichen ohne nennenswerte Beeinträchtigungen betrieben werden.

Beispiel 2

Beschrieben wird auch hier der stationäre Betriebszustand.

Ein als Hordenreaktor ausgeführter und adiabat gestalteter Schachtofenreaktor weist als ersten Abschnitt einer Reaktionszone A zwei in Strömungsrichtung hintereinander angeordnete Katalysatorbetten auf, die jeweils als Festbett mit einem Dehydrierkatalysator gemäß Vergleichsbeispiel 1 beschickt sind. Vor jedem Festbett befindet sich ein statischer Gasmischer.

Dem in Strömungsrichtung ersten Katalysatorbett wird ein Reaktionsgasausgangsgemisch zugeführt, das sich wie folgt zusammen setzt:

a) rückgeführtes Produktgasgemisch A, das eine Temperatur von 617°C und einen Druck von 2,75 bar aufweist in einer Menge von 132872 Nm$^3$/h, enthaltend

26,4 Vol.-% Propan,
6,6 Vol.-% Propen (Propylen),
0,1 Vol.-% $H_2$,
8,8 Vol.-% $H_2O$ und
0 Vol.% $O_2$

und

b) 8758 Nm$^3$/h Roh-Propan gemäß Vergleichsbeispiel 1.

Die Temperatur des Roh-Propan ist so beschaffen, dass die Temperatur des Reaktionsgasausgangsgemischs 560°C beträgt.

Die Reaktionsgasausgangsgemischmenge beträgt 141630 Nm$^3$/h, mit folgenden Gehalten:

31,0 Vol.-% Propan,
6,2 Vol.-% Propen,
0,1 Vol.% $H_2$,
8,2 Vol.-% $H_2O$ und
0 Vol.% $O_2$

Der Druck des Reaktionsgasausgangsgemisches beträgt vor dem Eingang in das erste Katalysatorbett 2,75 bar.

Die Schütthöhe des ersten vom Reaktionsgasausgangsgemisch durchströmten Katalysatorbetts ist so beschaffen, das das Reaktionsgasgemisch dieses Katalysatorfestbett mit folgenden Gehalten verlässt:

26,4 Vol.% Propan,
9,5 Vol.-% Propen,
3,6 Vol.% $H_2$,
7,9 Vol.% $H_2O$ und
0 Vol.% $O_2$

Die Verlassmenge beträgt 146533 Nm$^3$/h. Die Verlasstemperatur beträgt 500°C und der Verlassdruck liegt bei 2,65 bar. Der im ersten Katalysatorbett sich einstellende Propanumsatz liegt bei 11,8 mol-% des zugeführten Propans.

Dem das erste Katalysatorfestbett wie beschrieben verlassenden Reaktionsgasgemisch werden 6250 Nm$^3$/h

Luft zudosiert. Die Luft ist auf 500 °C vorerwärmt und ihr Druck ist so beschaffen, dass der Druck des nach der Luftdosierung resultierenden Reaktionsgasgemischs 2,65 bar beträgt.

Beim Durchströmen des zweiten Katalysatorfestbetts wird zunächst die Hälfte des im Reaktionsgasgemisch enthaltenen molekularen Wasserstoff mit dem in Form von Luft zudosierten molekularen Sauerstoff zu Wasser verbrannt. Dabei erwärmt sich das Reaktionsgasgemisch auf 550°C. Im weiteren Verlauf des Durchströmens des zweiten Katalysatorfestbetts erfolgt heterogen katalysierte Dehydrierung des Propans. Die Schütthöhe des zweiten Katalysatorfestbetts ist dabei so bemessen, dass das Reaktionsgasgemisch das zweite Katalysatorfestbett mit einer Temperatur von 513°C und einem Druck von 2,55 bar mit folgenden Gehalten in einer Menge von 154746 Nm$^3$/h verlässt:

22,9 Vol.-% Propan,
11,9 Vol.-% Propen,
3,9 Vol.-% $H_2$,
9,2 Vol.-% $H_2O$ und
0 Vol.-% $O_2$

Diesem Produktgasgemisch A* werden zunächst 761,6 Nm$^3$/h an molekularem Wasserstoff zugeführt. Anschließend werden mit einer Temperatur von 567°C 112792 Nm$^3$/h an Gesamtrestgas aus der Trennzone B zugeführt. Das Gesamtrestgas weist folgende Gehalte auf:

31,1 Vol.-% Propan,
0 Vol.-% Propen,
0 Vol.-% $H_2$,
1,9 Vol.-% $H_2O$ und
3,0 Vol.-% $O_2$

Die Zufuhr des Gesamtrestgases erfolgt nach dem Prinzip einer mit diesem Gesamtrestgas als Treibstrahl betriebenen Strahlpumpe, wobei die Förderrichtung des durch eine Treibdüse über eine Mischstrecke und einen Diffusor entspannten Treibstrahls in den zweiten Abschnitt der Reaktionszone A sowie die Saugwirkung des Saugstutzens in Richtung des Gemischs aus molekularem Wasserstoff und Produktgasgemisch A* weist.

Das dabei resultierende Reaktionsgasgemisch A* strömt in einer Menge von 267538 Nm$^3$/h und mit einer Temperatur von 536°C und einem Druck von 2,85 bar in einen zweiten Schachtofenreaktor, der den zweiten Abschnitt der Reaktionszone A bildet, ebenfalls adiabat gestaltet ist und ein Katalysatorfestbett enthält, das ebenfalls mit dem Dehydrierkatalysator gemäß Vergleichsbeispiel 1 beschickt ist. Es weist folgende Gehalte auf:

26,3 Vol.-% Propan,
6,4 Vol.-% Propen,
2,6 Vol.-% $H_2$,
6,1 Vol.-% $H_2O$ und
1,3 Vol.-% $O_2$

Beim Durchströmen dieses dritten Katalysatorfestbetts wird zunächst der im Reaktionsgasgemisch A* enthaltene molekulare Sauerstoff mit dem darin enthaltenen molekularen Wasserstoff im wesentlichen vollständig zu Wasser verbrannt, wobei sich das Reaktionsgasgemisch auf 620°C erwärmt.

Die Schütthöhe des dritten Katalysatorfestbetts ist so beschaffen, dass im Reaktionsgasgemisch bis zum Verlassen desselben noch in geringfügigem Umfang heterogen katalysierte Propandehydrierung erfolgt.

Im Ergebnis verlassen 265745 Nm$^3$/h Produktgasgemisch A mit einer Temperatur von 617°C und einem Druck von 2,75 bar das dritte Katalysatorfestbett. Es enthält die nachfolgenden Gehalte:

26,4 Vol.-% Propan,
6,6 Vol.-% Propen,
0,1 Vol.-% $H_2$,
8,8 Vol.-% $H_2O$ und
0 Vol.-% $O_2$

Das Produktgasgemisch A wird in zwei Hälften identischer Zusammensetzung aufgeteilt.

Davon wird die eine Hälfte als Bestandteil des Reaktionsgasausgangsgemischs für den ersten Abschnitt der Reaktionszone A in selbigen zurückgeführt.

Die andere Hälfte wird über einen indirekten Wärmetauscher W für Gase der Trennzone A zugeführt. Im Wärmetauscher wird das in die Reaktionszone A zurückgeführte Restgas aus der Trennzone B auf die 567°C erwärmt.

In der Trennzone B wird aus der zweiten Hälfte des Produktgasgemisch A das in selbigem enthaltene Propan und Propen wie in Vergleichsbeispiel 1 absorbtiv abgetrennt und wie im Vergleichsbeispiel 1 in der Reaktionszone B der zweistufigen heterogen katalysierten Partialoxidation des Propens zu Acrylsäure unterworfen.

Aus dem dabei resultierenden Produktgasgemisch B wird die Acrylsäure in der Trennzone B wie im Vergleichsbeispiel 1 abgetrennt, und das dabei verbleibende Gesamtrestgas verdichtet (auf ca. 4 bar) und wie beschrieben über den Wärmetauscher W der mit dem Gesamtrestgas als Treibstrahl betriebenen Strahlpumpe zugeführt und mit dem mit molekularem Wasserstoff ergänzten Produktgasgemisch A* vereint. Die in der Trennzone B abgetrennte Menge an Acrylsäure beläuft sich auf 332 kmol/h. Die Restgasmenge in den vorstehend beschriebenen Gasgemischen besteht im wesentlichen aus molekularem Stickstoff und aus geringen Mengen an Kohlenoxiden. Das beschriebene Verfahren ist im wesentlichen ohne Minderung langanhaltend betreibbar.

Beispiel 3

Beschrieben wird auch hier der stationäre Betriebszustand.

Ein als Hordenreaktor ausgeführter und adiabat gestalteter Schachtofenreaktor weist als erster Abschnitt einer Reaktionszone A zwei in Strömungsrichtung hintereinander angeordnete Katalysatorbetten auf, die jeweils als Festbett mit einem Dehydrierkatalysator gemäß Vergleichsbeispiel 1 beschickt sind. Vor jedem Festbett befindet sich ein statischer Gasmischer.

Dem in Strömungsrichtung ersten Katalysatorbett wird ein Reaktionsgasausgangsgemisch zugeführt, das sich wie folgt zusammen setzt:

a) rückgeführtes Produktgasgemisch A, das eine Temperatur von 676°C und einen Druck von 1,75 bar aufweist in einer Menge von 106510 $Nm^3$/h, enthaltend

12,3 Vol.-% Propan,
8,2 Vol.-% Propen (Propylen),
2,2 Vol.% $H_2$,
7,7 Vol.-% $H_2O$ und
0 Vol.-% $O_2$

und

b) 8758 $Nm^3$/h Roh-Propan gemäß Vergleichsbeispiel 1.

Die Temperatur des Roh-Propan ist so beschaffen, dass die Temperatur des Reaktionsausgangsgemischs 652°C beträgt.

Die Reaktionsgasausgangsgemischmenge beträgt 115268 $Nm^3$/h, mit folgenden Gehalten:

19,0 Vol.-% Propan,
7,6 Vol.-% Propen,
2,0 Vol.-% $H_2$,
7,1 Vol.-% $H_2O$ und
0 Vol.-% $O_2$

Der Druck des Reaktionsgasausgangsgemisches beträgt vor dem Eingang in das erste Katalysatorbett 1,75 bar.

Die Schütthöhe des ersten vom Reaktionsgasausgangsgemisch durchströmten Katalysatorbetts ist so beschaffen, das das Reaktionsgasgemisch dieses Katalysatorfestbett mit folgenden Gehalten verlässt:

15,1 Vol.-% Propan,
10,6 Vol.-% Propen,
5,1 Vol.-% $H_2$,
6,9 Vol.-% $H_2O$ und
0 Vol.-% $O_2$

Die Verlassmenge beträgt 119308 $Nm^3$/h. Die Verlasstemperatur beträgt 590°C und der Verlassdruck liegt bei 1,65 bar. Der im ersten Katalysatorbett sich einstellende Propanumsatz liegt bei 17,5 mol-% des zugeführten Propans.

Dem das erste Katalysatorfestbett wie beschrieben verlassenden Reaktionsgasgemisch werden 2598 $Nm^3$/h

Luft zudosiert. Die Luft ist auf 590°C vorerwärmt und ihr Druck ist so beschaffen, dass der Druck des nach der Luftdosierung resultierenden Reaktionsgasgemischs 1,65 bar beträgt.

Beim Durchströmen des zweiten Katalysatorfestbetts werden zunächst etwa 18 % des im Reaktionsgasgemisch enthaltenen molekularen Wasserstoff mit dem in Form von Luft zudosierten molekularen Sauerstoff zu Wasser verbrannt. Dabei erwärmt sich das Reaktionsgasgemisch auf 618°C. Im weiteren Verlauf des Durchströmens des zweiten Katalysatorfestbetts erfolgt heterogen katalysierte Dehydrierung des Propans. Die Schütthöhe des zweiten Katalysatorfestbetts ist dabei so bemessen, dass das Reaktionsgasgemisch das zweite Katalysatorfestbett mit einer Temperatur von 546°C und einem Druck von 1,55 bar mit folgenden Gehalten in einer Menge von 124387 Nm$^3$/h verlässt:

12,0 Vol.-% Propan,
12,6 Vol.-% Propen,
6,6 Vol.-% $H_2$,
7,5 Vol.-% $H_2O$ und
0 Vol.-% $O_2$

Diesem Produktgasgemisch A* werden anschließend mit einer Temperatur von 627°C 89715 Nm$^3$/h an Gesamtrestgas aus der Trennzone B zugeführt. Das Gesamtrestgas weist folgende Gehalte auf:

14,6 Vol.-% Propan,
0 Vol.-% Propen,
0 Vol.-% $H_2$,
1,9 Vol.-% $H_2O$ und
3,0 Vol.-% $O_2$

Die Zufuhr des Gesamtrestgases erfolgt nach dem Prinzip einer mit diesem Gesamtrestgas als Treibstrahl betriebenen Strahlpumpe, wobei die Förderrichtung des durch eine Treibdüse über eine Mischstrecke und einen Diffusor entspannten Treibstrahls in den zweiten Abschnitt der Reaktionszone A sowie die Saugrichtung des Saugstutzens in Richtung des Gemischs aus molekularem Wasserstoff und Produktgasgemisch A* weist.

Das dabei resultierende Reaktionsgasgemisch A* strömt in einer Menge von 214102 Nm$^3$/h und mit einer Temperatur von 580°C und einem Druck von 1,85 bar in einen zweiten Schachtofenreaktor, der den zweiten Abschnitt der Reaktionszone A bildet, ebenfalls adiabat gestaltet ist und ein Katalysatorfestbett enthält, das ebenfalls mit dem Dehydrierkatalysator gemäß Vergleichsbeispiel 1 beschickt ist. Es weist folgende Gehalte auf:

13,1 Vol.-% Propan,
7,3 Vol.-% Propen,
3,8 Vol.-% $H_2$,
5,2 Vol.-% $H_2O$ und
1,3 Vol.-% $O_2$

Beim Durchströmen dieses dritten Katalysatorfestbetts wird zunächst der im Reaktionsgasgemisch A* enthaltene molekulare Sauerstoff mit dem darin enthaltenen molekularen Wasserstoff im wesentlichen vollständig zu Wasser verbrannt, wobei sich das Reaktionsgasgemisch auf 692°C erwärmt.

Die Schütthöhe des dritten Katalysatorfestbetts ist so beschaffen, dass im Reaktionsgasgemisch bis zum Verlassen desselben noch in geringfügigem Umfang heterogen katalysierte Propandehydrierung erfolgt.

Im Ergebnis verlassen 213021 Nm$^3$/h Produktgasgemisch A mit einer Temperatur von 677°C und einem Druck von 1,75 bar das dritte Katalysatorfestbett. Es enthält die nachfolgenden Gehalte:

12,3 Vol.% Propan,
8,2 Vol.% Propen,
2,2 Vol.-% $H_2$,
7,7 Vol.-% $H_2O$ und
0 Vol.-% $O_2$

Das Produktgasgemisch A wird in zwei Hälften identischer Zusammensetzung aufgeteilt.

Davon wird die eine Hälfte als Bestandteil des Reaktionsgasausgangsgemischs für den ersten Abschnitt der Reaktionszone A in selbigen zurückgeführt.

Die andere Hälfte wird über einen indirekten Wärmetauscher W für Gase der Trennzone A zugeführt. Im Wärmetauscher wird das in die Reaktionszone A zurückgeführte Restgas aus der Trennzone B auf die 627°C erwärmt.

In der Trennzone B wird aus der zweiten Hälfte des Produktgasgemisch A das in selbigem enthaltene Propan und Propen wie in Vergleichsbeispiel 1 absorbtiv abgetrennt und wie im Vergleichsbeispiel 1 in der Reaktionszone B der zweistufigen heterogen katalysierten Partialoxidation des Propens zu Acrylsäure unterworfen.

Aus dem dabei resultierenden Produktgasgemisch B wird die Acrylsäure in der Trennzone B wie im Vergleichsbeispiel 1 abgetrennt, und das dabei verbleibende Gesamtrestgas verdichtet (auf ca. 4 bar) und wie beschrieben über den Wärmetauscher W der mit dem Gesamtrestgas als Treibstrahl betriebenen Strahlpumpe zugeführt und mit dem Produktgasgemisch A* vereint. Die in der Trennzone B abgetrennte Menge an Acrylsäure beläuft sich auf 332 kmol/h. Die Restgasmenge in den vorstehend beschriebenen Gasgemischen besteht im wesentlichen aus molekularem Stickstoff und aus geringen Mengen an Kohlenoxiden. Das beschriebene Verfahren ist im wesentlichen ohne Minderung langanhaltend betreibbar.


**Patentansprüche**

1.   Verfahren zur Herstellung von Acrolein, oder Acrylsäure, oder deren Gemisch aus Propan, bei dem man

   A) einer ersten Reaktionszone A wenigstens zwei gasförmige, Propan enthaltende Zufuhrströme zuführt, von denen wenigstens einer Frischpropan enthält, und in der Reaktionszone A ihr so zugeführtes Propan einer heterogen katalysierten Dehydrierung unterwirft, wobei ein Propan und Propylen enthaltendes Produktgasgemisch A erhalten wird,
   B) aus der Reaktionszone A Produktgasgemisch A herausführt und in einer ersten Trennzone A von den im Produktgasgemisch A enthaltenen, von Propan und Propylen verschiedenen, Bestandteilen wenigstens eine Teilmenge abtrennt, und dabei verbleibendes, Propan und Propylen enthaltendes, Produktgasgemisch A'
   C) in einer zweiten Reaktionszone B zur Beschickung wenigstens eines Oxidationsreaktors verwendet und in dem wenigstens einen Oxidationsreaktor das im Produktgasgemisch A' enthaltene Propylen einer heterogen katalysierten Gasphasenpartialoxidation mit molekularem Sauerstoff zu einem Acrolein, oder Acrylsäure, oder deren Gemisch als Zielprodukt sowie überschüssigen molekularen Sauerstoff enthaltenden Produktgasgemisch B unterwirft,
   D) aus der Reaktionszone B Produktgasgemisch B herausführt und in einer zweiten Trennzone B im Produktgasgemisch B enthaltenes Zielprodukt abtrennt und von dem dabei verbleibenden, nicht umgesetztes Propan, molekularen Sauerstoff sowie gegebenenfalls nicht umgesetztes Propylen enthaltenden Restgas wenigstens eine nicht umgesetztes Propan, molekularen Sauerstoff und gegebenenfalls nicht umgesetztes Propylen enthaltende Teilmenge als einen der wenigstens zwei Propan enthaltenden Zufuhrströme in die Reaktionszone A rückführt, **dadurch gekennzeichnet, dass** diese Rückführung in die Reaktionszone A längs des Reaktionspfads der heterogen katalysierten Dehydrierung des Propans in der Reaktionszone A so erfolgt, dass an der Zufuhrstelle bereits wenigstens 5 mol-% des der Reaktionszone A über die anderen Zufuhrströme zugeführten Propan in der Reaktionszone A dehydrierend umgesetzt sind.

2.   Verfahren nach Anspruch 1, **dadurch gekennzeichnet, dass** wenigstens die Hälfte des in der Trennzone B verbleibenden, nicht umgesetztes Propan, molekularen Sauerstoff sowie gegebenenfalls nicht umgesetztes Propylen enthaltenden Restgases in die Reaktionszone A rückgeführt wird.

3.   Verfahren nach Anspruch 1, **dadurch gekennzeichnet, dass** die Gesamtmenge des in der Trennzone B verbleibenden, nicht umgesetztes Propan, molekularen Sauerstoff sowie gegebenenfalls nicht umgesetztes Propylen enthaltenden Restgases in die Reaktionszone A rückgeführt wird.

4.   Verfahren nach einem der Ansprüche 1 bis 3, **dadurch gekennzeichnet, dass** die Rückführung von in der Trennzone B verbleibendem, nicht umgesetztes Propan, molekularen Sauerstoff sowie gegebenenfalls nicht umgesetztes Propylen enthaltendem Restgas in die Reaktionszone A nur an einer Stelle der Reaktionszone A erfolgt.

5.   Verfahren nach einem der Ansprüche 1 bis 3, **dadurch gekennzeichnet, dass** die Rückführung von in der Trennzone B verbleibendem, nicht umgesetztes Propan, molekularen Sauerstoff sowie gegebenenfalls nicht umgesetztes Propylen enthaltendem Restgas in die Reaktionszone A über mehrere hintereinander angeordnete Zufuhrstellen verteilt erfolgt.

6.   Verfahren nach einem der Ansprüche 1 bis 5, **dadurch gekennzeichnet, dass** die Rückführung von in der Trennzone B verbleibendem, nicht umgesetztes Propan, molekularen Sauerstoff sowie gegebenenfalls nicht umgesetztes Propylen enthaltendem Restgas in die Reaktionszone A so erfolgt, dass an der Zufuhrstelle wenigstens 20 mol-% des der Reaktionszone A über die anderen Zufuhrströme zugeführten Propan in der Reaktionszone A dehydrierend umgesetzt sind.

7. Verfahren nach einem der Ansprüche 1 bis 5, **dadurch gekennzeichnet, dass** die Rückführung von in der Trennzone B verbleibendem, nicht umgesetztes Propan, molekularen Sauerstoff sowie gegebenenfalls nicht umgesetztes Propylen enthaltendem Restgas in die Reaktionszone A so erfolgt, dass an der Zufuhrstelle wenigstens 30 mol-% des der Reaktionszone A über die anderen Zufuhrströme zugeführten Propan in der Reaktionszone A dehydrierend umgesetzt sind.

8. Verfahren nach einem der Ansprüche 1 bis 7, **dadurch gekennzeichnet, dass** der Gehalt des in die Reaktionszone A rückgeführten, in der Trennzone B verbleibenden, nicht umgesetztes Propan, molekularen Sauerstoff sowie gegebenenfalls nicht umgesetztes Propylen enthaltenden, Restgases an molekularem Sauerstoff 0,5 bis 10 Vol.-% beträgt.

9. Verfahren nach einem der Ansprüche 1 bis 7, **dadurch gekennzeichnet, dass** der Gehalt des in die Reaktionszone A rückgeführten, in der Trennzone B verbleibenden, nicht umgesetztes Propan, molekularen Sauerstoff sowie gegebenenfalls nicht umgesetztes Propylen enthaltenden, Restgases an molekularem Sauerstoff 2 bis 5 Vol.-% beträgt.

10. Verfahren nach einem der Ansprüche 1 bis 9, **dadurch gekennzeichnet, dass** das Verhältnis der Propanmenge, die der Reaktionszone A über aus der Trennzone B stammendes rückgeführtes Restgas zugeführt wird, zu der Propanmenge, die der Reaktionszone A über andere Zufuhrströme insgesamt zugeführt wird, 0,1 bis 10 beträgt.

11. Verfahren nach einem der Ansprüche 1 bis 10, **dadurch gekennzeichnet, dass** der Reaktionszone A als Propan enthaltende Zufuhrströme nur aus der Trennzone B stammendes Restgas und Roh-Propan als Frischpropan zugeführt werden.

12. Verfahren nach einem der Ansprüche 1 bis 11, **dadurch gekennzeichnet, dass** das in der Reaktionszone A befindliche Reaktionsgasgemisch an der Zufuhrstelle für das aus der Trennzone B stammende Restgas, bezogen auf die in ihm enthaltene molare Menge an Propan, einen höheren molaren Wasserstoffgehalt aufweist, als das der Reaktionszone A zugeführte Reaktionsgasausgangsgemisch.

13. Verfahren nach einem der Ansprüche 1 bis 12, **dadurch gekennzeichnet, dass** das sich in der Reaktionszone A an der Zufuhrstelle für das aus der Trennzone B stammende Restgas aus diesem zugeführten Restgas und dem vor dieser Restgaszuführung an der Zufuhrstelle in der Reaktionszone A vorliegenden Reaktionsgasgemisch bildende Reaktionsgasgemisch, bezogen auf die in ihm enthaltene molare Menge an Propan, einen höheren molaren Wasserstoffgehalt aufweist, als das der Reaktionszone A zugeführte Reaktionsgasausgangsgemisch.

14. Verfahren nach einem der Ansprüche 1 bis 13, **dadurch gekennzeichnet, dass** die Reaktionstemperaturen in der Reaktionszone A 300 bis 800°C betragen.

15. Verfahren nach einem der Ansprüche 1 bis 14, **dadurch gekennzeichnet, dass** in der Reaktionszone A Dehydrierkatalysatoren mitverwendet werden, die 10 bis 99,9 Gew.-% Zirkondioxid, 0 bis 60 Gew.-% Aluminiumoxid, Siliciumdioxid und/oder Titandioxid und 0,1 bis 10 Gew.-% mindestens eines Elements der ersten oder zweiten Hauptgruppe, eines Elements der dritten Nebengruppe, eines Elements der achten Nebengruppe des Periodensystems der Elemente, Lanthan und/oder Zinn mit der Maßgabe enthalten, dass die Summe der Gewichtsprozente 100 Gew.-% ergibt.

16. Verfahren nach einem der Ansprüche 1 bis 15, **dadurch gekennzeichnet, dass** in der Reaktionszone A als Katalysatoren Katalysatorstränge und/oder Katalysatorringe verwendet werden.

17. Verfahren nach einem der Ansprüche 1 bis 16, **dadurch gekennzeichnet, dass** als Reaktionszone A ein Hordenreaktor verwendet wird.

18. Verfahren nach einem der Ansprüche 1 bis 17, **dadurch gekennzeichnet, dass** der in der Reaktionszone A erzielte Umsatz an Propan, bezogen auf der Reaktionszone A insgesamt zugeführtes Propan und bezogen auf einmaligen Durchgang durch die Reaktionszone A, 30 bis 60 mol-% beträgt.

19. Verfahren nach einem der Ansprüche 1 bis 17, **dadurch gekennzeichnet, dass** der in der Reaktionszone A erzielte Umsatz an Propan, bezogen auf der Reaktionszone A insgesamt zugeführtes Propan und bezogen auf einmaligen Durchgang durch die Reaktionszone A, 40 bis 50 mol-% beträgt.

20. Verfahren nach einem der Ansprüche 1 bis 10 sowie 12 bis 19, **dadurch gekennzeichnet, dass** man das in der Reaktionszone A gebildete Produktgasgemisch A in zwei Teilmengen identischer Zusammensetzung aufteilt, eine der beiden Teilmengen als einen der Propan enthaltenden Zufuhrströme in die Reaktionszone A zurückführt und von der anderen Teilmenge aus der Reaktionszone A heraus in die erste Trennzone A führt.

21. Verfahren nach Anspruch 20, **dadurch gekennzeichnet, dass** die in die Reaktionszone A rückgeführte Teilmenge des Produktgasgemischs A, bezogen auf die Gesamtmenge an in der Reaktionszone A gebildetem Produktgasgemisch A, 30 bis 70 Vol.-% beträgt.

22. Verfahren nach Anspruch 20, **dadurch gekennzeichnet, dass** die in die Reaktionszone A rückgeführte Teilmenge des Produktgasgemischs A, bezogen auf die Gesamtmenge an in der Reaktionszone A gebildetem Produktgasgemisch A, 40 bis 60 Vol.-% beträgt.

23. Verfahren nach einem der Ansprüche 20 bis 22, **dadurch gekennzeichnet, dass** die in die Reaktionszone A rückgeführte Teilmenge des Produktgasgemischs A als Bestandteil des der Reaktionszone A zugeführten Reaktionsgasausgangsgemischs in die Reaktionszone A rückgeführt wird.

24. Verfahren nach einem der Ansprüche 1 bis 23, **dadurch gekennzeichnet, dass** man die Reaktionszone A mit der Maßgabe gestaltet und betreibt, dass sie aus einem ersten und aus einem zweiten Abschnitt besteht, wobei man

I. dem ersten Abschnitt der Reaktionszone A wenigstens einen, gasförmiges Propan enthaltenden Zufuhrstrom zuführt, der Frischpropan enthält, und in diesem ersten Abschnitt der Reaktionszone A das ihm so zugeführte Propan einer heterogen katalysierten Dehydrierung unterwirft, wobei ein Propan, Propylen und molekularer Wasserstoff enthaltendes Produktgasgemisch A* erhalten wird, bei dessen Erhalt wenigstens 5 mol-% des dem ersten Abschnitt der Reaktionszone A zugeführten Propan in diesem ersten Abschnitt der Reaktionszone A dehydrierend umgesetzt worden sind und das, bezogen auf die in ihm enthaltene molare Menge an Propan, eine größere molare Menge an molekularem Wasserstoff enthält, als das dem ersten Abschnitt der Reaktionszone A zugeführte Reaktionsgasausgangsgemisch;

II. dem Produktgasgemisch A* anschließend molekularen Sauerstoff, nicht umgesetztes Propan und gegebenenfalls nicht umgesetztes Propylen enthaltendes Restgas aus der Trennzone B zuführt und das dabei resultierende Reaktionsgasgemisch A* dem zweiten Abschnitt der Reaktionszone A zuführt, und

III. in diesem zweiten Abschnitt der Reaktionszone A unter Ausbildung des Propan und Propylen enthaltenden Produktgasgemischs A im Reaktionsgasgemisch A* enthaltenen molekularen Sauerstoff mit im Reaktionsgasgemisch A* enthaltenem molekularen Wasserstoff heterogen katalysiert zu Wasser verbrennt und im Reaktionsgasgemisch A* enthaltenes Propan gegebenenfalls heterogen katalysiert zu Propylen dehydriert.

25. Verfahren nach Anspruch 24, **dadurch gekennzeichnet, dass** der erste Abschnitt der Reaktionszone A adiabat gestaltet wird.

26. Verfahren nach Anspruch 24 oder 25, **dadurch gekennzeichnet, dass** der zweite Abschnitt der Reaktionszone A adiabat gestaltet wird.

27. Verfahren nach einem der Ansprüche 24 bis 26, **dadurch gekennzeichnet, dass** sowohl der erste als auch der zweite Abschnitt der Reaktionszone A mit der Maßgabe adiabat gestaltet wird, dass

- die auf den einmaligen Durchgang des dem ersten Abschnitt der Reaktionszone A zugeführten Reaktionsgasausgangsgemisch durch den ersten Abschnitt der Reaktionszone A bezogene Bruttowärmetönung endotherm bis autotherm, und
- die auf den einmaligen Durchgang des dem zweiten Abschnitt des Reaktionszone A zugeführten Reaktionsgasgemischs A* durch den zweiten Abschnitt der Reaktionszone A bezogene Bruttowärmetönung exotherm ist.

28. Verfahren nach einem der Ansprüche 24 bis 27, **dadurch gekennzeichnet, dass** der erste Abschnitt der Reaktionszone Hordenstruktur aufweist.

29. Verfahren nach einem der Ansprüche 24 bis 28, **dadurch gekennzeichnet, dass** das Produktgasgemisch A weniger als 5 Vol-% molekularen Wasserstoff enthält.

30. Verfahren nach einem der Ansprüche 24 bis 29, **dadurch gekennzeichnet, dass** der erste und der zweite Abschnitt der Reaktionszone A in einem Reaktor untergebracht sind.

31. Verfahren nach einem der Ansprüche 24 bis 30, **dadurch gekennzeichnet, dass** das Produktgasgemisch A in zwei Teilmengen identischer Zusammensetzung aufgeteilt und die eine der beiden Teilmengen in den ersten Abschnitt der Reaktionszone A rückgeführt wird.

32. Verfahren nach einem der Ansprüche 24 bis 31, **dadurch gekennzeichnet, dass** man dem Produktgasgemisch A* das nicht umgesetztes Propan, molekularen Sauerstoff und gegebenenfalls nicht umgesetztes Propylen enthaltende Restgas aus der Trennzone B nach dem Prinzip einer mit diesem Restgas als Treibstrahl betriebenen Strahlpumpe zuführt, wobei die Förderrichtung des durch eine Treibdüse über eine Mischstrecke und einen Diffusor entspannten Treibstrahls in den zweiten Abschnitt der Reaktionszone A und die Saugwirkung des Saugstutzens in Richtung des ersten Abschnitts der Reaktionszone A weist und die Verbindung Saugstutzen-Mischstrecke-Diffusor die alleinige Verbindung zwischen den beiden Abschnitten der Reaktionszone A bildet.

33. Verfahren nach Anspruch 32, **dadurch gekennzeichnet, dass** man den Druck des Treibstrahls so wählt, dass der Druck des sich im zweiten Abschnitt der Reaktionszone A ausbildenden Produktgasgemischs A oberhalb des Drucks, des sich im ersten Abschnitt der Reaktionszone A ausbildenden Produktgasgemischs A* liegt.

34. Verfahren nach Anspruch 33, **dadurch gekennzeichnet, dass** das Produktgasgemisch A in zwei Teilmengen identischer Zusammensetzung aufgeteilt und die eine der beiden Teilmengen in einer Art natürlichem Umlauf dem Druckgefälle zwischen dem Produktgasgemisch A und dem Produktgasgemisch A* folgend in den ersten Abschnitt der Reaktionszone A rückgeführt wird.

35. Verfahren nach einem der Ansprüche 1 bis 34, **dadurch gekennzeichnet, dass** das in die Trennzone A geführte Produktgasgemisch über einen indirekten Wärmeaustauscher in die Trennzone A geführt wird, um in diesem indirekten Wärmeaustauscher das Frischpropan und/oder das Restgas aus der Trennzone B zu erwärmen.

36. Verfahren nach einem der Ansprüche 1 bis 35, **dadurch gekennzeichnet, dass** keiner der beiden Reaktionszonen A, B und keiner der beiden Trennzonen A, B externer Wasserdampf zugeführt wird.

37. Verfahren nach einem der Ansprüche 1 bis 36, **dadurch gekennzeichnet, dass** das Produktgasgemisch A in der Trennzone B mit einem organischen Lösungsmittel in Kontakt gebracht wird, in welchem Propan und Propylen absorbiert werden.

38. Verfahren nach einem der Ansprüche 1 bis 37, **dadurch gekennzeichnet, dass** die Reaktionszone B als zweistufige heterogen katalysierte GasphasenPartialoxidation von Propylen zu Acrylsäure gestaltet wird.

39. Verfahren nach einem der Ansprüche 1 bis 38, **dadurch gekennzeichnet, dass** die Zielproduktabtrennung in der Reaktionszone B durch Absorption des Zielproduktes in einem Absorptionsmittel oder durch fraktionierende Kondensation des Produktgasgemischs B erfolgt.

40. Verfahren nach einem der Ansprüche 1 bis 39, **dadurch gekennzeichnet, dass** das in der Trennzone B verbleibende und in die Reaktionszone A rückgeführte Restgas folgende Bestandteile mit den nachfolgenden Gehalten aufweist:

10 bis 40 Vol.-% Propan,
0 bis 1 Vol.-% Propen,
> 0 bis 5 Vol.-% molekularer Sauerstoff,
1 bis 10 Vol.-% Wasserdampf und
0 bis 0,5 Vol.-% molekularer Wasserstoff.

41. Verfahren nach einem der Ansprüche 1 bis 40, **dadurch gekennzeichnet, dass** in der Reaktionszone A das molare Verhältnis von im Reaktionsgasgemisch enthaltenem Propylen zu im Reaktionsgasgemisch enthaltenem molekularem Wasserstoff den Wert 10 an keiner Stelle überschreitet.

**EP 1 765 754 B1**

**Claims**

1.  A process for preparing acrolein or acrylic acid or a mixture thereof from propane, by

    A) feeding to a first reaction zone A at least two gaseous, propane-comprising feed streams, at least one of which comprises fresh propane, and, in reaction zone A, subjecting their propane fed in this way to a heterogeneously catalyzed dehydrogenation to obtain a product gas mixture A comprising propane and propylene,
    B) conducting product gas mixture A out of reaction zone A and, in a first separation zone A, removing at least a portion of the constituents, other than propane and propylene, present in product gas mixture A, and using remaining product gas mixture A' comprising propane and propylene
    C) in a second reaction zone B to charge at least one oxidation reactor and, in the at least one oxidation reactor, subjecting the propylene present in product gas mixture A' to a heterogeneously catalyzed gas phase partial oxidation with molecular oxygen to give a product gas mixture B comprising acrolein or acrylic acid or a mixture thereof as the target product and also excess molecular oxygen,
    D) conducting product gas mixture B out of reaction zone B and, in a second separation zone B, removing target product present in product gas mixture B, and, of the remaining residual gas comprising unconverted propane, molecular oxygen and possibly unconverted propylene, recycling at least a portion comprising unconverted propane, molecular oxygen and possibly unconverted propylene as one of the at least two propane-comprising feed streams into reaction zone A, wherein this recycling into reaction zone A along the reaction path of the heterogeneously catalyzed dehydrogenation of propane in reaction zone A is effected such that, at the feed point, at least 5 mol% of the propane fed to reaction zone A via the other feed streams has already been converted under dehydrogenating conditions in reaction zone A.

2.  The process according to claim 1, wherein at least half of the residual gas which remains in separation zone B and comprises unconverted propane, molecular oxygen and, if appropriate, unconverted propylene is recycled into reaction zone A.

3.  The process according to claim 1, wherein the entire amount of the residual gas which remains in separation zone B and comprises unconverted propane, molecular oxygen and possibly unconverted propylene is recycled into reaction zone A.

4.  The process according to any of claims 1 to 3, wherein residual gas which remains in separation zone B and comprises unconverted propane, molecular oxygen and possibly unconverted propylene is recycled into reaction zone A only at one point in reaction zone A.

5.  The process according to any of claims 1 to 3, wherein residual gas which remains in separation zone B and comprises unconverted propane, molecular oxygen and possibly unconverted propylene is recycled into reaction zone A distributed over a plurality of feed points arranged in series.

6.  The process according to any of claims 1 to 5, wherein residual gas which remains in separation zone B and comprises unconverted propane, molecular oxygen and possibly unconverted propylene is recycled into reaction zone A such that, at the feed point, at least 20 mol% of the propane which is fed to reaction zone A via the other feed streams has been converted under dehydrogenating conditions in reaction zone A.

7.  The process according to any of claims 1 to 5, wherein residual gas which remains in separation zone B and comprises unconverted propane, molecular oxygen and possibly unconverted propylene is recycled into reaction zone A such that, at the feed point, at least 30 mol% of the propane which is fed to reaction zone A via the other feed streams has been converted under dehydrogenating conditions in reaction zone A.

8.  The process according to any of claims 1 to 7, wherein the molecular oxygen content of the residual gas which remains in separation zone B, comprises unconverted propane, molecular oxygen and possibly unconverted propylene and is recycled into reaction zone A is from 0.5 to 10% by volume.

9.  The process according to any of claims 1 to 7, wherein the molecular oxygen content of the residual gas which remains in separation zone B, comprises unconverted propane, molecular oxygen and possibly unconverted propylene and is recycled into reaction zone A is from 2 to 5% by volume.

10. The process according to any of claims 1 to 9, wherein the ratio of the amount of propane which is fed to reaction zone A via recycled residual gas stemming from separation zone B to the total amount of propane which is fed to reaction zone A via other feed streams is from 0.1 to 10.

11. The process according to any of claims 1 to 10, wherein only residual gas stemming from separation zone B and crude propane as fresh propane are fed as propane-comprising feed streams to reaction zone A.

12. The process according to any of claims 1 to 11, wherein the reaction gas mixture in reaction zone A, at the feed point for the residual gas stemming from separation zone B, based on the molar amount of propane comprised therein, has a higher molar hydrogen content than the starting reaction gas mixture fed to reaction zone A.

13. The process according to any of claims 1 to 12, wherein the reaction gas mixture which forms in reaction zone A at the feed point for the residual gas, stemming from separation zone B, composed of this fed residual gas and the reaction gas mixture present at the feed point in reaction zone A upstream of this residual gas feed, based on the molar amount of propane comprised therein, has a higher molar hydrogen content than the starting reaction gas mixture fed to reaction zone A.

14. The process according to any of claims 1 to 13, wherein the reaction temperatures in reaction zone A are from 300 to 800°C.

15. The process according to any of claims 1 to 14, wherein dehydrogenation catalysts are also used in reaction zone A which comprise from 10 to 99.9% by weight of zirconium dioxide, from 0 to 60% by weight of aluminum oxide, silicon dioxide and/or titanium dioxide and from 0.1 to 10% by weight of at least one element of the first or second main group, of an element of the third transition group, of an element of the eighth transition group, of the Periodic Table of the Elements, lanthanum and/or tin, with the proviso that the sum of the percentages by weight is 100% by weight.

16. The process according to any of claims 1 to 15, wherein the catalysts used in reaction zone A are catalyst extrudates and/or catalyst rings.

17. The process according to any of claims 1 to 16, wherein the reaction zone A used is a tray reactor.

18. The process according to any of claims 1 to 17, wherein the conversion of propane achieved in reaction zone A, based on the total amount of propane fed to reaction zone A and based on single pass through reaction zone A, is from 30 to 60 mol%.

19. The process according to any of claims 1 to 17, wherein the conversion of propane achieved in reaction zone A, based on the total amount of propane fed to reaction zone A and based on single pass through reaction zone A, is from 40 to 50 mol%.

20. The process according to any of claims 1 to 10 or 12 to 19, wherein the product gas mixture A formed in reaction zone A is divided into two portions of identical composition, one of the two portions is recycled into reaction zone A as one of the propane-comprising feed streams and of the other portion is conducted out of reaction zone A into the first separation zone A.

21. The process according to claim 20, wherein the portion of product gas mixture A recycled into reaction zone A, based on the total amount of product gas mixture A formed in reaction zone A, is from 30 to 70% by volume.

22. The process according to claim 20, wherein the portion of product gas mixture A recycled into reaction zone A, based on the total amount of product gas mixture A formed in reaction zone A, is from 40 to 60% by volume.

23. The process according to any of claims 20 to 22, wherein the portion of product gas mixture A recycled into reaction zone A is recycled into reaction zone A as a constituent of the starting reaction gas mixture fed to reaction zone A.

24. The process according to any of claims 1 to 23, wherein reaction zone A is configured and operated with the proviso that it consists of a first and of a second section, by

  I. feeding to the first section of reaction zone A at least one feed stream comprising gaseous propane, which comprises fresh propane, and, in this first section of reaction zone A, subjecting the propane fed to it in such a way to a heterogeneously catalyzed dehydrogenation to obtain a product gas mixture A* comprising propane, propylene and molecular hydrogen and which has been obtained by converting under dehydrogenating conditions at least 5 mol% of the propane fed to the first section of reaction zone A in this first section of reaction zone A and which, based on the molar amount of propane comprised therein, comprises a larger molar amount of molecular hydrogen than the starting reaction gas mixture fed to the first section of reaction zone A;

II. subsequently feeding residual gas, comprising molecular oxygen, unconverted propane and possibly unconverted propylene, from separation zone B to product gas mixture A* and feeding the resulting reaction gas mixture A* to the second section of reaction zone A, and

III. in this second section of reaction zone A, with formation of product gas mixture A comprising propane and propylene, combusting molecular oxygen present in reaction gas mixture A* with molecular hydrogen present in reaction gas mixture A* under heterogeneous catalysis to give water and dehydrogenating propane present in reaction gas mixture A* optionally under heterogeneous catalysis, to give propylene.

25. The process according to claim 24, wherein the first section of reaction zone A is configured adiabatically.

26. The process according to claim 24 or 25, wherein the second section of reaction zone A is configured adiabatically.

27. The process according to any of claims 24 to 26, wherein both the first and the second section of reaction zone A are configured adiabatically, with the proviso that

- the gross thermal character based on single pass of the starting reaction gas mixture fed to the first section of reaction zone A through the first section of reaction zone A is endothermic to autothermic, and
- the gross thermal character based on single pass of the reaction gas mixture A* fed to the second section of reaction zone A through the second section of reaction zone A is exothermic.

28. The process according to any of claims 24 to 27, wherein the first section of the reaction zone has tray structure.

29. The process according to any of claims 24 to 28, wherein product gas mixture A comprises less than 5% by volume of molecular hydrogen.

30. The process according to any of claims 24 to 29, wherein the first and the second section of reaction zone A are accommodated in one reactor.

31. The process according to any of claims 24 to 30, wherein product gas mixture A is divided into two portions of identical composition and one of the two portions is recycled into the first section of reaction zone A.

32. The process according to any of claims 24 to 31, wherein the residual gas, comprising unconverted propane, molecular oxygen and possibly unconverted propylene, from separation zone B is fed to product gas mixture A* by the principle of a jet pump operated with this residual gas as the driving jet, and the conveying direction of the driving jet decompressed through a driving nozzle via a mixing zone and a diffuser points into the second section of reaction zone A, and the sucking action of the sucking nozzle points in the direction of the first section of reaction zone A, and the sucking nozzle-mixing zone-diffuser connection forms the sole connection between the two sections of reaction zone A.

33. The process according to claim 32, wherein the pressure of the driving jet is selected such that the pressure of the product gas mixture A which forms in the second section of reaction zone A is above the pressure of the product gas mixture A* which forms in the first section of reaction zone A.

34. The process according to claim 33, wherein product gas mixture A is divided into two portions of identical composition and one of the two portions is recycled into the first section of reaction zone A in a kind of natural circulation following the pressure gradient between product gas mixture A and product gas mixture A*.

35. The process according to any of claims 1 to 34, wherein the product gas mixture conducted into separation zone A is conducted into separation zone A via an indirect heat exchanger in order to heat the fresh propane and/or the residual gas from separation zone B in this indirect heat exchanger.

36. The process according to any of claims 1 to 35, wherein no external steam is fed to either of the two reaction zones A, B or either of the two separation zones A, B.

**37.** The process according to any of claims 1 to 36, wherein product gas mixture A is contacted in separation zone B with an organic solvent in which propane and propylene are absorbed.

**38.** The process according to any of claims 1 to 37, wherein reaction zone B is configured as a two-stage heterogeneously catalyzed gas phase partial oxidation of propylene to acrylic acid.

**39.** The process according to any of claims 1 to 38, wherein the target product removal in reaction zone B is effected by absorption of the target product in an absorbent or by fractional condensation of product gas mixture B.

**40.** The process according to any of claims 1 to 39, wherein the residual gas which remains in separation zone B and is recycled into reaction zone A has the following constituents with the following contents:

from 10 to 40% by volume of propane,
from 0 to 1% by volume of propene,
from > 0 to 5% by volume of molecular oxygen,
from 1 to 10% by volume of steam and
from 0 to 0.5% by volume of molecular hydrogen.

**41.** The process according to any of claims 1 to 40, wherein the molar ratio of propylene present in the reaction gas mixture to molecular hydrogen present in the reaction gas mixture in reaction zone A does not at any point exceed the value of 10.

**Revendications**

**1.** Procédé de fabrication d'acroléine ou d'acide acrylique ou leur mélange à partir de propane, selon lequel

A) au moins deux courants d'alimentation gazeux contenant du propane sont introduits dans une première zone de réaction A, parmi lesquels au moins un contient du propane frais, et le propane ainsi introduit dans la zone de réaction A y est soumis à une déshydrogénation sous catalyse hétérogène, un mélange gazeux de produits A contenant du propane et du propylène étant obtenu,
B) le mélange gazeux de produits A est déchargé de la zone de réaction A et au moins une partie des constituants différents du propane et du propylène contenus dans le mélange gazeux de produits A est séparée dans une première zone de séparation A, et le mélange gazeux de produits A' restant, contenant du propane et du propylène,
C) est utilisé dans une seconde zone de réaction B pour l'alimentation d'au moins un réacteur d'oxydation, et le propylène contenu dans le mélange gazeux de produits A' est soumis dans ledit au moins un réacteur d'oxydation à une oxydation partielle en phase gazeuse sous catalyse hétérogène avec de l'oxygène moléculaire pour former un mélange gazeux de produits B contenant de l'acroléine ou de l'acide acrylique ou leur mélange en tant que produit cible et l'oxygène moléculaire en excès,
D) le mélange gazeux de produits B est déchargé de la zone de réaction B et le produit cible contenu dans le mélange gazeux de produits B est séparé dans une seconde zone de séparation B et, à partir du gaz résiduel restant, contenant du propane non réagi, de l'oxygène moléculaire et éventuellement du propylène non réagi, au moins une partie contenant du propane non réagi, de l'oxygène moléculaire et éventuellement du propylène non réagi est recyclée dans la zone de réaction A en tant qu'un desdits au moins deux courants d'alimentation contenant du propane, **caractérisé en ce que** ce recyclage dans la zone de réaction A a lieu le long du chemin réactionnel de la déshydrogénation sous catalyse hétérogène du propane dans la zone de réaction A de manière à ce qu'à l'emplacement d'alimentation, au moins 5 % en moles du propane introduit dans la zone de réaction A par les autres courants d'alimentation ait déjà été transformé par déshydrogénation dans la zone de réaction A.

**2.** Procédé selon la revendication 1, **caractérisé en ce qu'**au moins la moitié du gaz résiduel restant dans la zone de séparation B, contenant du propane non réagi, de l'oxygène moléculaire et éventuellement du propylène non réagi, est recyclé dans la zone de réaction A.

**3.** Procédé selon la revendication 1, **caractérisé en ce que** la totalité du gaz résiduel restant dans la zone de séparation B, contenant du propane non réagi, de l'oxygène moléculaire et éventuellement du propylène non réagi, est recyclé dans la zone de réaction A.

**4.** Procédé selon l'une quelconque des revendications 1 à 3, **caractérisé en ce que** le recyclage du gaz résiduel restant dans la zone de séparation B, contenant du propane non réagi, de l'oxygène moléculaire et éventuellement du propylène non réagi, dans la zone de réaction A n'a lieu qu'à un emplacement de la zone de réaction A.

**5.** Procédé selon l'une quelconque des revendications 1 à 3, **caractérisé en ce que** le recyclage du gaz résiduel restant dans la zone de séparation B, contenant du propane non réagi, de l'oxygène moléculaire et éventuellement du propylène non réagi, dans la zone de réaction A a lieu par répartition sur plusieurs emplacements d'alimentation agencés les uns après les autres.

**6.** Procédé selon l'une quelconque des revendications 1 à 5, **caractérisé en ce que** le recyclage du gaz résiduel restant dans la zone de séparation B, contenant du propane non réagi, de l'oxygène moléculaire et éventuellement du propylène non réagi, dans la zone de réaction A a lieu de manière à ce qu'à l'emplacement d'alimentation, au moins 20 % en moles du propane introduit dans la zone de réaction A par les autres courants d'alimentation ait été transformé par déshydrogénation dans la zone de réaction A.

**7.** Procédé selon l'une quelconque des revendications 1 à 5, **caractérisé en ce que** le recyclage du gaz résiduel restant dans la zone de séparation B, contenant du propane non réagi, de l'oxygène moléculaire et éventuellement du propylène non réagi, dans la zone de réaction A a lieu de manière à ce qu'à l'emplacement d'alimentation, au moins 30 % en moles du propane introduit dans la zone de réaction A par les autres courants d'alimentation ait été transformé par déshydrogénation dans la zone de réaction A.

**8.** Procédé selon l'une quelconque des revendications 1 à 7, **caractérisé en ce que** la teneur du gaz résiduel restant dans la zone de séparation B, contenant du propane non réagi, de l'oxygène moléculaire et éventuellement du propylène non réagi, recyclé dans la zone de réaction A, en oxygène moléculaire est de 0,5 à 10 % en volume.

**9.** Procédé selon l'une quelconque des revendications 1 à 7, **caractérisé en ce que** la teneur du gaz résiduel restant dans la zone de séparation B, contenant du propane non réagi, de l'oxygène moléculaire et éventuellement du propylène non réagi, recyclé dans la zone de réaction A, en oxygène moléculaire est de 2 à 5 % en volume.

**10.** Procédé selon l'une quelconque des revendications 1 à 9, **caractérisé en ce que** le rapport entre la quantité de propane introduite dans la zone de réaction A par le gaz résiduel recyclé provenant de la zone de séparation B et la quantité de propane introduite au total dans la zone de réaction A par les autres courants d'alimentation de 0,1 à 10.

**11.** Procédé selon l'une quelconque des revendications 1 à 10, **caractérisé en ce que** seul le gaz résiduel provenant de la zone de séparation B et du propane brut en tant que propane frais sont introduits dans la zone de réaction A en tant que courants d'alimentation contenant du propane.

**12.** Procédé selon l'une quelconque des revendications 1 à 11, **caractérisé en ce que** le mélange réactionnel gazeux se trouvant dans la zone de réaction A présente, à l'emplacement d'alimentation du gaz résiduel provenant de la zone de séparation B, par rapport à la quantité molaire de propane qu'il contient, une teneur en hydrogène molaire plus élevée que le mélange réactionnel gazeux initial introduit dans la zone de réaction A.

**13.** Procédé selon l'une quelconque des revendications 1 à 12, **caractérisé en ce que** le mélange réactionnel gazeux qui se forme dans la zone de réaction A à l'emplacement d'alimentation du gaz résiduel provenant de la zone de séparation B à partir de ce gaz résiduel introduit et du mélange réactionnel gazeux présent dans la zone de réaction A avant cette introduction de gaz résiduel à l'emplacement d'alimentation présente, par rapport à la quantité molaire de propane qu'il contient, une teneur en hydrogène molaire plus élevée que le mélange réactionnel gazeux initial introduit dans la zone de réaction A.

**14.** Procédé selon l'une quelconque des revendications 1 à 13, **caractérisé en ce que** les températures de réaction dans la zone de réaction A sont de 300 à 800 °C.

**15.** Procédé selon l'une quelconque des revendications 1 à 14, **caractérisé en ce que** des catalyseurs de déshydrogénation sont utilisés dans la zone de réaction A, qui contiennent 10 à 99,9 % en poids de dioxyde de zirconium, 0 à 60 % en poids d'oxyde d'aluminium, de dioxyde de silicium et/ou de dioxyde de titane, et 0,1 à 10 % en poids d'au moins un élément du premier ou du deuxième groupe principal, un élément du troisième groupe de transition, un élément du huitième groupe de transition du tableau périodique des éléments, de lanthane et/ou d'étain, à condition que la somme des pourcentages en poids soit de 100 % en poids.

**16.** Procédé selon l'une quelconque des revendications 1 à 15, **caractérisé en ce que** des filaments de catalyseur et/ou des anneaux de catalyseur sont utilisés dans la zone de réaction A en tant que catalyseurs.

**17.** Procédé selon l'une quelconque des revendications 1 à 16, **caractérisé en ce qu'**un réacteur à étages est utilisé en tant que zone de réaction A.

**18.** Procédé selon l'une quelconque des revendications 1 à 17, **caractérisé en ce que** la conversion de propane obtenue dans la zone de réaction A, par rapport au propane introduit au total dans la zone de réaction A et par rapport à un passage unique dans la zone de réaction A, est de 30 à 60 % en moles.

**19.** Procédé selon l'une quelconque des revendications 1 à 17, **caractérisé en ce que** la conversion de propane obtenue dans la zone de réaction A, par rapport au propane introduit au total dans la zone de réaction A et par rapport à un passage unique dans la zone de réaction A, est de 40 à 50 % en moles.

**20.** Procédé selon l'une quelconque des revendications 1 à 10, ainsi que 12 à 19, **caractérisé en ce que** le mélange gazeux de produits A formé dans la zone de réaction A est divisé en deux parties de composition identique, une des deux parties est recyclée dans la zone de réaction A en tant qu'un des courants d'alimentation contenant du propane et l'autre partie est conduite depuis la zone de réaction A dans la première zone de séparation A.

**21.** Procédé selon la revendication 20, **caractérisé en ce que** la partie du mélange gazeux de produits A recyclée dans la zone de réaction A représente, par rapport à la totalité du mélange gazeux de produits A formé dans la zone de réaction A, 30 à 70 % en volume.

**22.** Procédé selon la revendication 20, **caractérisé en ce que** la partie du mélange gazeux de produits A recyclée dans la zone de réaction A représente, par rapport à la totalité du mélange gazeux de produits A formé dans la zone de réaction A, 40 à 60 % en volume.

**23.** Procédé selon l'une quelconque des revendications 20 à 22, **caractérisé en ce que** la partie du mélange gazeux de produits A recyclée dans la zone de réaction A est recyclée dans la zone de réaction A en tant que constituant du mélange réactionnel gazeux initial introduit dans la zone de réaction A.

**24.** Procédé selon l'une quelconque des revendications 1 à 23, **caractérisé en ce que** la zone de réaction A est configurée et exploitée de manière à être constituée d'une première et d'une seconde section,

I. au moins un courant d'alimentation gazeux contenant du propane, qui contient du propane frais, étant introduit dans la première section de la zone de réaction A et le propane ainsi introduit dans cette première section de la zone de réaction A y étant soumis à une déshydrogénation sous catalyse hétérogène, un mélange gazeux de produits A* contenant du propane, du propylène et de l'hydrogène moléculaire étant obtenu, à l'obtention duquel au moins 5 % en moles du propane introduit dans la première section de la zone de réaction A ayant été transformé par déshydrogénation dans cette première section de la zone de réaction A, et qui contient, par rapport à la quantité molaire de propane qu'il contient, une quantité molaire d'hydrogène moléculaire plus élevée que le mélange réactionnel gazeux initial introduit dans la première section de la zone de réaction A ;
II. le gaz résiduel contenant de l'oxygène moléculaire, du propane non réagi et éventuellement du propylène non réagi, provenant de la zone de séparation B, étant ensuite introduit dans le mélange gazeux de produits A* et le mélange réactionnel gazeux A* résultant étant introduit dans la seconde section de la zone de réaction A, et
III. dans cette seconde section de la zone de réaction A, l'oxygène moléculaire contenu dans le mélange réactionnel gazeux A* étant brûlé en eau sous catalyse hétérogène avec l'hydrogène moléculaire contenu dans le mélange réactionnel gazeux A* pour former le mélange gazeux de produits A contenant du propane et du propylène, et le propane contenu dans le mélange réactionnel gazeux A* étant éventuellement déshydrogéné sous catalyse hétérogène en propylène.

**25.** Procédé selon la revendication 24, **caractérisé en ce que** la première section de la zone de réaction A est configurée sous forme adiabatique.

**26.** Procédé selon la revendication 24 ou 25, **caractérisé en ce que** la seconde section de la zone de réaction A est configurée sous forme adiabatique.

**27.** Procédé selon l'une quelconque des revendications 24 à 26, **caractérisé en ce qu'**aussi bien la première que la seconde section de la zone de réaction A est configurée sous forme adiabatique, à condition que

- la chaleur de réaction brute relative au passage unique dans la première section de la zone de réaction A du mélange réactionnel gazeux initial introduit dans la première section de la zone de réaction A soit endotherme à autotherme, et
- la chaleur de réaction brute relative au passage unique dans la seconde section de la zone de réaction A du mélange réactionnel gazeux A* introduit dans la seconde section de la zone de réaction A soit exotherme.

**28.** Procédé selon l'une quelconque des revendications 24 à 27, **caractérisé en ce que** la première section de la zone de réaction présente une structure à étages.

**29.** Procédé selon l'une quelconque des revendications 24 à 28, **caractérisé en ce que** le mélange gazeux de produits A contient moins de 5 % en volume d'hydrogène moléculaire.

**30.** Procédé selon l'une quelconque des revendications 24 à 29, **caractérisé en ce que** la première et la seconde section de la zone de réaction A sont placées dans un réacteur.

**31.** Procédé selon l'une quelconque des revendications 24 à 30, **caractérisé en ce que** le mélange gazeux de produits A est divisé en deux parties de composition identique et une des deux parties est recyclée dans la première section de la zone de réaction A.

**32.** Procédé selon l'une quelconque des revendications 24 à 31, **caractérisé en ce que** le gaz résiduel contenant du propane non réagi, de l'oxygène moléculaire et éventuellement du propylène non réagi, provenant de la zone de séparation B, est introduit dans le mélange gazeux de produits A* selon le principe d'une pompe à jet entraînée avec ce gaz résiduel en tant que jet d'entraînement, la direction de transport du jet d'entraînement détendu par une buse d'entraînement par le biais d'une section de mélange et d'un diffuseur étant vers la seconde section de la zone de réaction A et l'action d'aspiration de l'embout d'aspiration étant dans la direction de la première section de la zone de réaction A, et le raccordement embout d'aspiration-section de mélange-diffuseur formant le seul raccordement entre les deux sections de la zone de réaction A.

**33.** Procédé selon la revendication 32, **caractérisé en ce que** la pression du jet d'entraînement est choisie pour que la pression du mélange gazeux de produits A formé dans la seconde section de la zone de réaction A soit supérieure à la pression du mélange gazeux de produits A* formé dans la première section de la zone de réaction A.

**34.** Procédé selon la revendication 33, **caractérisé en ce que** le mélange gazeux de produits A est divisé en deux parties de composition identique et une des deux parties est recyclée dans la première section de la zone de réaction A par circulation naturelle en suivant le gradient de pression entre le mélange gazeux de produits A et le mélange gazeux de produits A*.

**35.** Procédé selon l'une quelconque des revendications 1 à 34, **caractérisé en ce que** le mélange gazeux de produits introduit dans la zone de séparation A est conduit dans la zone de séparation A par le biais d'un échangeur de chaleur indirect afin de chauffer le propane frais et/ou le gaz résiduel provenant de la zone de séparation B dans cet échangeur de chaleur indirect.

**36.** Procédé selon l'une quelconque des revendications 1 à 35, **caractérisé en ce que** de la vapeur d'eau externe n'est introduite dans aucune des deux zones de réaction A, B et aucune des deux zones de séparation A, B.

**37.** Procédé selon l'une quelconque des revendications 1 à 36, **caractérisé en ce que** le mélange gazeux de produits A est mis en contact dans la zone de séparation B avec un solvant organique dans lequel le propane et le propylène sont absorbés.

**38.** Procédé selon l'une quelconque des revendications 1 à 37, **caractérisé en ce que** la zone de réaction B est configurée sous la forme d'une oxydation partielle en phase gazeuse sous catalyse hétérogène à deux étapes de propylène en acide acrylique.

**39.** Procédé selon l'une quelconque des revendications 1 à 38, **caractérisé en ce que** la séparation du produit cible dans la zone de réaction B a lieu par absorption du produit cible dans un agent d'absorption ou par condensation fractionnée du mélange gazeux de produits B.

**40.** Procédé selon l'une quelconque des revendications 1 à 39, **caractérisé en ce que** le gaz résiduel restant dans la zone de séparation B et recyclé dans la zone de réaction A comprend les constituants suivants en les teneurs suivantes :

10 à 40 % en volume de propane,
0 à 1 % en volume de propène,
> 0 à 5 % en volume d'oxygène moléculaire,
1 à 10 % en volume de vapeur d'eau et
0 à 0,5 % en volume d'hydrogène moléculaire.

**41.** Procédé selon l'une quelconque des revendications 1 à 40, **caractérisé en ce que**, dans la zone de réaction A, le rapport molaire entre le propylène contenu dans le mélange réactionnel gazeux et l'hydrogène moléculaire contenu dans le mélange réactionnel gazeux ne dépasse à aucun emplacement la valeur de 10.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0196270 A
- US 20030187299 A1
- DE 10245585 A
- DE 10246119 A
- DE 10211275 A
- WO 0200587 A
- DE 3313573 A
- DE 10131297 A
- EP 731077 A
- WO 9946039 A
- US 4788371 A
- EP 0705136 A
- WO 9929420 A
- US 4220091 A
- US 5430220 A
- US 5877369 A
- EP 0117146 A
- DE 19937196 A
- DE 19937105 A
- DE 19937107 A
- WO 0185333 A
- WO 0185330 A
- US 4902849 A
- US 4996387 A
- US 5389342 A
- US 5073662 A
- US 4886928 A
- US 5430209 A
- US 5530171 A
- US 5527979 A
- US 5563314 A
- US 5430203 A
- EP 117146 A
- US 3161670 A
- US 55530171 A
- DE 4308087 A
- DE 10313210 A
- DE 10313214 A
- DE 10313213 A
- DE 10313212 A
- DE 10308824 A
- DE 10313208 A
- EP 253409 A
- DE 4431957 A
- DE 102004025445 A
- DE 4431949 A
- DE 10325488 A
- DE 10325487 A
- DE 10353954 A
- DE 10344149 A
- DE 10351269 A
- DE 10350812 A
- DE 10350822 A
- DE 19955176 A
- DE 19948523 A
- DE 10101695 A
- DE 19948248 A
- DE 19955168 A
- EP 700714 A
- DE 10046957 A
- DE 10063162 A
- DE 3338380 C
- DE 19902562 A
- EP 15565 A
- DE 2380765 C
- EP 807465 A
- EP 279374 A
- DE 3300044 A
- EP 575897 A
- US 4438217 A
- DE 19855913 A
- WO 9824746 A
- DE 19746210 A
- JP 3294239 A
- EP 293224 A
- DE 4023239 A
- DE 2909671 A
- EP 293859 A
- EP 714700 A
- DE 10046928 A
- DE 19815281 A
- DE 4335973 A
- EP 668104 A
- DE 19736105 A
- DE 19740493 A
- DE 19528646 A
- DE 19910506 A
- DE 19910508
- DE 10121592 A
- EP 911313 A
- EP 979813 A
- EP 990636 A
- DE 2830765 A

- DE 19929487 A
- DE 19952964 A
- DE 19837517 A
- DE 19910508 A
- DE 19837519 A
- EP 1388533 A
- EP 1388532 A
- DE 10235847 A
- EP 792867 A
- WO 9801415 A
- EP 1015411 A
- EP 1015410 A
- WO 9950219 A
- WO 0053560 A
- WO 0209839 A
- WO 03041833 A
- DE 10223058 A
- DE 10243625 A
- DE 10336386 A
- EP 854129 A
- US 4317926 A
- DE 19837520 A
- DE 19606877 A
- DE 190501325 A
- DE 10247240 A
- DE 19740253 A
- EP 695736 A
- EP 982287 A
- EP 1041062 A
- DE 4335172 A
- DE 4436243 A
- DE 19924532 A
- DE 10332758 A
- DE 19924533 A
- EP 982289 A
- DE 10115277 A
- DE 19740252 A
- DE 19627847 A
- DE 10053086 A
- EP 982288 A
- WO 0196271 A
- DE 10219879 A
- DE 10028582 A
- DE 10360057 A
- DE 10360058 A
- DE 10313211 A
- DE 10313209 A
- EP 616998 A
- EP 912486 A
- EP 648732 A
- DE 10143565 A
- EP 270999 A
- EP 1189861 A
- WO 9801404 A